# EUROPEAN PATENT APPLICATION

(11) **EP 4 725 503 A2**
(43) Date of publication of application: **15.04.2026**
(21) Application number: 26156880.2
(22) Date of filing: 29.03.2017
(51) Int. Cl.: A61K 40/42

(54) **CHIMERIC ANTIGEN RECEPTORS TARGETING CANCER**

(30) Priority: 29.03.2016 US 201662314864 P
(62) Divisional of application: 17776591.4
(71) Applicant: University Of Southern California, Los Angeles, CA 90015 (US)
(72) Inventor: CHAUDHARY, Preet M, Toluca Lake, CA 91602 (US)
(74) Representative: Page White Farrer

(57) **Abstract**

Provided herein is a composition comprising, a cell, comprising nucleic acids encoding a chimeric antigen receptor (CAR) and one or more of signaling proteins selected from K13-vFLIP, MC159-vFLIP, cFLIP-L, cFLIP-p22, HTLV1-Tax and HTLV2-Tax, wherein the CAR comprises an a) extracellular antigen specific domain, b) a transmembrane domain and c) an intracellular signaling domain comprising an immunoreceptor tyrosine-based activation motif (ITAM); wherein c) is located at the C-terminus of the chimeric receptor. In some embodiments, the CAR further comprises one or more co-stimulatory domains. Also provided herein are methods for treating diseases using the compositions described herein. Further provided herein is a kinase inhibitor for use in therapeutic methods described herein.

## Description

### STATEMENT REGARDING FEDERALLY SPONSORED RESEARCH OR DEVELOPMENT

This invention was made with government support under Grant No. DE019811 awarded by National Institutes of Health. The government has certain rights in the invention.

### CROSS-REFERENCE TO RELATED APPLICATIONS

This application claims benefit of priority under 35 U.S.C. §119(e) of U.S. Provisional Patent Application No. 62/314,864 filed on March 29, 2016, the contents of which are incorporated by reference in their entirety.

### FIELD OF INVENTION

Provided herein are chimeric antigen receptors for treating cancers.

### BACKGROUND

All publications herein are incorporated by reference to the same extent as if each individual publication or patent application was specifically and individually indicated to be incorporated by reference. The following description includes information that may be useful in understanding the present invention. It is not an admission that any of the information provided herein is prior art or relevant to the presently claimed invention, or that any publication specifically or implicitly referenced is prior art.

Adoptive T-cell immunotherapy has risen to the forefront of treatment approaches for cancer. T cells can be engineered to express the genes of chimeric antigen receptors (CARs) that recognize tumor associated antigens. CARs are synthetic immune-receptors, which can redirect T cells to selectively kill tumor cells. The general premise for their use in cancer immunotherapy is to rapidly generate tumor-targeted T cells, bypassing the barriers and incremental kinetics of active immunization and thereby act as 'living drugs'. Unlike the physiologic T-cell receptor (TCR), which engages HLA-peptide complexes, CARs engage molecules that do not require peptide processing or HLA expression to be recognized. CARs therefore recognize antigen on any HLA background, in contrast to TCRs, which need to be matched to the haplotype of the patient. Furthermore, CARs can target tumor cells that have down-regulated HLA expression or proteasomal antigen processing, two mechanisms that contribute to tumor escape from TCR-mediated immunity. Another feature of the broad applicability of CARs is their ability to bind not only to proteins but also to carbohydrate and glycolipid structures, again expanding the range of potential targets.

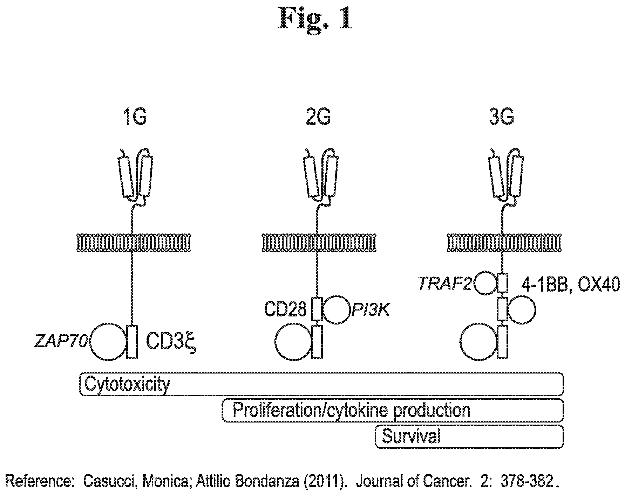

Initial first-generation CARs were constructed through the fusion of a scFv (single chain fragment variable)-based antigen binding domain to an inert CD8 transmembrane domain, linked to a cytoplasmic signaling domain derived from the CD3-ζ or Fc receptor γ chains (Fig. 1).

Although CD3-ζ chain aggregation is sufficient to enable lytic activity of T-cells, they failed to elicit a robust cytokine response, including interleukin-2 (IL-2), and support T-cell expansion upon repeated exposure to antigen. For optimal activation and proliferation, T cells require both T-cell receptor engagement and signaling, as well as costimulatory signaling through costimulatory receptors (i.e., CD28, 4-1BB, OX-40) on T cells binding to cognate ligands (i.e., CD80/86, 4-1BBL, OX-40L) expressed either by the targeted tumor cell or the antigen-presenting cells. To overcome the lack of T-cell co-stimulation, first generation CARs were further modified by incorporating the cytoplasmic signaling domains of T-cell costimulatory receptors. These second-generation CARs (Fig. 1) enhanced signaling strength and persistence of the modified T cells, leading to superior antitumor activity. But, which second-generation CAR is the best is not known and each one has their own strengths and weakness. For example, in clinics the 4-1BB design appears to favor persistence, with CD19-CAR T cells detectable out to at least 6 months in a majority of patients, whereas the CD28 costimulatory domain containing CD19 CAR T cells were typically undetectable beyond 3 months, but less severe cytokine release syndrome (CRS) and a lower CD19-negative relapse rate were observed using the CD28-based CAR T cells.

Despite the success with CAR-T cells, there are several limitation to this approach. In majority of patients who respond to engineered CAR T cells, excessive release of proinflammatory cytokines causes symptoms that include fevers, hypotension, hypoxemia, cardiac dysfunction, kidney failure and electrolyte abnormalities, collectively termed as "Cytokine release syndrome' (CRS). In some cases, CAR therapy can lead to neurologic symptoms including tremor, seizures and can be fatal. Strategies to counteract CRS and neurological complications include immunosuppressive agents (e.g., steroids) and Tocilizumab, a monoclonal antibody against IL-6 receptor. However, these strategies are not uniformly successful. The instant invention provides novel approaches to control the activity of CAR-T cells so as to prevent and/or treat the above complications.

The CAR constructs in current clinical use are artificial in design as they represent fusion of several different proteins. In particular, inclusion of costimulatory domain in the CAR construct results in non-physiological signaling through the receptor, which in turn could contribute to their toxicity. Some CARs show tonic antigen-independent signaling, which leads to unrestrained cellular activation, eventually resulting in apoptosis, excessive cytokine release independent of cognate antigens, and immunologic exhaustion. Frigault *et al* demonstrated that expression of some CARs containing CD28 and CD3z tandem signaling domains leads to constitutive activation and proliferation of the transduced primary human T cells which was related to inferior in vivo efficacy (Frigault et al., Cancer immunology research 3:356-67, 2015). One mechanism that was found to result in the phenotype of CARs with continuous T-cell proliferation was high density of CARs at the cell surface (Frigault et al., Cancer immunology research 3:356-67, 2015). Long *et al* demonstrated that early T cell exhaustion is a primary factor limiting the antitumor efficacy of CAR-expressing T cells and that CAR structure has a central role in predisposing CAR T cells to chronic activation and exhaustion (Long et al., Nat Med 21:581-90, 2015). They showed that tonic CAR CD3-z phosphorylation, triggered by antigen-independent clustering of CAR single-chain variable fragments, can induce early exhaustion of CAR T cells that limits antitumor efficacy (Long et al., Nat Med 21:581-90, 2015). Thus, there is a need for improving the CAR design to achieve long term persistence of CAR modified T cells without the risk of excessive toxicity, such as CRS.

Most CAR constructs in current clinical trials are based on murine monoclonal antibodies. Immunogenicity of these murine monoclonal antibodies based construct has been also postulated to limit their *in vivo* persistence and efficacy. This problem can be overcome with the use of CAR constructs described herein that are based on human or humanized antibodies.

The polyclonal nature of the immune response is key to its success in controlling various infections. In contrast, the current CAR therapies generally rely on targeting of a single antigen and/or single epitope of a single antigen. Loss of the targeted antigen or the targeted epitope is a frequent cause of failure of the current CAR therapies. To overcome this limitation, the instant invention provides CAR against multiple antigens and against multiple epitopes of a single antigen. These CARs can be used in suitable combinations to provide a polyclonal adaptive immune response for the prevention or treatment of diseases, such as cancer, infectious diseases, autoimmune diseases, allergic diseases and degenerative diseases.

CD19 is an attractive target for immunotherapy because it is uniformly expressed by the vast majority of B-cell malignancies, it is not expressed by normal non-hematopoietic tissues, and among hematopoietic cells, it is only expressed by B-lineage lymphoid cells. Early experiments demonstrated that anti-CD19 CARs could activate T cells in a CD19-specific manner. T cells genetically modified to express these CARs could kill CD19⁺ primary leukemia cells *in vitro* and eliminate CD19⁺ target cells in murine xenograft models. Even though, initial clinical results with first-generation CD19-targeted CAR-modified T cells were disappointing, T cells engineered to express second-generation CARs have demonstrated impressive clinical efficacy with significant improvements in patient outcomes for a number of B-cell malignancies, the notable being the dramatic clinical responses observed in patients with relapsed B-cell ALL.

Anti-CD19 CART therapy as proof-of-concept has been successful in part due to the tissue restriction of CD19 to B cells and by the clinical tolerability of prolonged B-cell depletion. However, in other settings, CART-based targeting of antigens expressed at low levels by normal tissues has led to significant toxicities. The paucity of well-characterized, truly tumor-specific surface antigens in myeloid malignancies, such as acute myeloid leukemia (AML), chronic myeloid leukemia (CML) and myelodysplastic syndrome MDS, has necessitated consideration of CAR-T tumor-targeting strategies that may also affect normal tissues, such as bone marrow.

### SUMMARY

The following embodiments and aspects thereof are described and illustrated in conjunction with systems, compositions and methods which are meant to be exemplary and illustrative, not limiting in scope.

In certain embodiments, the present invention provides compositions comprising genetically engineered effector cells (such as NK cells and T cells) that include polynucleotides that encode chimeric antigen receptors that can be used on adoptive cell therapy for treatment of cancer, infectious, autoimmune and degenerative diseases.

In some embodiments, provided herein are polynucleotides encoding chimeric antigen receptors (CARs) comprising an antigen specific domain (ASD), an extracellular hinge or spacer region (HR), a transmembrane domain (TMD), a costimulatory domain (CSD) and intracellular signaling domain (ISD), wherein the antigen binding domain targets any one or more of the targets set forth in **Table 21.** In one embodiment, the CAR targets CD19 and comprises the V_{L} and V_{H} of Bu12 described in **Table 21** and set forth in SEQ ID NO: 1470 and SEQ ID NO: 3215. In one embodiment, the CAR targets CD19 and comprises the V_{L} and V_{H} of MM described in **Table 21** and set forth in SEQ ID NO: 1471 and SEQ ID NO: 3216.

In some embodiments, provided herein are polynucleotides encoding any of conventional CARs **(Table 1)** and backbones 1-62 **(Table 2),** wherein each backbone comprises a conventional CAR component **(Table 1)** and one or more accessory modules as shown in **Table 2.** In some embodiments, the conventional CAR component and the one or more accessory components of each backbone are encoded by a single polynucleotide molecule. In some embodiments, the conventional CAR component is encoded by a first polynucleotide molecule and the one or more accessory modules are encoded by a second polynucleotide molecule. In some embodiments, backbones comprising a conventional CAR component and more than one accessory module, the first polynucleotide molecule encodes the conventional CAR component, a second polynucleotide molecule encodes the first accessory molecule and a third polynucleotide molecule encodes the second accessory molecule. For example, backbone 1 comprising conventional CAR I and K13-vFLIP may be encoded by a single polynucleotide molecule or by two polynucleotide molecules, wherein the first polynucleotide molecule encodes the conventional CAR I and the second polynucleotide molecule encodes K13-vFLIP. In various embodiments, the polynucleotide molecules encoding the CAR component of the conventional CARs I to III **(Table 1)** or the backbones **(Table 2)** described herein encodes one or more antigen specific domains. In some embodiments, the antigen specific domain comprises one or more V_{L} (or vL) fragments. In some embodiments, the antigen specific domain comprises one or more V_{H} (or vH) fragments. In some embodiments, the antigen specific domain comprises one or more scFVs (or scFvs) specific to the antigens on target cells such as cancer cells. In some embodiments, the antigen specific domain comprises one or more Fv fragments. In some embodiments, the antigen specific domain comprises one or more Fab fragments. In some embodiments, the antigen specific domain comprises one or more (Fab')2 fragments. In some embodiments, the antigen specific domain comprises one or more single domain antibodies (SDAB). In some embodiments, the antigen specific domain comprises one or more camelid V_{HH} (or vHH) domains. In some embodiments, the antigen specific domain comprises one or more non-immunoglobulin scaffold such as a DARPIN, an affibody, an affilin, an adnectin, an affitin, an obodies, a repebody, a fynomer, an alphabody, an avimer, an atrimer, a centyrin, a pronectin, an anticalin, a kunitz domain, an Armadillo repeat protein, an autoantigen, a receptor or a ligand. In some embodiments, the antigen specific domain comprises one or more ligands.

Also provided herein are polypeptides encoded by the polynucleotides described herein. In various embodiments, polypeptides encoded by the polynucleotide molecule encoding the CAR component of the conventional CARs I to III **(Table 1)** or the backbones **(Table** 2) described herein encodes one or more antigen specific domains. In some embodiments, the antigen specific domain comprises one or more V_{L} (or vL) fragments. In some embodiments, the antigen specific domain comprises one or more V_{H} (or vH) fragments. In some embodiments, the antigen specific domain comprises one or more scFVs specific to the antigens on target cells such as cancer cells. In some embodiments, the antigen specific domain comprises one or more Fv fragments. In some embodiments, the antigen specific domain comprises one or more Fab fragments. In some embodiments, the antigen specific domain comprises one or more (Fab')2 fragments. In some embodiments, the antigen specific domain comprises one or more single domain antibodies (SDAB). In some embodiments, the antigen specific domain comprises one or more camelid V_{HH} (vHH) domains. In some embodiments, the antigen specific domain comprises one or more Fv fragments. In some embodiments, the antigen specific domain comprises one or more non-immunoglobulin scaffold such as a DARPIN, an affibody, an affilin, an adnectin, an affitin, an obodies, a repebody, a fynomer, an alphabody, an avimer, an atrimer, a centyrin, a pronectin, an anticalin, a kunitz domain, an Armadillo repeat protein, an autoantigen, a receptor or a ligand. In some embodiments, the antigen specific domain comprises one or more ligands.

Further provided herein are one or more vectors comprising nucleic acids encoding any of conventional CARS I to III **(Table 1)** or backbones 1-62 **(Table 2).** In some embodiments, all the components of each backbone are encoded by a single vector. In some embodiments, each component of the backbone is encoded by a different vector. For examples, a backbone comprising a conventional CAR component and a single accessory module, a first vector comprises polynucleotides encoding the conventional CAR component and a second vector comprises polynucleotides encoding the accessory module. For example, a backbone comprising a conventional CAR component and a more than one accessory module, a first vector comprises polynucleotides encoding the conventional CAR component and a second vector comprises polynucleotides encoding the accessory modules. Alternately, a first vector comprises polynucleotides encoding the conventional CAR component, a second vector comprises polynucleotides encoding the first accessory module and a third vector comprises polynucleotides encoding the second accessory module. In various embodiments, the vectors encoding the polynucleotide molecule encoding the CAR component of the conventional CARs I to III or the backbones described herein encodes one or more antigen specific domains. In some embodiments, the antigen specific domain comprises one or more V_{L} fragments. In some embodiments, the antigen specific domain comprises one or more V_{H} fragments. In some embodiments, the antigen specific domain comprises one or more scFVs specific to the antigens on target cells such as cancer cells. In some embodiments, the antigen specific domain comprises one or more Fv fragments. In some embodiments, the antigen specific domain comprises one or more Fab fragments. In some embodiments, the antigen specific domain comprises one or more (Fab')2 fragments. In some embodiments, the antigen specific domain comprises one or more single domain antibodies (SDAB). In some embodiments, the antigen specific domain comprises one or more camelid V_{HH} domains. In some embodiments, the antigen specific domain comprises one or more Fv fragments. In some embodiments, the antigen specific domain comprises one or more non-immunoglobulin scaffold such as a DARPIN, an affibody, an affilin, an adnectin, an affitin, an obodies, a repebody, a fynomer, an alphabody, an avimer, an atrimer, a centyrin, a pronectin, an anticalin, a kunitz domain, an Armadillo repeat protein, an autoantigen, a receptor or a ligand. In some embodiments, the antigen specific domain comprises one or more ligand.

Further provided herein are genetically modified cells comprising vectors encoding polynucleotides encoding the conventional CARs I to III or backbones 1-62 described herein, wherein the CARs of the conventional CARs I to III or the backbones comprise antigen specific domains as described herein. In various embodiments, the genetically modified cells target antigen on target cells such as cancer cells via the V_{L} fragments, V_{H} fragments, V_{HH} domains, scFvs, Fv fragments, Fab fragments, (Fab')2 fragments, single domain antibody (SDAB) fragments, and/or non-immunoglobulin scaffold each of which are encoded by the antigen specific domains.

In some embodiments, the antigen specific domains of the CARs comprise one or more V_{L} fragments. In exemplary embodiments, the one or more V_{L} fragments are described in **Table 4.** In some embodiments, the polynucleotides encoding the one more V_{L} fragments comprise, consist of or consist essentially of sequences set forth in any one or more of SEQ ID NOs: 11-249. In some embodiments, the polypeptides encoding the one more V_{L} fragments comprise, consist of or consist essentially of sequences set forth in any one or more of SEQ ID NOS: 1785-2023 or sequences with 80-99% identity to sequences set forth in any one or more of SEQ ID NOS: 1785-2023 or sequences with 80-99% identity in the three complementarity determining regions (CDRs) to the sequences set forth in any one or more of SEQ ID NOS: 1785-2023 or sequences that bind to the same target antigens or the same epitopes on the target antigens as the sequences set forth in any one or more of SEQ ID NOS: 1785-2023. In some embodiments, the antigen specific domains of the CARs comprise one or more V_{H} fragments. In exemplary embodiments, the one or more V_{H} fragments are described in **Table 6.** In some embodiments, the polynucleotides encoding the one more V_{H} fragments comprise, consist of or consist essentially of sequences set forth in any one or more of SEQ ID NOS: 259-498. In some embodiments, the polypeptides encoding the one more V_{H} fragments comprise, consist of or consist essentially of sequences set forth in any one or more of SEQ ID NOS: 2028-2268 or sequences with 80-99% identity to sequences set forth in any one or more of SEQ ID NOS: 2028-2268 or sequences with 80-99% identity in the three complementarity determining regions (CDRs) to the sequences set forth in any one or more of SEQ ID NOS: 2028-2268 or sequences that bind to the same target antigens or the same epitopes on the target antigens as the sequences set forth in any one or more of SEQ ID NOS:2028-2268.

In some embodiments, the antigen specific domains of the CARs comprise one or more nanobodies (V_{HH} domains). In exemplary embodiments, the one or more V_{HH} domains are described in **Table** 7. In some embodiments, the polynucleotides encoding the one more V_{HH} fragments comprise, consist of or consist essentially of sequences set forth in any one or more of SEQ ID NOS: 499-523. In some embodiments, the polypeptides encoding the one more V_{HH} fragments comprise, consist of or consist essentially of sequences set forth in any one or more of SEQ ID NOS: 2269-2293 or sequences with 80-99% identity to sequences set forth in any one or more of SEQ ID NOS: 2269-2293 or sequences with 80-99% identity in the three complementarity determining regions (CDRs) to sequences set forth in any one or more of SEQ ID NOS: 2269-2293 or sequences that bind to the same target antigens or the same epitopes on the target antigens as the sequences set forth in any one or more of SEQ ID NOS: 2269-2293.

In some embodiments, the antigen specific domains of the CARs comprise one or more non-immunoglobulin antigen binding scaffolds. In exemplary embodiments, the one or more non-immunoglobulin antigen binding scaffolds are described in **Table 8.** In some embodiments, the polynucleotides encoding the one more non-immunoglobulin antigen binding scaffolds comprise, consist of or consist essentially of sequences set forth in any one or more of SEQ ID NOS: 524-528. In some embodiments, the polypeptides encoding the one more non-immunoglobulin antigen binding scaffolds comprise, consist of or consist essentially of sequences set forth in any one or more of SEQ ID NOS: 2294-2298 or sequences with 80-99% identity to sequences set forth in any one or more of SEQ ID NOS:2294-2298 or sequences that bind to the same target antigens or the same epitopes on the target antigens as the sequences set forth in any one or more of SEQ ID NOS: 2269-2293.

In some embodiments, the antigen specific domains of the CARs comprise one or more ligands. In exemplary embodiments, the ligands are described in **Table 10.** In some embodiments, the polynucleotides encoding the one more ligands comprise, consist of or consist essentially of sequences set forth in any one or more of SEQ ID NOS: 553-563. In some embodiments, the polypeptides encoding the one more ligand binding domains comprise, consist of or consist essentially of sequences set forth in any one or more of SEQ ID NOS: 2323-2333 or sequences with 80-99% identity to sequences set forth in any one or more of SEQ ID NOS: 2323-2333 or sequences that bind to the same target antigens or the same epitopes on the target antigens as the sequences set forth in any one or more of SEQ ID NOS: 2323-2333.

In some embodiments, the antigen specific domains of the CARs comprise one or more scFvs. In exemplary embodiments, the scFv fragments are described in **Table 11.** In some embodiments, the polynucleotides encoding the one more scFv fragments comprise, consist of or consist essentially of sequences set forth in any one or more of SEQ ID NOS: 564-798. In some embodiments, the polynucleotides encoding the one more scFv fragments comprise, consist of or consist essentially of sequences that encode for polypeptide sequences set forth in any one or more of SEQ ID NOS: 2334-2568 or that encode for polypeptide sequences with 80-99% identity in the six complementarity determining regions (CDRs) to sequences set forth in any one or more of SEQ ID NOS: 2334-2568 or that encode for polypeptide sequences that bind to the same target antigens or the same epitopes on the target antigens as the sequences set forth in any one or more of SEQ ID NOS: 2334-2568. In some embodiments, the polypeptides encoding the one more scFv fragments comprise, consist of or consist essentially of sequences set forth in any one or more of SEQ ID NOS: 2334-2568 or sequences with 80-99% identity to sequences set forth in any one or more of SEQ ID NOS: 2334-2568 or sequences with 80-99% identity in the six complementarity determining regions (CDRs) to sequences set forth in any one or more of SEQ ID NOS: 2334-2568 or sequences that bind to the same target antigens or the same epitopes on the target antigens as the sequences set forth in any one or more of SEQ ID NOS: 2334-2568.

In some embodiments, provided herein are polynucleotides encoding conventional CAR **(Table 1)** and any of backbones 1-62 as described herein in **Table 2,** wherein each backbone comprises a conventional CAR component **(Table 1)** and one or more accessory modules as shown in **Table 2.** In some embodiments, the conventional CAR component and the one or more accessory components of each backbone are encoded by a single polynucleotide molecule. In some embodiments, the conventional CAR component is encoded by a first polynucleotide molecule and the one or more accessory modules are encoded by a second polynucleotide molecule. In some embodiments, backbones comprising a conventional CAR component and more than one accessory module, the first polynucleotide molecule encodes the conventional CAR component, a second polynucleotide molecule encodes the first accessory molecule and a third polynucleotide molecule encodes the second accessory molecule.

In some embodiments, provided herein are polypeptides encoding conventional CAR **(Table 1)** and any of backbones 1-62 as described herein in **Table 2,** wherein each backbone comprises a conventional CAR component **(Table 1)** and one or more accessory modules as shown in **Table 2.** In some embodiments, the polypeptide encoding the conventional CAR component and the one or more accessory module components of each backbone are encoded by a single polynucleotide molecule. In some embodiments, the polypeptide encoding the conventional CAR component is encoded by a first polynucleotide molecule and the one or more accessory modules are encoded by a second polynucleotide molecule. In some embodiments, the polypeptide encoding the conventional CAR component and the one or more accessory module components of each backbone are encoded by a single polypeptide molecule. In some embodiments, the polypeptide encoding the conventional CAR component is encoded by a first polypeptide molecule and the one or more accessory modules are encoded by a second or more polypeptide molecules. In some embodiments, backbones comprising a conventional CAR component and more than one accessory module, the first polynucleotide molecule encodes the conventional CAR component, a second polynucleotide molecule encodes the first accessory molecule and a third polynucleotide molecule encodes the second accessory molecule. In some embodiments, backbones comprising a conventional CAR component and more than one accessory module, the first polypeptide molecule encodes the conventional CAR component, a second polypeptide molecule encodes the first accessory molecule and a third polypeptide molecule encodes the second accessory molecule.

In some embodiments, the polynucleotides encoding the one more accessory module comprise, consist of or consist essentially of sequences set forth in any one or more of SEQ ID NOS: 850-904. In some embodiments, the polynucleotides encoding the one more accessory module comprise, consist of or consist essentially of sequences that encode for polypeptide sequences as set forth in any one or more of SEQ ID NOS: 2613-2667 or encode for polypeptide sequences with 70-99% identity to sequences set forth in any one or more of SEQ ID NOS: 2613-2667. In some embodiments, the polypeptides encoding the one more accessory modules comprise, consist of or consist essentially of sequences set forth in any one or more of SEQ ID NOS: 2613-2667 or sequences with 70-99% identity to sequences set forth in any one or more of SEQ ID NOS: 2613-2667.

In exemplary embodiments, nucleic acids encoding backbone-1 comprising conventional CAR I and K13-vFLIP, wherein the antigen specific domain of the CAR targets antigens of interest are described in **Table 19.** The sequences of nucleic acid fragments encoding backbone-1 comprising conventional CAR I and K13-vFLIP and targeting antigens of interest as described in **Table 19** are set forth in SEQ ID NOs: 927-1196. The sequences of polypeptides encoding backbone-1 comprising conventional CAR I and K13-vFLIP and targeting antigens of interest as described in **Table 19** are set forth in SEQ ID NOs: 2672-2941. The nucleic acid sequences in SEQ ID NOs: 927-1196 and polypeptide sequences in SEQ ID NOs: 2672-2941 also encode for a puromycin acetyl transferase (PuroR) fragment, which fragment can be used to select cells but is not essential for the function of the CAR.

In one embodiment, provided herein is an isolated nucleic acid encoding backbone-1 comprising conventional CAR I and K13-vFLIP, wherein the antigen specific domain of the CAR targets CD19 **(Table 19).** In exemplary embodiments, the sequences of isolated nucleic acid fragments targeting CD19 and coexpressing K13-vFLIP are set forth in SEQ ID NOs: 927-941. In exemplary embodiments, the sequences of isolated polypeptide targeting CD19 and coexpressing K13-vFLIP are set forth in SEQ ID NOs: 2672-2686. In some embodiments, the scFv fragments targeting CD19 are described in **Table 11** and set forth in SEQ ID NOs: 564-577 and SEQ ID NOs: 2334-2347. Also provided herein are polypeptides encoded by nucleic acids encoding backbone-1 comprising conventional CAR I and K13-vFLIP, wherein the antigen specific domain of the CAR targets CD19. Further provided herein are vectors encoding nucleic acids encoding backbone-1 comprising conventional CAR I and K13-vFLIP, wherein the antigen specific domain of the CAR targets CD19. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets CD19.

In one embodiment, provided herein is an isolated nucleic acid encoding backbone-1 comprising conventional CAR I and K13-vFLIP, wherein the antigen specific domain of the CAR targets MPL or the Thrombopoietin Receptor **(Table 19).** In exemplary embodiments, the sequences of isolated nucleic acid fragments targeting MPL and coexpressing K13-vFLIP are set forth in SEQ ID NOs: 1117-1124. In exemplary embodiments, the sequences of isolated polypeptide targeting MPL are set forth in SEQ ID NOs: 2862-2869. In some embodiments, the scFv fragments targeting MPL are described in **Table 11** and set forth in SEQ ID NOs: 729-736 and SEQ ID NOs: 2499-2506. Also provided herein are polypeptides encoded by nucleic acids encoding backbone-1 comprising conventional CAR I and K13-vFLIP, wherein the antigen specific domain of the CAR targets MPL. Further provided herein are vectors encoding nucleic acids encoding backbone-1 comprising conventional CAR I and K13-vFLIP, wherein the antigen specific domain of the CAR targets MPL. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets MPL.

In one embodiment, provided herein is an isolated nucleic acid encoding backbone-1 comprising conventional CAR I and K13-vFLIP, wherein the antigen specific domain of the CAR targets Lym1 **(Table 19).** In exemplary embodiments, the sequence of nucleic acid fragment targeting Lym1 is set forth in SEQ ID NO: 1109. In exemplary embodiments, the sequence of isolated polypeptide fragment targeting Lym1 is set forth in SEQ ID NO: 2854. In some embodiments, the scFv fragment targeting Lym1 is described in **Table 11** and set forth in SEQ ID NO: 723 and SEQ ID NO: 2493. Also provided herein are polypeptides encoded by nucleic acids encoding backbone-1 comprising conventional CAR I and K13-vFLIP, wherein the antigen specific domain of the CAR targets Lym1. Further provided herein are vectors encoding nucleic acids encoding backbone-1 comprising conventional CAR I and K13-vFLIP, wherein the antigen specific domain of the CAR targets Lym1. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets Lym1.

In one embodiment, provided herein is an isolated nucleic acid encoding backbone-1 comprising conventional CAR I and K13-vFLIP, wherein the antigen specific domain of the CAR targets Lym2 **(Table 19).** In exemplary embodiments, sequence of the isolated nucleic acid fragment targeting Lym2 is set forth in SEQ ID NO: 1110. In exemplary embodiments, the sequence of isolated polypeptide targeting Lym2 is set forth in SEQ ID NO: 2855. In some embodiments, the scFv fragment targeting Lym2 is described in **Table 11** and set forth in SEQ ID NO: 724 and SEQ ID NO: 2494. Also provided herein are polypeptides encoded by nucleic acids encoding backbone-1 comprising conventional CAR I and K13-vFLIP, wherein the antigen specific domain of the CAR targets Lym2. Further provided herein are vectors encoding nucleic acids encoding backbone-1 comprising conventional CAR I and K13-vFLIP, wherein the antigen specific domain of the CAR targets Lym2. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets Lym2.

In one embodiment, provided herein is an isolated nucleic acid encoding backbone-1 comprising conventional CAR I and K13-vFLIP, wherein the antigen specific domain of the CAR targets GRP-78 **(Table 19).** In exemplary embodiments, sequence of the isolated nucleic acid targeting GRP-78 is set forth in SEQ ID NO: 1078. In exemplary embodiments, sequence of the polypeptide targeting GRP-78 is set forth in SEQ ID NO: 2823. In some embodiments, the scFv fragment targeting GRP-78 is described in **Table 11** and set forth in SEQ ID NO: 703 and SEQ ID NO: 2473. Also provided herein are polypeptides encoded by nucleic acids encoding backbone-1 comprising conventional CAR I and K13-vFLIP, wherein the antigen specific domain of the CAR targets GRP-78. Further provided herein are vectors encoding nucleic acids encoding backbone-1 comprising conventional CAR I and K13-vFLIP, wherein the antigen specific domain of the CAR targets GRP-78. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets GRP-78.

In one embodiment, provided herein is an isolated nucleic acid encoding backbone-1 comprising conventional CAR I and K13-vFLIP, wherein the antigen specific domain of the CAR targets CD79b **(Table 19).** In exemplary embodiments, the sequences of the nucleic acid fragments targeting CD79b are set forth in SEQ ID NOs: 995-996. In exemplary embodiments, the sequences of the polypeptide fragments targeting CD79b are set forth in SEQ ID NO: 2740-2741. In some embodiments, the scFv fragment targeting CD79b is described in **Table 11** and set forth in SEQ ID NO: 628 and SEQ ID NO: 2398. Also provided herein are polypeptides encoded by nucleic acids encoding backbone-1 comprising conventional CAR I and K13-vFLIP, wherein the antigen specific domain of the CAR targets CD79b. Further provided herein are vectors encoding nucleic acids encoding backbone-1 comprising conventional CAR I and K13-vFLIP, wherein the antigen specific domain of the CAR targets CD79b. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets CD79b.

In one embodiment, provided herein is an isolated nucleic acid encoding backbone-1 comprising conventional CAR I and K13-vFLIP, wherein the antigen specific domain of the CAR targets ALK **(Table 19).** In exemplary embodiments, the sequences of the nucleic acid fragments targeting ALK are set forth in SEQ ID NOs: 946-947. In exemplary embodiments, the sequences of the polypeptide fragments targeting ALK are set forth in SEQ ID NO: 2691-2692. In some embodiments, the scFv fragments targeting ALK are described in **Table 11** and set forth in SEQ ID NO: 582-583 and SEQ ID NO: 2352-2353. Also provided herein are polypeptides encoded by nucleic acids encoding backbone-1 comprising conventional CAR I and K13-vFLIP, wherein the antigen specific domain of the CAR targets ALK. Further provided herein are vectors encoding nucleic acids encoding backbone-1 comprising conventional CAR I and K13-vFLIP, wherein the antigen specific domain of the CAR targets ALK. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets ALK.

In one embodiment, provided herein is an isolated nucleic acid encoding backbone-1 comprising conventional CAR I and K13-vFLIP, wherein the antigen specific domain of the CAR targets BCMA **(Table 19).** In exemplary embodiments, the sequences of the nucleic acid fragments targeting BCMA are set forth in SEQ ID NOs: 951-957. In exemplary embodiments, the sequences of the polypeptide fragments targeting BCMA are set forth in SEQ ID NO: 2696-2702. In some embodiments, the scFv fragments targeting BCMA are described in **Table 11** and set forth in SEQ ID NO: 586-592 and SEQ ID NO: 2356-2362. Also provided herein are polypeptides encoded by nucleic acids encoding backbone-1 comprising conventional CAR I and K13-vFLIP, wherein the antigen specific domain of the CAR targets BCMA. Further provided herein are vectors encoding nucleic acids encoding backbone-1 comprising conventional CAR I and K13-vFLIP, wherein the antigen specific domain of the CAR targets BCMA. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets BCMA.

In one embodiment, provided herein is an isolated nucleic acid encoding backbone-1 comprising conventional CAR I and K13-vFLIP, wherein the antigen specific domain of the CAR targets CD20 **(Table 19).** In exemplary embodiments, the sequences of the nucleic acid fragments targeting CD20 are set forth in SEQ ID NOs: 964-976. In exemplary embodiments, the sequences of the polypeptide fragments targeting CD20 are set forth in SEQ ID NO: 2709-2721. In some embodiments, the scFv fragments targeting CD20 are described in **Table 11** and set forth in SEQ ID NO: 596-597;599-610 and SEQ ID NO: 2366-2367;2369-2380. Also provided herein are polypeptides encoded by nucleic acids encoding backbone-1 comprising conventional CAR I and K13-vFLIP, wherein the antigen specific domain of the CAR targets CD20. Further provided herein are vectors encoding nucleic acids encoding backbone-1 comprising conventional CAR I and K13-vFLIP, wherein the antigen specific domain of the CAR targets CD20. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets CD20.

In one embodiment, provided herein is an isolated nucleic acid encoding backbone-1 comprising conventional CAR I and K13-vFLIP, wherein the antigen specific domain of the CAR targets CD22 **(Table 19).** In exemplary embodiments, the sequences of the nucleic acid fragments targeting CD22 are set forth in SEQ ID NOs: 977-980 and SEQ ID NOs: 1192-1196. In exemplary embodiments, the sequences of the polypeptide fragments targeting CD22 are set forth in SEQ ID NOs: 2722-2725 and SEQ ID NOs: 2937-2941. In some embodiments, the scFv fragments targeting CD22 are described in **Table 11** and set forth in SEQ ID NO: 598, 611-613 and SEQ ID NO: 2368, 2381-2384. Also provided herein are polypeptides encoded by nucleic acids encoding backbone-1 comprising conventional CAR I and K13-vFLIP, wherein the antigen specific domain of the CAR targets CD22. Further provided herein are vectors encoding nucleic acids encoding backbone-1 comprising conventional CAR I and K13-vFLIP, wherein the antigen specific domain of the CAR targets CD22. Also provided herein are genetically-engineered cells (such as T cells, NK cells) comprising vectors encoding nucleic acids encoding backbone-1 comprising conventional CAR I and K13-vFLIP, wherein the antigen specific domain of the CAR targets CD22.

In one embodiment, provided herein is an isolated nucleic acid encoding backbone-1 comprising conventional CAR I and K13-vFLIP, wherein the antigen specific domain of the CAR targets CD30 (Table 19). In exemplary embodiments, the sequences of the nucleic acid fragments targeting CD30 are set forth in SEQ ID NOs: 981-982. In exemplary embodiments, the sequences of the polypeptide fragments targeting CD30 are set forth in SEQ ID NOs: 2726-2727. In some embodiments, the scFv fragments targeting CD30 are described in Table 11 and set forth in SEQ ID NO: 614-615 and SEQ ID NO: 2384-2385. Also provided herein are polypeptides encoded by nucleic acids encoding backbone-1 comprising conventional CAR I and K13-vFLIP, wherein the antigen specific domain of the CAR targets CD30. Further provided herein are vectors encoding nucleic acids encoding backbone-1 comprising conventional CAR I and K13-vFLIP, wherein the antigen specific domain of the CAR targets CD30. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets CD30.

In one embodiment, provided herein is an isolated nucleic acid encoding backbone-1 comprising conventional CAR I and K13-vFLIP, wherein the antigen specific domain of the CAR targets CD32 **(Table 19).** In exemplary embodiments, the sequences of the nucleic acid fragment targeting CD32 is set forth in SEQ ID NO: 983. In exemplary embodiments, the sequence of the polypeptide fragments targeting CD32 is set forth in SEQ ID NO: 2728 In some embodiments, the scFv fragment targeting CD32 is described in **Table 11** and set forth in SEQ ID NO: 616 and SEQ ID NO: 2386. Also provided herein are polypeptides encoded by nucleic acids encoding backbone-1 comprising conventional CAR I and K13-vFLIP, wherein the antigen specific domain of the CAR targets CD32. Further provided herein are vectors encoding nucleic acids encoding backbone-1 comprising conventional CAR I and K13-vFLIP, wherein the antigen specific domain of the CAR targets CD32. Also provided herein are genetically-engineered cells (such as T cells, NK cells) comprising vectors encoding nucleic acids encoding backbone-1 comprising conventional CAR I and K13-vFLIP, wherein the antigen specific domain of the CAR targets CD32.

In one embodiment, provided herein is an isolated nucleic acid encoding backbone-1 comprising conventional CAR I and K13-vFLIP, wherein the antigen specific domain of the CAR targets CD33 (Table 19). In exemplary embodiments, the sequences of the nucleic acid fragments targeting CD33 are set forth in SEQ ID NOs: 984-991. In exemplary embodiments, the sequences of the polypeptide fragments targeting CD33 are set forth in SEQ ID NOs: 2729-2736. In some embodiments, the scFv fragments targeting CD33 are described in Table 11 and set forth in SEQ ID NO: 617-624 and SEQ ID NO: 2387-2394. Also provided herein are polypeptides encoded by nucleic acids encoding backbone-1 comprising conventional CAR I and K13-vFLIP, wherein the antigen specific domain of the CAR targets CD33. Further provided herein are vectors encoding nucleic acids encoding backbone-1 comprising conventional CAR I and K13-vFLIP, wherein the antigen specific domain of the CAR targets CD33. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets CD33.

In one embodiment, provided herein is an isolated nucleic acid encoding backbone-1 comprising conventional CAR I and K13-vFLIP, wherein the antigen specific domain of the CAR targets CD123 (Table 19). In exemplary embodiments, the sequences of the nucleic acid fragments targeting CD123 are set forth in SEQ ID NOs: 998-1010. In exemplary embodiments, the sequences of the polypeptide fragments targeting CD123 are set forth in SEQ ID NOs: 2743-2755. In some embodiments, the scFv fragments targeting CD123 are described in Table 11 and set forth in SEQ ID NO: 630-642 and SEQ ID NO: 2400-2412. Also provided herein are polypeptides encoded by nucleic acids encoding backbone-1 comprising conventional CAR I and K13-vFLIP, wherein the antigen specific domain of the CAR targets CD123. Further provided herein are vectors encoding nucleic acids encoding backbone-1 comprising conventional CAR I and K13-vFLIP, wherein the antigen specific domain of the CAR targets CD123. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets CD123.

In one embodiment, provided herein is an isolated nucleic acid encoding backbone-1 comprising conventional CAR I and K13-vFLIP, wherein the antigen specific domain of the CAR targets CD138 **(Table 19).** In exemplary embodiments, the sequences of the nucleic acid fragment targeting CD138 is set forth in SEQ ID NO: 1011. In exemplary embodiments, the sequence of the polypeptide fragments targeting CD138 is set forth in SEQ ID NO: 2756 In some embodiments, the scFv fragment targeting CD138 is described in **Table 11** and set forth in SEQ ID NOs: 643 and 2413. Also provided herein are polypeptides encoded by nucleic acids encoding backbone-1 comprising conventional CAR I and K13-vFLIP, wherein the antigen specific domain of the CAR targets CD138. Further provided herein are vectors encoding nucleic acids encoding backbone-1 comprising conventional CAR I and K13-vFLIP, wherein the antigen specific domain of the CAR targets CD138. Also provided herein are genetically-engineered cells (such as T cells, NK cells) comprising vectors encoding nucleic acids encoding backbone-1 comprising conventional CAR I and K13-vFLIP, wherein the antigen specific domain of the CAR targets CD138.

In one embodiment, provided herein is an isolated nucleic acid encoding backbone-1 comprising conventional CAR I and K13-vFLIP, wherein the antigen specific domain of the CAR targets CLL1 (Table 19). In exemplary embodiments, the sequences of the nucleic acid fragments targeting CLL1 are set forth in SEQ ID NOs: 1024-1029. In exemplary embodiments, the sequences of the polypeptide fragments targeting CLL1 are set forth in SEQ ID NOs:2769-2774. In some embodiments, the scFv fragments targeting CLL1 are described in Table 11 and set forth in SEQ ID NO: 655-660 and SEQ ID NO: 2425-2430. Also provided herein are polypeptides encoded by nucleic acids encoding backbone-1 comprising conventional CAR I and K13-vFLIP, wherein the antigen specific domain of the CAR targets CLL1. Further provided herein are vectors encoding nucleic acids encoding backbone-1 comprising conventional CAR I and K13-vFLIP, wherein the antigen specific domain of the CAR targets CLL1. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets CLL1.

In one embodiment, provided herein is an isolated nucleic acid encoding backbone-1 comprising conventional CAR I and K13-vFLIP, wherein the antigen specific domain of the CAR targets CS1 (Table 19). In exemplary embodiments, the sequences of the nucleic acid fragments targeting CS1 are set forth in SEQ ID NOs: 1031-1039. In exemplary embodiments, the sequences of the polypeptide fragments targeting CS1 are set forth in SEQ ID NOs:2776-2784. In some embodiments, the scFv fragments targeting CS1 are described in Table 11 and set forth in SEQ ID NO: 662-670 and SEQ ID NO: 2432-2440. Also provided herein are polypeptides encoded by nucleic acids encoding backbone-1 comprising conventional CAR I and K13-vFLIP, wherein the antigen specific domain of the CAR targets CS1. Further provided herein are vectors encoding nucleic acids encoding backbone-1 comprising conventional CAR I and K13-vFLIP, wherein the antigen specific domain of the CAR targets CD79b. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets CS1.

In one embodiment, provided herein is an isolated nucleic acid encoding backbone-1 comprising conventional CAR I and K13-vFLIP, wherein the antigen specific domain of the CAR targets CSF2RA (Table 19). In exemplary embodiments, the sequences of the nucleic acid fragments targeting CSF2RA are set forth in SEQ ID NOs: 1040-1041. In exemplary embodiments, the sequences of the polypeptide fragments targeting CSF2RA are set forth in SEQ ID NOs: 2785-2786. In some embodiments, the scFv fragments targeting CSF2RA are described in Table 11 and set forth in SEQ ID NO: 671-672 and SEQ ID NO: 2441-2442. Also provided herein are polypeptides encoded by nucleic acids encoding backbone-1 comprising conventional CAR I and K13-vFLIP, wherein the antigen specific domain of the CAR targets CSF2RA. Further provided herein are vectors encoding nucleic acids encoding backbone-1 comprising conventional CAR I and K13-vFLIP, wherein the antigen specific domain of the CAR targets CSF2RA. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets CSF2RA.

In one embodiment, provided herein is an isolated nucleic acid encoding backbone-1 comprising conventional CAR I and K13-vFLIP, wherein the antigen specific domain of the CAR targets DLL3 (Table 19). In exemplary embodiments, the sequences of the nucleic acid fragments targeting DLL3 are set forth in SEQ ID NOs: 1044-1045. In exemplary embodiments, the sequences of the polypeptide fragments targeting DLL3 are set forth in SEQ ID NOs: 2789-2790. In some embodiments, the scFv fragments targeting DLL3 are described in Table 11 and set forth in SEQ ID NO: 673-674 and SEQ ID NO: 2443-2444. Also provided herein are polypeptides encoded by nucleic acids encoding backbone-1 comprising conventional CAR I and K13-vFLIP, wherein the antigen specific domain of the CAR targets DLL3. Further provided herein are vectors encoding nucleic acids encoding backbone-1 comprising conventional CAR I and K13-vFLIP, wherein the antigen specific domain of the CAR targets DLL3. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets DLL3.

In one embodiment, provided herein is an isolated nucleic acid encoding backbone-1 comprising conventional CAR I and K13-vFLIP, wherein the antigen specific domain of the CAR targets GPRC5D (Table 19). In exemplary embodiments, the sequences of the nucleic acid fragments targeting GPRC5D are set forth in SEQ ID NOs: 1070-1073. In exemplary embodiments, the sequences of the polypeptide fragments targeting GPRC5D are set forth in SEQ ID NOs: 2815-2818. In some embodiments, the scFv fragments targeting GPRC5D are described in Table 11 and set forth in SEQ ID NO: 695-674 and SEQ ID NO: 2465-2468. Also provided herein are polypeptides encoded by nucleic acids encoding backbone-1 comprising conventional CAR I and K13-vFLIP, wherein the antigen specific domain of the CAR targets GPRC5D. Further provided herein are vectors encoding nucleic acids encoding backbone-1 comprising conventional CAR I and K13-vFLIP, wherein the antigen specific domain of the CAR targets GPRC5D. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets GPRC5D.

In one embodiment, provided herein is an isolated nucleic acid encoding backbone-1 comprising conventional CAR I and K13-vFLIP, wherein the antigen specific domain of the CAR targets HIV1-envelop glycoprotein gp120 (Table 19). In exemplary embodiments, the sequences of the nucleic acid fragments targeting HIV1-envelop glycoprotein gp120 are set forth in SEQ ID NOs: 959, 1089-1092. In exemplary embodiments, the sequences of the polypeptide fragments targeting HIV1-envelop glycoprotein gp120 are set forth in SEQ ID NOs: 2704, 2834-2837. In some embodiments, the scFv fragments targeting HIV1-envelop glycoprotein gp120 are described in Table 11 and set forth in SEQ ID NO: 581, 705-708 and SEQ ID NO: 2351, 2475-2478. Also provided herein are polypeptides encoded by nucleic acids encoding backbone-1 comprising conventional CAR I and K13-vFLIP, wherein the antigen specific domain of the CAR targets HIV1-envelop glycoprotein gp120. Further provided herein are vectors encoding nucleic acids encoding backbone-1 comprising conventional CAR I and K13-vFLIP, wherein the antigen specific domain of the CAR targets HIV1-envelop glycoprotein gp120. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets HIV1-envelop glycoprotein gp120.

In one embodiment, provided herein is an isolated nucleic acid encoding backbone-1 comprising conventional CAR I and K13-vFLIP, wherein the antigen specific domain of the CAR targets IL11Ra **(Table 19).** In exemplary embodiments, the sequences of the nucleic acid fragment targeting IL11Ra is set forth in SEQ ID NO: 1099. In exemplary embodiments, the sequence of the polypeptide fragments targeting IL11Ra is set forth in SEQ ID NO: 2844 In some embodiments, the scFv fragments targeting IL11Ra is described in **Table 11** and set forth in SEQ ID NOs: 715 and 2485. Also provided herein are polypeptides encoded by nucleic acids encoding backbone-1 comprising conventional CAR I and K13-vFLIP, wherein the antigen specific domain of the CAR targets IL11Ra. Further provided herein are vectors encoding nucleic acids encoding backbone-1 comprising conventional CAR I and K13-vFLIP, wherein the antigen specific domain of the CAR targets IL11Ra. Also provided herein are genetically-engineered cells (such as T cells, NK cells) comprising vectors encoding nucleic acids encoding backbone-1 comprising conventional CAR I and K13-vFLIP, wherein the antigen specific domain of the CAR targets IL11Ra.

In one embodiment, provided herein is an isolated nucleic acid encoding backbone-1 comprising conventional CAR I and K13-vFLIP, wherein the antigen specific domain of the CAR targets IL13Ra2 (Table 19). In exemplary embodiments, the sequences of the nucleic acid fragments targeting IL13Ra2 are set forth in SEQ ID NOs: 1101-1102. In exemplary embodiments, the sequences of the polypeptide fragments targeting IL13Ra2 are set forth in SEQ ID NOs: 2846-2847. In some embodiments, the scFv fragments targeting IL13Ra2 are described in Table 11 and set forth in SEQ ID NO: 716-717 and SEQ ID NO: 2486-2487. Also provided herein are polypeptides encoded by nucleic acids encoding backbone-1 comprising conventional CAR I and K13-vFLIP, wherein the antigen specific domain of the CAR targets IL13Ra2. Further provided herein are vectors encoding nucleic acids encoding backbone-1 comprising conventional CAR I and K13-vFLIP, wherein the antigen specific domain of the CAR targets IL13Ra2. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets IL13Ra2.

In one embodiment, provided herein is an isolated nucleic acid encoding backbone-1 comprising conventional CAR I and K13-vFLIP, wherein the antigen specific domain of the CAR targets LAMP1 (Table 19). In exemplary embodiments, the sequences of the nucleic acid fragments targeting LAMP1 are set forth in SEQ ID NOs: 1104-1105. In exemplary embodiments, the sequences of the polypeptide fragments targeting LAMP1 are set forth in SEQ ID NOs: 2849-2850. In some embodiments, the scFv fragments targeting LAMP1 are described in Table 11 and set forth in SEQ ID NO: 719-720 and SEQ ID NO: 2489-2490. Also provided herein are polypeptides encoded by nucleic acids encoding backbone-1 comprising conventional CAR I and K13-vFLIP, wherein the antigen specific domain of the CAR targets LAMP1. Further provided herein are vectors encoding nucleic acids encoding backbone-1 comprising conventional CAR I and K13-vFLIP, wherein the antigen specific domain of the CAR targets LAMP1. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets LAMP1.

In one embodiment, provided herein is an isolated nucleic acid encoding backbone-1 comprising conventional CAR I and K13-vFLIP, wherein the antigen specific domain of the CAR targets NY-BR1 **(Table 19).** In exemplary embodiments, the sequences of the nucleic acid fragment targeting NY-BR1 is set forth in SEQ ID NO: 1131. In exemplary embodiments, the sequence of the polypeptide fragments targeting NY-BR1 is set forth in SEQ ID NO: 2876 In some embodiments, the scFv fragments targeting NY-BR1 are described in **Table 11** and set forth in SEQ ID NOs: 742 and 2512. Also provided herein are polypeptides encoded by nucleic acids encoding backbone-1 comprising conventional CAR I and K13-vFLIP, wherein the antigen specific domain of the CAR targets NY-BR1. Further provided herein are vectors encoding nucleic acids encoding backbone-1 comprising conventional CAR I and K13-vFLIP, wherein the antigen specific domain of the CAR targets NY-BR1. Also provided herein are genetically-engineered cells (such as T cells, NK cells) comprising vectors encoding nucleic acids encoding backbone-1 comprising conventional CAR I and K13-vFLIP, wherein the antigen specific domain of the CAR targets NY-BR1.

In one embodiment, provided herein is an isolated nucleic acid encoding backbone-1 comprising conventional CAR I and K13-vFLIP, wherein the antigen specific domain of the CAR targets TIM1/HAVCR (Table 19). In exemplary embodiments, the sequences of the nucleic acid fragments targeting TIM1/HAVCR are set forth in SEQ ID NOs: 1160-1161. In exemplary embodiments, the sequences of the polypeptide fragments targeting TIM1/HAVCR are set forth in SEQ ID NOs: 2905-2906. In some embodiments, the scFv fragments targeting TIM1/HAVCR are described in Table 11 and set forth in SEQ ID NO: 770-771 and SEQ ID NO: 2540-2541. Also provided herein are polypeptides encoded by nucleic acids encoding backbone-1 comprising conventional CAR I and K13-vFLIP, wherein the antigen specific domain of the CAR targets TIM1/HAVCR. Further provided herein are vectors encoding nucleic acids encoding backbone-1 comprising conventional CAR I and K13-vFLIP, wherein the antigen specific domain of the CAR targets TIM1/HAVCR. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets TIM1/HAVCR.

In one embodiment, provided herein is an isolated nucleic acid encoding backbone-1 comprising conventional CAR I and K13-vFLIP, wherein the antigen specific domain of the CAR targets TROP2 (Table 19). In exemplary embodiments, the sequences of the nucleic acid fragments targeting TROP2 are set forth in SEQ ID NOs: 1165-1166. In exemplary embodiments, the sequences of the polypeptide fragments targeting TROP2 are set forth in SEQ ID NOs: 2910-2911. In some embodiments, the scFv fragments targeting TROP2 are described in Table 11 and set forth in SEQ ID NO: 774-775 and SEQ ID NO: 2544-2545. Also provided herein are polypeptides encoded by nucleic acids encoding backbone-1 comprising conventional CAR I and K13-vFLIP, wherein the antigen specific domain of the CAR targets TROP2. Further provided herein are vectors encoding nucleic acids encoding backbone-1 comprising conventional CAR I and K13-vFLIP, wherein the antigen specific domain of the CAR targets TROP2. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets TROP2.

In one embodiment, provided herein is an isolated nucleic acid encoding backbone-1 comprising conventional CAR I and K13-vFLIP, wherein the antigen specific domain of the CAR targets TSHR (Table 19). In exemplary embodiments, the sequences of the nucleic acid fragments targeting TSHR are set forth in SEQ ID NOs: 1167-1170. In exemplary embodiments, the sequences of the polypeptide fragments targeting TSHR are set forth in SEQ ID NOs: 2912-2914. In some embodiments, the scFv fragments targeting TSHR are described in Table 11 and set forth in SEQ ID NO: 776-778 and SEQ ID NO: 2546-2548. Also provided herein are polypeptides encoded by nucleic acids encoding backbone-1 comprising conventional CAR I and K13-vFLIP, wherein the antigen specific domain of the CAR targets TSHR. Further provided herein are vectors encoding nucleic acids encoding backbone-1 comprising conventional CAR I and K13-vFLIP, wherein the antigen specific domain of the CAR targets TSHR. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets TSHR.

In one embodiment, provided herein is an isolated nucleic acid encoding backbone-1 comprising conventional CAR I and K13-vFLIP, wherein the antigen specific domain of the CAR targets TSLPR **(Table 19).** In exemplary embodiments, the sequences of the nucleic acid fragment targeting TSLPR is set forth in SEQ ID NO: 1171. In exemplary embodiments, the sequence of the polypeptide fragment targeting TSLPR is set forth in SEQ ID NO: 2916 In some embodiments, the scFv fragment targeting TSLPR is described in **Table 11** and set forth in SEQ ID NOs: 779 and 2549. Also provided herein is a polypeptide encoded by a nucleic acid fragment encoding backbone-1 comprising conventional CAR I and K13-vFLIP, wherein the antigen specific domain of the CAR targets TSLPR. Further provided herein are vectors encoding nucleic acids encoding backbone-1 comprising conventional CAR I and K13-vFLIP, wherein the antigen specific domain of the CAR targets TSLPR. Also provided herein are genetically-engineered cells (such as T cells, NK cells) comprising vectors encoding nucleic acids encoding backbone-1 comprising conventional CAR I and K13-vFLIP, wherein the antigen specific domain of the CAR targets TSLPR.

In one embodiment, provided herein is an isolated nucleic acid encoding backbone-1 comprising conventional CAR I and K13-vFLIP, wherein the antigen specific domain of the CAR targets CDH19 **(Table 19).** In exemplary embodiments, the sequences of the nucleic acid fragments targeting CDH19 are set forth in SEQ ID NO: 1019 and 1180. In exemplary embodiments, the sequences of the polypeptide fragments targeting CDH19 are set forth in SEQ ID NO: 2764 and 2925 In some embodiments, the scFv fragments targeting CDH19 are described in **Table 11** and set forth in SEQ ID NOs: 651, 788 and SEQ ID NOs: 2421 and 2558. Also provided herein are polypeptides encoded by nucleic acids encoding backbone-1 comprising conventional CAR I and K13-vFLIP, wherein the antigen specific domain of the CAR targets CDH19. Further provided herein are vectors encoding nucleic acids encoding backbone-1 comprising conventional CAR I and K13-vFLIP, wherein the antigen specific domain of the CAR targets CDH19. Also provided herein are genetically-engineered cells (such as T cells, NK cells) comprising vectors encoding nucleic acids encoding backbone-1 comprising conventional CAR I and K13-vFLIP, wherein the antigen specific domain of the CAR targets CDH19.

In one embodiment, provided herein is an isolated nucleic acid encoding backbone-1 comprising conventional CAR I and K13-vFLIP, wherein the antigen specific domain of the CAR targets CDH6 (Table 19). In exemplary embodiments, the sequences of the nucleic acid fragments targeting CDH6 are set forth in SEQ ID NOs: 1017-1018. In exemplary embodiments, the sequences of the polypeptide fragments targeting CDH6 are set forth in SEQ ID NOs: 2761-2762. In some embodiments, the scFv fragments targeting CDH6 are described in Table 11 and set forth in SEQ ID NO: 648-649 and SEQ ID NO: 2418-2419. Also provided herein are polypeptides encoded by nucleic acids encoding backbone-1 comprising conventional CAR I and K13-vFLIP, wherein the antigen specific domain of the CAR targets CDH6. Further provided herein are vectors encoding nucleic acids encoding backbone-1 comprising conventional CAR I and K13-vFLIP, wherein the antigen specific domain of the CAR targets CDH6. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets CDH6.

In one embodiment, provided herein is an isolated nucleic acid encoding backbone-1 comprising conventional CAR I and K13-vFLIP, wherein the antigen specific domain of the CAR targets CD324 (Table 19). In exemplary embodiments, the sequences of the nucleic acid fragments targeting CD324 are set forth in SEQ ID NOs: 1014-1015. In exemplary embodiments, the sequences of the polypeptide fragments targeting CD324 are set forth in SEQ ID NOs: 2759-2760. In some embodiments, the scFv fragments targeting CD324 are described in Table 11 and set forth in SEQ ID NO: 646-647 and SEQ ID NO: 2416-2417. Also provided herein are polypeptides encoded by nucleic acids encoding backbone-1 comprising conventional CAR I and K13-vFLIP, wherein the antigen specific domain of the CAR targets CD324. Further provided herein are vectors encoding nucleic acids encoding backbone-1 comprising conventional CAR I and K13-vFLIP, wherein the antigen specific domain of the CAR targets CD324. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets CD324.

In one embodiment, provided herein is an isolated nucleic acid encoding backbone-1 comprising conventional CAR I and K13-vFLIP, wherein the antigen specific domain of the CAR targets TCR gamma-delta (TCRgd) **(Table 19).** In exemplary embodiments, the sequence of the nucleic acid fragment targeting TCR gamma-delta (TCRgd) is set forth in SEQ ID NO: 1155. In exemplary embodiments, the sequence of the polypeptide fragments targeting TCR gamma-delta (TCRgd) is set forth in SEQ ID NO: 2900 In some embodiments, the scFv fragment targeting TCR gamma-delta (TCRgd) is described in **Table 11** and set forth in SEQ ID NOs: 765 and SEQ ID NOs: 2535. Also provided herein are polypeptides encoded by nucleic acids encoding backbone-1 comprising conventional CAR I and K13-vFLIP, wherein the antigen specific domain of the CAR targets TCR gamma-delta (TCRgd). Further provided herein are vectors encoding nucleic acids encoding backbone-1 comprising conventional CAR I and K13-vFLIP, wherein the antigen specific domain of the CAR targets TCR gamma-delta (TCRgd). Also provided herein are genetically-engineered cells (such as T cells, NK cells) comprising vectors encoding nucleic acids encoding backbone-1 comprising conventional CAR I and K13-vFLIP, wherein the antigen specific domain of the CAR targets TCR gamma-delta (TCRgd).

In one embodiment, provided herein is an isolated nucleic acid encoding backbone-1 comprising conventional CAR I and K13-vFLIP, wherein the antigen specific domain of the CAR targets TCRB1 (TCR beta 1 constant chain) **(Table 19).** In exemplary embodiments, the sequences of the nucleic acid fragments targeting TCRB1 (TCR beta 1 constant chain) are set forth in SEQ ID NOs: 1151-1152. In exemplary embodiments, the sequences of the polypeptide fragments targeting TCRB1 (TCR beta 1 constant chain) are set forth in SEQ ID NOs: 2896-2897. In some embodiments, the scFv fragments targeting TCRB1 (TCR beta 1 constant chain) are described in Table 11 and set forth in SEQ ID NO: 761-762 and SEQ ID NO: 2531-2532. Also provided herein are polypeptides encoded by nucleic acids encoding backbone-1 comprising conventional CAR I and K13-vFLIP, wherein the antigen specific domain of the CAR targets TCRB1 (TCR beta 1 constant chain). Further provided herein are vectors encoding nucleic acids encoding backbone-1 comprising conventional CAR I and K13-vFLIP, wherein the antigen specific domain of the CAR targets TCRB1 (TCR beta 1 constant chain). Also provided herein are genetically engineered cells (such as T cells, NK cells) comprising vectors encoding nucleic acids encoding backbone-1 comprising conventional CAR I and K13-vFLIP, wherein the antigen specific domain of the CAR targets TCRB1 (TCR beta 1 constant chain).

In one embodiment, provided herein is an isolated nucleic acid encoding backbone-1 comprising conventional CAR I and K13-vFLIP, wherein the antigen specific domain of the CAR targets TCRB2 (TCR beta 2 constant chain) **(Table 19).** In exemplary embodiments, the sequences of the nucleic acid fragments targeting TCRB2 (TCR beta 2 constant chain) are set forth in SEQ ID NOs: 1153-1154. In exemplary embodiments, the sequences of the polypeptide fragments targeting TCRB2 (TCR beta 2 constant chain) are set forth in SEQ ID NOs: 2898-2899. In some embodiments, the scFv fragments targeting TCRB2 (TCR beta 2 constant chain) are described in Table 11 and set forth in SEQ ID NO: 763-764 and SEQ ID NO: 2533-2534. Also provided herein are polypeptides encoded by nucleic acids encoding backbone-1 comprising conventional CAR I and K13-vFLIP, wherein the antigen specific domain of the CAR targets TCRB2 (TCR beta 2 constant chain). Further provided herein are vectors encoding nucleic acids encoding backbone-1 comprising conventional CAR I and K13-vFLIP, wherein the antigen specific domain of the CAR targets TCRB2 (TCR beta 2 constant chain). Also provided herein are genetically engineered cells (such as T cells, NK cells) comprising vectors encoding nucleic acids encoding backbone-1 comprising conventional CAR I and K13-vFLIP, wherein the antigen specific domain of the CAR targets TCRB2 (TCR beta 2 constant chain).

In one embodiment, provided herein is an isolated nucleic acid encoding backbone-1 comprising conventional CAR I and K13-vFLIP, wherein the antigen specific domain of the CAR targets LHR (Luteinizing hormone Receptor) **(Table 19).** In exemplary embodiments, the sequences of the nucleic acid fragments targeting LHR (Luteinizing hormone Receptor) are set forth in SEQ ID NOs: 1182-1183. In exemplary embodiments, the sequences of the polypeptide fragments targeting LHR (Luteinizing hormone Receptor) are set forth in SEQ ID NOs: 2927-2928. In some embodiments, the scFv fragments targeting LHR (Luteinizing hormone Receptor) are described in Table 11 and set forth in SEQ ID NO: 790-791 and SEQ ID NO: 2560-2561. Also provided herein are polypeptides encoded by nucleic acids encoding backbone-1 comprising conventional CAR I and K13-vFLIP, wherein the antigen specific domain of the CAR targets LHR (Luteinizing hormone Receptor). Further provided herein are vectors encoding nucleic acids encoding backbone-1 comprising conventional CAR I and K13-vFLIP, wherein the antigen specific domain of the CAR targets LHR (Luteinizing hormone Receptor). Also provided herein are genetically engineered cells (such as T cells, NK cells) comprising vectors encoding nucleic acids encoding backbone-1 comprising conventional CAR I and K13-vFLIP, wherein the antigen specific domain of the CAR targets LHR (Luteinizing hormone Receptor).

In one embodiment, provided herein is an isolated nucleic acid encoding backbone-1 comprising conventional CAR I and K13-vFLIP, wherein the antigen specific domain of the CAR targets Fc region of an immunoglobulin **(Table 19).** In exemplary embodiments, the sequences of the nucleic acid fragments targeting Fc region of an immunoglobulin are set forth in SEQ ID NOs: 961-962. In exemplary embodiments, the sequences of the polypeptide fragments targeting Fc region of an immunoglobulin are set forth in SEQ ID NOs: 2707-2708. In some embodiments, the receptor targeting Fc region of an immunoglobulin comprises, consists of or essentially consists of extracellular ligand-binding domain of CD16 (FCGR3A) or its variant V158 or its variant F158. Also provided herein are polypeptides encoded by nucleic acids encoding backbone-1 comprising conventional CAR I and K13-vFLIP, wherein the antigen specific domain of the CAR targets Fc region of an immunoglobulin. Further provided herein are vectors encoding nucleic acids encoding backbone-1 comprising conventional CAR I and K13-vFLIP, wherein the antigen specific domain of the CAR targets Fc region of an immunoglobulin. Also provided herein are genetically engineered cells (such as T cells, NK cells) comprising vectors encoding nucleic acids encoding backbone-1 comprising conventional CAR I and K13-vFLIP, wherein the antigen specific domain of the CAR targets Fc region of an immunoglobulin. The genetically engineered cells comprising vectors encoding nucleic acids encoding backbone-1 comprising conventional CAR I and K13-vFLIP, wherein the antigen specific domain of the CAR targets Fc region of an immunoglobulin can be used to enhance antibody dependent cell mediated cytotoxicity and/or enhance antibody based immunotherapy, such as cancer immunotherapy, as described in US 2015/0139943A1.

In exemplary embodiments, nucleic acids encoding backbone-32 comprising conventional CAR II and K13-vFLIP, wherein the antigen specific domain of the CAR targets antigens of interest are described in **Table 20.** The sequences of nucleic acid fragments encoding backbone-32 comprising conventional CAR II and K13-vFLIP and targeting antigens of interest as described in **Table 20** are set forth in SEQ ID NOs: 1197-1466. The sequences of polypeptides encoding backbone-32 comprising conventional CAR I and K13-vFLIP and targeting antigens of interest as described in **Table 20** are set forth in SEQ ID NOs: 2942-3211. The nucleic acid sequences in SEQ ID NOs: 1197-1466 and polypeptide sequences in SEQ ID NOs: 2942-3211 also encode for a puromycin acetyl transferase (PuroR) fragment, which fragment can be used to select cells and is not essential for the function of the CAR.

In one embodiment, provided herein is an isolated nucleic acid encoding backbone-32 comprising conventional CAR II and K13-vFLIP, wherein the antigen specific domain of the CAR targets CD19 **(Table 20).** In exemplary embodiments, nucleic acid sequences of the V_{L}-V_{H} targeting CD19 are set forth in SEQ ID NOs: 1197-1211. In exemplary embodiments, the polypeptide sequences of the V_{L}-V_{H} targeting CD19 are set forth in SEQ ID NOs: 2942-2956. In some embodiments, the scFv fragments targeting CD19 are described in **Table 11** and set forth in SEQ ID NOs: 564-577 and SEQ ID NOs: 2334-2347. Also provided herein are polypeptides encoded by nucleic acids encoding backbone-32 comprising conventional CAR II and K13-vFLIP, wherein the antigen specific domain of the CAR targets CD19. Further provided herein are vectors encoding nucleic acids encoding backbone-32 comprising conventional CAR II and K13-vFLIP, wherein the antigen specific domain of the CAR targets CD19. Also provided herein are genetically engineered cells (such as T cells, NK cells) comprising vectors encoding nucleic acids encoding backbone-32 comprising conventional CAR II and K13-vFLIP, wherein the antigen specific domain of the CAR targets CD19.

In one embodiment, provided herein is an isolated nucleic acid encoding backbone-32 comprising conventional CAR II and K13-vFLIP, wherein the antigen specific domain of the CAR targets MPL **(Table 20).** In exemplary embodiments, nucleic acid sequences of the V_{L}-V_{H} targeting MPL are set forth in SEQ ID NOs: 1387-1394. In exemplary embodiments, the polypeptide sequences of the V_{L}-V_{H} targeting MPL are set forth in SEQ ID NOs: 3132-3139. In some embodiments, the scFv fragments targeting MPL are described in **Table 11** and set forth in SEQ ID NOs: 729-736 and SEQ ID NOs: 2499-2506. Also provided herein are polypeptides encoded by nucleic acids encoding backbone-32 comprising conventional CAR II and K13-vFLIP, wherein the antigen specific domain of the CAR targets MPL. Further provided herein are vectors encoding nucleic acids encoding backbone-32 comprising conventional CAR II and K13-vFLIP, wherein the antigen specific domain of the CAR targets MPL. Also provided herein are genetically engineered cells (such as T cells, NK cells) comprising vectors encoding nucleic acids encoding backbone-32 comprising conventional CAR II and K13-vFLIP, wherein the antigen specific domain of the CAR targets MPL.

In one embodiment, provided herein is an isolated nucleic acid encoding backbone-32 comprising conventional CAR II and K13-vFLIP, wherein the antigen specific domain of the CAR targets Lym1 **(Table 20).** In exemplary embodiments, the nucleic acid sequence of the V_{L}-V_{H} targeting Lym1 is set forth in SEQ ID NO: 1379. In exemplary embodiments, the polypeptide sequence of the V_{L}-V_{H} targeting Lym1 is set forth in SEQ ID NO: 3124. In some embodiments, the scFv fragment targeting Lym1 is described in **Table 11** and set forth in SEQ ID NO: 723 and SEQ ID NO: 2493. Also provided herein are polypeptides encoded by nucleic acids encoding backbone-32 comprising conventional CAR II and K13-vFLIP, wherein the antigen specific domain of the CAR targets Lym1. Further provided herein are vectors encoding nucleic acids encoding backbone-32 comprising conventional CAR II and K13-vFLIP, wherein the antigen specific domain of the CAR targets Lym1. Also provided herein are genetically engineered cells (such as T cells, NK cells) comprising vectors encoding nucleic acids encoding backbone-32 comprising conventional CAR II and K13-vFLIP, wherein the antigen specific domain of the CAR targets Lym1.

In one embodiment, provided herein is an isolated nucleic acid encoding backbone-32 comprising conventional CAR II and K13-vFLIP, wherein the antigen specific domain of the CAR targets Lym2 **(Table 20).** In exemplary embodiments, nucleic acid sequence of the V_{L}-V_{H} targeting Lym2 is set forth in SEQ ID NO: 1380. In exemplary embodiments, the polypeptide sequence of the V_{L}-V_{H} targeting Lym2 is set forth in SEQ ID NO: 3125. In some embodiments, the scFv fragment targeting Lym2 is described in **Table 11** and set forth in SEQ ID NO: 724 and SEQ ID NO: 2494. Also provided herein are polypeptides encoded by nucleic acids encoding backbone-32 comprising conventional CAR II and K13-vFLIP, wherein the antigen specific domain of the CAR targets Lym2. Further provided herein are vectors encoding nucleic acids encoding backbone-32 comprising conventional CAR II and K13-vFLIP, wherein the antigen specific domain of the CAR targets Lym2. Also provided herein are genetically engineered cells (such as T cells, NK cells) comprising vectors encoding nucleic acids encoding backbone-32 comprising conventional CAR II and K13-vFLIP, wherein the antigen specific domain of the CAR targets Lym2.

In one embodiment, provided herein is an isolated nucleic acid encoding backbone-32 comprising conventional CAR I and K13-vFLIP, wherein the antigen specific domain of the CAR targets GRP-78 **(Table 20).** In exemplary embodiments, the nucleic acid sequence of the V_{L}-V_{H} targeting GRP-78 is set forth in SEQ ID NO: 1348. In exemplary embodiments, the polypeptide sequence of the V_{L}-V_{H} targeting GRP-78 is set forth in SEQ ID NO: 3093. In some embodiments, the scFv fragment targeting GRP-78 is described in **Table 11** and set forth in SEQ ID NO: 703 and SEQ ID NO: 2473. Also provided herein are polypeptides encoded by nucleic acids encoding backbone-32 comprising conventional CAR II and K13-vFLIP, wherein the antigen specific domain of the CAR targets GRP-78. Further provided herein are vectors encoding nucleic acids encoding backbone-32 comprising conventional CAR II and K13-vFLIP, wherein the antigen specific domain of the CAR targets GRP-78. Also provided herein are genetically engineered cells (such as T cells, NK cells) comprising vectors encoding nucleic acids encoding backbone-32 comprising conventional CAR II and K13-vFLIP, wherein the antigen specific domain of the CAR targets GRP-78.

In one embodiment, provided herein is an isolated nucleic acid encoding backbone-32 comprising conventional CAR I and K13-vFLIP, wherein the antigen specific domain of the CAR targets CD79b **(Table 20).** In exemplary embodiments, the nucleic acid sequence of the V_{L}-V_{H} targeting CD79b are set forth in SEQ ID NO: 1381. In exemplary embodiments, the polypeptide sequence of the V_{L}-V_{H} targeting CD79b is set forth in SEQ ID NO: 3126. In some embodiments, the scFv fragment targeting CD79b is described in **Table 11** and set forth in SEQ ID NO: 628 and SEQ ID NO: 2398. Also provided herein are polypeptides encoded by nucleic acids encoding backbone-32 comprising conventional CAR II and K13-vFLIP, wherein the antigen specific domain of the CAR targets CD79b. Further provided herein are vectors encoding nucleic acids encoding backbone-32 comprising conventional CAR II and K13-vFLIP, wherein the antigen specific domain of the CAR targets CD79b. Also provided herein are genetically engineered cells (such as T cells, NK cells) comprising vectors encoding nucleic acids encoding backbone-32 comprising conventional CAR II and K13-vFLIP, wherein the antigen specific domain of the CAR targets CD79b.

In one embodiment, provided herein is an isolated nucleic acid encoding backbone-1 comprising conventional CAR II and K13-vFLIP, wherein the antigen specific domain of the CAR targets Fc region of an immunoglobulin **(Table 20).** In exemplary embodiments, the sequences of the nucleic acid fragments targeting Fc region of an immunoglobulin are set forth in SEQ ID NOs: 1232-1233. In exemplary embodiments, the sequences of the polypeptide fragments targeting Fc region of an immunoglobulin are set forth in SEQ ID NOs: 2977-2978. In some embodiments, the receptor targeting Fc region of an immunoglobulin comprises, consists of or essentially consists of extracellular ligand-binding domain of CD16 (FCGR3A) or its variant V158 or its variant F158. Also provided herein are polypeptides encoded by nucleic acids encoding backbone-1 comprising conventional CAR II and K13-vFLIP, wherein the antigen specific domain of the CAR targets Fc region of an immunoglobulin. Further provided herein are vectors encoding nucleic acids encoding backbone-1 comprising conventional CAR II and K13-vFLIP, wherein the antigen specific domain of the CAR targets Fc region of an immunoglobulin. Also provided herein are genetically engineered cells (such as T cells, NK cells) comprising vectors encoding nucleic acids encoding backbone-1 comprising conventional CAR II and K13-vFLIP, wherein the antigen specific domain of the CAR targets Fc region of an immunoglobulin. The genetically engineered cells comprising vectors encoding nucleic acids encoding backbone-1 comprising conventional CAR II and K13-vFLIP, wherein the antigen specific domain of the CAR targets Fc region of an immunoglobulin can be used to enhance antibody dependent cell mediated cytotoxicity and/or enhance antibody based immunotherapy, such as cancer immunotherapy, as described in US 2015/0139943A1.

In exemplary embodiments, nucleic acids encode conventional CAR II wherein the antigen specific domain of the CAR targets antigens of interest **(Table 21).** The sequences of nucleic acid fragments encoding conventional CAR II and targeting antigens of interest as described in **Table 21** are set forth in SEQ ID NOs: 1467-1730. The sequences of polypeptides comprising conventional CAR II and targeting antigens of interest as described in Table 21 are set forth in SEQ ID NOs: 3212-3475. The nucleic acid sequences in SEQ ID NOs: 1467-1730 and polypeptide sequences in SEQ ID NOs: 3212-3475 also encode for a puromycin acetyl transferase (PuroR) fragment, which fragment can be used to select cells and is not essential for the function of the CAR. Similarly, several CAR constructs described in Table 22 encode for PuroR or Enhanced Green Fluorescent Protein (EGFP), which are not essential for the function of these CAR constructs but may be used to select or enrich for cells expressing these CAR constructs.

In one embodiment, provided herein is an isolated nucleic acid encoding conventional CAR II, wherein the antigen specific domain of the CAR targets CD19 **(Table 21).** In exemplary embodiments, the sequences of isolated nucleic acid fragments targeting CD19 are set forth in SEQ ID NOs: 1470-1481, 1772. In exemplary embodiments, the sequences of isolated polypeptide targeting CD19 are set forth in SEQ ID NOs: 3215-3225, 3517. In some embodiments, the scFv fragments targeting CD19 are described in **Table 11** and set forth in SEQ ID NOs: 566-577 and SEQ ID NOs: 2336-2347. Also provided herein are polypeptides encoded by nucleic acids encoding conventional CAR II, wherein the antigen specific domain of the CAR targets CD19. Further provided herein are vectors encoding nucleic acids encoding conventional CAR II, wherein the antigen specific domain of the CAR targets CD19. Also provided herein are genetically engineered cells (such as T cells, NK cells) comprising vectors encoding nucleic acids encoding conventional CAR II, wherein the antigen specific domain of the CAR targets CD19.

In one embodiment, provided herein is an isolated nucleic acid encoding conventional CAR II, wherein the antigen specific domain of the CAR targets MPL or the Thrombopoietin Receptor **(Table 21).** In exemplary embodiments, the sequences of isolated nucleic acid fragments targeting MPL are set forth in SEQ ID NOs: 1657-1664, 1731-1737. In exemplary embodiments, the sequences of isolated polypeptide targeting MPL are set forth in SEQ ID NOs: 3402-3409, 3476-3484. In some embodiments, the scFv fragments targeting MPL are described in **Table 11** and set forth in SEQ ID NOs: 729-736 and SEQ ID NOs: 2499-2506. Also provided herein are polypeptides encoded by nucleic acids encoding conventional CAR II, wherein the antigen specific domain of the CAR targets MPL. Further provided herein are vectors encoding nucleic acids encoding conventional CAR II, wherein the antigen specific domain of the CAR targets MPL. Also provided herein are genetically engineered cells (such as T cells, NK cells) comprising vectors encoding nucleic acids encoding conventional CAR II, wherein the antigen specific domain of the CAR targets MPL.

In one embodiment, provided herein is an isolated nucleic acid encoding conventional CAR II, wherein the antigen specific domain of the CAR targets Lym1 **(Table** 21). In exemplary embodiments, the sequence of nucleic acid fragment targeting Lym1 is set forth in SEQ ID NO: 1649. In exemplary embodiments, the sequence of isolated polypeptide fragment targeting Lym1 is set forth in SEQ ID NO: 3394. In some embodiments, the scFv fragment targeting Lym1 is described in **Table 11** and set forth in SEQ ID NO: 723 and SEQ ID NO: 2493. Also provided herein are polypeptides encoded by nucleic acids encoding conventional CAR II, wherein the antigen specific domain of the CAR targets Lym1. Further provided herein are vectors encoding nucleic acids encoding conventional CAR II, wherein the antigen specific domain of the CAR targets Lym1. Also provided herein are genetically engineered cells (such as T cells, NK cells) comprising vectors encoding nucleic acids encoding conventional CAR II, wherein the antigen specific domain of the CAR targets Lym1.

In one embodiment, provided herein is an isolated nucleic acid encoding conventional CAR II, wherein the antigen specific domain of the CAR targets Lym2 **(Table 21).** In exemplary embodiments, sequence of the isolated nucleic acid fragment targeting Lym2 is set forth in SEQ ID NO: 1650. In exemplary embodiments, the sequence of isolated polypeptide targeting Lym2 is set forth in SEQ ID NO: 3395. In some embodiments, the scFv fragment targeting Lym2 is described in **Table 11** and set forth in SEQ ID NO: 724 and SEQ ID NO: 2494. Also provided herein are polypeptides encoded by nucleic acids encoding conventional CAR II, wherein the antigen specific domain of the CAR targets Lym2. Further provided herein are vectors encoding nucleic acids encoding conventional CAR II, wherein the antigen specific domain of the CAR targets Lym2. Also provided herein are genetically engineered cells (such as T cells, NK cells) comprising vectors encoding nucleic acids encoding conventional CAR II, wherein the antigen specific domain of the CAR targets Lym2.

In one embodiment, provided herein is an isolated nucleic acid encoding conventional CAR II, wherein the antigen specific domain of the CAR targets DLL3 **(Table 21).** In exemplary embodiments, the sequences of isolated nucleic acid fragments targeting DLL3 are set forth in SEQ ID NOs: 1314-1315. In exemplary embodiments, the sequences of isolated polypeptide targeting DLL3 are set forth in SEQ ID NOs: 3059-3060. In some embodiments, the scFv fragments targeting DLL3 are described in **Table 11** and set forth in SEQ ID NOs: 673-674 and SEQ ID NOs: 2443-2444. Also provided herein are polypeptides encoded by nucleic acids encoding conventional CAR II, wherein the antigen specific domain of the CAR targets DLL3. Further provided herein are vectors encoding nucleic acids encoding conventional CAR II, wherein the antigen specific domain of the CAR targets DLL3. Also provided herein are genetically engineered cells (such as T cells, NK cells) comprising vectors encoding nucleic acids encoding conventional CAR II, wherein the antigen specific domain of the CAR targets DLL3.

In one embodiment, provided herein is an isolated nucleic acid encoding conventional CAR II, wherein the antigen specific domain of the CAR targets CD324 **(Table 21).** In exemplary embodiments, the sequences of isolated nucleic acid fragments targeting CD324 are set forth in SEQ ID NOs: 1554-1555. In exemplary embodiments, the sequences of isolated polypeptide targeting CD324 are set forth in SEQ ID NOs: 3299-3300. In some embodiments, the scFv fragments targeting CD324 are described in **Table 11** and set forth in SEQ ID NOs: 646-647 and SEQ ID NOs: 2416-2417. Also provided herein are polypeptides encoded by nucleic acids encoding conventional CAR II, wherein the antigen specific domain of the CAR targets CD324. Further provided herein are vectors encoding nucleic acids encoding conventional CAR II, wherein the antigen specific domain of the CAR targets CD324. Also provided herein are genetically engineered cells (such as T cells, NK cells) comprising vectors encoding nucleic acids encoding conventional CAR II, wherein the antigen specific domain of the CAR targets CD324.

In one embodiment, provided herein is an isolated nucleic acid encoding conventional CAR II, wherein the antigen specific domain of the CAR targets CD276 **(Table 21).** In exemplary embodiments, the sequence of nucleic acid fragment targeting CD276 is set forth in SEQ ID NO: 1553. In exemplary embodiments, the sequence of isolated polypeptide fragment targeting CD276 is set forth in SEQ ID NO: 3298. In some embodiments, the scFv fragment targeting CD276 is described in **Table 11** and set forth in SEQ ID NO: 645 and SEQ ID NO: 2415. Also provided herein are polypeptides encoded by nucleic acids encoding conventional CAR II, wherein the antigen specific domain of the CAR targets CD276. Further provided herein are vectors encoding nucleic acids encoding conventional CAR II, wherein the antigen specific domain of the CAR targets CD276. Also provided herein are genetically engineered cells (such as T cells, NK cells) comprising vectors encoding nucleic acids encoding conventional CAR II, wherein the antigen specific domain of the CAR targets CD276.

In one embodiment, provided herein is an isolated nucleic acid encoding conventional CAR II, wherein the antigen specific domain of the CAR targets PTK7 **(Table 21).** In exemplary embodiments, the sequences of isolated nucleic acid fragments targeting PTK7 are set forth in SEQ ID NOs: 1683-1684. In exemplary embodiments, the sequences of isolated polypeptide targeting PTK7 are set forth in SEQ ID NOs: 3428-3429. In some embodiments, the scFv fragments targeting PTK7 are described in **Table 11** and set forth in SEQ ID NOs: 753-754 and SEQ ID NOs:2523-2524. Also provided herein are polypeptides encoded by nucleic acids encoding conventional CAR II, wherein the antigen specific domain of the CAR targets PTK7. Further provided herein are vectors encoding nucleic acids encoding conventional CAR II, wherein the antigen specific domain of the CAR targets PTK7. Also provided herein are genetically engineered cells (such as T cells, NK cells) comprising vectors encoding nucleic acids encoding conventional CAR II, wherein the antigen specific domain of the CAR targets PTK7.

In one embodiment, provided herein is an isolated nucleic acid encoding conventional CAR II, wherein the antigen specific domain of the CAR targets HIV1-envelop glycoprotein gp120 **(Table 21).** In exemplary embodiments, sequence of the isolated nucleic acid fragment targeting HIV1-envelop glycoprotein gp120 is set forth in SEQ ID NO: 1485. In exemplary embodiments, the sequence of isolated polypeptide targeting HIV1-envelop glycoprotein gp120 is set forth in SEQ ID NO: 3230. In some embodiments, the scFv fragment targeting HIV1-envelop glycoprotein gp120 is described in **Table 11** and set forth in SEQ ID NO: 581 and SEQ ID NO: 2351. Also provided herein are polypeptides encoded by nucleic acids encoding conventional CAR II, wherein the antigen specific domain of the CAR targets HIV1-envelop glycoprotein gp120. Further provided herein are vectors encoding nucleic acids encoding conventional CAR II, wherein the antigen specific domain of the CAR targets HIV1-envelop glycoprotein gp120. Also provided herein are genetically engineered cells (such as T cells, NK cells) comprising vectors encoding nucleic acids encoding conventional CAR II, wherein the antigen specific domain of the CAR targets HIV1-envelop glycoprotein gp120.

In one embodiment, provided herein is an isolated nucleic acid encoding conventional CAR II, wherein the antigen specific domain of the CAR targets tyrosinase-derived peptides in association with MHC class I complex **(Table 21).** In exemplary embodiments, sequence of the isolated nucleic acid fragments targeting tyrosinase-derived peptides in association with MHC class I complex (HLA-A2) is set forth in SEQ ID NO: 1712-1714. In exemplary embodiments, the sequence of isolated polypeptides targeting tyrosinase-derived peptides in association with MHC class I complex (HLA-A2) is set forth in SEQ ID NO: 3457-3459. In some embodiments, the scFv fragments targeting tyrosinase-derived peptide in association with MHC class II (HLA-A2) complex is described in **Table 11** and set forth in SEQ ID NO:780-782 and SEQ ID NO: 2550-2552. Also provided herein are polypeptides encoded by nucleic acids encoding conventional CAR II, wherein the antigen specific domain of the CAR targets tyrosinase-derived peptides in association with MHC class I complex (HLA-A2). Further provided herein are vectors encoding nucleic acids encoding conventional CAR II, wherein the antigen specific domain of the CARs target tyrosinase-derived peptides in association with MHC class I complex (HLA-A2). Also provided herein are genetically engineered cells (such as T cells, NK cells) comprising vectors encoding nucleic acids encoding conventional CAR II, wherein the antigen specific domain of the CAR targets target tyrosinase-derived peptides in association with MHC class I complex (HLA-A2). In exemplary embodiment, the sequence of the tyrosinase-derived peptide that is targeted by the CAR in association with MHC class I complex (HLA-A2) is - YMDGTMSQV- (Table 23).

In some embodiments, provided herein are vectors comprising nucleic acid sequences encoding the conventional CARs or novel backbones (comprising conventional CARs and accessory modules) described herein. In exemplary embodiments, the vector is any of a DNA vector, an RNA vector, a plasmid, a lentivirus vector, an adenoviral vector or a retrovirus vector. In an embodiment, the vector is a lentivirus vector as described herein. In an embodiment, the vector is pLENTI-EF1α (SEQ ID NO: 905). In another embodiment, the vector is pLenti-EF1a-DWPRE (SEQ ID NO: 906). In another embodiment, the vector is MSCV-Bgl2-AvrII-Bam-EcoR1-Xho-BstB1-Mlu-Sal-ClaI.I03 (SEQ ID NO: 907). In another embodiment, the vector is a sleeping beauty transposon vector. In another embodiment, the sleeping beauty transposon vector is pSBbi-Pur (SEQ ID NO: 908). In one embodiment, the vector further comprises a promoter. Non-limiting examples of a promoter include from an EF-1 promoter, a CMV IE gene promoter, an EF-lα promoter, an ubiquitin C promoter, or a phosphoglycerate kinase (PGK) promoter. In one embodiment, the promoter is an EF-1 promoter (SEQ ID NO: 909). In one embodiment, the promoter is an inducible promoter that provides a molecular switch capable of turning on expression of the polynucleotide sequence encoding conventional CARs I to III and backbones 1-62 which it is operatively linked when such expression is desired, or turning off the expression when expression is not desired. Examples of inducible promoters include, but are not limited to a metallothionine inducible promoter, a glucocorticoid inducible promoter, a progesterone inducible promoter, and a tetracycline inducible promoter. RheoSwitch^{®} system (Agarwal et al, 2012, AACR-NCI-EORTC international conference on Molecular Targets and Cancer Therapeutics) represents another transcriptional regulator platform for controlling the expression of conventional CARs and backbones 1-62 of the present invention. In one embodiment, a combinatorially activated T cell circuit can be engineered in which a synthetic Notch receptor for one antigen induces the expression of a conventitonal CAR or backbones 1-62 targeting a second antigen as described recently for controlling the activity of CARs (Roybal KT et al., Cell, 164:1-10 2016). In one embodiment, the vector is an *in vitro* transcription vector, (for example, a vector that transcribes RNA from a DNA molecule) described herein. In one embodiment, the nucleic acid sequence in the vector further comprises a poly(A) tail, e.g., a poly A tail described herein, e.g., comprising about 100-150 adenosine bases. For example, contemplated herein is a poly(A) tail comprising about 150 adenosine bases (SEQ ID NO: 922). In one embodiment, the nucleic acid sequence in the vector further comprises a 3'UTR, e.g., a 3' UTR described herein, e.g., comprising at least one repeat of a 3'UTR derived from human beta-globulin.

In some embodiments, the vector comprising a nucleic acid sequence encoding a CAR may further comprise a nucleic acid sequence encoding one or more inhibitory molecules. Non-limiting examples of inhibitory molecules contemplated herein include: an inhKIR cytoplasmic domain; a transmembrane domain, e.g., a KIR transmembrane domain; and an inhibitor cytoplasmic domain, e.g., an ITIM domain, e.g., an inhKIR ITIM domain. An exemplary inhibitory molecule comprising the transmembrane and cytoplasmic domains of LAILR1 is represented by SEQ ID NOS: 849 and 2612. Inhibitory conventional CARs and backbones 1-62 targeting different antigens can be easily constructed by joining the scFv fragments described in Table 11 to the transmembrane and cytoplasmic domains of LAILR1 (SEQ ID NOS: 849 and 2612). Such inhibitory CARs can be expressed alone or in combination with the activating CARs, such as the CARs described in Tables 19-22.

In some embodiments, the ASD of the CARs described herein have a binding affinity (KD) of 10⁻⁴ M to 10⁻⁸ M, 10⁻⁵ M to 10⁻⁷ M, 10⁻⁶ M or 10⁻⁷ M, for the target antigen.

Also provided herein are genetically engineered cells e.g., an immune effector cell, (e.g., a population of cells, e.g., a population of immune effector cells) and/or a stem cell (e.g., a hematopoietic stem cell, a peripheral blood stem cell, a bone marrow derived stem cell, an immune stem cell, an induced pluripotent stem cell or iPSC) comprising nucleic acids, polypeptides or vectors as described herein.

In one embodiment, the cell is a human T cell. In some embodiments, the cell is an immune cell. Non-limiting examples of immune cells include T-cells and NK-cells. Further, non-limiting examples of T-cells include Tregs, CD8+ T cells, and CD4+ T cells. Immune cells, (e.g., T cells and NK cells) can be obtained from a number of sources, including peripheral blood mononuclear cells, bone marrow, lymph node tissue, cord blood, thymus tissue, tissue from a site of infection, ascites, pleural effusion, spleen tissue, and tumors. T cells could be tissue resident gamma-delta T cells or tumor infiltrating lymphocytes (TILs), which can be cultured and expanded in vitro prior to and/or after the expression of the CARs and the novel backbones of this invention. In some embodiments, the cell is a mammalian cell. In some embodiment, the cell is a human cell. In some embodiments, the cell is a dog cell. In some embodiments, the cell is a monkey cell.

In one embodiment, the cell is a T cell and the T cell is deficient in one or more of endogenous T cell receptor chains. T cells stably lacking expression of a functional TCR according to the invention may be produced using a, variety of approaches such as use of Zn finger nucleases (ZFN), CRISP/Cas9 and shRNA targeting the endogenous T cell receptor chains. A non-limiting exemplary method relating to shRNAs is described in US 2012/0321667 A1. Another non-limiting exemplary method relating to eliminating endogenous TCR expression using ZFNs targeting constant regions of α and β chains of TCRs has been described (Torikai H et al.,Blood 119:24, 2012).

In one embodiment, the cell is a stem cell and the stem cell is deficient in one or more of endogenous T cell receptor chains. In another embodiment, the cell is a stem cell in which one or more target antigens of the CAR have been deleted or mutated to a form that is no longer recognized by the CAR. In a non-limiting example, a CAR targeting CD19 is expressed in stem cells that have been made deficient in CD19 using CRISP/Cas9 or Zn finger nucleases so that the B cells produced by such stem cells are not eliminated by the T cells expressing the CD19-targeting CAR. In another non-limiting example, a CAR targeting CD19 is expressed in stem cells in which the endogenous CD19 gene has been mutated using CRISP/Cas9 or Zn finger nucleases to a form that is not targeted by CAR so that the B cells produced by such stem cells are not eliminated by the T cells expressing the CD19-targeting CAR. In another non-limiting example, the CAR is expressed in immune effector cells and the stem cells from an autologous or an allogeneic donor are genetically engineered to either lack the expression of the target antigen of the CAR or to express a mutated form of the target antigen of the CAR which is not recognized by the CAR. In another non-limiting example, a CAR targeting CD19 is expressed in T cells that are infused into a patient along with autologous or allogeneic hematopoietic stem cells that have been made deficient in CD19 using CRISP/Cas9 or Zn finger nucleases so that the B cells produced by such stem cells are not eliminated by the T cells expressing the CD19-targeting CAR. In another non-limiting example, a CAR targeting CD19 is expressed in T cells that are infused into a patient along with autologous or allogeneic hematopoietic stem cells in which the endogenous CD19 gene has been mutated using CRISP/Cas9 or Zn finger nucleases to a form that is not targeted by CAR so that the B cells produced by such stem cells are not eliminated by the T cells expressing the CD19-targeting CAR. In other non-limiting examples, CRISP/Cas9, Zn finger nucleases or siRNA/shRNA approaches are used to mutate or eliminate other endogenous antigens (e.g., MPL, CD33, CD123 etc.) in stem cells in subjects receiving CAR-T cells targeting these antigens for the treatment of specific diseases in which these antigens are expressed on disease-associated or disease-causing cells. In a non-limiting example, a CAR targeting MPL is expressed in stem cells that have been made deficient in MPL using CRISP/Cas9 or Zn finger nucleases so that the blood cells produced by such stem cells are not eliminated by the T cells expressing the MPL-targeting CAR. In another non-limiting example, a CAR targeting MPL (e.g., SEQ ID NO: 1657-1660) is expressed in stem cells in which the endogenous MPL gene has been mutated using CRISP/Cas9 or Zn finger nucleases to a form that is not targeted by MPL-directed CAR so that the blood cells produced by such stem cells are not eliminated by the T cells expressing the MPL-targeting CAR. In another non-limiting example, a CAR targeting MPL is expressed in T cells that are infused into a patient along with autologous or allogeneic hematopoietic stem cells in which the endogenous MPL gene has been mutated using CRISP/Cas9 or Zn finger nucleases to a form that is not targeted by CAR. In an exemplary embodiment the region of MPL gene that is targeted for mutation by CRISP/Cas9 or Zn finger nucleases to a form that is not targeted by MPL-directed CAR encodes for the peptide sequence -PWQDGPK-.

In various embodiments, the CARs target one, two, three, four or more antigens on the diseased-causing or disease-associated cells, such as the cancer cells. In some embodiments, the CARs comprising more than one ASD target the same antigen on the diseased-causing or disease-associated cells. In another embodiment, the CARs comprising more than one ASD target different antigens on the diseased-causing or disease-associated cells.

In some embodiments, the genetically engineered cells further comprise nucleic acids, polypeptides or vectors that encode accessory modules. In some embodiments, the accessory modules reduce or prevent toxicity associated with CARs. In some embodiments, the accessory modules are inhibitors of cytokines or chemokines, for examples, siRNA (either shRNA or Mir based shRNAs) against the involved cytokine and/or chemokine and are expressed from the same vector as the CAR. For example, any one or more of IL6, IL10 and IFNγ can be targeted. In some embodiments, the expression of the chemokine or cytokine can be blocked by expression of a blocking scFv fragment targeting the chemokine or cytokine or its receptor. Non-limiting examples of such accessory modules include IL6R-304-vHH (SEQ ID NOs: 884 and 2647), IGHSP2-IL6R-304-VHH-ALB8-VHH (SEQ ID NOs: 886 and 2649), CD8SP2-PD1-4H1-scFv (SEQ ID NOs: 889 and 2652), CD8SP2-PD1-5C4-scFv CD8SP2-CTLA4-Ipilimumab-scFv (SEQ ID NOs: 891 and 2654), CD8SP2-PD1-4H1-Alb8-vHH (SEQ ID NOs: 892 and 2655), CD8SP2-PD1-5C4-Alb8-vHH (SEQ ID NOs: 893 and 2656), CD8SP2-CTLA4-Ipilimumab-Alb8-vHH (SEQ ID NOs: 894 and 2657), IgSP-IL6-19A-scFV (SEQ ID NOs: 899 and 2662), CD8SP2-sHVEM (SEQ ID NOs: 901 and 2664), CD8SP2-sHVEM-Alb8-vHH (SEQ ID NOs: 902 and 2665), hTERT (SEQ ID NOs: 903 and 2666), Heparinase (SEQ ID NOs: 904 and 2667), IL12F (SEQ ID NOs: 863 and 2626), 41BB-L (SEQ ID NOs: 864 2627), CD40L (SEQ ID NOs: 865 2628) and shRNA targeting Brd4 (SEQ ID NO: 919). An exemplary vector expressing H1 promoter-driven shRNA targeting Brd4 that can be used to clone the conventional CARs and novel backbones 1-62 of the invention is pLenti-EF1a-shRNA-BRD4-DWPRE (SEQ ID NO: 920). An exemplary CAR in backbone 1 coexpressing shRNA targeting Brd4 is pLenti-EF1a-CD8SP-FMC63-(vL-vH)-Myc-z-P2A-K13-Flag-T2A-PAC-H1-prom-shRNA-BRD4-DWPRE (SEQ ID NO: 921).

Capillary leak syndrome is another major complication of CARs. A natural plasmin digest product of fibrin, peptide BB 15-42 (also called FX06), has been shown to significantly reduce vascular leak (Groger et al., PLoS ONE 4:e5391, 2009). FX06 peptide has been shown to block capillary leak. In some embodiments, as described herein, FX06 (SEQ ID NOs: 900 and 2663) can be coexpressed with the CARs described herein from the same vector to mitigate capillary leak associated with CAR therapy. The nucleic acid and protein sequences of an exemplary CAR co-expressing FX06 are represented by SEQ ID NOs: 1764 and 3509, respectively.

A limitation of the current technology for adoptive cellular therapy with T cells is the limited life span of genetically modified T cells, such as CAR-T cells, when infused into patients. To extend the life span of genetically-modified T cells, provided herein are vectors expressing CARs in conjunction with a number of viral and cellular proteins that stimulate the proliferation of T cells and/or to protect them from activation-induced cell death. The use of these proteins, however, is not limited to CAR-expressing T cells and they can be used to extend the life-span of any immune cells that are to be used for the purpose of adoptive immunotherapy, including but not limited to any T cell (e.g., autologous T cells or allogeneic T cells), T cell subsets (e.g., CD8, CD4, TILs), T cells directed against specific tumor antigens or infectious agents, T cells expressing an exogenous T cell receptor (i.e., TCR gene therapy), and T cells expressing a synthetic or chimeric T cell receptor. The DNA and protein sequences of several proteins that can promote the proliferation and survival of CAR-expressing T cells are provided in SEQ ID NOs; 862-882 and SEQ ID NOs: 2625-2645, respectively (Table 16). In exemplary embodiments, these proteins include any one or more of viral proteins K13-vFLIP (SEQ ID NOs: 866 and 2629) andMC159-vFLIP (SEQ ID NOs: 867 and 2630) as described herein. In further embodiments, these proteins include cFLIP-L/MRIT-alpha (SEQ ID NOs: 873 and 2636) and cFLIP-p22 (SEQ ID NOs: 874 and 2637). In further embodiments, these proteins include any one, two or more of Tax and Tax-mutants from human T cell leukemia/lymphoma virus (HTLV-1 and HTLV-2) (SEQ ID NOs: 875-877 and SEQ ID NOs: 2638-2640), Tio protein from herpes virus ateles, stpC Tip from Herpes virus Saimiri. The use of the viral and cellular proteins to promote the survival and proliferation of T cells, is however, not limited to CAR-expressing T cells. In further embodiment, these viral and cellular proteins can be used to promote the survival and proliferation of any T cell, including those expressing an exogenous T cell receptor or synthetic T cell receptor.

In one embodiment, the viral and cellular proteins that are known to stimulate the proliferation of T cells and/or to protect them from activation- induced cell death can be expressed in the CAR-expressing cells constitutively. In another embodiment, the viral and cellular proteins that are known to stimulate the proliferation of T cells and/or to protect them from activation- induced cell death can be expressed in the CAR-expressing cells in an inducible manner, for example, using an inducible promoter. Examples of inducible promoters include, but are not limited to a metallothionine inducible promoter, a glucocorticoid inducible promoter, a progesterone inducible promoter, and a tetracycline inducible promoter. RheoSwitch^{®} system represents another transcriptional regulator platform for controlling the expression of CAR of the present invention (Agarwal et al, 2012, AACR-NCI-EORTC international conference on Molecular Targets and Cancer Therapeutics). In another embodiment, the activity of the viral and cellular proteins that are known to stimulate the proliferation of T cells and/or to protect them from activation-induced cell death is controlled by expressing them in fusion with a dimerization domain. Non-limiting examples of dimerization domains include FKBP (SEQ ID NOs: 857-858 and SEQ ID NOs: 2620 and 2621), FKBPx2 (SEQ ID NOs: 859 and 2622) and Myr-FKBP. Non-limiting examples of several viral and cellular proteins in fusion with FKBP domains are provided in SEQ ID NOs: 878-882 and SEQ ID NOs: 2641 to 2645 (Table 16). The DNA and protein sequences of exemplary CAR constructs coexpressing a viral protein in fusion with FKBP dimerization domain(s) are provided in SEQ ID NOs: 1755 to 1758 and SEQ ID NOs: 3500 to 3503.

Further, a Src inhibitor can be used to prevent and/or treat the toxicity associated with the administration of CAR-expressing cells. In some embodiments, the Src inhibitors are particularly useful for the treatment of cytokine release syndrome and neurological complications associated with the use of genetically modified T cells (e.g., T cells expressing CAR or exogenous TCR or chimeric TCR),Bispecific T cell engagers (BiTE) and DARTs (Dual Affinity Re-targeting proteins).

Also provided herein are methods for treating a disease in a subject in need thereof comprising administering to the subject a therapeutically effective amount of a nucleic acid encoding one or more conventional CARs I to III, a therapeutically effective amount of a nucleic acid encoding one or more CARs and at least one accessory module (backbones 1-62), a therapeutically effective amount of polypeptide encoded by the nucleic acid encoding one or more conventional CARs I to III, a therapeutically effective amount of polypeptide encoded by the nucleic acid encoding one or more CARs and at least one accessory module (backbones 1-62), a therapeutically effective amount of genetically modified cells, as described herein, comprising nucleic acid encoding one or more conventional CARs I to III, or a therapeutically effective amount of genetically modified cells, as described herein, comprising nucleic acid encoding one or more conventional CARs and at least one accessory module (backbones 1-62), as described herein. In one embodiment, the nucleic acids, polypeptides, vectors and genetically modified cells comprise a conventional CAR II. In one embodiment, the nucleic acids, polypeptides, vectors and genetically modified cells comprise backbone-1, comprising a conventional CAR I and K13-vFLIP. In one embodiment, the nucleic acids, polypeptides, vectors and genetically modified cells comprise backbone-32, comprising a conventional CAR II and K13-vFLIP. In one embodiment, the disease treated or prevented by CAR is a cancer. In other embodiments, the diseases treated or prevented by CARs are infectious diseases, allergic diseases, autoimmune diseases, a degenerative diseases, or a combination thereof.

In one embodiment, provided herein is a method for treating, inhibiting, reducing the severity of, preventing metastasis of and/or reducing the relapse of a disease in a subject in need thereof, comprising administering to the subject a therapeutically effective amount of genetically modified cells comprising nucleic acid encoding one or more conventional CARs. In one embodiment, the genetically modified cells comprise vectors encoding a conventional CAR II as described herein. In one embodiment, provided herein is a method for treating, inhibiting, reducing the severity of, preventing metastasis of and/or reducing the relapse of cancer in a subject in need thereof, comprising administering to the subject a therapeutically effective amount of genetically modified cells comprising nucleic acid encoding one or more CARs and at least one accessory module (backbones 1-62). In one embodiment, the genetically modified cells comprise vectors encoding backbone-1, comprising a conventional CAR I and K13-vFLIP as described herein. In one embodiment, the genetically modified cells comprise vectors encoding backbone-32, comprising a conventional CAR II and K13-vFLIP as described herein. In some embodiments, the vectors encoding CARs target two, three or more antigens on cancer cells. In one embodiment, the disease treated or prevented by administration of genetically modified cells comprising nucleic acid encoding one or more CARs is a cancer. In other embodiments, the diseases treated or prevented by administration of genetically modified cells comprising nucleic acid encoding one or more CARs are infectious disease, allergic diseases, autoimmune diseases, a degenerative diseases, or combination thereof.

In exemplary embodiments, the cancer is any of colon cancer, rectal cancer, renal-cell carcinoma, liver cancer, non-small cell carcinoma of the lung, cancer of the small intestine, cancer of the esophagus, melanoma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, primary effusion lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers, combinations of said cancers, and metastatic lesions of said cancers.

Also provided herein are methods for treating, inhibiting, reducing the severity of, preventing metastasis of and/or reducing the relapse of hematological cancer in a subject in need thereof comprising administering to the subject a therapeutically effective amount of genetically modified cells, as described herein, wherein the genetically modified cells comprise vectors encoding a conventional CAR II as described herein.

Also provided herein are methods for treating, inhibiting, reducing the severity of, preventing metastasis of and/or reducing the relapse of hematological cancer in a subject in need thereof comprising administering to the subject a therapeutically effective amount of genetically modified cells, as described herein, wherein the genetically modified cells comprise vectors encoding backbone-1, comprising conventional CAR I and K13-vFLIP or vectors encoding backbone-32, comprising CAR II and K13-vFLIP. In one embodiment, the ASD of the CAR targets MPL. In exemplary embodiments, conventional CAR II targeting MPL are described in **Table 21** and **22** and comprise nucleic acid sequences set forth in SEQ ID NOs: 1657-1664 and 1731-1739 and comprise polypeptide sequences set forth in SEQ ID NOs: 3402-3409 and 3476-3484. In exemplary embodiments, conventional CAR I targeting MPL and coexpressing K13-vFLIP (backbone 1) are described in **Table 19** and comprise nucleic acid sequences set forth in SEQ ID NOs: 1117-1124 and 1747 and comprise polypeptide sequences set forth in SEQ ID NOs: 2862-2869 and 3492. In exemplary embodiments, conventional CAR II targeting MPL and coexpressing K13-vFLIP (backbone 32) are described in **Tables 20 and 22** and comprise nucleic acid sequences set forth in SEQ ID NOs: 1387-1394 and 1747 and comprise polypeptide sequences set forth in SEQ ID NOs: 3132-3139 and 3492. Non-limiting examples of hematological cancers treated or prevented by administration of cells expressing CARs targeting MPL include acute myeloid leukemia, chronic myeloid leukemia and myelodysplastic syndrome.

Also provided herein are methods for treating, inhibiting, reducing the severity of, preventing metastasis of and/or reducing the relapse of cancers expressing CD19 (e.g., B cell acute lymphocytic leukemia (B-ALL), chronic lymphocytic leukemia, diffuse large cell lymphoma, indolent lymphoma) in a subject in need thereof comprising administering to the subject a therapeutically effective amount of genetically modified cells, as described herein, wherein the genetically modified cells comprise vectors encoding a conventional CAR II targeting CD19 as described herein.

Also provided herein are methods for treating, inhibiting, reducing the severity of, preventing metastasis of and/or reducing the relapse of a CD19 expressing cancer in a subject in need thereof comprising administering to the subject a therapeutically effective amount of genetically modified cells, as described herein, wherein the genetically modified cells comprise vectors encoding backbone-1, comprising conventional CAR I targeting CD19 and K13-vFLIP or vectors encoding backbone-32, comprising CAR II targeting CD19 and K13-vFLIP. In exemplary embodiments, conventional CAR II targeting CD19 are described in **Table 21** and comprise nucleic acid sequences set forth in SEQ ID NOs: 1470-1480 and comprise polypeptide sequences set forth in SEQ ID NOs: 3215-3225. In exemplary embodiments, conventional CAR I targeting CD19 and coexpressing K13-vFLIP (backbone 1) are described in **Table 19** and comprise nucleic acid sequences set forth in SEQ ID NOs: 927-941 and comprise polypeptide sequences set forth in SEQ ID NOs: 2672-2686. In exemplary embodiments, conventional CAR II targeting CD19 and coexpressing K13-vFLIP (backbone 32) are described in **Tables** 20 **and** 22 and comprise nucleic acid sequences set forth in SEQ ID NOs: 1197-1211 and 1759 and comprise polypeptide sequences set forth in SEQ ID NOs: 2942-2956 and 3504.

Also provided herein are methods for treating, inhibiting, reducing the severity of, preventing metastasis of and/or reducing the relapse of GRP-78 expressing cancer in a subject in need thereof comprising administering to the subject a therapeutically effective amount of genetically modified cells, as described herein, wherein the genetically modified cells comprise vectors encoding backbone-1, comprising conventional CAR I and K13-vFLIP or vectors encoding backbone-32, comprising CAR II and K13-vFLIP. In one embodiment, the ASD of the CAR targets GRP-78. In exemplary embodiments, CARs targeting GRP-78 and accessory modules are described in **Table 22** and comprise nucleic acid sequence set forth in SEQ ID NOs: 1771 and 1775 and comprise polypeptide sequence set forth in SEQ ID NO: 3516 and 3520.

In various embodiments, additional antigen targets, CAR constructs and accessory modules for use with the instant invention will be apparent to a person of skill in the art. Exemplary embodiments are set forth in **Tables 2-22.**

In some embodiments, the therapeutically effective amount of the genetically modified cells is administered at a dosage of 10⁴ to 10⁹ cells/kg body weight, in some instances 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng. J. of Med. 319:1676, 1988). The cells can be administered by injection into the site of the lesion (e.g., intra-tumoral injection).

In some embodiments, the therapeutic methods described herein further comprise administering to the subject, sequentially or simultaneously, existing therapies. Examples of existing cancer treatment include, but are not limited to, active surveillance, observation, surgical intervention, chemotherapy, immunotherapy, radiation therapy (such as external beam radiation, stereotactic radiosurgery (gamma knife), and fractionated stereotactic radiotherapy (FSR)), focal therapy, systemic therapy, vaccine therapies, viral therapies, molecular targeted therapies, or combinations thereof.

In some embodiments, the therapeutic methods described herein further comprise administering to the subject, sequentially or simultaneously, any one or more of a kinase inhibitor, a BTK inhibitor, an mTOR inhibitor, a MNK inhibitor, BRD4 inhibitor or a dual PI3K/mTOR inhibitor, or combinations thereof.

In one embodiment, the kinase inhibitor is a Src family kinase inhibitor, such as dasatinib.

In one embodiment, the kinase inhibitor is a CDK4 inhibitor, such as CDK4/6 inhibitors, selected from ribociclib, abemaciclib and palbociclib.

In one embodiment, the kinase inhibitor is an mTOR inhibitor, for example, rapamycin, a rapamycin analog. OSI-027. The mTOR inhibitor can be, for example, an mTORCl inhibitor and/or an mTORC2 inhibitor.

In one embodiment, the kinase inhibitor is a MNK inhibitor, for example, 4-amino-5-(4-fluoroanilino)-pyrazolo [3,4-d] pyrimidine. The MNK inhibitor can be, for example, an inhibitor of MNKla, MNK1b, MNK2a and/or MNK2b. The dual PI3K/mTOR inhibitor can be, e.g., PF-04695102.

In one embodiment, the inhibitor is a Brd4 inhibitor selected from JQ1, MS417, OTXO15, LY 303511 and Brd4 inhibitor as described in US 20140256706 A1 and any analogs thereof.

In one embodiment of the methods described herein, the kinase inhibitor is a CDK4 inhibitor, e.g., palbociclib (PD0332991), and the palbociclib is administered at a dose of about 50 mg, 60 mg, 70 mg, 75 mg, 80 mg, 90 mg, 100 mg, 105 mg, 110 mg, 115 mg, 120 mg, 125 mg, 130 mg, 135 mg (e.g., 75 mg, 100 mg or 125 mg) daily for a period of time, e.g., daily for 14-21 days of a 28 day cycle, or daily for 7-12 days of a 21 day cycle. In one embodiment, 1, 2, 3, 4, 5, 6, 7, 8, 9, 10, 11, 12 or more cycles of palbociclib are administered.

In one embodiment of the methods described herein, the kinase inhibitor is a BTK inhibitor selected from ibrutinib (PCI-32765); GDC-0834; RN-486; CGI-560; CGI-1764; HM-71224; CC-292; ONO-4059; CNX-774; and LFM-A13. In one embodiment, the BTK inhibitor does not reduce or inhibit the kinase activity of interleukin- 2-inducible kinase (ITK), and is selected from GDC-0834; RN-486; CGI-560; CGI-1764; HM-71224; CC-292; ONO-4059; CNX-774; and LFM-A13. In one embodiment of the methods or uses described herein, the kinase inhibitor is a BTK inhibitor, e.g., ibrutinib (PCI-32765), and the ibrutinib is administered at a dose of about 250 mg, 300 mg, 350 mg, 400 mg, 420 mg, 440 mg, 460 mg, 480 mg, 500 mg, 520 mg, 540 mg, 560 mg, 580 mg, 600 mg (e.g., 250 mg, 420 mg or 560 mg) daily for a period of time, e.g., daily for 21 day cycle, or daily for 28 day cycle. In one embodiment, 1, 2, 3, 4, 5, 6, 7, 8, 9, 10, 11, 12 or more cycles of ibrutinib are administered.

In one embodiment of the methods or uses described herein, the kinase inhibitor is a BTK inhibitor that does not inhibit the kinase activity of ITK, e.g., RN-486, and RN- 486 is administered at a dose of about 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg (e.g., 150 mg, 200 mg or 250 mg) daily for a period of time, e.g., daily a 28 day cycle. In one embodiment, 1, 2, 3, 4, 5, 6, 7, or more cycles of RN-486 are administered.

In one embodiment of the methods described herein, the kinase inhibitor is an mTOR inhibitor selected from temsirolimus; ridaforolimus (1R,2R,4S)-4-[(2R)-2 [(1R,9S,12S,15R,16E,18R,19R,21R, 23S,24E,26E,28Z,30S,32S,35R)-1,18-dihydroxy- 19,30-dimethoxy-15, 17,21,23, 29,35-hexamethyl-2,3,10,14,20-pentaoxo-1 l,36-dioxa-4-azatricyclo[30.3.1.0] hexatriaconta-16,24,26,28-tetraen-12-yl]propyl]-2- methoxycyclohexyl dimethylphosphinate, also known as AP23573 and MK8669; everolimus (RAD001); rapamycin (AY22989); simapimod; (5-{2.4-bis[(35")-3- methylmorpholin-4-yl]pyrido[2,3-(i]pyrimidin-7-yl}-2-methoxyphenyl)methanol (AZD8055), 2-amino-8-[iran5-4-(2-hydroxyethoxy)cyclohexyl]-6-(6-methoxy-3- pyridinyl)-4-methyl-pyrido[2,3-J|pyrimidin-7(8H)-one (PF04691502); and N²-[1,4-dioxo- 4-[[4-(4-oxo-8-phenyl-4H-1-benzopyran-2-yl)morpholinium-4-yl]methoxy]butyl]-L- arginylglycyl-L-a-aspartylL-serine-, inner salt (SF1126); and XL765.

In one embodiment of the methods or uses described herein, the kinase inhibitor is an mTOR inhibitor, e.g., rapamycin, and the rapamycin is administered at a dose of about 3 mg, 4 mg, 5 mg, 6 mg, 7 mg, 8 mg, 9 mg, 10 mg (e.g., 6 mg) daily for a period of time, e.g., daily for 21 day cycle, or daily for 28 day cycle. In one embodiment, 1, 2, 3, 4, 5, 6, 7, 8, 9, 10, 11, 12 or more cycles of rapamycin are administered. In one embodiment, the kinase inhibitor is an mTOR inhibitor, e.g., everolimus and the everolimus is administered at a dose of about 2 mg, 2.5 mg, 3 mg, 4 mg, 5 mg, 6 mg, 7 mg, 8 mg, 9 mg, 10 mg, 11 mg, 12 mg, 13 mg, 14 mg, 15 mg (e.g., 10 mg) daily for a period of time, e.g., daily for 28 day cycle. In one embodiment, 1, 2, 3, 4, 5, 6, 7, 8, 9, 10, 11, 12 or more cycles of everolimus are administered.

Also provided herein are methods for preparing genetically engineered cells comprising transfecting the cells with vectors comprising nucleic acid encoding the conventional CARs or the CARs on novel backbones described herein. In some embodiments, the cells are immune effector cells, such as human T cells or human NK cells, or stem cells that give rise to immune effector cells. In some embodiments, the cells are autologous human T cells or autologous human NK cells or autologous human stem cells. In some embodiments, the cells are allogeneic human T cells or allogeneic human NK cells or allogeneic human stem cells.

In one embodiment, the method for preparing the genetically modified cells comprise obtaining a population of cells and depleting the cells of the CD25+ T regulatory cells, for example by using antibodies specific to CD25. Methods for depleting CD25+ T regulatory cells from the population of cells will be apparent to a person of skill in the art. In some embodiments, the Treg depleted cells comprise less than 30%, 20%, 10%, 5% or less Treg cells. In some embodiments, the vectors encoding the CARs described herein are transfected into Treg depleted cells.

In some embodiments, prior to transfection, the Treg depleted cells are further depleted of cells which express checkpoint inhibitors. The checkpoint inhibitor may be any one or more of PD-1, LAG-3, TIM3, B7-H1, CD160, P1H, 2B4, CEACAM (e.g., CEACAM-1, CEACAM-3, and/or CEACAM-5), TIGIT, CTLA-4, BTLA, and LAIR1. In some embodiments, the cells comprise less than comprise less than 30%, 20%, 10%, 5% or less cells that express checkpoint inhibitors. In some embodiments, the vectors encoding the CARs described herein are transfected into Treg depleted and checkpoint inhibitor depleted cells.

In some embodiments, methods for preparing the genetically modified cells comprise obtaining a population of cells and selecting cells that express any one or more of CD3, CD28, CD4, CD8, CD45RA, and/or CD45RO. In certain embodiments, the population of immune effector cells provided are CD3+ and/or CD28+.

In one embodiment, the method for preparing the genetically modified cells comprise obtaining a population of cells and enriching for the CD25+ T regulatory cells, for example by using antibodies specific to CD25. Methods for enriching CD25+ T regulatory cells from the population of cells will be apparent to a person of skill in the art. In some embodiments, the Treg enriched cells comprise less than 30%, 20%, 10%, 5% or less non-Treg cells. In some embodiments, the vectors encoding the CARs described herein are transfected into Treg-enriched cells. Treg enriched cells expressing a CAR may be used to induced tolerance to antigen targeted by the CAR.

In some embodiments, the method further comprises expanding the population of cells after the vectors comprising nucleic acids encoding the CARs described herein have been transfected into the cells. In embodiments, the population of cells is expanded for a period of 8 days or less. In certain embodiments, the population of cells is expanded in culture for 5 days, and the resulting cells are more potent than the same cells expanded in culture for 9 days under the same culture conditions. In other embodiments, the population of cells is expanded in culture for 5 days show at least a one, two, three or four fold increase in cell doublings upon antigen stimulation as compared to the same cells expanded in culture for 9 days under the same culture conditions. In some embodiments, the population of cells is expanded in an appropriate media that includes one or more interleukins that result in at least a 200-fold, 250-fold, 300- fold, or 350-fold increase in cells over a 14 day expansion period, as measured by flow cytometry.

In various embodiments, the expanded cells comprise one or more CARs as described herein. In some embodiments, the expanded cells comprise one CAR with one, two, three or more ASDs. In some embodiments, the expanded cells further comprise accessory modules and therapeutic controls as described herein.

Provided herein is a cell comprising nucleic acids encoding a chimeric antigen receptor (CAR) and one or more of signaling proteins selected from K13-vFLIP, MC159-vFLIP, cFLIP-L, cFLIP-p22, HTLV1-Tax and HTLV2-Tax , wherein the CAR comprises an a) extracellular antigen specific domain, b)a transmembrane domain and c) an intracellular signaling domain comprising an immunoreceptor tyrosine-based activation motif (ITAM); wherein c) is located at the C-terminus of the chimeric receptor. In some embodiments, the CAR further comprises one or more co-stimulatory domains.

Also provided herein is a cell comprising nucleic acids encoding a chimeric antigen receptor (CAR) and K13-vFLIP signaling protein, wherein the CAR comprises an a) extracellular antigen specific domain, b) a transmembrane domain and c) an intracellular signaling domain comprising an immunoreceptor tyrosine-based activation motif (ITAM); wherein c) is located at the C-terminus of the chimeric receptor. In some embodiments, the CAR further comprises one or more co-stimulatory domains.

Also provided herein is a cell comprising nucleic acids encoding a chimeric antigen receptor (CAR) and MC159-vFLIP signaling protein, wherein the CAR comprises an a) extracellular antigen specific domain, b) a transmembrane domain and c) an intracellular signaling domain comprising an immunoreceptor tyrosine-based activation motif (ITAM); wherein c) is located at the C-terminus of the chimeric receptor. In some embodiments, the CAR further comprises one or more co-stimulatory domains.

In some embodiment, the cell described herein further comprises nucleic acids encoding MC159-vFLIP signaling protein.

In some embodiments, the cell further comprises nucleic acids encoding scFvs targeting IL6 and/or IL6 receptor alpha. In some embodiments, the cell further comprises nucleic acid encoding peptide FX06 so as to mitigate capillary leak associated with CAR therapy. In some embodiments, the signaling protein is expressed in fusion with one or more copies of FKBP domain. In some embodiments, the activity of the signaling protein is controlled post-translationally by dimerization of the FKBP domain in the presence of a dimerizing agent. In some embodiments, the dimerizing agent is AP20187.

In an exemplary embodiment, wherein the antigen specific domain of the CAR targets MPL. In some embodiments, the antigen specific domain targets two antigens, wherein the two antigens are MPL and CD123.

In some embodiments, the antigen specific domain of the CAR targets CD19, CD23, Lym1, Lym2, CLEC5A, CDH179b, FLT3, GCC, Muc1, CSF2RA, GFRa4, CD32, IL11Ra, IL13Ra, NYBR1, SLea, CD200R, TGFBetaR2, CD276, TROP2, LAMP1, PTK7, DLL3, CDH1, CDH6, CDH17, CDH19, TSHR and tyrosinase.

In some embodiments, the antigen specific domain of the CAR targets MPL and comprises one or more scFv fragments selected from 161 (SEQ ID NO: 2500 and 2501), 175 (SEQ ID NO: 2499), 178 (SEQ ID NO: 2503), 111 (SEQ ID NO: 2502), AB317 (SEQ ID NO: 2404), 12E10 (SEQ ID NO: 2505) or huVB22Bw5 (SEQ ID NO: 2506) or ligands selected from extracellular receptor binding domains of hTPO (SEQ ID NO: 2323) or mTPO (SEQ ID NO: 2323).

In some embodiments, the antigen specific domain of the CAR targets CD19 and comprises one or more scFv fragments selected from CD19Bu12 or CD19MM.

In one embodiment, the cell is a T-lymphocyte (T-cell). In another embodiment, the cell is a Natural Killer (NK) cell.

Also provided herein are nucleic acids comprising a first polynucleotide encoding the CAR of claim and a second polynucleotide encoding the K13-vFLIP signaling protein. In some embodiments, the nucleic acids further comprise a third polynucleotide encoding the MC159-vFLIP signaling protein. Also provided herein are polypeptides encoded by the nucleic acids described herein and vectors encoding the nucleic acids described herein.

Further provided herein is a pharmaceutical composition comprising the cells described herein, the nucleic acids described herein, the polypeptides encoded by the nucleic acids described herein or vectors comprising the nucleic acids described herein, and a pharmaceutically acceptable carrier.

In some embodiments, provided herein is a method for treating a MPL-expressing cancer comprising administering to the subject, a therapeutically effective amount of the cell described herein, wherein the CAR targets MPL. In some embodiments, the cancer is a blood cancer, wherein the blood cancer is any one or more of acute myeloid leukemia, chronic myeloid leukemia, myelodyplastic syndrome, lymphoma, multiple myeloma and acute lymphocytic leukemia. In some embodiments, the method further comprises administering to the subject a tyrosine kinase inhibitor, wherein the inhibitor inhibits the Src family of kinases. In some embodiments, the inhibitor inhibits Lck. In some embodiments, the inhibitor is any one or more of Dasatinib, Ponatinib or A-770041.

### BRIEF DESCRIPTION OF THE DRAWINGS

Exemplary embodiments are illustrated in referenced figures. It is intended that the embodiments and figures disclosed herein are to be considered illustrative rather than restrictive.
**Fig. 1** depicts, in accordance with various embodiments of the invention, three generations of CARs. Depiction of first, second, and third generation chimeric antigen receptors with the scFv segments in green and the various TCR signaling components in red, blue and yellow (Casucci, Monica and Attilio Bondanza (2011). Journal of Cancer. 2: 378-382)

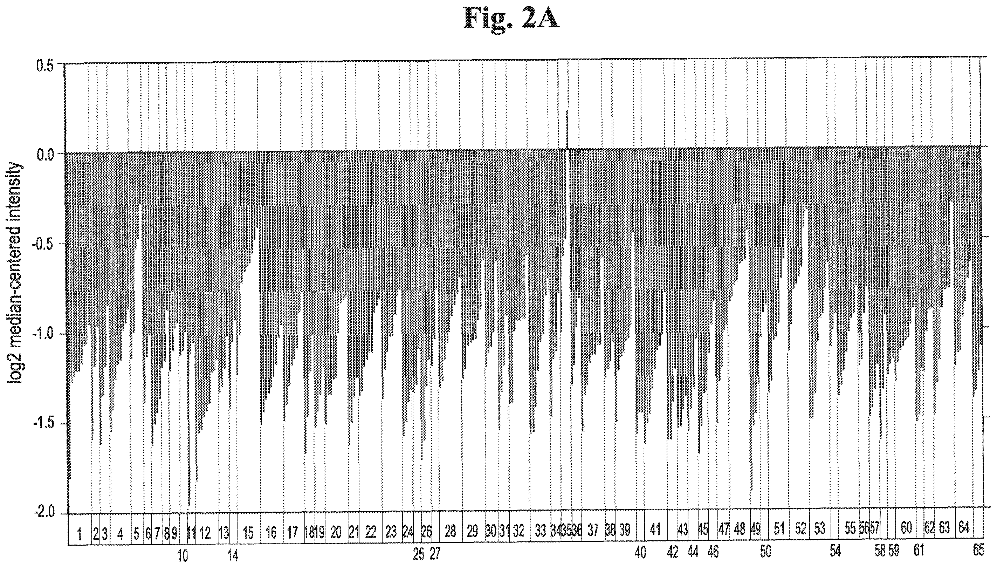

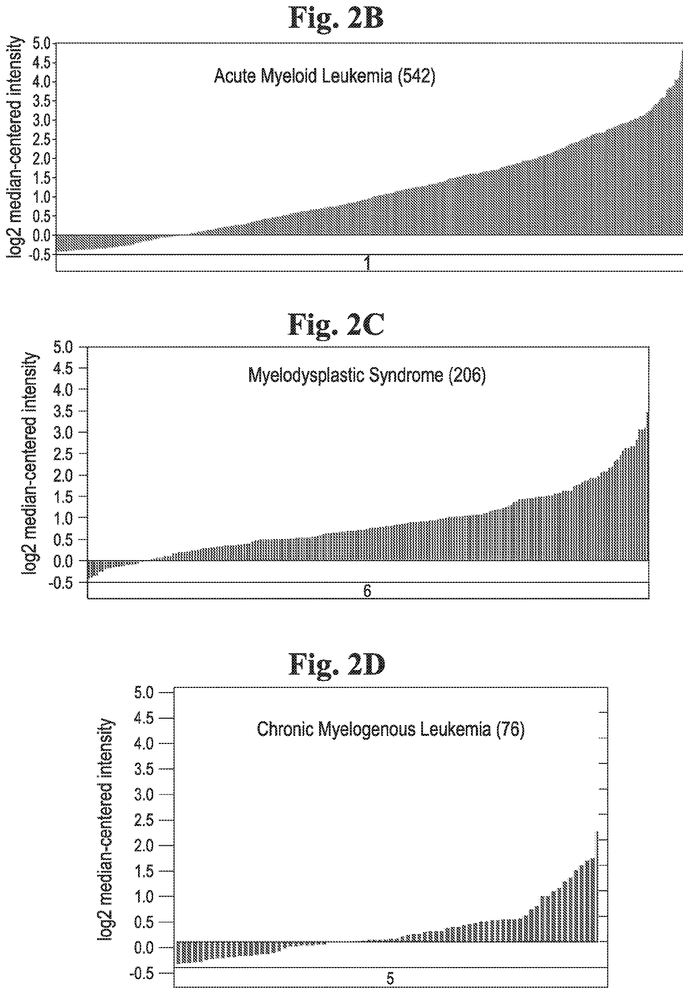

**Fig. 2A****-****Fig. 2D** depict, in accordance with various embodiments of the invention, **Fig. 2A****:** MPL gene expression in normal tissues. MPL has very restricted expression in normal tissues; **Fig. 2B****:** MPL gene expression in 542 patient samples of Acute Myeloid Leukemia. MPL is expressed in most AML samples; **Fig. 2C****:** MPL gene expression in 206 samples of Myelodysplastic syndrome (MDS). MPL is expressed in most MDS samples. **Fig. 2D****:** MPL gene expression in 76 samples of CML. MPL is expressed in most CML samples.

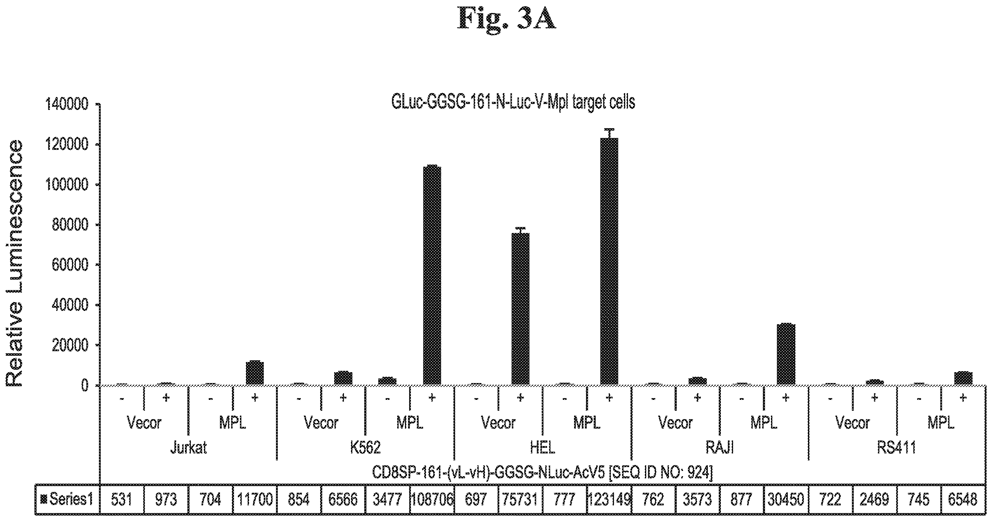

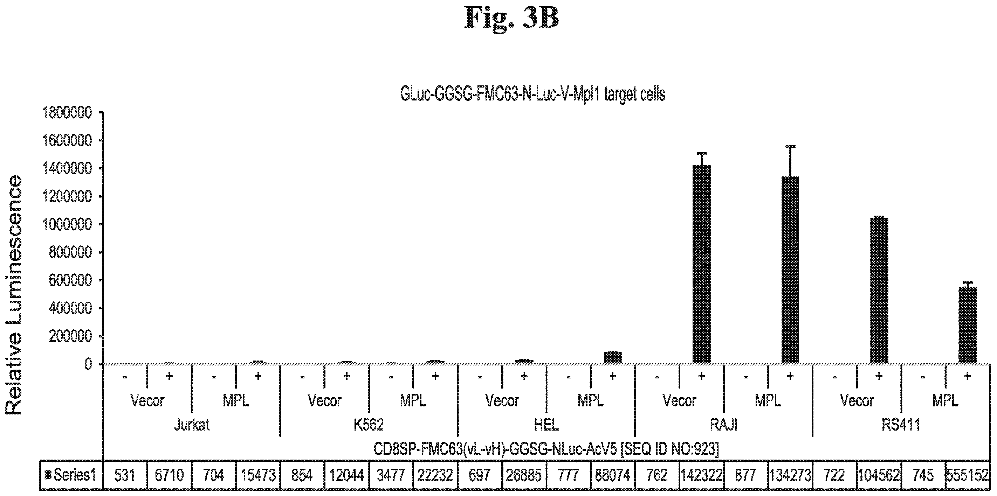

**Fig. 3A****-****Fig.3B** depict, in accordance with various embodiments of the invention, strong binding with 161-GGSG-NLuc-AcV5 was observed on HEL.92.1.7-Gluc-vector cells suggesting significant expression of MPL endogenously.

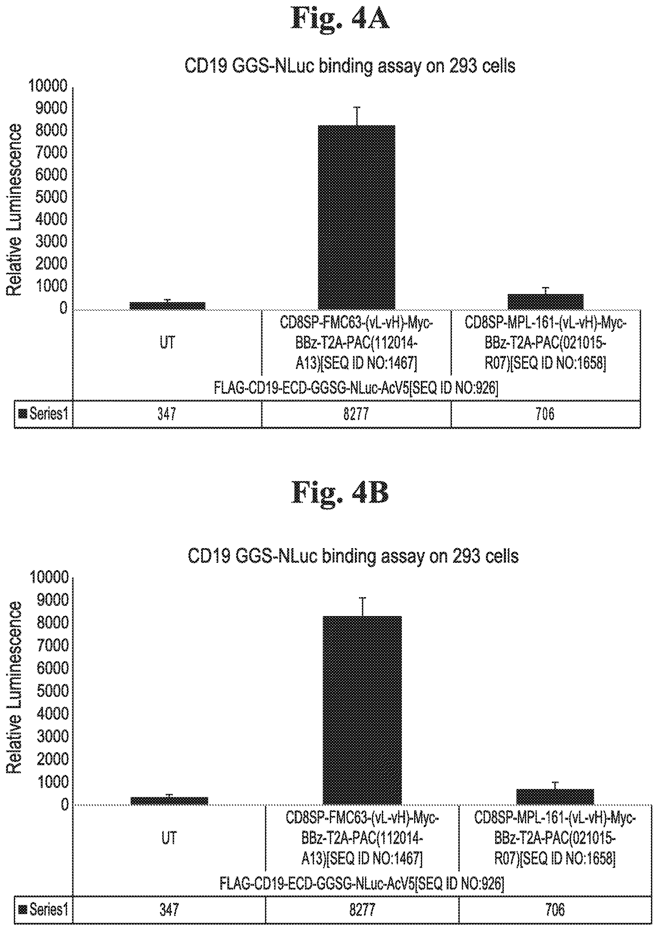

**Fig. 4A****-****Fig. 4B** depicts in accordance with various embodiments of the invention, NLuc assay to measure expression of CAR in 293FT cells. The untransfected 293FT cells, and those transfected with CAR CD8SP-FMC63-(vL-vH)-Myc-BBz-T2A-PAC(112014-A13)[SEQ ID NO:1467] and CD8SP-MPL-161-(vL-vH)-Myc-BBz-T2A-PAC(021015-R07)[SEQ ID NO:1658] CAR were incubated with FLAG-CD19-ECD-GGSG-NLuc-AcV5[SEQ ID NO: 926] and MPL-ECD-GGSG-NLuc-AcV5[SEQ ID NO: 925]supernatants as described in material and methods section followed by washing with PBS and measurement of NLuc activity by Coeleoentrazine (CTZ; Nanolight) diluted in PBS. Luminescence was quantified using a BioTek plate reader. Data represents mean values of triplicate wells +/- standard deviation (SD).

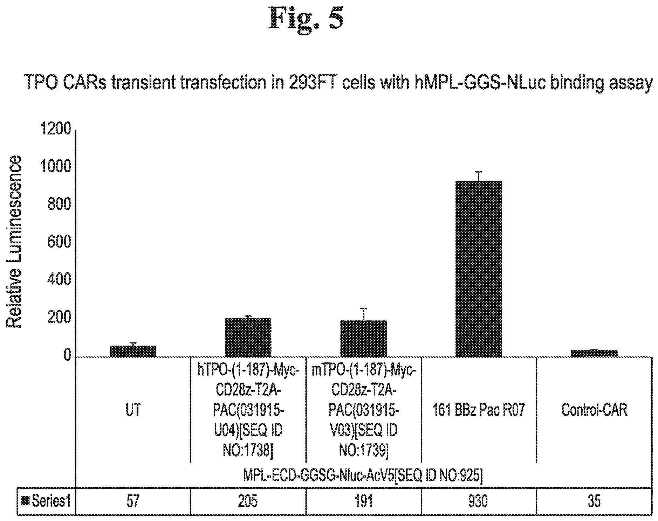

**Fig. 5** depicts, in accordance with various embodiments of the invention, modest binding of MPL-ECD-GGSG-NLuc-AcV5 to 293FT cells transfected with hTPO-(1-187)-Myc-CD28z-T2A-PAC(031915-U04)[SEQ ID NO:1738] and mTPO-(1-187)-Myc-CD28z-T2A-PAC(031915-V03)[SEQ ID NO:1739] constructs and strong binding to 293FT cells transfected with CD8SP-MPL-161-(vL-vH)-Myc-BBz-T2A-PAC(021015-R07)[SEQ ID NO:1658] construct as compared to untransfected cells or cells that had been transfected with control CAR construct.

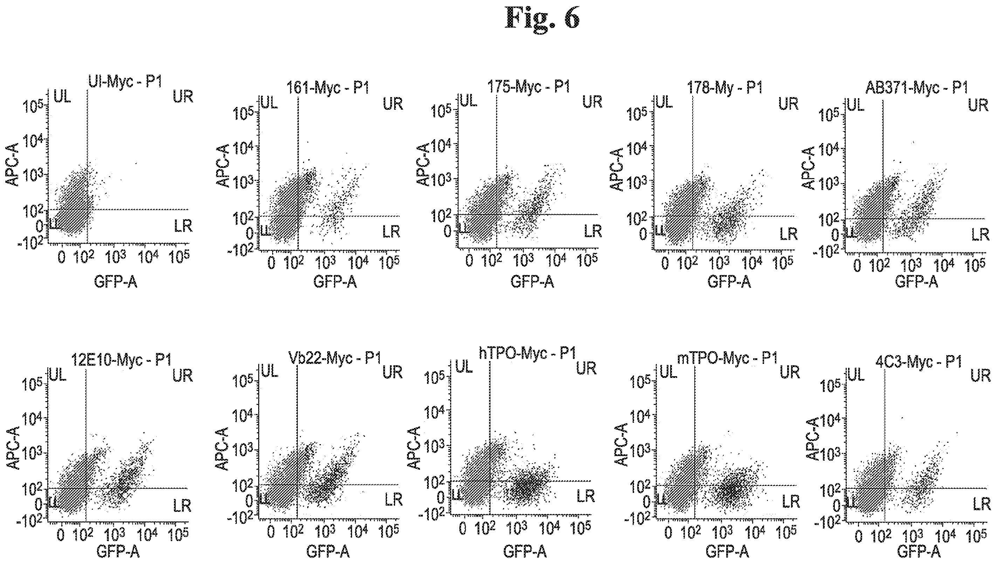

**Fig. 6** depicts, in accordance with various embodiments of the invention, expression of MPL CARs on the surface of T cells as determined by increased EGFP fluorescence and increased staining with APC-Myc as compared to the uninfected T cells (UI).

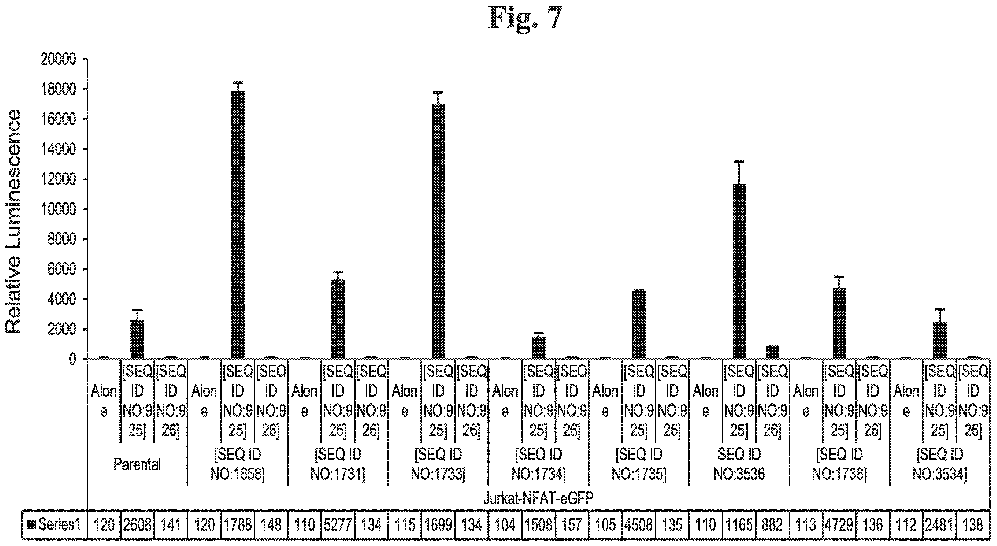

**Fig. 7** depicts in accordance with various embodiments, of the invention, strong binding of T cells expressing CD8SP-MPL-161-(vL-vH)-Myc-BBz-T2A-PAC(021015-R07)[SEQ ID NO:1658], CD8SP-175-(vL-vH)-Myc-CD28z-T2A-PAC(031615-Q04)[SEQ ID NO:1733], and CD8SP-MPL-VB22Bw4-(vL-vH)-Myc-CD28z-T2A-PAC(031615-B06)[SEQ ID NO:3536] CARs and modest binding of T cells expressing CD8SP-161-(vL-vH)-Myc-CD28z-T2A-PAC(021715-Z07)[SEQ ID NO:1731], CD8SP-AB317-(vL-vH)-Myc-CD28z-T2A-PAC(031615-T04)[SEQ ID NO:1735] and CD8SP-12E10-(vL-vH)-Myc-CD28z-T2A-PAC(031615-S03)[SEQ ID NO:1736] to MPL-GGSG-NLuc AcV5 supernatant as measured by NLuc assay. SEQ ID NO. 925 comprises MPL-ECD-GGSG-Nluc-AcV5. SEQ ID NO.: 926 comprises FLAG-CD19-ECD-GGSG-NLuc-AcV5. SEQ ID NO: 1734 comprises CD8SP-178-(vL-vH)-Myc-CD28z-T2A-PAC(031615-R04). SEQ ID NO: 3534 comprises CD8SP-4C3-(vL-vH)-Myc-CD28z-T2A-Pac(031615-WO5).

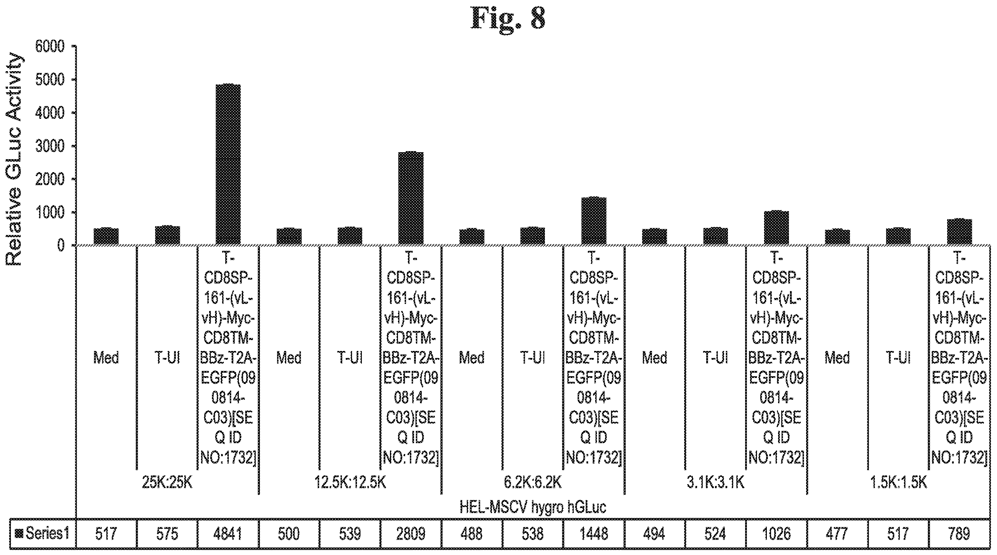

**Fig. 8** depicts, in accordance with various embodiments of the invention, increase in GLuc activity following co-culture with T cells expressing MPL-specific CD8SP-161-(vL-vH)-Myc-CD8TM-BBz-T2A-EGFP(090814-C03)[SEQ ID NO:1732] as compared to the uninfected T cells indicating lysis of target cells by the different MPL-CAR-T cells.

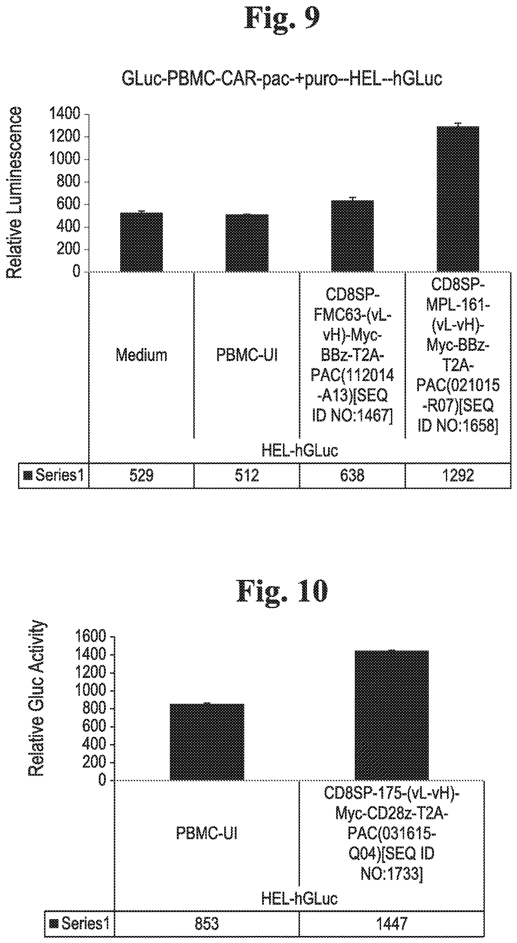

**Fig. 9** depicts, in accordance with various embodiments of the invention, increased killing of HEL-GLuc cells by CD8SP-MPL-161-(vL-vH)-Myc-BBz-T2A-PAC(021015-R07)[SEQ ID NO:1658] CAR expressing PBMC as compared to FMC63-(vL-vH)-Myc-BBz-T2A-PAC(112014-A13)[SEQ ID NO:1467] PBMC or uninfected PBMC.
**Fig. 10** depicts, in accordance with an embodiment of the invention, increased killing of HEL-GLuc cells by CD8SP-175-(vL-vH)-Myc-CD28z-T2A-PAC(031615-Q04)[SEQ ID NO:1733] CAR expressing PBMC as compared to uninfected PBMC.

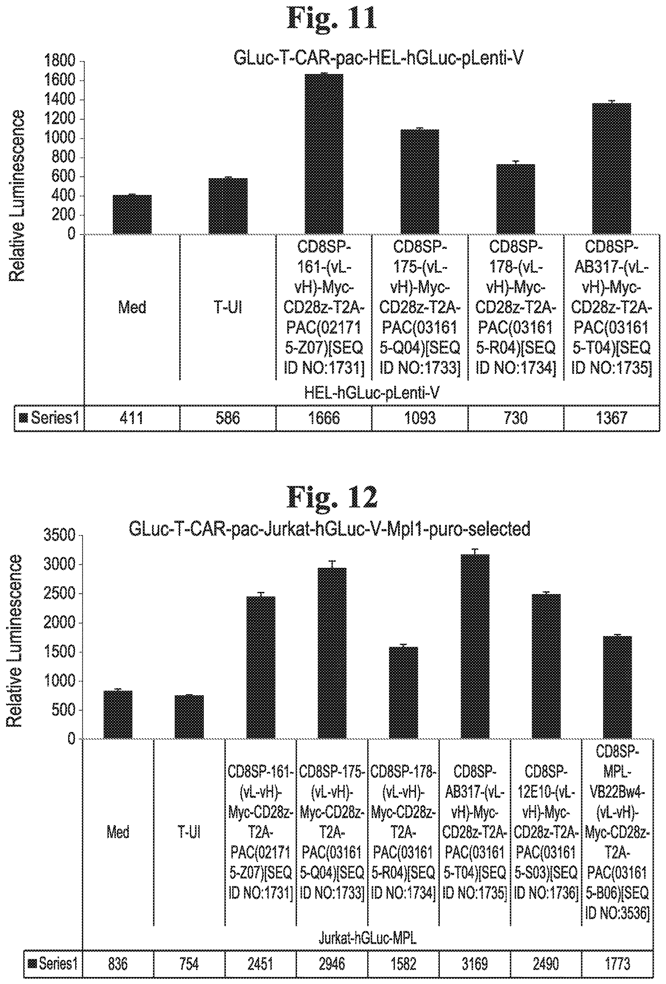

**Fig. 11** depicts, in accordance with an embodiments of the invention, increase in GLuc activity, indicating lysis of target cells, following co-culture with T cells expressing MPL-specific CARs, which was stronger with CD8SP-161-(vL-vH)-Myc-CD28z-T2A-PAC(021715-Z07)[SEQ ID NO:1731], CD8SP-175-(vL-vH)-Myc-CD28z-T2A-PAC(031615-Q04)[SEQ ID NO:1733], and CD8SP-AB317-(vL-vH)-Myc-CD28z-T2A-PAC(031615-T04)[SEQ ID NO:1735] constructs and modest with CD8SP-178-(vL-vH)-Myc-CD28z-T2A-PAC(031615-R04)[SEQ ID NO:1734] construct as compared to the uninfected T cells.
**Fig. 12** depicts, in accordance with various embodiments of the invention, increase in GLuc activity, indicating lysis of target cells, following co-culture with T cells expressing MPL-specific CARs, which was stronger with CD8SP-161-(vL-vH)-Myc-CD28z-T2A-PAC(021715-Z07)[SEQ ID NO:1731], CD8SP-175-(vL-vH)-Myc-CD28z-T2A-PAC(031615-Q04)[SEQ ID NO:1733], CD8SP-AB317-(vL-vH)-Myc-CD28z-T2A-PAC(031615-T04)[SEQ ID NO:1735], and CD8SP-12E10-(vL-vH)-Myc-CD28z-T2A-PAC(031615-S03)[SEQ ID NO:1736] constructs and modest with CD8SP-178-(vL-vH)-Myc-CD28z-T2A-PAC(031615-R04)[SEQ ID NO:1734] and CD8SP-VB22Bw4-(vL-vH)-Myc-CD28z-T2A-PAC(031615-B06)[SEQ ID NO:3536] constructs as compared to the uninfected T cells.

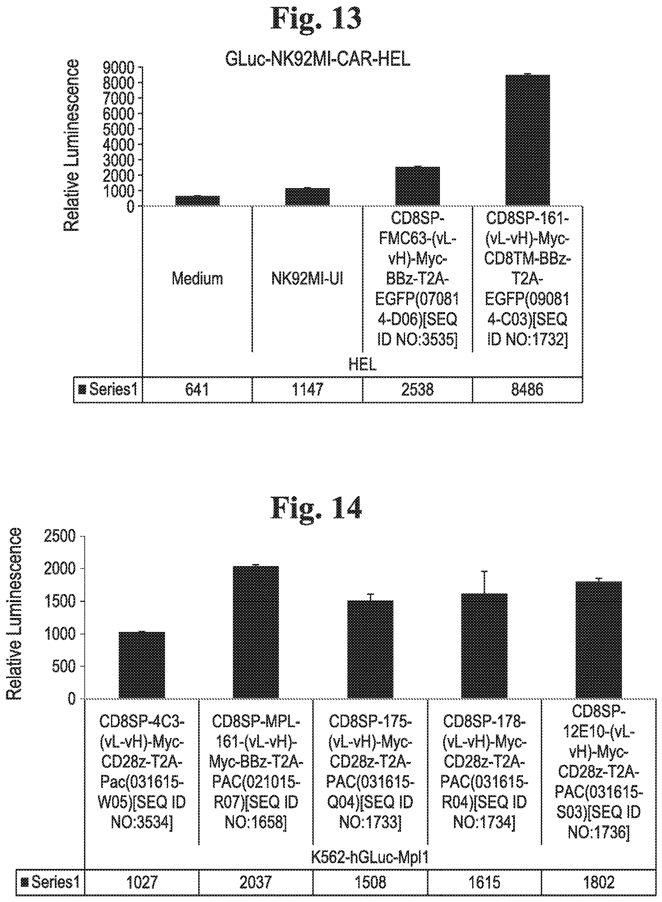

**Fig. 13** depicts, in accordance with an embodiment of the invention, increase in GLuc activity, indicating lysis of target cells, following co-culture with NK92MI cells expressing MPL-specific CD8SP-161-(vL-vH)-Myc-CD8TM-BBz-T2A-EGFP(090814-C03)[SEQ ID NO:1732] CAR.
**Fig. 14** depicts, in accordance with an embodiment of the invention, increase in GLuc activity, indicating lysis of target cells, following co-culture with NK92MI cells expressing MPL-specific CARs.

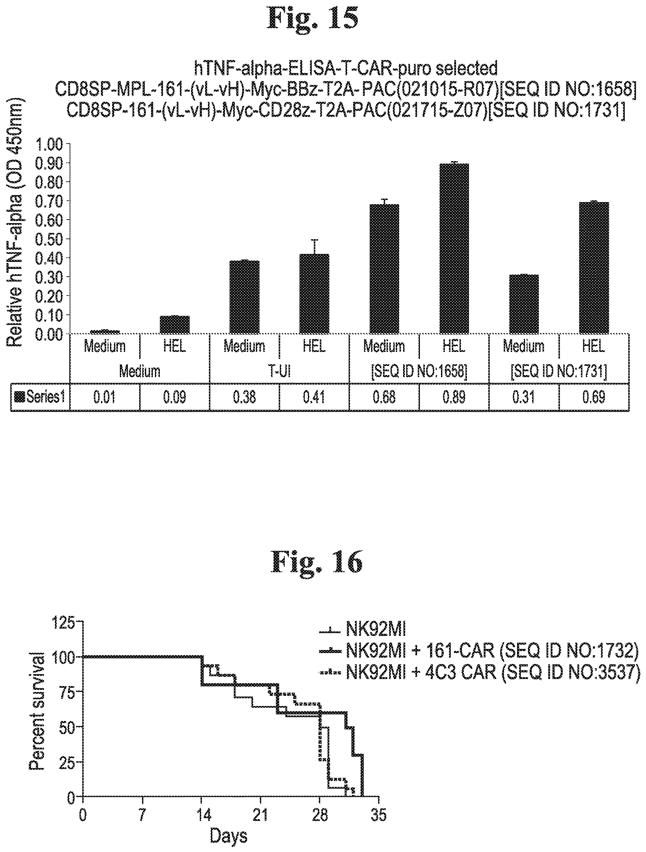

**Fig. 15** depicts, in accordance with an embodiment of the invention, increased secretion of TNFα upon coculture of T cells expressing CAR targeting MPL with HEL cells as compared to uninfected T cells used as controls.
**Fig. 16** depicts, in accordance with an embodiment of the invention, that the median survival of mice given CD8SP-161-(vL-vH)-Myc-CD8TM-BBz-T2A-EGFP(090814-C03)[SEQ ID NO:1732] expressing NK92MI cells was 31.5 days, which was significantly higher than mice given unmodified NK92MI cells (28.5 days; p = 0.03) or those given NK92MI cells expressing a control CD8SP-4C3-(vL-vH)-Myc-BBz-T2A-EGFP(100814-K06)[SEQ ID NO:3537] CAR (28 days; p=.04).

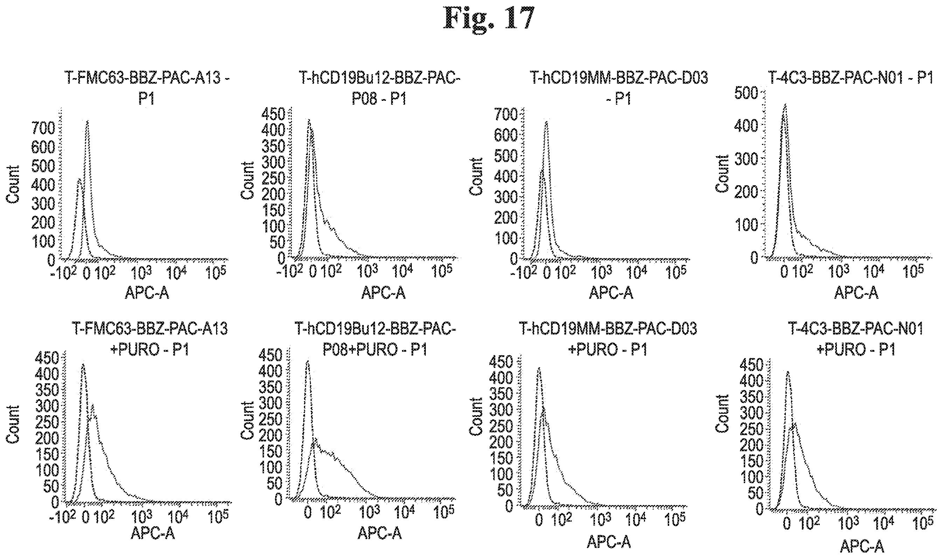

**Fig. 17** depicts, in accordance with an embodiment of the invention, increased expression of the different CD19 CARs (shown with grey lines) on the surface of T cells as determined by staining with APC-Myc as compared to the uninfected T cells (UI; shown with dotted lines). Constructs depicted include CD8SP-FMC63-(vL-vH)-Myc-BBz-T2A-PAC(112014-A13)[SEQ ID NO:1467]; CD8SP-CD19Bu12-(vL-vH)-Myc-BBz-T2A-PAC(082815-P08)[SEQ ID NO:1470]; CD8SP-2-CD19MM-(vL-vH)-Myc-BBz-T2A-PAC(062915-D03)[SEQ ID NO:1471]; CD8SP-KSHV-4C3-(vL-vH)-Myc-BBz-T2A-PAC(042315-N01)[SEQ ID NO:1643].

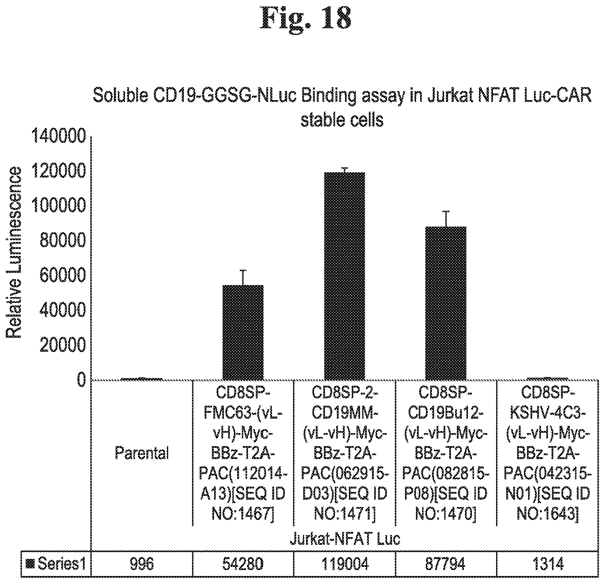

**Fig. 18** depicts, in accordance with various embodiments of the invention, strong binding of Jurkats expressing CD8SP-CD19Bu12-(vL-vH)-Myc-BBz-T2A-PAC(082815-P08)[SEQ ID NO:1470], CD8SP-2-CD19MM-(vL-vH)-Myc-BBz-T2A-PAC(062915-D03)[SEQ ID NO:1471] and CD8SP-FMC63-(vL-vH)-Myc-BBz-T2A-PAC(112014-A13)[SEQ ID NO:1467] CAR constructs to FLAG-CD19-ECD-GGSG-NLuc-AcV5 [SEQ ID NO:2671] supernatant, while no significant binding was observed on parental cells or those expressing the CD8SP-KSHV-4C3-(vL-vH)-Myc-BBz-T2A-PAC(042315-N01)[SEQ ID NO:1643] control CAR.

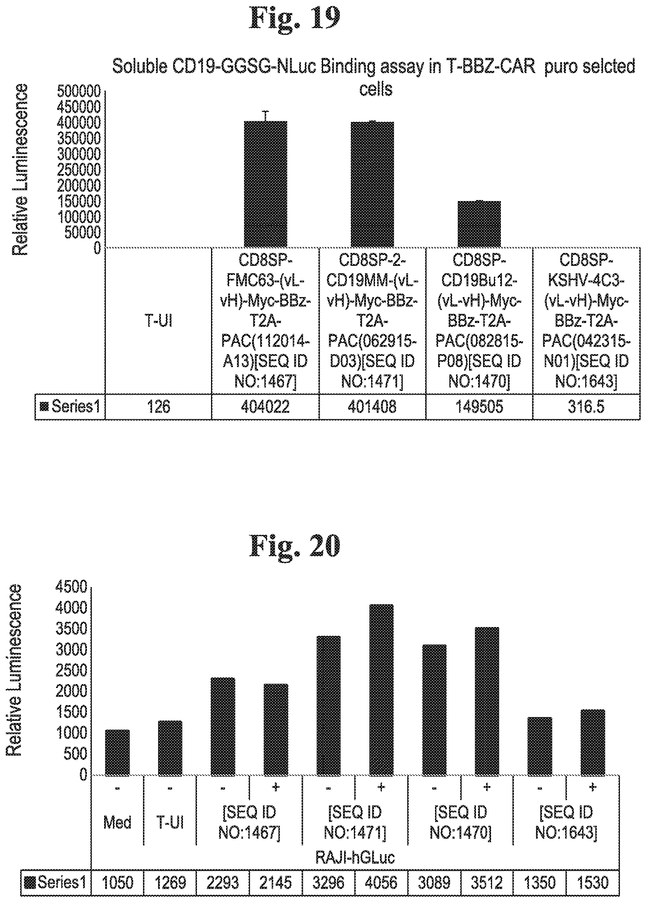

**Fig. 19** depicts, in accordance with various embodiments of the invention, strong binding of T cells expressing CD8SP-CD19Bu12-(vL-vH)-Myc-BBz-T2A-PAC(082815-P08)[SEQ ID NO:1470], CD8SP-2-CD19MM-(vL-vH)-Myc-BBz-T2A-PAC(062915-D03)[SEQ ID NO:1471] and CD8SP-FMC63-(vL-vH)-Myc-BBz-T2A-PAC(112014-A13)[SEQ ID NO:1467] CAR constructs to FLAG-CD19-ECD-GGSG-NLuc-AcV5 [SEQ ID NO:2671] supernatant, while no significant binding was observed on parental cells or those expressing CD8SP-KSHV-4C3-(vL-vH)-Myc-BBz-T2A-PAC(042315-NO1)[SEQ ID NO:1643] control CAR.
**Fig. 20** depicts, in accordance with various embodiments of the invention, increase in GLuc activity, indicating lysis of RAJI target cells, following co-culture with T cells expressing CD19-specific CARs as compared to uninfected T cells (T-UI) or those expressing the control CAR 4C3. Constructs depicted include CD8SP-FMC63-(vL-vH)-Myc-BBz-T2A-PAC(112014-A13)[SEQ ID NO:1467]; CD8SP-2-CD19MM-(vL-vH)-Myc-BBz-T2A-PAC(062915-D03)[SEQ ID NO:1471]; CD8SP-CD19Bu12-(vL-vH)-Myc-BBz-T2A-PAC(082815-P08)[SEQ ID NO:1470]; CD8SP-KSHV-4C3-(vL-vH)-Myc-BBz-T2A-PAC(042315-N01)[SEQ ID NO:1643]; (-) indicates CAR-transduced T cells were used without puromycin selection; (+) indicates CAR-transduced T cells were used with puromycin selection.

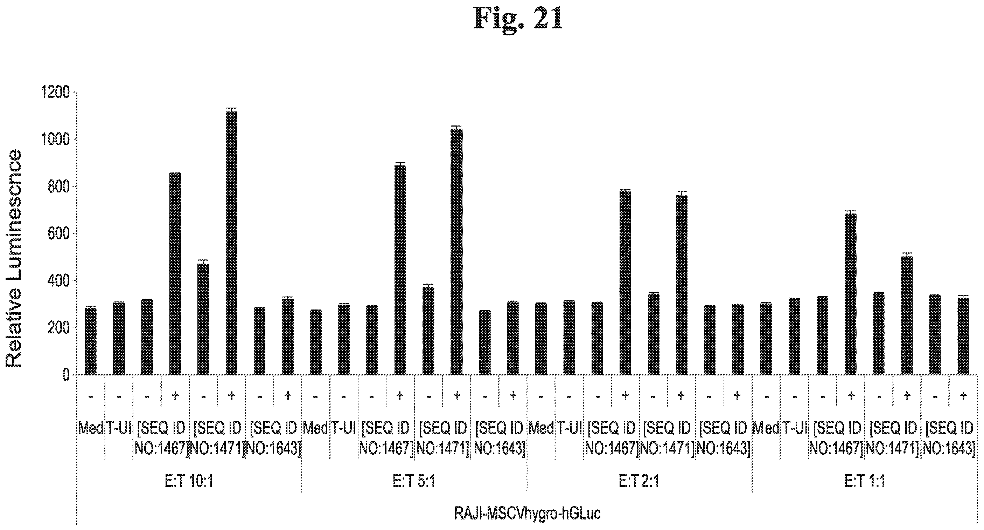

**Fig. 21** depicts, in accordance with various embodiments of the invention, increase in GLuc activity, indicating lysis of RAJI target cells, following co-culture with T cells expressing CD19-specific CARs as compared to uninfected T cells (T-UI) or those expressing the control CAR 4C3. Constructs depicted include CD8SP-FMC63-(vL-vH)-Myc-BBz-T2A-PAC(112014-A13)[SEQ ID NO:1467]; CD8SP-2-CD19MM-(vL-vH)-Myc-BBz-T2A-PAC(062915-D03)[SEQ ID NO:1471]; CD8SP-KSHV-4C3-(vL-vH)-Myc-BBz-T2A-PAC(042315-N01)[SEQ ID NO:1643]; (-) indicates CAR-transduced T cells were used without puromycin selection; (+) indicates CAR-transduced T cells were used with puromycin selection.

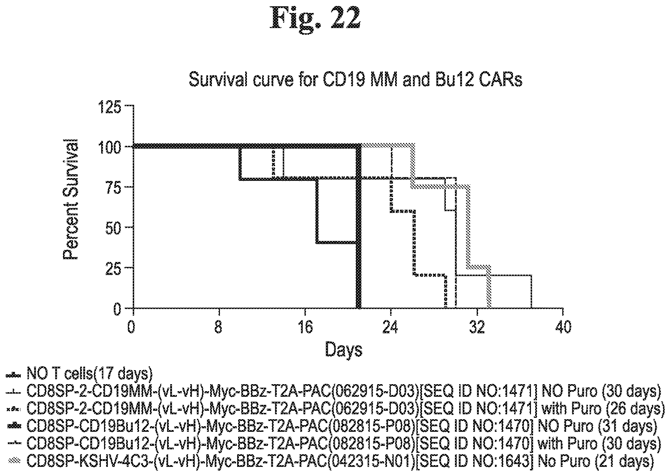

**Fig. 22** depicts, in accordance with various embodiments of the invention, the survival of mice in specific groups.

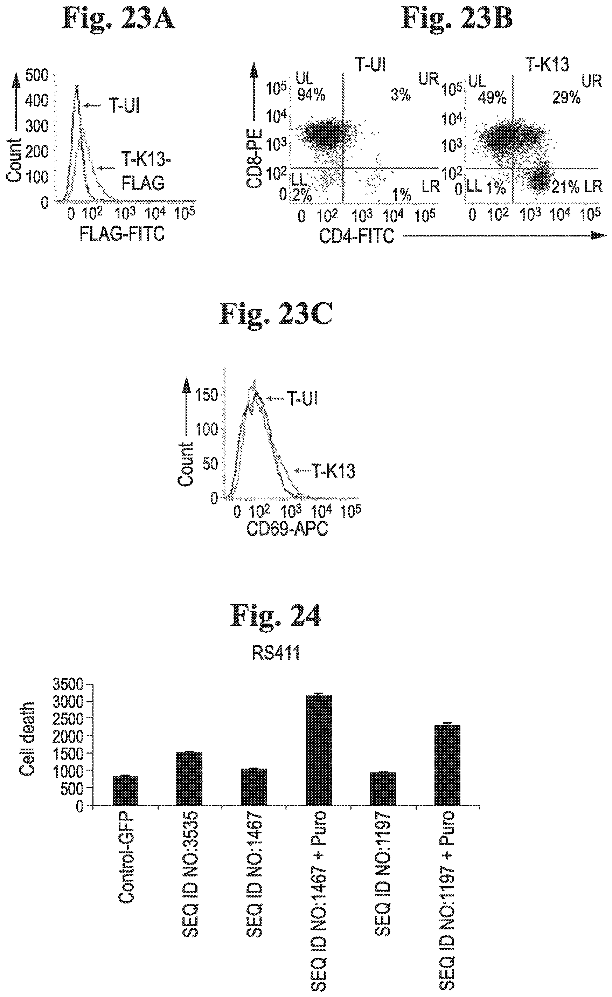

**Fig. 23** depicts, in accordance with various embodiments of the invention, increased staining with FITC-FLAG antibody in K13-expressing T cells as compared to uninfected T cells. K13 immortalized T cells are CD8+/CD4- (49%), CD8-/CD4+ (21%) and CD8+/CD4+ (29%).
**Fig. 24** depicts, in accordance with various embodiments of the invention, that CD8SP-FMC63-(vL-vH)-Myc-BBz-T2A-PAC(112014-A13)[SEQ ID NO:1467], and CD8SP-FMC63-(vL-vH)-Myc-BBz-P2A-K13-Flag-T2A-PAC(111014-Y11)[SEQ ID NO:1197] expressing T-cells that have been selected with puromycin induce specific lysis of RS411 cells as measured by Gluc assay.

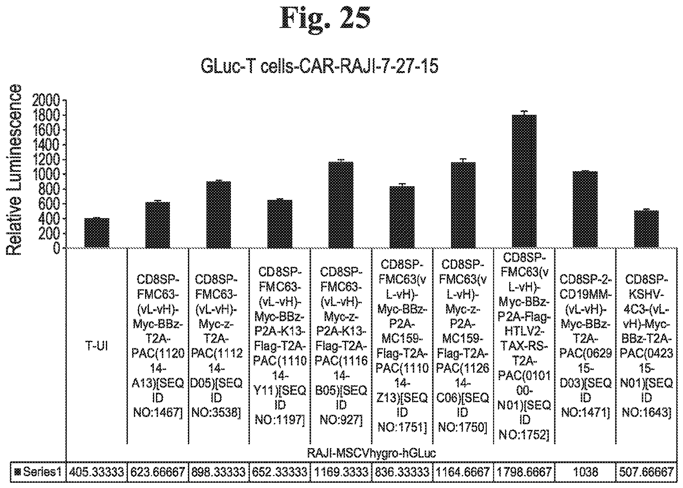

**Fig. 25** depicts, in accordance with various embodiments of the invention, increase in GLuc activity, indicating lysis of target cells, following co-culture with T cells expressing CD19-specific CARs as compared to uninfected T cells (T-UI) or those expressing the control CAR 4C3.

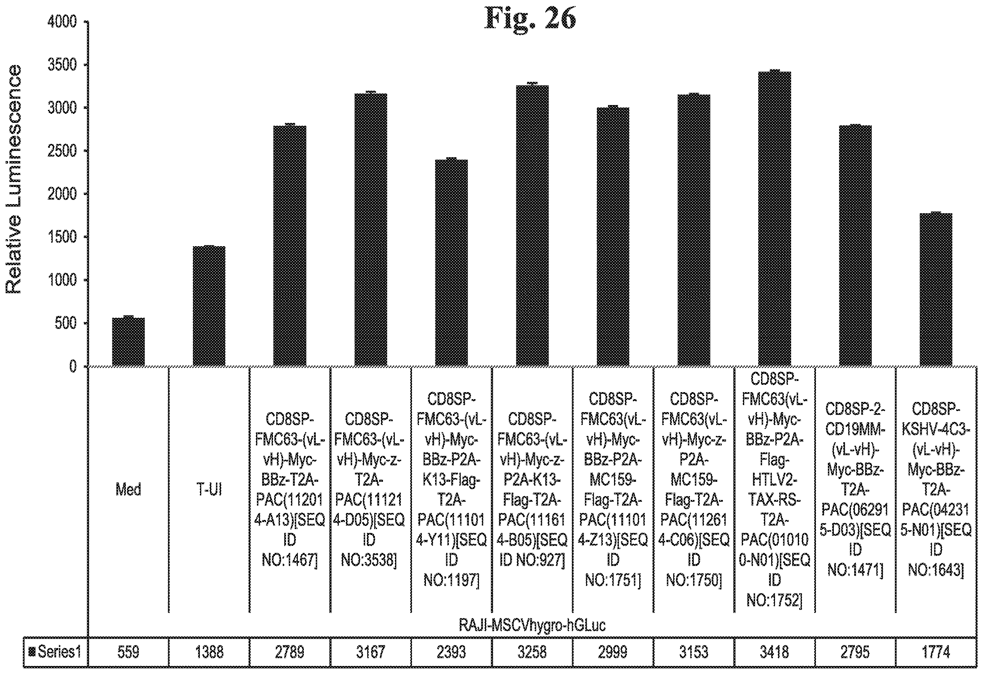

**Fig. 26** depicts, in accordance with various embodiments of the invention, confirms that incorporation of K13, MC159 or HTLV2-Tax (Tax2) does not negatively impact the killing ability of CARs with or without the presence of 41BB costimulatory domain.

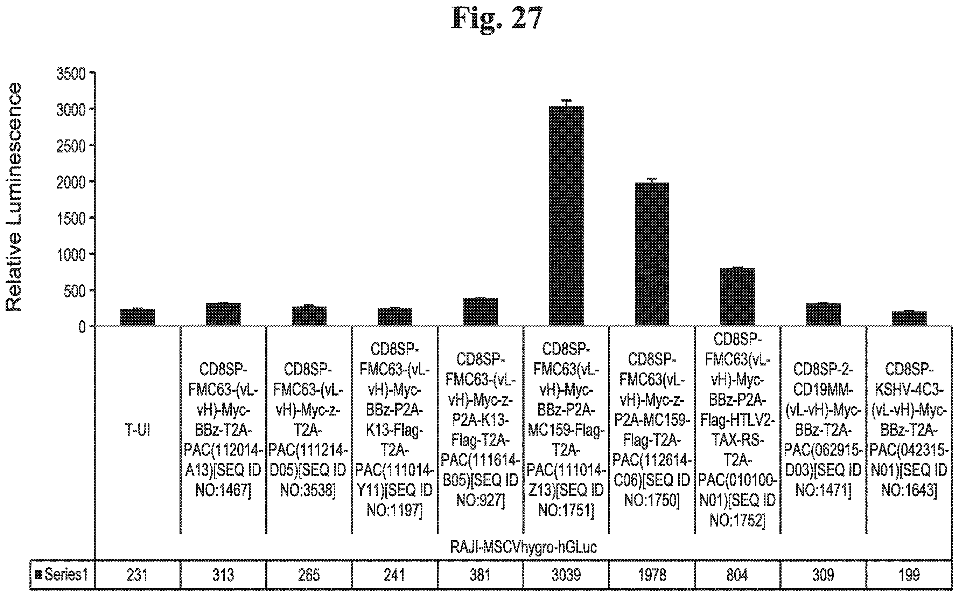

**Fig. 27** depicts, in accordance with various embodiments of the invention, that the T cells expressing CAR constructs that co-expressed MC159 and HTL V2-Tax continue to exert cytotoxicity after 2 months in culture while the T cells expressing the constructs lacking MC159 and HTLV2-Tax lost this ability.

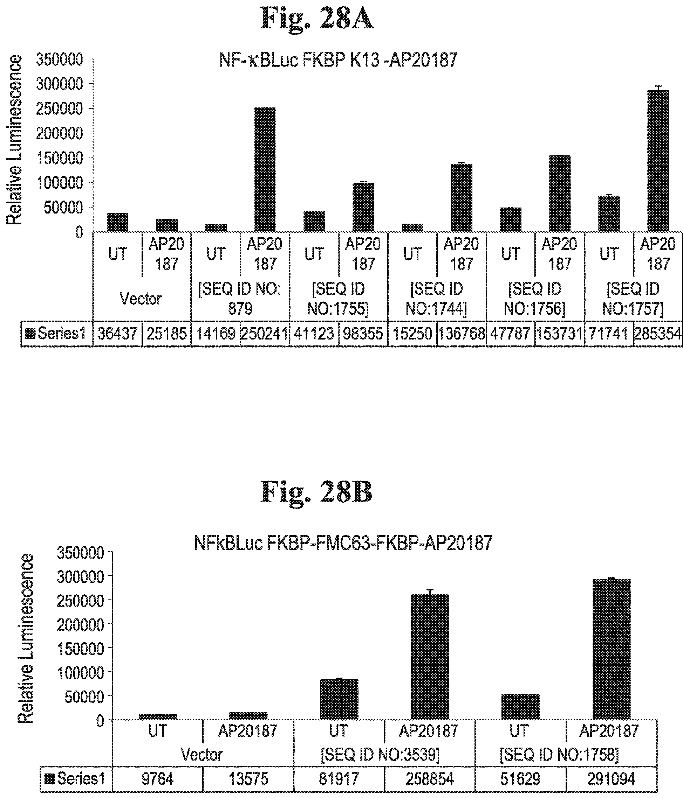

**Fig. 28A****-****Fig. 28B** depict, in accordance with various embodiments of the invention, treatment with AP20187 led to increase in K13- and HTLV2-Tax RS mutant-induced NF-κB-Luc activity in all constructs in which K13 and HTLV2-Tax mutant were expressed in fusion with FKBP. Constructs depicted in Fig. 28A include FKBPX2-K13[SEQ ID NO: 879]; CD8SP-FMC63(vL-vH)-Myc-z-P2A-FKBP-K13-FLAG-T2A-EGFP(112614-B06)[SEQ ID NO:1755]; CD8SP-161-(vL-vH)-Myc-BBz-P2A-FKBP-K13-Flag-T2A-EGFP(112614-E05)[SEQ ID NO:1744]; CD8SP-FMC63(vL-vH)-Myc-z-P2A-FKBPx2-K13-FLAG-T2A-EGFP(120314-J03)[SEQ ID NO:1756]; and CD8SP-FMC63(vL-vH)-Myc-z-P2A-Myr-FKBPx2-K13-FLAG-T2A-EGFP(120314-N07)[SEQ ID NO:1757]. Constructs depicted in **Fig. 28B** include CD8SP-FMC63(vL-vH)-Myc-BBz-P2A-FKBPx2-FLAG-TAX2U2RS-T2A-eGFP-M03(012315-03)[SEQ ID NO:3539]; and CD8SP-FMC63(vL-vH)-Myc-BBz-P2A-FKBPx2-HTLV2-Tax-RS-T2A-EGFP(012315-001)[SEQ ID NO:1758].

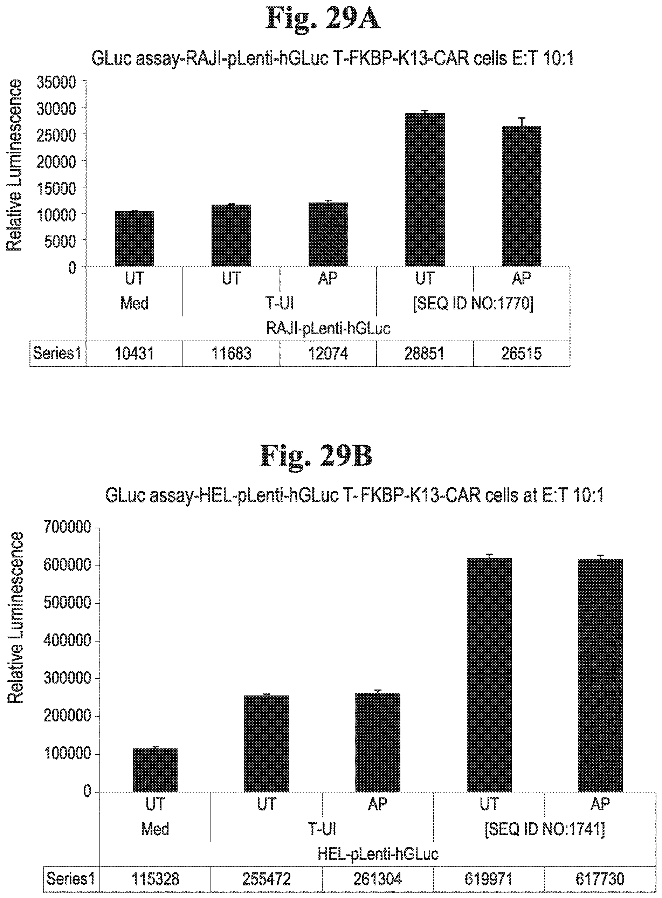

**Fig. 29A****-****Fig. 29B** depict, in accordance with various embodiments of the invention, activation of K13-mediated NF-κB activity by addition of AP20187 does not adversely affect cell killing induced by the CAR constructs that coexpress FKBP-K13. Construct depicted in **Fig. 29A** include CD8SP-FMC63(vL-vH)-Myc-z-P2A-FKBP-K13-FLAG-T2A-PAC(112316-S06)[SEQ ID NO:1770]. Construct depicted in **Fig. 29B** include CD8SP-161-(vL-vH)-Myc-z-P2A-FKBP-K13-FLAG-T2A-PAC(112916-U06)[SEQ ID NO: 1741].

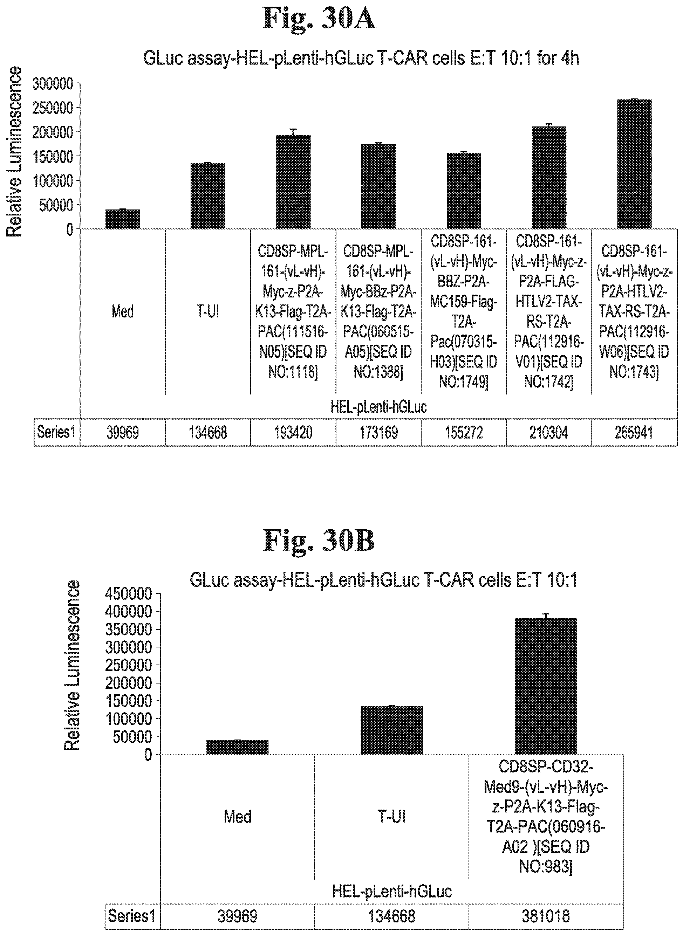

**Fig. 30A****-****Fig 30B** depict, in accordance with various embodiments of the invention, induction of target cell death by different MPL-directed and CD32-directed CAR constructs that coexpress K13, MC159, HTLV2-Tax or HTLV2-Tax RS mutant.

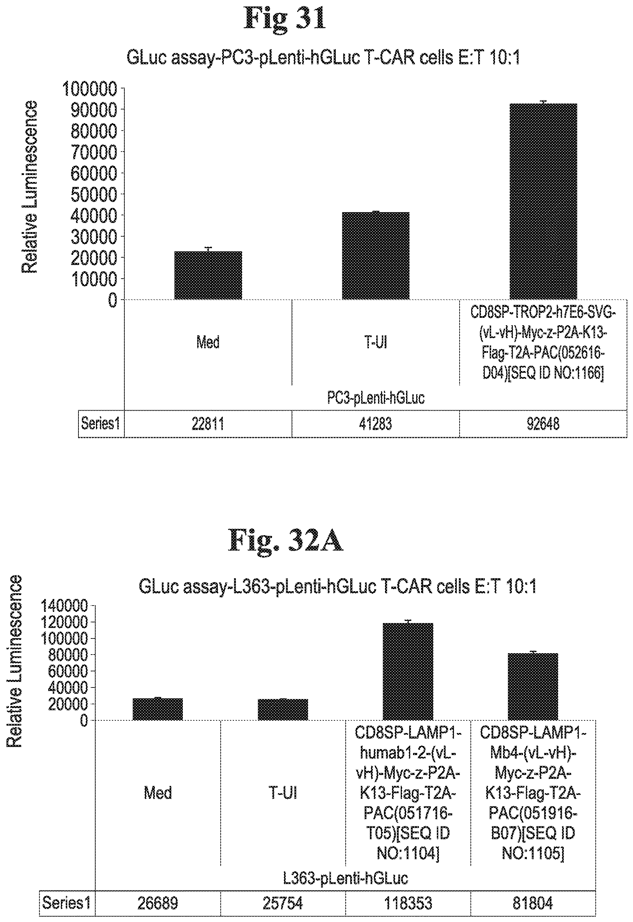

**Fig. 31** depicts, in accordance with various embodiments of the invention, induction of PC3-target cell death by a TROP2-directed conventional CAR I construct that coexpresses K13.
**Fig. 32A****-****Fig 32B** depict, in accordance with various embodiments of the invention, induction of target cell death by different LAMP1-directed CAR constructs that coexpress K13,

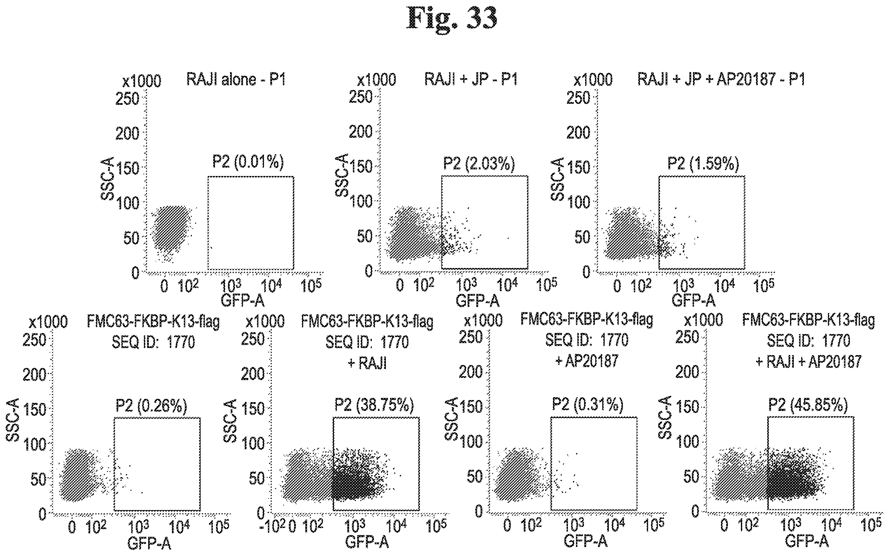

**Fig. 33** depicts, in accordance with various embodiments of the invention, that activation of K13 signaling in T cells expressing the FKBP-K13 fusion protein by addition of AP20187 compound does not adversely affect activation of CAR-induced NFAT signaling.

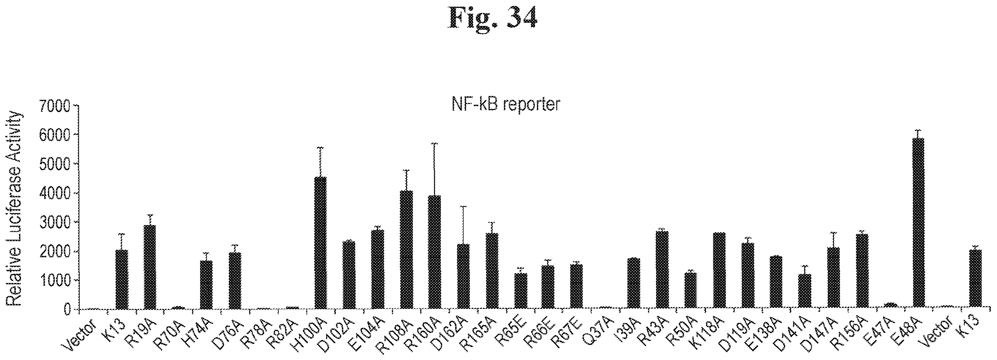

**Fig. 34** depicts, in accordance with various embodiments of the invention, NF-κB reporter activity by wild-type K13 and various K13 mutants.

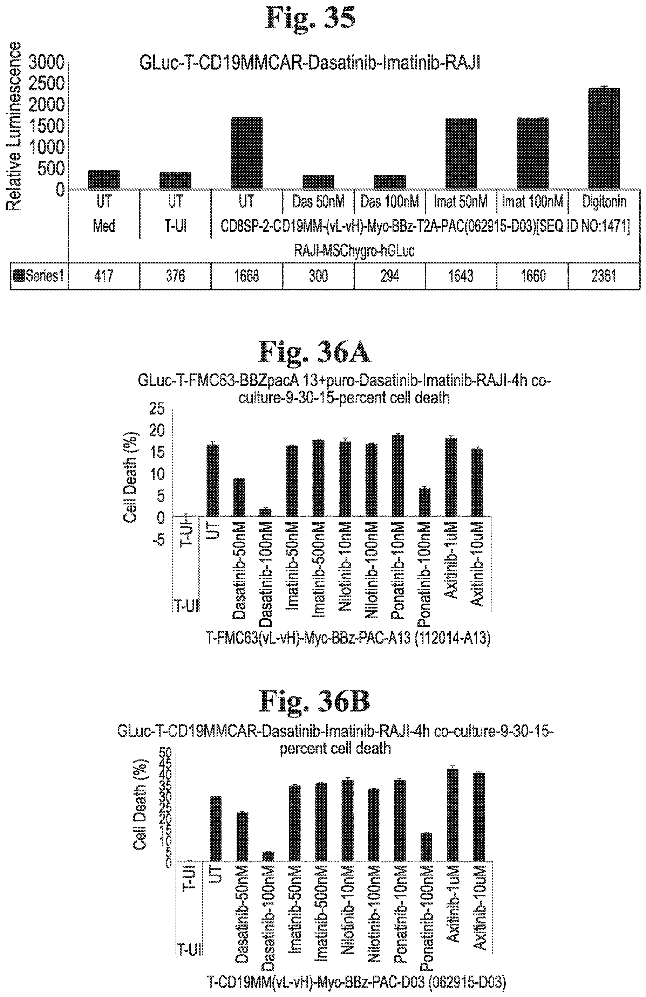

**Fig. 35** depict, in accordance with various embodiments of the invention, shows inhibition of CAR CD8SP-2-CD19MM-(vL-vH)-Myc-BBz-T2A-PAC(062915-D03)[SEQ ID NO:1471]-induced cell death by 50nM and 100nM Dasatinib, while Imatinib has no effect.
**Fig. 36** depict, in accordance with various embodiments of the invention, increase in GLuc activity following co-culture with T cells expressing CD19-specific CARs CD8SP-FMC63-(vL-vH)-Myc-BBz-T2A-PAC(112014-A13)[SEQ ID NO:1467] and CD8SP-2-CD19MM-(vL-vH)-Myc-BBz-T2A-PAC(062915-D03)[SEQ ID NO:1471] as compared to the uninfected T cells (T-UI) indicating lysis of target cells by the different CD19-CAR-T cells and its inhibition by Dasatinib and Ponatinib at the indicated doses.

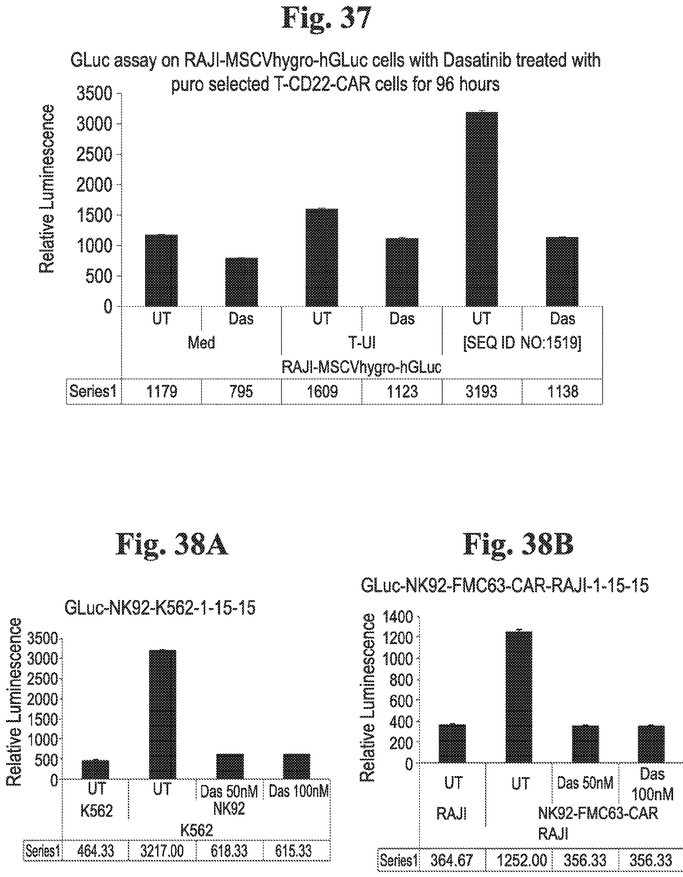

**Fig. 37** depict, in accordance with various embodiments of the invention, that Dasatinib effectively blocks killing of RAJI-GLuc cells by T cells expressing the CD8SP-CD22-m971-(vL-vH)-Myc-BBz-T2A-PAC(091515-A02)[SEQ ID NO:1519] CAR as determined by GLuc assay.
**Fig. 38A****-****Fig. 38B** depict, in accordance with various embodiments of the invention, that **Fig. 38A****:** Dasatinib blocks NK92 cells mediated death of K562 cells as determined by Gluc assay and **Fig. 38B****:** Dasatinib blocks death of RAJI-GLuc cells by CD8SP-FMC63-(vL-vH)-Myc-BBz-T2A-EGFP(070814-D06)[SEQ ID NO:3535] expressing NK92MI cells as determined by Gluc assay.

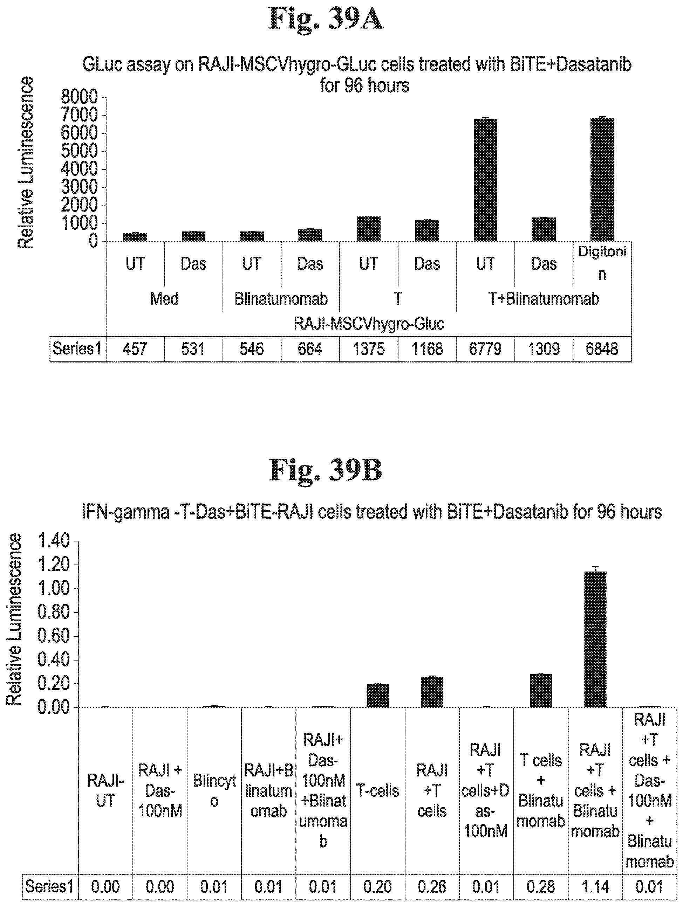

**Fig. 39A****-****Fig. 39B** depict, in accordance with various embodiments of the invention, that **Fig. 39A****:** treatment with Blinatumomab in the presence of T cells result in strong induction of death of RAJI-GLuc cells which is effectively blocked by 100nM Dasatinib and **Fig. 39B****:** that co-culture of RAJI-GLuc cells with Blinatumomab in the presence of T cells result in strong induction of IFNγ production which is effectively blocked by 100nM Dasatinib.

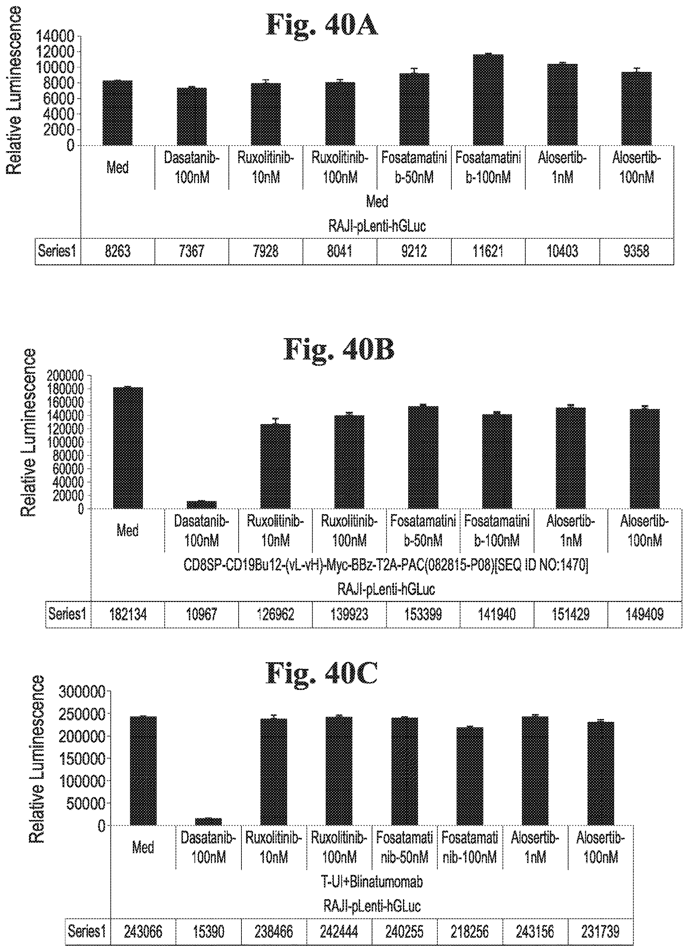

**Fig. 40A****-****Fig. 40C** depict, in accordance with various embodiments of the invention, **Fig. 40A** shows no significant toxic effect of the various drugs on RAJI-pLenti-Gluc cells that had not been co-cultured with CAR T cells. **Fig. 40B** shows mild to modest inhibition of CD8SP-CD19Bu12-(vL-vH)-Myc-BBz-T2A-PAC(082815-P08)[SEQ ID NO:1470] CAR-T cells-induced death of RAJI-pLenti-GLuc cells by Ruxolitinib, Fosatamatinib and Alisertib as compared to cells treated with media (Med) alone **(****Fig 40A****).** Dasatinib was used as a positive control. **Fig. 40C** shows that the above compounds have only a minimal effect on Blinatumomab induced cell death mediated by T cells at the indicated concentrations.

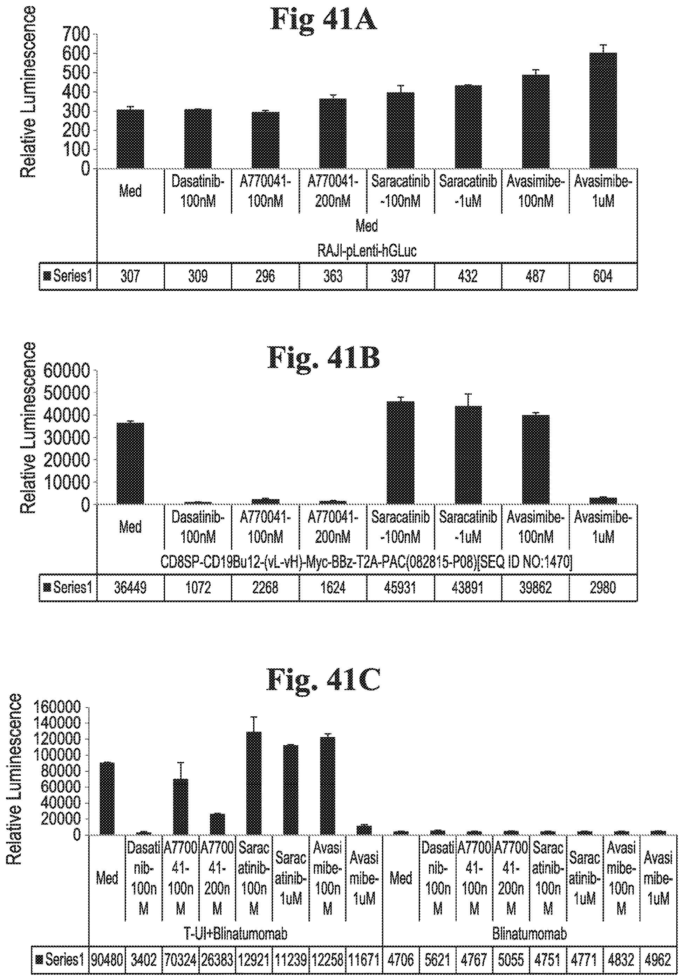

**Fig. 41A****-****Fig. 41C** depict, in accordance with various embodiments of the invention, **Fig. 41A** shows the release of Gluc in cells treated with the indicated drugs in the presence of media alone without any CAR-T cells. **Fig.41B** shows near complete inhibition of CD8SP-CD19Bu12-(vL-vH)-Myc-BBz-T2A-PAC(082815-P08)[SEQ ID NO:1470] CAR-T cells-induced death of RAJI-pLenti-GLuc cells by A-770041 at 100nM and 200nM while Saracatinib had no significant effect. Avasimibe had no significant effect on CAR-T cells induced cell death at 100nM but completely blocked it at 1µM. **Fig. 41C** shows that treatment with 100nM A770041 partially blocked cell death induced by combination of Blinatumomab with T cells while treatment with 200nM A770041 led to complete inhibition.

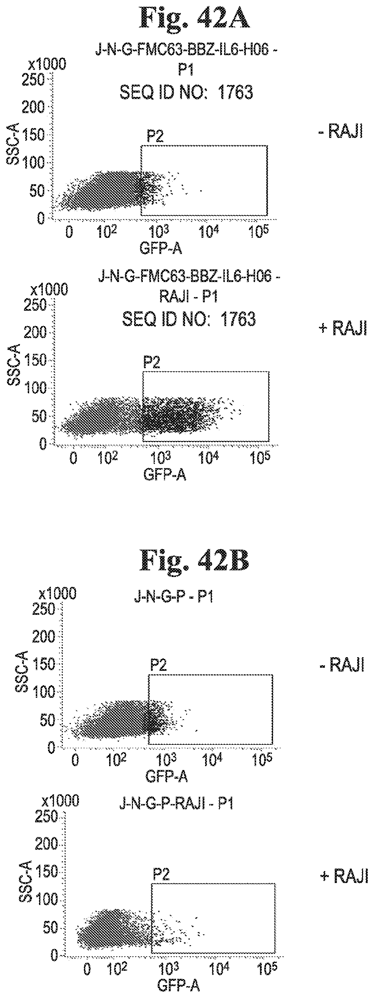

**Fig. 42A****-****Fig. 42B** depict, in accordance with various embodiments of the invention, that coculturing of Jurkat cells expressing CD8SP-FMC63(vL-vH)-BBz-P2A-IgHSP-IL6R-M83(vL-vH)-Flag-T2A-PAC(011315-K04)[SEQ ID NO:1763] with RAJI cells led to increase in EGFP expression as compared to cells that had not been exposed to RAJI cells **(****Fig. 42A****).** This indicates activation of NFAT signaling. No increase in EGFP expression was observed in parental Jurkats (J-N-G-P) without or with culturing with RAJI **(****Fig. 42B****).**

### DETAILED DESCRIPTION

All references cited herein are incorporated by reference in their entirety as though fully set forth. Unless defined otherwise, technical and scientific terms used herein have the same meaning as commonly understood by one of ordinary skill in the art to which this invention belongs. Allen et al., Remington: The Science and Practice of Pharmacy 22nd ed., Pharmaceutical Press (September 15, 2012); Hornyak et al., Introduction to Nanoscience and Nanotechnology, CRC Press (2008); Singleton and Sainsbury, Dictionary of Microbiology and Molecular Biology 3rd ed, revised ed, J. Wiley & Sons (New York, NY 2006); Smith, March's Advanced Organic Chemistry Reactions, Mechanisms and Structure 7th ed., J. Wiley & Sons (New York, NY 2013); Singleton, Dictionary of DNA and Genome Technology 3rd ed., Wiley-Blackwell (November 28, 2012); and Green and Sambrook, Molecular Cloning: A Laboratory Manual 4th ed., Cold Spring Harbor Laboratory Press (Cold Spring Harbor, NY 2012), provide one skilled in the art with a general guide to many of the terms used in the present application. For references on how to prepare antibodies, see Greenfield, Antibodies A Laboratory Manual 2nd ed., Cold Spring Harbor Press (Cold Spring Harbor NY, 2013); Köhler and Milstein, Derivation of specific antibody-producing tissue culture and tumor lines by cell fusion, Eur. J. Immunol. 1976 Jul, 6(7):511-9; Queen and Selick, Humanized immunoglobulins*,* U. S. Patent No. 5,585,089 (1996 Dec); and Riechmann et al., Reshaping human antibodies for therapy, Nature 1988 Mar 24, 332(6162):323-7..

One skilled in the art will recognize many methods and materials similar or equivalent to those described herein, which could be used in the practice of the present invention. Other features and advantages of the invention will become apparent from the following detailed description, taken in conjunction with the accompanying drawings, which illustrate, by way of example, various features of embodiments of the invention. Indeed, the present invention is in no way limited to the methods and materials described. For convenience, certain terms employed herein, in the specification, examples and appended claims are collected here.

Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention, because the scope of the invention is limited only by the claims. Unless otherwise defined, all technical and scientific terms used herein have the same meaning as commonly understood by one of ordinary skill in the art to which this invention belongs.

Chimeric antigen receptors (CAR) are artificial T cell receptors that are under investigation as a therapy for cancer, using a technique called adoptive cell transfer. Hematopoietic cells are removed from a patient and modified so that they express receptors that recognize proteins that are specific to the particular form of cancer. The cells, which can then recognize and kill the cancer cells, are reintroduced into the patient. CAR targeting the human CD19 antigen have shown impressive activity against B cell lineage human blood cancers, such as Acute Lymphocytic Leukemia, chronic lymphocytic leukemia and lymphoma and are under commercial development by several companies. However, there is a need to develop CAR targeting antigens that are expressed on non-lymphoid cells. This invention pertains to CARs targeting several such antigens.

### Definitions

As used herein the term "comprising" or "comprises" is used in reference to compositions, methods, and respective component(s) thereof, that are useful to an embodiment, yet open to the inclusion of unspecified elements, whether useful or not. It will be understood by those within the art that, in general, terms used herein are generally intended as "open" terms (e.g., the term "including" should be interpreted as "including but not limited to," the term "having" should be interpreted as "having at least," the term "includes" should be interpreted as "includes but is not limited to," etc.).

Unless stated otherwise, the terms "a" and "an" and "the" and similar references used in the context of describing a particular embodiment of the application (especially in the context of claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (for example, "such as") provided with respect to certain embodiments herein is intended merely to better illuminate the application and does not pose a limitation on the scope of the application otherwise claimed. The abbreviation, "e.g." is derived from the Latin exempli gratia, and is used herein to indicate a non-limiting example. Thus, the abbreviation "e.g." is synonymous with the term "for example." No language in the specification should be construed as indicating any non-claimed element essential to the practice of the application.

As used herein, the term "about" refers to a measurable value such as an amount, a time duration, and the like, and encompasses variations of ±20%, ±10%, ±5%, ±1%, ±0.5% or ±0.1% from the specified value.

"Chimeric antigen receptor" or "CAR" or "CARs" as used herein refers to engineered receptors, which graft an antigen specificity onto cells (for example T cells such as naive T cells, central memory T cells, effector memory T cells or combination thereof). CARs are also known as artificial T-cell receptors, chimeric T-cell receptors or chimeric immunoreceptors. In various embodiments, CARs are recombinant polypeptides comprising an antigen-specific domain (ASD), a hinge region (HR), a transmembrane domain (TMD), co-stimulatory domain (CSD) and an intracellular signaling domain (ISD).

"Antigen-specific domain" (ASD) refers to the portion of the CAR that specifically binds the antigen on the target cell. In some embodiments, the ASD of the CARs comprising any of the backbones described herein comprises an antibody or a functional equivalent thereof or a fragment thereof or a derivative thereof. The targeting regions may comprise full length heavy chain, Fab fragments, single chain Fv (scFv) fragments, divalent single chain antibodies or diabodies, each of which are specific to the target antigen. There are, however, numerous alternatives, such as linked cytokines (which leads to recognition of cells bearing the cytokine receptor), affibodies, ligand binding domains from naturally occurring receptors, soluble protein/peptide ligand for a receptor (for example on a tumor cell), peptides, and vaccines to prompt an immune response, which may each be used in various embodiments of the invention. In some embodiments, almost any molecule that binds a given antigen with high affinity can be used as an ASD, as will be appreciated by those of skill in the art. In some embodiments, the ASD comprises T cell receptors (TCRs) or portions thereof. In exemplary embodiments, nucleic acids encoding ASDs comprising scFVs are set forth herein in SEQ ID NOs: 564-798.

"Hinge region" (HR) as used herein refers to the hydrophilic region which is between the ASD and the TMD. The hinge regions include but are not limited to Fc fragments of antibodies or fragments or derivatives thereof, hinge regions of antibodies or fragments or derivatives thereof, CH2 regions of antibodies, CH3 regions of antibodies, artificial spacer sequences or combinations thereof. Examples of hinge regions include but are not limited to CD8a hinge, and artificial spacers made of polypeptides which may be as small as, for example, Gly3 or CH1 and CH3 domains of IgGs (such as human IgG4). In some embodiments, the hinge region is any one or more of (i) a hinge, CH2 and CH3 regions of IgG4, (ii) a hinge region of IgG4, (iii) a hinge and CH2 of IgG4, (iv) a hinge region of CD8a, (v) a hinge, CH2 and CH3 regions of IgG1, (vi) a hinge region of IgG1 or (vi) a hinge and CH2 region of IgG1. Other hinge regions will be apparent to those of skill in the art and may be used in connection with alternate embodiments of the invention.

"Transmembrane domain" (TMD) as used herein refers to the region of the CAR which crosses the plasma membrane. The transmembrane domain of the CAR of the invention is the transmembrane region of a transmembrane protein (for example Type I transmembrane proteins), an artificial hydrophobic sequence or a combination thereof. Other transmembrane domains will be apparent to those of skill in the art and may be used in connection with alternate embodiments of the invention. In some embodiments, the TMD encoded CAR comprising any of the backbones described herein comprises a transmembrane domain selected from the transmembrane domain of an alpha, beta or zeta chain of a T-cell receptor, CD28, CD3 epsilon, CD45, CD4, CD5, CD8, CD9, CD16, CD22, CD33, CD37, CD64, CD80, CD86, CD134, CD137, CD154, KIRDS2, OX40, CD2, CD27, LFA-1 (CDl la, CD18), ICOS (CD278), 4-1BB (CD137), GITR, CD40, BAFFR, HVEM (LIGHTR), SLAMF7, NKp80 (KLRFl), CD160, CD19, IL2R beta, IL2R gamma, IL7R a, ITGA1, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGB1, CD29, ITGB2, CD18, LFA-1, ITGB7, TNFR2, DNAM1(CD226), SLAMF4 (CD244, 2B4), CD84, CD96 (Tactile), CEACAM1, CRT AM, Ly9 (CD229), CD160 (BY55), PSGL1, CD100 (SEMA4D), SLAMF6 (NTB-A, Lyl08), SLAM (SLAMF1, CD150, IPO-3), BLAME (SLAMF8), SELPLG (CD162), LTBR, PAG/Cbp, NKp44, NKp30, NKp46, NKG2D, and/or NKG2C.

"Co-stimulatory domain" (CSD) as used herein refers to the portion of the CAR comprising any of the backbones described herein which enhances the proliferation, survival and/or development of memory cells. The CARs of the invention may comprise one or more co-stimulatory domains. Each co-stimulatory domain comprises the costimulatory domain of any one or more of, for example, members of the TNFR superfamily, CD28, CD137 (4-1BB), CD134 (OX40), Dap10, CD27, CD2, CD5, ICAM-1, LFA-1(CD11a/CD18), Lck, TNFR-I, TNFR-II, Fas, CD30, CD40 or combinations thereof. Other co-stimulatory domains (e.g., from other proteins) will be apparent to those of skill in the art and may be used in connection with alternate embodiments of the invention.

"Intracellular signaling domain" (ISD) or "cytoplasmic domain" as used herein refers to the portion of the CAR comprising any of the backbones described herein which transduces the effector function signal and directs the cell to perform its specialized function. Examples of domains that transduce the effector function signal include but are not limited to the z chain of the T-cell receptor complex or any of its homologs (e.g., h chain, FceR1g and b chains, MB1 (Iga) chain, B29 (Igb) chain, etc.), human CD3 zeta chain, CD3 polypeptides (D, d and e), syk family tyrosine kinases (Syk, ZAP 70, etc.), src family tyrosine kinases (Lck, Fyn, Lyn, etc.) and other molecules involved in T-cell transduction, such as CD2, CD5 and CD28. Other intracellular signaling domains will be apparent to those of skill in the art and may be used in connection with alternate embodiments of the invention.

"Linker" (L) or "linker domain" or "linker region" as used herein refer to an oligo- or polypeptide region from about 1 to 100 amino acids in length, which links together any of the domains/regions of the CAR of the invention. Linkers may be composed of flexible residues like glycine and serine so that the adjacent protein domains are free to move relative to one another. Longer linkers may be used when it is desirable to ensure that two adjacent domains do not sterically interfere with one another. Linkers may be cleavable or non-cleavable. Examples of cleavable linkers include 2A linkers (for example T2A), 2A-like linkers or functional equivalents thereof and combinations thereof. In some embodiments, the linkers include the picornaviral 2A-like linker, CHYSEL sequences of porcine teschovirus (P2A), *Thosea asigna* virus (T2A) or combinations, variants and functional equivalents thereof. In other embodiments, the linker sequences may comprise Asp-Val/Ile-Glu-X-Asn-Pro-Gly^{(2A)}-Pro^{(2B)} motif, which results in cleavage between the 2A glycine and the 2B proline. The nucleic sequences of several exemplary cleavable linkers are provided in SEQ ID NO: 831 to SEQ ID NO: 836 and amino acid sequences of several exemplary linkers are provide in SEQ ID NO: 2598 to SEQ ID NO: 2602. Other linkers will be apparent to those of skill in the art and may be used in connection with alternate embodiments of the invention. In an embodiment, a Ser-Gly-Ser-Gly (SGSG) motif (SEQ ID NOs: 837-838 and SEQ ID NO: 2603) is also added upstream of the cleavable linker sequences to enhance the efficiency of cleavage. A potential drawback of the cleavable linkers is the possibility that the small 2A tag left at the end of the N-terminal protein may affect protein function or contribute to the antigenicity of the proteins. To overcome this limitation, in some embodiments, a furine cleavage site (RAKR) (SEQ ID NO: 839-841 and SEQ ID NO: 2604) is added upstream of the SGSG motifs to facilitate cleavage of the residual 2A peptide following translation.

"Effector function" refers to the specialized function of a differentiated cell. Effector function of a T-cell, for example, may be cytolytic activity or helper activity including the secretion of cytokines.

"Enhancer", as used herein, denotes sequence elements that augment, improve or ameliorate transcription of a nucleic acid sequence irrespective of its location and orientation in relation to the nucleic acid sequence to be expressed. An enhancer may enhance transcription from a single promoter or simultaneously from more than one promoter. As long as this functionality of improving transcription is retained or substantially retained (e.g., at least 70%, at least 80%, at least 90% or at least 95% of wild-type activity, that is, activity of a full-length sequence), any truncated, mutated or otherwise modified variants of a wild-type enhancer sequence are also within the above definition.

"Accessory module" or "accessory modules" as used herein refers to an element that is co-expressed with a CAR to increase, decrease, regulate or modify the expression or activity of a CAR or CAR-expressing cells. In exemplary embodiments, accessory modules include but are not limited to examples described in **Table 16.** In one embodiment, the accessory module comprises K13-vFLIP **(Table 16).** In another embodiment, the accessory module comprises MC159-vFLIP **(Table 16).** In a further embodiment, the accessory module comprises an FKBP((FK506 binding protein)-fusion protein, such as FKBP-K13, in which whose activity can be controlled by the administration of a dimerizer molecule **(Table 16).**

"Switch domain," or a "dimerization domain" as used herein, typically refers to a polypeptide-based entity, that, in the presence of a dimerization molecule, associates with another switch domain. The association results in a functional coupling of a first entity linked to, e.g., fused to, a first switch domain, and a second entity linked to, e.g., fused to, a second switch domain. A first and second switch domain are collectively referred to as a dimerization switch. In embodiments, the first and second switch domains are the same as one another, e.g., they are polypeptides having the same primary amino acid sequence, and are referred to collectively as a homodimerization switch. In embodiments, the switch is intracellular. In embodiments, the switch domain is a polypeptide-based entity, e.g., FKBP (FK506 binding protein), and the dimerization molecule is small molecule, e.g., AP20187.

"Dimerization molecule," as that term is used herein refers to a molecule that promotes the association of a first switch domain with a second switch domain. In embodiments, the dimerization molecule does not naturally occur in the subject, or does not occur in concentrations that would result in significant dimerization. In embodiments, the dimerization molecule is a small molecule, e.g., rapamycin or a rapalogue, e.g, RAD001 or AP20187.

"Therapeutic Controls" as used herein refers to an element used for controlling the activity of a CAR expressing cell.. In some embodiments, therapeutic controls for controlling the activity of the CAR expressing cells of the invention comprise any one or more of truncated epidermal growth factor receptor (tEGFR), truncated epidermal growth factor receptor viii (tEGFRviii), truncated CD30 (tCD30), truncated BCMA (tBCMA), truncated CD19 (tCD19), thymidine kinase, cytosine deaminase, nitroreductase, xanthine-guanine phosphoribosyl transferase, human caspase 8, human caspase 9, inducible caspase 9, purine nucleoside phosphorylase, linamarase/linamarin/glucose oxidase, deoxyribonucleoside kinase, horseradish peroxidase (HRP)/indole-3-acetic (IAA), Gamma-glutamylcysteine synthetase, CD20/alphaCD20, CD34/thymidine kinase chimera, dox-depedent caspase-2, mutant thymidine kinase (HSV-TKSR39), AP1903/Fas system, a chimeric cytokine receptor (CCR), a selection marker, and combinations thereof. The nucleic acid sequences of several therapeutic controls are provided in SEQ ID NO: 850 to SEQ ID NO: 856 and SEQ ID NO: 883 **(Table 16).**

"Disease targeted by genetically modified cells" as used herein encompasses the targeting of any cell involved in any manner in any disease by the genetically modified cells of the invention, irrespective of whether the genetically modified cells target diseased cells or healthy cells to effectuate a therapeutically beneficial result. The genetically modified cells include but are not limited to genetically modified T-cells, NK cells, hematopoietic stem cells, pluripotent embryonic stem cells or embryonic stem cells. The genetically modified cells express the conventional CARs and novel backbones containing conventional CARs with accessory modules of the invention, which CARs may target any of the antigens expressed on the surface of target cells. Examples of antigens which may be targeted include but are not limited to antigens expressed on B-cells; antigens expressed on carcinomas, sarcomas, lymphomas, leukemia, germ cell tumors, and blastomas, antigens expressed on various immune cells; and antigens expressed on cells associated with various hematologic diseases, autoimmune diseases, and/or inflammatory diseases. Other antigens that may be targeted will be apparent to those of skill in the art and may be targeted by the CARs of the invention in connection with alternate embodiments thereof.

"Co-express" as used herein refers to simultaneous expression of two or more genes. Genes may be nucleic acids encoding, for example, a single protein or a chimeric protein as a single polypeptide chain. In one embodiment, simultaneous expression includes a single vector encoding a CAR and proteins that modulate an immune response. In another embodiment, simultaneous expression includes a first vector that encodes the CAR and a second vector that encodes one or more proteins that modulate an immune response.

"Autologous" cells as used herein refers to cells derived from the same individual as to whom the cells are later to be re-administered into.

"Genetically modified cells", "redirected cells", "genetically engineered cells" or "modified cells" as used herein refer to cells that express the CAR of the invention. In some embodiments, the genetically modified cells comprise vectors that encode a CAR. In some embodiments, the genetically modified cells comprise vectors that encode a CAR and one or more accessory molecules in the same vector. In some embodiments, the genetically modified cells comprise a first vector that encodes a CAR and a second vector that encodes the accessory molecule. In some embodiments, the genetically modified cells comprise a first vector that encodes a CAR and a second vector that encodes more than one accessory molecule. In some embodiments, the genetically modified cells comprise a first vector that encodes a CAR and a second vector that encodes the first accessory molecule and a third vector that encodes a second accessory molecule.

As used herein, the term "backbone" refers to the specific combination of conventional CARs **(Table 1)** and accessory modules as described in **Table 2.** In exemplary embodiments, specific combinations of CARs and accessory modules which comprise various backbones are described in **Table 2.** In one embodiment, the CAR and the accessory module are encoded by a single nucleic acid molecule. In another embodiment, the CAR is encoded by the first nucleic acid molecule and the accessory module is encoded by a second nucleic acid molecule. In some embodiments, the accessory module is encoded by more than one nucleic acid molecule, depending on the number of components in the accessory modules.

"Immune cell" as used herein refers to the cells of the mammalian immune system including but not limited to antigen presenting cells, B-cells, basophils, cytotoxic T-cells, dendritic cells, eosinophils, granulocytes, helper T-cells, leukocytes, lymphocytes, macrophages, mast cells, memory cells, monocytes, natural killer cells, neutrophils, phagocytes, plasma cells and T-cells.

The term "immune effector function" of the CAR-containing cell refers to any of the activities shown by the CAR-expressing cell upon stimulation by a stimulatory molecule. Examples of immune effector function, e.g., in a CAR-T cell, include cytolytic activity and helper activity, including the secretion of cytokines.

"Immune effector cell" as used herein refers to the T cells and natural killer (NK) cells.

"Immune response" as used herein refers to immunities including but not limited to innate immunity, humoral immunity, cellular immunity, immunity, inflammatory response, acquired (adaptive) immunity, autoimmunity and/or overactive immunity.

As used herein, "CD4 lymphocytes" refer to lymphocytes that express CD4, i.e., lymphocytes that are CD4+. CD4 lymphocytes may be T cells that express CD4.

The terms "T-cell" and "T-lymphocyte" are interchangeable and used synonymously herein. Examples include but are not limited to naive T cells, central memory T cells, effector memory T cells or combinations thereof.

As used herein, the term "antibody" refers to an intact immunoglobulin or to a monoclonal or polyclonal antigen-binding fragment with the Fc (crystallizable fragment) region or FcRn binding fragment of the Fc region, referred to herein as the "Fc fragment" or "Fc domain". Antigen-binding fragments may be produced by recombinant DNA techniques or by enzymatic or chemical cleavage of intact antibodies. Antigen-binding fragments include, inter alia, Fab, Fab', F(ab')2, Fv, dAb, and complementarity determining region (CDR) fragments, single-chain antibodies (scFv), single domain antibodies, chimeric antibodies, diabodies and polypeptides that contain at least a portion of an immunoglobulin that is sufficient to confer specific antigen binding to the polypeptide. The Fc domain includes portions of two heavy chains contributing to two or three classes of the antibody. The Fc domain may be produced by recombinant DNA techniques or by enzymatic (e.g. papain cleavage) or via chemical cleavage of intact antibodies.

The term "antibody fragment," as used herein, refer to a protein fragment that comprises only a portion of an intact antibody, generally including an antigen binding site of the intact antibody and thus retaining the ability to bind antigen. Examples of antibody fragments encompassed by the present definition include: (i) the Fab fragment, having VL, CL, VH and CH1 domains; (ii) the Fab' fragment, which is a Fab fragment having one or more cysteine residues at the C-terminus of the CH1 domain; (iii) the Fd fragment having VH and CH1 domains; (iv) the Fd' fragment having VH and CH1 domains and one or more cysteine residues at the C-terminus of the CH1 domain; (v) the Fv fragment having the VL and VH domains of a single arm of an antibody; (vi) the dAb fragment (Ward et al., Nature 341, 544-546 (1989)) which consists of a VH domain; (vii) isolated CDR regions; (viii) F(ab')2 fragments, a bivalent fragment including two Fab' fragments linked by a disulphide bridge at the hinge region; (ix) single chain antibody molecules (e.g., single chain Fv; scFv) (Bird et al., Science 242:423-426 (1988); and Huston et al., PNAS (USA) 85:5879-5883 (1988)); (x) "diabodies" with two antigen binding sites, comprising a heavy chain variable domain (VH) connected to a light chain variable domain (VL) in the same polypeptide chain (see, e.g., EP 404,097; WO 93/11161; and Hollinger et al., Proc. Natl. Acad. Sci. USA, 90:6444-6448 (1993)); (xi) "linear antibodies" comprising a pair of tandem Fd segments (VH-CH1-VH-CH1) which, together with complementary light chain polypeptides, form a pair of antigen binding regions (Zapata et al. Protein Eng. 8(10):1057-1062 (1995); and U.S. Pat. No. 5,641,870).

"Single chain variable fragment", "single-chain antibody variable fragments" or "scFv" antibodies as used herein refer to forms of antibodies comprising the variable regions of only the heavy (V_{H}) and light (V_{L}) chains, connected by a linker peptide. The scFvs are capable of being expressed as a single chain polypeptide. The scFvs retain the specificity of the intact antibody from which it is derived. The light and heavy chains may be in any order, for example, V_{H}-linker-V_{L} or V_{L}-linker-V_{H}, so long as the specificity of the scFv to the target antigen is retained.

"Complementarity determining region" (CDR) as used herein refers to the amino acid sequences within the variable regions of antibodies which regions confer specificity and binding affinity. In general, there are three CDRs in each of the light chain variable regions (LCDR1, LCDR2 and LCDR3) and three CDRs in each of the heavy chain variable regions (HCD1, HCDr2 and HCDR3). The boundaries of the CDRs may be determined using methods well known in the art including the "Kabat" numbering scheme and/or "Chothia" number scheme (Kabat et al. Sequences of Proteins of Immunological Interest, 5th Ed. Public Health Services, National Institutes of Health, Bethesda, MD; Al-Lazikani et al., (1997) JMB 273,927-948).

As used herein, the term "specific binding" means the contact between an antibody and an antigen with a binding affinity of at least 10⁻⁶ M. In certain aspects, antibodies bind with affinities of at least about 10⁻⁷M, and preferably 10⁻⁸M, 10⁻⁹M, 10⁻¹⁰M, 10⁻¹¹M, or 10⁻¹²M.

"Binds the same epitope as" means the ability of an antibody, scFv, antigen specific domain of a CAR or other binding agent to bind to a target and having the same epitope as the exemplified antibody, scFv, antigen specific domain of a CAR or other binding agent. As an example, the epitopes of the exemplified antibody, scFv, or other binding agent and other antibodies can be determined using standard epitope mapping techniques. Epitope mapping techniques, well known in the art include Epitope Mapping Protocols in Methods in Molecular Biology, Vol. 66 (Glenn E.Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871 ; Geysen et al, (1984) Proc. Natl. Acad. Sci. USA 8:3998-4002; Geysen et al, (1985) Proc. Natl. Acad. Sci. USA 82:78-182; Geysen et al, (1986) Mol. lmmunol. 23: 709-715. Similarly, conformational epitopes are readily identified by determining spatial conformation of amino acids such as by, e.g., hydrogen/deuterium exchange, x-ray crystallography and two-dimensional nuclear magnetic resonance. See, e.g., Epitope Mapping Protocols, supra. Antigenic regions of proteins can also be identified using standard antigenicity and hydropathy plots, such as those calculated using, e.g., the Omiga version 1.0 software program available from the Oxford Molecular Group. This computer program employs the Hopp/Woods method, Hopp et al, (1981) Proc. Natl. Acad. Sci USA 78:3824-3828; for determining antigenicity profiles, and the Kyte-Doolittle technique, Kyte et al, (1982) J.Mol. Bioi. 157: 1 05-132; for hydropathy plots (FROM MORPHOSYS CD19 patent WO 2013/024097). To determine if selected monoclonal antibodies against a target (e.g., CD19) bind to unique epitopes, each antibody can be biotinylated using commercially available reagents (Pierce, Rockford, Ill.). Competition studies using unlabeled monoclonal antibodies and biotinylated monoclonal antibodies can be performed using CD19-extracellualr domain coated-ELISA plates as described above. Biotinylated mAb binding can be detected with a strep-avidin-alkaline phosphatase probe.

The epitope recognized by a CAR can be also determined from the epitope recognized by the scFv comprising the CAR. For example, since the antigen specific domain of the CAR CD8SP-MPL-161-(vL-vH)-Myc-BBz-T2A-PAC (SEQ ID NO: 1658 and SEQ ID NO: 3403) targeting MPL is comprised of scFv MPL-161-(vL-vH) (SEQ ID NO: 730 and SEQ ID NO: 2500), it is expected that the CAR would target the same epitope as the scFv and the parental antibody from which the scFv is derived. The epitopes recognized by several scFv and/or their parental antibodies used in the construction of the CARs and backbones of this invention are known in the art. Alternatively, the epitope targeted by a CAR (including the CARs that are present as parat of backbones) can be determined by generating a series of mutants of its target antigen and testing the ability of the mutants to bind to the CAR-expressing cells. As an example, the epitope recognized by the CAR CD8SP-MPL-161-(vL-vH)-Myc-BBz-T2A-PAC (SEQ ID NO: 1658 and SEQ ID NO: 3403) targeting MPL can be determined by generating a panel of mutants of the MPL-ECD-GGSG-Nluc-AcV5 fusion construct (SEQ ID NO: 925 and SEQ ID NO: 2670). The mutant constructs would be transfected into a suitable cell line (e.g., 293FT cells) and the supernatant containing the fusion protein collected and assayed for NLuc activity to assure that the different mutant MPL-ECD-GGSG-Nluc-AcV5 fusion proteins are being secreted in the supernatant. Subsequently, the fusion proteins would be tested for their ability to bind to cells (e.g., Jurkat cells or T cells) expressing the CD8SP-MPL-161-(vL-vH)-Myc-BBz-T2A-PAC (SEQ ID NO: 1658 and SEQ ID NO: 3403) CAR construct. The mutant that fails to bind to the CAR-expressing cells is a candidate for containing the epitope targeted by the MPL-specific CAR. An alternate approach to determine the epitope recognized by a particular CAR could include a functional competitive assay with different test antibodies. For example, T cells expressing the CD8SP-MPL-161-(vL-vH)-Myc-BBz-T2A-PAC (SEQ ID NO: 1658 and SEQ ID NO: 3403) CAR would be co-cultured with a cell line expressing MPL (e.g., HEL cells) in the absence and presence of increasing concentrations of different test MPL antibodies. In case the epitope recognized by a test MPL antibody overlaps with the epitope recognized by the CD8SP-MPL-161-(vL-vH)-Myc-BBz-T2A-PAC (SEQ ID NO: 1658 and SEQ ID NO: 3403) CAR, then the test antibody would be expected to block target-cell killing and cytokine production induced by T cells expressing the CD8SP-MPL-161-(vL-vH)-Myc-BBz-T2A-PAC (SEQ ID NO: 1658 and SEQ ID NO: 3403) CAR in a dose-dependent manner. A nonspecific antibody of the same isotype as the test antibody would be included as a control and would be expected to have no effect on the target-cell killing and cytokine production induced by T cells expressing the CAR. Similarly, a specific CAR can be expressed in Jurkat-NFAT-EGFP cells and the ability of a test antibody to block EGFP induction by the CAR-expressing Jurkat-NFAT-GFP cells upon coculture with a target cell line can be used to determine whether the epitope recognized by the test antibody overlaps with the epitope recognized by the said CAR.

"Therapeutic agents" as used herein refers to agents that are used to, for example, treat, inhibit, prevent, mitigate the effects of, reduce the severity of, reduce the likelihood of developing, slow the progression of and/or cure, a disease. Diseases targeted by the therapeutic agents include but are not limited to infectious diseases, carcinomas, sarcomas, lymphomas, leukemia, germ cell tumors, blastomas, antigens expressed on various immune cells, and antigens expressed on cells associated with various hematologic diseases, and/or inflammatory diseases.

"Cancer" and "cancerous" refer to or describe the physiological condition in mammals that is typically characterized by unregulated cell growth. The term "cancer" is meant to include all types of cancerous growths or oncogenic processes, metastatic tissues or malignantly transformed cells, tissues, or organs, irrespective of histopathologic type or stage of invasiveness. Examples of solid tumors include malignancies, e.g., sarcomas, adenocarcinomas, and carcinomas, of the various organ systems, such as those affecting liver, lung, breast, lymphoid, gastrointestinal (e.g., colon), genitourinary tract (e.g., renal, urothelial cells), prostate and pharynx. Adenocarcinomas include malignancies such as most colon cancers, rectal cancer, renal-cell carcinoma, liver cancer, non-small cell carcinoma of the lung, cancer of the small intestine and cancer of the esophagus. In one embodiment, the cancer is a melanoma, e.g., an advanced stage melanoma. Metastatic lesions of the aforementioned cancers can also be treated or prevented using the methods and compositions of the invention. Examples of other cancers that can be treated include bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin Disease, non-Hodgkin lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, and combinations of said cancers. Treatment of metastatic cancers, e.g., metastatic cancers that express PD-L1 (Iwai et al. (2005) Int. Immunol. 17:133-144) can be effected using the antibody molecules described herein.

"Polynucleotide" as used herein includes but is not limited to DNA, RNA, cDNA (complementary DNA), mRNA (messenger RNA), rRNA (ribosomal RNA), shRNA (small hairpin RNA), snRNA (small nuclear RNA), snoRNA (short nucleolar RNA), miRNA (microRNA), genomic DNA, synthetic DNA, synthetic RNA, and/or tRNA.

It is to be inferred without explicit recitation and unless otherwise intended, that when the present disclosure relates to a polypeptide, protein, polynucleotide, antibody or fragment thereof, an equivalent or a biologically equivalent of such is intended within the scope of this disclosure. As used herein, the term "biological equivalent thereof" is intended to be synonymous with "equivalent thereof" when referring to a reference protein, antibody or fragment thereof, polypeptide or nucleic acid, intends those having minimal homology while still maintaining desired structure or functionality. Unless specifically recited herein, it is contemplated that any of the above also includes equivalents thereof. For example, an equivalent intends at least about 70% homology or identity, or at least 80% homology or identity and alternatively, or at least about 85%, or alternatively at least about 90%, or alternatively at least about 95%, or alternatively at least 98% percent homology or identity and exhibits substantially equivalent biological activity to the reference protein, polypeptide, antibody or fragment thereof or nucleic acid. Alternatively, when referring to polynucleotides, an equivalent thereof is a polynucleotide that hybridizes under stringent conditions to the reference polynucleotide or its complement. Alternatively, when referring to polypeptides or proteins, an equivalent thereof is a expressed polypeptide or protein from a polynucleotide that hybridizes under stringent conditions to the polynucleotide or its complement that encodes the reference polypeptide or protein.

A polynucleotide or polynucleotide region (or a polypeptide or polypeptide region) having a certain percentage (for example, 80%, 85%, 90%, or 95%) of "sequence identity" to another sequence means that, when aligned, that percentage of bases (or amino acids) are the same in comparing the two sequences. The alignment and the percent homology or sequence identity can be determined using software programs known in the art, for example those described in Current Protocols in Molecular Biology (Ausubel et al., eds. 1987) Supplement 30, section 7.7.18, Table 7.7.1. Preferably, default parameters are used for alignment. A preferred alignment program is BLAST, using default parameters. In particular, preferred programs are BLASTN and BLASTP, using the following default parameters: Genetic code=standard; filter=none; strand=both; cutoff=60; expect=10; Matrix=BLOSUM62; Descriptions=50 sequences; sort by=HIGH SCORE; Databases=non-redundant, GenBank+EMBL+DDBJ+PDB+GenBank CDS translations+SwissProtein+SPupdate+PIR. Details of these programs can be found at the following Internet address: ncbi.nlm.nih.gov/cgi-bin/BLAST.

The term "isolated" as used herein refers to molecules or biologicals or cellular materials being substantially free from other materials. In one aspect, the term "isolated" refers to nucleic acid, such as DNA or RNA, or protein or polypeptide (e.g., an antibody or derivative thereof), or cell or cellular organelle, or tissue or organ, separated from other DNAs or RNAs, or proteins or polypeptides, or cells or cellular organelles, or tissues or organs, respectively, that are present in the natural source. The term "isolated" also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides. The term "isolated" is also used herein to refer to cells or tissues that are isolated from other cells or tissues and is meant to encompass both, cultured and engineered cells or tissues.

"Naked DNA" as used herein refers to DNA encoding a CAR cloned in a suitable expression vector in proper orientation for expression. Viral vectors which may be used include but are not limited SIN lentiviral vectors, retroviral vectors, foamy virus vectors, adeno-associated virus (AAV) vectors, hybrid vectors and/or plasmid transposons (for example sleeping beauty transposon system) or integrase based vector systems. Other vectors that may be used in connection with alternate embodiments of the invention will be apparent to those of skill in the art.

"Target cell" as used herein refers to cells which are involved in a disease and can be targeted by the genetically modified cells of the invention (including but not limited to genetically modified T-cells, NK cells, hematopoietic stem cells, pluripotent stem cells, and embryonic stem cells). Other target cells will be apparent to those of skill in the art and may be used in connection with alternate embodiments of the invention.

The terms "T-cell" and "T-lymphocyte" are interchangeable and used synonymously herein. Examples include but are not limited to naive T cells, central memory T cells, effector memory T cells or combinations thereof.

"Vector", "cloning vector" and "expression vector" as used herein refer to the vehicle by which a polynucleotide sequence (e.g. a foreign gene) can be introduced into a host cell, so as to transform the host and promote expression (e.g. transcription and translation) of the introduced sequence. Vectors include plasmids, phages, viruses, etc.

The term "functional" when used in conjunction with "derivative" or "variant" or "fragment" refers to a polypeptide which possess a biological activity that is substantially similar to a biological activity of the entity or molecule of which it is a derivative or variant or fragment thereof.

As used herein, the term "administering," refers to the placement an agent as disclosed herein into a subject by a method or route which results in at least partial localization of the agents at a desired site.

"Beneficial results" may include, but are in no way limited to, lessening or alleviating the severity of the disease condition, preventing the disease condition from worsening, curing the disease condition, preventing the disease condition from developing, lowering the chances of a patient developing the disease condition and prolonging a patient's life or life expectancy. As non-limiting examples, "beneficial results" or "desired results" may be alleviation of one or more symptom(s), diminishment of extent of the deficit, stabilized (i.e., not worsening) state of cancer progression, delay or slowing of metastasis or invasiveness, and amelioration or palliation of symptoms associated with the cancer.

As used herein, the terms "treat," "treatment," "treating," or "amelioration" refer to therapeutic treatments, wherein the object is to reverse, alleviate, ameliorate, inhibit, slow down or stop the progression or severity of a condition associated with, a disease or disorder. The term "treating" includes reducing or alleviating at least one adverse effect or symptom of a condition, disease or disorder, such as cancer. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation of at least slowing of progress or worsening of symptoms that would be expected in absence of treatment. Beneficial or desired clinical results include, but are not limited to, alleviation of one or more symptom(s), diminishment of extent of disease, stabilized (i.e., not worsening) state of disease, delay or slowing of disease progression, amelioration or palliation of the disease state, and remission (whether partial or total), whether detectable or undetectable. The term "treatment" of a disease also includes providing relief from the symptoms or side-effects of the disease (including palliative treatment). In some embodiments, treatment of cancer includes decreasing tumor volume, decreasing the number of cancer cells, inhibiting cancer metastases, increasing life expectancy, decreasing cancer cell proliferation, decreasing cancer cell survival, or amelioration of various physiological symptoms associated with the cancerous condition.

"Conditions" and "disease conditions," as used herein may include, cancers, tumors or infectious diseases. In exemplary embodiments, the conditions include but are in no way limited to any form of malignant neoplastic cell proliferative disorders or diseases. In exemplary embodiments, conditions include any one or more of kidney cancer, melanoma, prostate cancer, breast cancer, glioblastoma, lung cancer, colon cancer, or bladder cancer.

The term "effective amount" or "therapeutically effective amount" as used herein refers to the amount of a pharmaceutical composition comprising one or more peptides as disclosed herein or a mutant, variant, analog or derivative thereof, to decrease at least one or more symptom of the disease or disorder, and relates to a sufficient amount of pharmacological composition to provide the desired effect. The phrase "therapeutically effective amount" as used herein means a sufficient amount of the composition to treat a disorder, at a reasonable benefit/risk ratio applicable to any medical treatment.

A therapeutically or prophylactically significant reduction in a symptom is, e.g. at least about 10%, at least about 20%, at least about 30%, at least about 40%, at least about 50%, at least about 60%, at least about 70%, at least about 80%, at least about 90%, at least about 100%, at least about 125%, at least about 150% or more in a measured parameter as compared to a control or non-treated subject or the state of the subject prior to administering the oligopeptides described herein. Measured or measurable parameters include clinically detectable markers of disease, for example, elevated or depressed levels of a biological marker, as well as parameters related to a clinically accepted scale of symptoms or markers for diabetes. It will be understood, however, that the total daily usage of the compositions and formulations as disclosed herein will be decided by the attending physician within the scope of sound medical judgment. The exact amount required will vary depending on factors such as the type of disease being treated, gender, age, and weight of the subject.

The phrase "first line" or "second line" or "third line" refers to the order of treatment received by a patient. First line therapy regimens are treatments given first, whereas second or third line therapy are given after the first line therapy or after the second line therapy, respectively. The National Cancer Institute defines first line therapy as "the first treatment for a disease or condition. In patients with cancer, primary treatment can be surgery, chemotherapy, radiation therapy, or a combination of these therapies. First line therapy is also referred to those skilled in the art as "primary therapy and primary treatment." See National Cancer Institute website at www.cancer.gov, last visited on May 1, 2008. Typically, a patient is given a subsequent chemotherapy regimen because the patient did not show a positive clinical or sub-clinical response to the first line therapy or the first line therapy has stopped.

"Mammal" as used herein refers to any member of the class *Mammalia,* including, without limitation, humans and nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be included within the scope of this term.

### Chimeric Antigen Receptors

Provided herein are compositions comprising a CAR and an accessory module and method of using same to treat diseases, including cancer. As described herein, specific combinations of conventional CARs **(Table 1)** and accessory modules as described in **Table 2** define a 'backbone' **(Table 2).**

**Table 1:** Conventional CAR architectures. First generation conventional CARs (Conventional CAR I) have an intracellular signaling (ISD) domain (e.g. CD3z) and no costimulatory domain. Second generation conventional CARs (Conventional CAR 2 or CAR II) have one costimulatory domain (e.g. 41BB or CD28) and an intracellular signaling (ISD) domain (e.g. CD3z). Third generation conventional CARs (Conventional CAR 3 or CAR III) have two costimulatory domains (e.g. 41BB and CD28) and an intracellular signaling (ISD) domain (e.g. CD3z).

| **Table 1 Conventional CAR Architectures** | | | | | | | |
|---|---|---|---|---|---|---|---|
| 1 | Conventional CAR 1 (CAR I) | ASD | HR | TMD | ISD | | |
| 2 | Conventional CAR 2 (CAR II) | ASD | HR | TMD | CSD | ISD | |
| 3 | Conventional CAR 3 (CAR III) | ASD | HR | TMD | CSD-I | CSD-II | ISD |

**Table 2: Exemplary Backbones**

| **Backbone No.** | **CAR Component** | **Accessory Module** | | |
|---|---|---|---|---|
| | | **NAME** | **SEQ ID (DNA)** | **SEQ ID (PRT)** |
| Backbone 1 | Conventional CAR I | K13-vFLIP | 866 | 2629 |
| Backbone 2 | Conventional CAR I | MC159-vFLIP | 867 | 2630 |
| Backbone 3 | Conventional CAR I | MC160-vFLIP | 868 | 2631 |
| Backbone 4 | Conventional CAR I | E8-vFLIP | 869 | 2632 |
| Backbone 5 | Conventional CAR I | Herpesvirus-saimiri-vFLIP | 870 | 2633 |
| Backbone 6 | Conventional CAR I | Mecaca-vFLIP | 871 | 2634 |
| Backbone 7 | Conventional CAR I | BHV-vFLIP | 872 | 2635 |
| Backbone 8 | Conventional CAR I | cFLIP-L/MRIT-alpha | 873 | 2636 |
| Backbone 9 | Conventional CAR I | cFLIP-p22 | 874 | 2637 |
| Backbone 10 | Conventional CAR I | HTLV1-TAX | 875 | 2638 |
| Backbone 11 | Conventional CAR I | HTLV2-TAX | 876 | 2639 |
| Backbone 12 | Conventional CAR I | HTLV2-TAX-RS | 877 | 2640 |
| Backbone 13 | Conventional CAR I | FKBP-K13 | 878 | 2641 |
| Backbone 14 | Conventional CAR I | FKBPX2-K13 | 879 | 2642 |
| Backbone 15 | Conventional CAR I | Myr-FKBPx2-K13 | 880 | 2643 |
| Backbone 16 | Conventional CAR I | FKBPx2-HTLV2-Tax-RS | 881 | 2644 |
| Backbone 17 | Conventional CAR I | FKBPx2-Flag-HTLV2-Tax-RS | 882 | 2645 |
| Backbone 18 | Conventional CAR I | IL6R-304-vHH | 884 | 2647 |
| Backbone 19 | Conventional CAR I | IGHSP2-IL6R-304-VHH-ALB8-VHH | 886 | 2649 |
| Backbone 20 | Conventional CAR I | IgSP-IL6-19A-scFV | 899 | 2662 |
| Backbone 21 | Conventional CAR I | IgSP-Fx06 | 900 | 2663 |
| Backbone 22 | Conventional CAR I | CD8SP2-PD1-4H1-scFv | 889 | 2652 |
| Backbone 23 | Conventional CAR I | CD8SP2-PD1-5C4-scFv | 890 | 2653 |
| Backbone 24 | Conventional CAR I | CD8SP2-CTLA4-Ipilimumab-scFv | 891 | 2654 |
| Backbone 25 | Conventional CAR I | CD8SP2-PD1-4H1-Alb8-vHH | 892 | 2655 |
| Backbone 26 | Conventional CAR I | CD8SP2-PD1-5C4-Alb8-vHH | 893 | 2656 |
| Backbone 27 | Conventional CAR I | CD8SP2-CTLA4-Ipilimumab-Alb8-vHH | 894 | 2657 |
| Backbone 28 | Conventional CAR I | CD8SP2-sHVEM-Alb8-vHH | 902 | 2665 |
| Backbone 29 | Conventional CAR I | tCD19 | 855 | 2618 |
| Backbone 30 | Conventional CAR I | tBCMA | 856 | 2619 |
| Backbone 31 | Conventional CAR I | CNB30 | 852 | 2615 |
| Backbone 32 | Conventional CAR II | K13-vFLIP | 866 | 2629 |
| Backbone 33 | Conventional CAR II | MC159-vFLIP | 867 | 2630 |
| Backbone 34 | Conventional CAR II | MC160-vFLIP | 868 | 2631 |
| Backbone 35 | Conventional CAR II | E8-vFLIP | 869 | 2632 |
| Backbone 36 | Conventional CAR II | Herpesvirus-saimiri-vFLIP | 870 | 2633 |
| Backbone 37 | Conventional CAR II | Mecaca-vFLIP | 871 | 2634 |
| Backbone 38 | Conventional CAR II | BHV-vFLIP | 872 | 2635 |
| Backbone 39 | Conventional CAR II | cFLIP-L/MRIT-alpha | 873 | 2636 |
| Backbone 40 | Conventional CAR II | cFLIP-p22 | 874 | 2637 |
| Backbone 41 | Conventional CAR II | HTLV1-TAX | 875 | 2638 |
| Backbone 42 | Conventional CAR II | HTLV2-TAX | 876 | 2639 |
| Backbone 43 | Conventional CAR II | HTLV2-TAX-RS | 877 | 2640 |
| Backbone 44 | Conventional CAR II | FKBP-K13 | 878 | 2641 |
| Backbone 45 | Conventional CAR II | FKBPX2-K13 | 879 | 2642 |
| Backbone 46 | Conventional CAR II | Myr-FKBPx2-K13 | 880 | 2643 |
| Backbone 47 | Conventional CAR II | FKBPx2-HTLV2-Tax-RS | 881 | 2644 |
| Backbone 48 | Conventional CAR II | FKBPx2-Flag-HTLV2-Tax-RS | 882 | 2645 |
| Backbone 49 | Conventional CAR II | IL6R-304-vHH | 884 | 2647 |
| Backbone 50 | Conventional CAR II | IGHSP2-IL6R-304-VHH-ALB8-VHH | 886 | 2649 |
| Backbone 51 | Conventional CAR II | IgSP-IL6-19A-scFV | 899 | 2662 |
| Backbone 52 | Conventional CAR II | IgSP-Fx06 | 900 | 2663 |
| Backbone 53 | Conventional CAR II | CD8SP2-PD1-4H1-scFv | 889 | 2652 |
| Backbone 54 | Conventional CAR II | CD8SP2-PD1-5C4-scFv | 890 | 2653 |
| Backbone 55 | Conventional CAR II | CD8SP2-CTLA4-Ipilimumab-scFv | 891 | 2654 |
| Backbone 56 | Conventional CAR II | CD8SP2-PD1-4H1-Alb8-vHH | 892 | 2655 |
| Backbone 57 | Conventional CAR II | CD8SP2-PD1-5C4-Alb8-vHH | 893 | 2656 |
| Backbone 58 | Conventional CAR II | CD8SP2-CTLA4-Ipilimumab-Alb8-vHH | 894 | 2657 |
| Backbone 59 | Conventional CAR II | CD8SP2-sHVEM-Alb8-vHH | 902 | 2665 |
| Backbone 60 | Conventional CAR II | tCD19 | 855 | 2618 |
| Backbone 61 | Conventional CAR II | tBCMA | 856 | 2619 |
| Backbone 62 | Conventional CAR II | CNB30 | 852 | 2615 |

Exemplary embodiments of the CAR components are described in **Tables 1-6, 8, 11, and 15.**

As described herein, the various backbones comprise a CAR component and accessory modules. Exemplary embodiments of the accessory modules are described in **Table** 16.

In some embodiments, the compositions comprise nucleic acids encoding conventional CARs I-III (Table 1), wherein the antigen specific domain of the CAR targets one or more specific antigens as described in Tables 21 and 22. In some embodiments, the compositions comprise nucleic acids encoding any one or more of backbones 1-62 **(Table** 2) where the antigen specific domain of the encoded CAR targets one or more specific antigens as described herein and in Tables 19, 20 and 22. In some embodiments, the compositions comprise nucleic acids encoding backbone-1, wherein the antigen specific domain of the CAR in backbone-1 targets one or more cancer specific antigens as described herein and in Table 19 and 22. In some embodiments, the compositions comprise nucleic acids encoding backbone-2, wherein the antigen specific domain of the CAR in backbone-2 targets one or more cancer specific antigens as described herein. In some embodiments, the compositions comprise nucleic acids encoding backbone-32, wherein the antigen specific domain of the CAR in backbone-32 targets one or more cancer specific antigens as described herein and in Table 20 and 22. In some embodiments, the compositions comprise nucleic acids encoding backbone-33, wherein the antigen specific domain of the CAR in backbone-33 targets one or more cancer specific antigens as described herein. In various embodiments, the nucleic acids are used in the treatment of cancer. In some embodiments, the nucleic acids encoding the CAR component of the backbones described herein comprise more than one antigen specific domain wherein each of the antigen specific domains are contiguous and in the same reading frame as the other antigen specific domains in the same CAR.

In some embodiments, the compositions comprise polypeptides encoded by nucleic acids encoding any conventional CARs 1-III, wherein the antigen specific domain of the CAR targets one or more specific antigens as described herein and in Tables 21 and 22. In some embodiments, the compositions comprise polypeptides encoded by nucleic acids encoding any one or more of backbones 1-62 **(Table 2)** where the antigen specific domain of the encoded CAR targets one or more specific antigens as described herein and in Tables 20 to 22. In some embodiments, the compositions comprise polypeptides encoded by nucleic acids encoding backbone-1, wherein the antigen specific domain of the CAR in backbone-1 targets one or more cancer specific antigens as described herein and in Table 19 and 22. In some embodiments, the compositions comprise polypeptides encoded by nucleic acids encoding backbone-2, wherein the antigen specific domain of the CAR in backbone-2 targets one or more cancer specific antigens as described herein. In some embodiments, the compositions comprise nucleic acids encoding backbone-32, wherein the antigen specific domain of the CAR in backbone-32 targets one or more cancer specific antigens as described herein and in Table 20 and 22. In some embodiments, the compositions comprise nucleic acids encoding backbone-33, wherein the antigen specific domain of the CAR in backbone-33 targets one or more cancer specific antigens as described herein. In various embodiments, the polypeptides are used in the treatment of cancer. In some embodiments, polypeptides encoded by the nucleic acids encoding the CAR component of the backbones described herein comprise more than one antigen specific domain wherein each of the antigen specific domains are contiguous and in the same reading frame as the other antigen specific domains in the same CAR.

In some embodiments, the compositions comprise vectors comprising nucleic acids encoding any of conventional CARs I-III, wherein the antigen specific domain of the CAR targets one or more cancer specific antigens as described herein and in Tables 21 and 22. In some embodiments, the compositions comprise vectors comprising nucleic acids encoding any one or more of backbones 1-62 **(Table 2)** where the antigen specific domain of the encoded CAR targets one or more specific antigens as described herein and in **Tables 20 to 22.** In some embodiments, the compositions comprise vectors encoding nucleic acids encoding backbone-1, wherein the antigen specific domain of the CAR in backbone-1 targets one or more cancer specific antigens as described herein and in Tables 19 and 22. In some embodiments, the compositions comprise vectors encoding nucleic acids encoding backbone-2, wherein the antigen specific domain of the CAR in backbone-2 targets one or more cancer specific antigens as described herein. In some embodiments, the compositions comprise vectors encoding nucleic acids encoding backbone-32, wherein the antigen specific domain of the CAR in backbone-32 targets one or more cancer specific antigens as described herein. In some embodiments, the compositions comprise vectors encoding nucleic acids encoding backbone-33, wherein the antigen specific domain of the CAR in backbone-33 targets one or more cancer specific antigens as described herein. In various embodiments, the vectors are used in the treatment of cancer. In some embodiments, vectors comprising nucleic acids encoding the conventional CARs I to III or CAR component of the backbones described herein comprise more than one antigen specific domain wherein each of the antigen specific domains are contiguous and in the same reading frame as the other antigen specific domains in the same CAR.

In some embodiments, the compositions comprise genetically modified cells which include vectors comprising nucleic acids encoding any one or more of conventional CARs I to III or backbones 1-62 **(Table 2)** where the antigen specific domain of the encoded CAR targets one or more specific antigens as described herein and in **Tables 19 to 22.** In some embodiments, the compositions comprise genetically modified cells which include vectors encoding nucleic acids encoding conventional CARs I to III, wherein the antigen specific domain of the CAR targets one or more cancer specific antigens as described herein and in Tables 19 and 22. In some embodiments, the compositions comprise genetically modified cells which include vectors encoding nucleic acids encoding backbone-1, wherein the antigen specific domain of the CAR in backbone-1 targets one or more cancer specific antigens as described herein. In some embodiments, the compositions comprise genetically modified cells which include vectors encoding nucleic acids encoding backbone-2, wherein the antigen specific domain of the CAR in backbone-2 targets one or more cancer specific antigens as described herein. In some embodiments, the compositions comprise genetically modified cells which include vectors encoding nucleic acids encoding backbone-32, wherein the antigen specific domain of the CAR in backbone-32 targets one or more cancer specific antigens as described herein. In some embodiments, the compositions comprise genetically modified cells which include vectors encoding nucleic acids encoding backbone-33, wherein the antigen specific domain of the CAR in backbone-33 targets one or more cancer specific antigens as described herein. In various embodiments, the genetically modified cells are used in treatment of cancer. In some embodiments, the genetically modified cells targets more than one antigen on a cancer cell.

In various embodiments, the isolated nucleic acid molecules encoding the CAR components of the backbones described herein, encode one, two, three or more antigen specific domains.

In various embodiments, the isolated nucleic acid molecules encoding the CAR components of the backbones described herein, encode one, two, three or more co-stimulatory domains.

In various embodiments, the isolated nucleic acid molecules encoding the CAR components of the backbones described herein, encode one, two, three or more intracellular signaling domain.

The nucleic acid sequences encoding for the desired components of the CARs and accessory modules described herein can be obtained using recombinant methods known in the art, such as, for example by screening libraries from cells expressing the nucleic acid molecule, by deriving the nucleic acid molecule from a vector known to include the same, or by isolating directly from cells and tissues containing the same, using standard techniques. Alternatively, the nucleic acid of interest can be produced synthetically, rather than cloned. In exemplary embodiments, the nucleic acid sequences encoding for the desired components of the CARs and accessory modules described herein are described in **Tables 3-22.**

In some embodiments, the genetically modified cells described herein that express the CARs and accessory components described herein also express agents that reduce toxicity of CARs. In exemplary embodiments, such agents are set forth in **Table 16.** In exemplary embodiments, such agents include but are not limited to any one, two, three or more of scFv against IL-6 (for example, SEQ ID NO: 899), a camelid vHH against IL-6 receptor alpha (for example, SEQ ID NO: 884), a bispecific camelid vHH targeting IL-6 receptor alpha and albumin (SEQ ID NO: 886), Fx06 peptide (SEQ ID NO: 900), or combinations thereof.

In some embodiments, the genetically modified cells described herein that express the CARs and accessory components described herein also express agents that enhance the activity of CARs. In exemplary embodiments, such agents are set forth in **Table 16.** In exemplary embodiments, such agents include but are not limited to any one, two, three or more of hTERT (SEQ ID NO: 903), heparinase (SEQ ID NO: 904), soluble HVEM (SEQ ID NO: 901), a fusion protein containing soluble HVEM and a camelid vHH targeting albumin (SEQ ID NO: 902), scFvs targeting PD1 (SEQ ID NOs: 889 and 890), scFv targeting CTLA4 (SEQ ID NO: 894), bispecific proteins targeting PD1 and albumin (SEQ ID NO: 892 and 893), bispecific proteins targeting CTLA4 and albumin (SEQ ID NO: 894) or combinations thereof. The rationale for targeting albumin in the bispecific proteins is to extend the half-lives of the scFvs and vHH fragments which are otherwise rapidly cleared by the kidneys due to their relatively small size.

In specific embodiments, exemplary constructs containing backbone-1, which comprises a conventional CAR I containing a CD3z activation domain and coexpresses a K13-vFLIP accessory module, are set forth in **Table 19.**

In specific embodiments, exemplary constructs containing backbone-32, which comprises a conventional CAR II containing a 41BB costimulatory domain, a CD3z activation domain and coexpresses a K 13-vFLIP accessory module, are set forth in **Table 20.**

In specific embodiments, exemplary conventional CAR II constructs containing a 41BB costimulatory domain and a CD3z activation domain are described in **Table 21.**

In specific embodiments, exemplary CAR constructs in various embodiments of the inventions are described in **Table 22.**

### Antigen specific domain

The compositions comprising various backbones as described herein comprise CARs which comprise one or more ASD that binds specifically to a cancer associated antigen as described herein. The sequences of the ASD are contiguous with and in the same reading frame as a nucleic acid sequence encoding the remainder of the CAR.

In one embodiment, each antigen specific region comprises the full-length IgG heavy chain (specific for the target antigen) having the V_{H}, CH1, hinge, and the CH2 and CH3 (Fc) Ig domains, if the V_{H} domain alone is sufficient to confer antigen-specificity ("single-domain antibodies"). The full length IgG heavy chain may be linked to the co-stimulatory domain and the intracellular signaling domain via the appropriate transmembrane domain. If both, the V_{H} and the V_{L} domains, are necessary to generate a fully active antigen-specific targeting region, the V_{H}-containing CAR and the full-length lambda light chain (IgL) are both introduced into the cells to generate an active antigen-specific targeting region.

In some embodiments, the antigen specific domain of the encoded CAR molecule comprises an antibody, an antibody fragment, an scFv, a Fv, a Fab, a (Fab')₂, a single domain antibody (SDAB), a VH or VL domain, or a camelid VHH domain. In some embodiments, the antigen binding domain of the CAR is a scFv antibody fragment that is humanized compared to the murine sequence of the scFv from which it is derived.

In some instances, scFvs can be prepared according to methods known in the art (for example, Bird et al., (1988) Science 242:423-426 and Huston et al., (1988) Proc.Natl. Acad. Sci. USA 85:5879-5883). ScFv molecules can be produced by linking V_{H} and V_{L} regions together using flexible polypeptide linkers. The scFv molecules comprise a linker (e.g., a Ser-Gly linker) with an optimized length and/or amino acid composition. The linker length can greatly affect how the variable regions of a scFv fold and interact. For example, if a short polypeptide linker is employed (for example, between 5-10 amino acids) intrachain folding is prevented. Interchain folding is also required to bring the two variable regions together to form a functional epitope binding site. For examples of linker orientation and size see, e.g., Hollinger et al. 1993 Proc Natl Acad. Sci. U.S.A. 90:6444-6448, U.S. Patent Application Publication Nos. 2005/0100543, 2005/0175606, 2007/0014794, and PCT publication Nos. WO2006/020258 and WO2007/024715, is incorporated herein by reference.

An scFv can comprise a linker of at least 1, 2, 3, 4, 5, 6, 7, 8, 9, 10, 11, 12, 13, 14, 15, 16, 17, 18, 19, 20, 25, 30, 35, 40, 45, 50, or more amino acid residues between its V_{L} and V_{H} regions. The linker sequence may comprise any naturally occurring amino acid. In some embodiments, the linker sequence comprises amino acids glycine and serine. In another embodiment, the linker sequence comprises sets of glycine and serine repeats such as (Gly4Ser)n, where n is a positive integer equal to or greater than 1 (SEQ ID NO:252, SEQ ID NO:253). In one embodiment, the linker can be (Gly4Ser)3 (SEQ ID NO:254, SEQ ID NO:254) or (Gly4Ser)3 (SEQ ID NO:250 - SEQ ID NO:255). Variation in the linker length may retain or enhance activity, giving rise to superior efficacy in activity studies.

In another aspect, the antigen binding domain is a T cell receptor ("TCR"), or a fragment thereof, for example, a single chain TCR (scTCR). Methods to make such TCRs are known in the art. See, e.g., Willemsen RA et al, Gene Therapy 7: 1369-1377 (2000); Zhang T et al, Cancer Gene Ther 11: 487-496 (2004); Aggen et al, Gene Ther. 19(4):365- 74 (2012) (references are incorporated herein by its entirety). For example, scTCR can be engineered that contains the Vα and vβ genes from a T cell clone linked by a linker (e.g., a flexible peptide). This approach is very useful to cancer associated target that itself is intracellular, however, a fragment of such antigen (peptide) is presented on the surface of the cancer cells by MHC

In one embodiment, the antigen specific domain comprises one, two or all three heavy chain (hc) CDRs, hcCDRl, hcCDR2 and hcCDR3 of an antibody listed herein, and/or one, two or all three light chain (lc) CDRs, lcCDRl, lcCDR2 and lcCDR3 of an antibody listed herein.

In another embodiment, the antigen specific domain comprises a humanized antibody or an antibody fragment. In some aspects, a non-human antibody is humanized, where specific sequences or regions of the antibody are modified to increase similarity to an antibody naturally produced in a human or fragment thereof. In one aspect, the antigen binding domain is humanized. A humanized antibody can be produced using a variety of techniques known in the art, including but not limited to, CDR-grafting (see, e.g., European Patent No. EP 239,400; International Publication No. WO 91/09967; and U.S. Pat. Nos. 5,225,539, 5,530,101, and 5,585,089, each of which is incorporated herein in its entirety by reference), veneering or resurfacing (see, e.g., European Patent Nos. EP 592,106 and EP 519,596; Padlan, 1991, Molecular Immunology, 28(4/5):489-498; Studnicka et al., 1994, Protein Engineering, 7(6):805-814; and Roguska et al., 1994, PNAS, 91:969-973, each of which is incorporated herein by its entirety by reference), chain shuffling (see, e.g., U.S. Pat. No. 5,565,332, which is incorporated herein in its entirety by reference), and techniques disclosed in, e.g., U.S. Patent Application Publication No. US2005/0042664, U.S. Patent Application Publication No. US2005/0048617, U.S. Pat. No. 6,407,213, U.S. Pat. No. 5,766,886, International Publication No. WO 9317105, Tan et al., J. Immunol., 169:1119-25 (2002), Caldas et al., Protein Eng., 13(5):353-60 (2000), Morea et al., Methods, 20(3):267- 79 (2000), Baca et al., J. Biol. Chem., 272(16): 10678-84 (1997), Roguska et al., Protein Eng., 9(10):895-904 (1996), Couto et al., Cancer Res., 55 (23 Supp):5973s-5977s (1995), Couto et al., Cancer Res., 55(8): 1717-22 (1995), Sandhu J S, Gene, 150(2):409-10 (1994), and Pedersen et al., J. Mol. Biol., 235(3):959-73 (1994), each of which is incorporated herein in its entirety by reference. In some embodiments, the portion of a CAR composition of the invention that comprises an antibody fragment is humanized with retention of high affinity for the target antigen and other favorable biological properties
In some embodiments, the antigen specific domain is a T cell receptor specific for the target antigen or a fragment of the T cell receptor, wherein the fragment retains the specificity for the target antigen.

In some embodiments, the antigen specific domain of a CAR described herein is a scFv antibody fragment. In some embodiments, the antigen specific scFv antibody fragments are functional in that they bind the same antigen with the same or comparable affinity as the IgG antibody from which it is derived. In other embodiments, the antibody fragment has a lower binding affinity to the antigen compared to the antibody from which it is derived but is functional in that it provides a biological response described herein. In one embodiment, the CAR molecule comprises an antibody fragment that has a binding affinity KD of 10⁻⁴ M to 10⁻⁸ M, 10⁻⁵ M to 10⁻⁷ M, 10⁻⁶ M or 10⁻⁸ M, for the target antigen.

In some embodiments, antigen specific domain of a CAR described herein binds to a MHC presented peptide. Normally, peptides derived from endogenous proteins fill the pockets of Major histocompatibility complex (MHC) class I molecules, and are recognized by T cell receptors (TCRs) on CD8+ T lymphocytes. The MHC class I complexes are constitutively expressed by all nucleated cells. In cancer, virus-specific and/or tumor-specific peptide/MHC complexes represent a unique class of cell surface targets for immunotherapy. TCR-like antibodies targeting peptides derived from viral or tumor antigens in the context of human leukocyte antigen (HLA)-A1 or HLA-A2 have been described (see, e.g., Sastry et al., J Viral. 2011 85(5):1935-1942; Sergeeva et al., Blood, 2011117(16):4262-4272; Verma et al., Jlmmunol2010 184(4):2156-2165; Willemsen et al., Gene Ther20018(21) :1601-1608; Dao et al., Sci Transl Med 2013 5(176) :176ra33; Tassev et al., Cancer Gene Ther 2012 19(2):84-100). For example, TCR-like antibody can be identified from screening a library, such as a human scFv phage displayed library. Exemplary CARs that are based on TCR-like antibodies targeting WT1 in association with HLA-A2 are represented by SEQ ID NO: 1176 to SEQ ID NO: 1179. In the instant invention, CARs were generated using antigen binding domain derived from TCR like antibodies against several HLA-A2 restricted intracellular peptides. The target protein antigens, the peptide fragment and the sequence of the peptide are shown in **Table 23.**

In some embodiments, when the CARs comprising functional fragments of antibodies (including scFv fragments) described herein bind the target antigen, a biological response is induced such as activation of an immune response, inhibition of signal-transduction originating from its target antigen, inhibition of kinase activity, and the like, as will be understood by a skilled artisan.

In some embodiments, the antigens specific for disease which may be targeted by the CARs when expressed alone or with the accessory modules as described herein include but are not limited to any one or more of CD19, CD22, CD23, MPL, CD123, CD32, CD138, CD200R, CD276, CD324, CD30, CD32, FcRH5, CD99, Tissue Factor, amyloid, Fc region of an immunoglobulin, CD171, CS-1, CLL-1 (CLECL1), CD33, EGFRvIII , GD2, GD3, BCMA, Tn Ag, PSMA, ROR1, FLT3, FAP, TAG72, CD38, CD44v6, CEA, EPCAM, B7H3, KIT, IL-13Ra2, IL11Ra, Mesothelin, PSCA, VEGFR2, Lewis Y, CD24, PDGFR-beta, PRSS21, SSEA-4, CD20, Folate receptor alpha, ERBB2 (Her2/neu), MUC1, EGFR, NCAM, Prostase, PAP, ELF2M, Ephrin B2, IGF-I receptor, CAIX, LMP2, gpl00, bcr-abl, tyrosinase, EphA2, Fucosyl GM1, sLea, GM3, TGS5, HMWMAA, o-acetyl-GD2, Folate receptor beta, TEM1/CD248, TEM7R, CLDN6, TSHR, TCR-beta1 constant chain, TCR beta2 constant chain, TCR gamma-delta, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WTl, NY-ESO-1, LAGE-la, legumain, HPV E6, E7, HTLV1-Tax, KSHV K8.1 protein, EBB gp350, HIV1-envelop glycoprotein gp120, MAGE-Al, MAGE Al, ETV6-AML, sperm protein 17, XAGE1, Tie 2, MAD-CT-1, MAD-CT-2, Fos-related antigen 1, p53, p53 mutant, prostein, survivin and telomerase, PCTA-l/Galectin 8, MelanA/MARTl, Ras mutant, hTERT, DLL3, TROP2, PTK7, GCC, AFP, sarcoma translocation breakpoints, ML-IAP, ERG (TMPRSS2 ETS fusion gene), NA17, PAX3, Androgen receptor, Cyclin Bl, MYCN, RhoC, TRP-2, CYP1B1, BORIS, SART3, PAX5, OY-TES1, LCK, AKAP-4, SSX2, RAGE-1, RUI, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, IGLL1, FITC, Leutenizing hormone receptor (LHR), Follicle stimulating hormone receptor (FSHR), Chorionic Gonadotropin Hormone receptor (CGHR), CCR4, GD3, SLAMF6, SLAMF4, FITC, Leutenizing hormone receptor (LHR), Follicle stimulating hormone receptor (FSHR), Chorionic Gonadotropin Hormone receptor (CGHR), CCR4, GD3, SLAMF6, SLAMF4, or combinations thereof.

In some embodiments, the antigens specific for a disease which may be targeted by the CARs when expressed alone or with the accessory modules as described herein include but are not limited to any one or more of 4-1BB, 5T4, adenocarcinoma antigen, alpha-fetoprotein, BAFF, B-lymphoma cell, C242 antigen, CA-125, carbonic anhydrase 9 (CA-IX), C-MET, CCR4, CD152, CD19, CD20, CD200, CD22, CD221, CD23 (IgE receptor), CD28, CD30 (TNFRSF8), CD33, CD4, CD40, CD44 v6, CD51, CD52, CD56, CD74, CD80, CD123, CEA, CNTO888, CTLA-4, DR5, EGFR, EpCAM, CD3, FAP, fibronectin extra domain-B, folate receptor 1, GD2, GD3 ganglioside, glycoprotein 75, GPNMB, HER2/neu, HGF, human scatter factor receptor kinase, IGF-1 receptor, IGF-I, IgG1, L1-CAM, IL-13, IL-6, insulin-like growth factor I receptor, integrin α5β1, integrin αvβ3, LAMP1, MORAb-009, MS4A1, MUC1, mucin CanAg, N-glycolylneuraminic acid, NPC-1C, PDGF-R α, PDL192, phosphatidylserine, prostatic carcinoma cells, RANKL, RON, ROR1, SCH 900105, SDC1, SLAMF7, TAG-72, tenascin C, TGF beta 2, TGF-β, TRAIL-R1, TRAIL-R2, tumor antigen CTAA16.88, VEGF-A, VEGFR-1, VEGFR2, vimentin or combinations thereof. Other antigens specific for cancer will be apparent to those of skill in the art and may be used in connection with alternate embodiments of the invention.

In some embodiments, the antigens specific for cancer which may be targeted by the CARs when expressed alone or with the accessory modules as described herein include but are not limited to any one or more of MPL,CD19, FLT3, GRP-78, CD79b, Ly-1, Lym2, CD23, CD179b, CDH1, CDH6, CDH19, CDH17, DLL3, PTK7, TROP2, TIM1, LAMP1 or combinations thereof. In some embodiments, the antigen specific domains of the CARs specific for MPL, CD19, FLT3, GRP-78, CD79b, Lym1, Lym2, CD23, CDH1, CDH6, CDH19, CDH17, DLL3, PTK7, TROP2, TIM1, LAMP1 comprise scFv sequences set forth in **Table 11.**

A CAR when used alone or with accessory modules as described herein can comprise an antigen binding domain (e.g., antibody or antibody fragment) that binds to a disease-supporting antigen (e.g., a disease-supporting antigen as described herein). In some embodiments, the disease-supporting antigen is an antigen present on cells that support the survival and proliferation of disease causing cells. In some embodiments, the disease-supporting antigen is an antigen present on a stromal cell or a myeloid-derived suppressor cell (MDSC). Stromal cells can secrete growth factors and cytokines to promote cell proliferation in the microenvironment. MDSC cells can block T cell proliferation and activation. Without wishing to be bound by theory, in some embodiments, the CAR-expressing cells destroy the disease-supporting cells, thereby indirectly blocking growth or survival of disease causing cells.

In embodiments, the stromal cell antigen is selected from one or more of: bone marrow stromal cell antigen 2 (BST2), fibroblast activation protein (FAP) and tenascin. In an embodiment, the FAP-specific antibody is, competes for binding with, or has the same CDRs as, sibrotuzumab. In embodiments, the MDSC antigen is selected from one or more of: CD33, CDllb, C14, CD15, and CD66b. Accordingly, in some embodiments, the disease supporting antigen is selected from one or more of: bone marrow stromal cell antigen 2 (BST2), fibroblast activation protein (FAP) or tenascin, CD33, CDllb, C14, CD15, and CD66b.

In a further embodiment, each antigen specific region of the CAR may comprise a divalent (or bivalent) single-chain variable fragment (di-scFvs, bi-scFvs). In CARs comprising di-scFVs, two scFvs specific for each antigen are linked together by producing a single peptide chain with two V_{H} and two V_{L} regions, yielding tandem scFvs. (Xiong, Cheng-Yi; Natarajan, A; Shi, XB; Denardo, GL; Denardo, SJ (2006). "Development of tumor targeting anti-MUC-1 multimer: effects of di-scFv unpaired cysteine location on PEGylation and tumor binding". Protein Engineering Design and Selection 19 (8): 359-367; Kufer, Peter; Lutterbüse, Ralf; Baeuerle, Patrick A. (2004). "A revival of bispecific antibodies". Trends in Biotechnology 22 (5): 238-244). CARs comprising at least two antigen-specific targeting regions would express two scFvs specific for each of the two antigens. The resulting ASD is joined to the co-stimulatory domain and the intracellular signaling domain via a hinge region and a transmembrane domain. Alternatively, CARs comprising two antigen specific targeting regions would express two vHH specific for each of the two antigens or two epitopes of the same antigen. Exemplary CARs targeting two antigens are represented by SEQ ID NOs: 1042, 1043, 1049, 1050 and 1087.

In an additional embodiment, each ASD of the CAR comprises a diabody. In a diabody, the scFvs are created with linker peptides that are too short for the two variable regions to fold together, driving the scFvs to dimerize. Still shorter linkers (one or two amino acids) lead to the formation of trimers, the so-called triabodies or tribodies. Tetrabodies may also be used.

In some embodiments, the ASD of the CAR comprises V_{L} fragments as described in **Table 4.**

In some embodiments, the ASD of the CAR comprises V_{H} fragments as described in **Table 6.**

In some embodiments, the ASD of the CAR comprises V_{HH} fragments (nanobodies) as described in **Table 7.**

In some embodiments, the ASD of the CAR comprises affibodies as described in **Table 8.**

In some embodiments, the ASD of the CAR comprises ligands as describes in **Table 10.**

In some embodiments, the ASD of the CAR comprises scFv fragments as describes in **Table 11.**

In one embodiment, an antigen specific domain of a CAR against a target antigen is an antigen binding portion, e.g., CDRs, of vL and vH fragments targeting this antigen as shown in **Tables 4** and **6,** respectively.

In one embodiment, an antigen specific domain of a CAR against a target antigen is an antigen binding portion, e.g., CDRs, of vHH fragments targeting this antigen as shown in Table 7.

In one embodiment, an antigen specific domain of a CAR against a target antigen is an antigen binding portion of a non-immunoglobulin scaffold targeting this antigen as shown in **Table 8.**

In one embodiment, an antigen specific domain of a CAR against a target antigen is an antigen binding portion of a receptor known to bind this target antigen.

In one embodiment, an antigen binding specific domain of a CAR against a target antigen is an antigen binding portion of a ligand known to bind this target antigen.

In one embodiment, an antigen specific domain of a CAR against a target antigen is an antigen binding portion, e.g., CDRs, of vL and vH fragments of a scFV targeting this antigen as shown in **Table 11.**

In another embodiment, the invention provides CARs that bind to the same epitope on the different targets described in Tables 19-22 as any of the CARs of the invention (i.e., CARs that have the ability to cross-compete for binding to the different targets with any of the CARs of the invention). In some embodiments, the antigen specific domains of these CARs could be derived from vL fragments, vH fragments or scFv fragments of antibodies. In some embodiments, the reference antibodies for cross-competition studies to determine the target-epitope recognized by a CAR of the invention described in Tables 19-22 are scFvs having sequences as shown in SEQ ID NOs: 2334-2568 **(Table 11).** In an exemplary embodiment, the reference scFv FMC63(vL-vH) represented by SEQ ID NO: 2334 can be used in cross-competiton studies to to determine the target-epitope recognized by FMC63-based conventional CARs and backbones of the invention (SEQ ID NOs:2672, 2942, 3212, 3495-3515) described in **Tables 19-22.** In some embodiments, the reference vHH fragments for cross-competition studies to determine the target-epitope recognized by a CAR of the invention described in **Tables 19-22** are are vHH fragments having sequences as shown in SEQ ID NOs: 2269-2293 **(Table 7).** In some embodiments, the reference non-immunoglobulin antigen binding scaffolds for cross-competition studies for cross-competition studies to determine the target-epitope recognized by a CAR of the invention described in **Tables 19-22** are non-immunoglobulin antigen binding scaffolds having sequences as shown in SEQ ID NOs: 2294-2298 **(Table 8).** ). In some embodiments, the reference ligands for cross-competition studies to determine the target-epitope recognized by a CAR of the invention described in **Tables 19-22** are ligands having sequences as shown in SEQ ID NOs: 2323-2333 **(Table 10).** In some embodiments, the reference CARs for cross-competition studies against different targets are CARs having sequences as shown in SEQ ID NOs: 2672-2941 **(Table 19).**

In an embodiment, the reference antibodies for cross-competition studies to determine the target-epitopes recognized by the MPL-targeting CARs of the invention (e.g., SEQ ID NOs: 1117-1125) are antibodies mAb-1.6, mAb-1.111, mAb-1.75, mAb-1.78, mAb-1.169, and mAb-1.36 described in patent application US 2012/0269814 A1.

In an embodiment, the reference scFvs for cross-competition studies to determine the target-epitopes recognized by the MPL-targeting CARs of the invention (e.g., SEQ ID NOs: 1117-1125) are scFvs having sequences as shown in SEQ ID NOs: 2499-2506 **(Table 11).**

In an embodiment, the reference ligands for cross-competition studies to determine the target-epitopes recognized by the MPL-targeting CARs of the invention (e.g., SEQ ID NOs: 1117-1125) are ligands having sequences as shown in SEQ ID NOs: 2323-2324 **(Table 10).**

In an embodiment, the reference CARs for cross-competition studies to determine the target-epitopes recognized by the MPL-targeting CARs of the invention are CARs having sequences as shown in SEQ ID NOs: 1117-1125 and 1164 **(Table 19).**

In the preferred embodiment, an MPL-targeting CAR of the invention binds to an MPL-epitope corresponding to or overlapping with the peptide sequence -PWQDGPK-.

In an embodiment, the reference scFvs for cross-competition studies to determine the target-epitopes recognized by the CD19-targeting CARs of the invention (e.g., SEQ ID NOs: 930-941) are scFvs having sequences as shown in SEQ ID NOs: 2336-2347 **(Table 11).**

In an embodiment, the reference CARs for cross-competition studies to determine the target-epitopes recognized by the CD19-targeting CARs of the invention are CARs having sequences as shown in SEQ ID NOs: 930-941 **(Table 19).**

In an embodiment, the reference scFvs for cross-competition studies to determine the target-epitopes recognized by the CD20-targeting CARs of the invention (e.g., SEQ ID NOs: 964-977) are scFvs having sequences as shown in SEQ ID NOs: 2366-2380 **(Table 11).**

In an embodiment, the reference CARs for cross-competition studies to determine the target-epitopes recognized by the CD20-targeting CARs of the invention are CARs having sequences as shown in SEQ ID NOs: 964-977 **(Table 19).**

In the preferred embodiment, the CD20-targeting CARs of the invention bind to the epitopes corresponding to one or more of the sequences -PAGIYAPI-, - FLKMESLNFIRAHTP-, -HFLKMESLNFIRAHTPY -, -YNAEPANPSEKNSPSTQY-, - YNAEPANPSEKNSPST- and -YNCEPANPSEKNSP-.

In an embodiment, the reference scFvs for cross-competition studies against DLL3-targeting CARs of the invention (e.g., SEQ ID NOs: 1044-1045) are scFvs having sequences as shown in SEQ ID NOs: 2443-2444 **(Table 11).**

In an embodiment, the reference CARs for cross-competition studies against DLL3-targeting CARs of the invention are CARs having sequences as shown in SEQ ID NOs: 1044-1045 **(Table 19).**

In an embodiment, the reference scFvs for cross-competition studies against LAMP1-targeting CARs of the invention (e.g., SEQ ID NOs: 1104-1105) are scFvs having sequences as shown in SEQ ID NOs: 2489-2490 **(Table 11).**

In an embodiment, the reference CARs for cross-competition studies against LAMP1-targeting CARs of the invention are CARs having sequences as shown in SEQ ID NOs: 1104-1105 **(Table 19).**

In an embodiment, the reference scFvs for cross-competition studies against TROP2-targeting CARs of the invention (e.g., SEQ ID NOs: 2910-2911) are scFvs having sequences as shown in SEQ ID NOs: 2544-2545 **(Table 11).**

In an embodiment, the reference CARs for cross-competition studies against TROP2-targeting CARs of the invention are CARs having sequences as shown in SEQ ID NOs: 2910-2911 **(Table 19).**

In an embodiment, the reference scFvs for cross-competition studies against PTK7-targeting CARs of the invention (e.g., SEQ ID NOs: 1143-1144) are scFvs having sequences as shown in SEQ ID NOs: 2523-2524 **(Table 11).**

In an embodiment, the reference CARs for cross-competition studies against PTK7-targeting CARs of the invention are CARs having sequences as shown in SEQ ID NOs: 1143-1144 **(Table 19).**

In an embodiment, the reference scFv for cross-competition studies against a CD23-targeting CAR (e.g., SEQ ID NO: 2932) is a scFv having sequence as shown in SEQ ID NO: 2565 **(Table 11).**

In an embodiment, the reference CAR for cross-competition studies against a CD23-targeting CAR of the invention is a CAR having sequences as shown in SEQ ID NO: 2932 **(Table 19).**

In an embodiment, the reference scFvs for cross-competition studies against a TCR-gamma-delta-targeting CAR (e.g., SEQ ID NO: 1155) is a scFv having sequence as shown in SEQ ID NO: 2535 **(Table 11).**

In an embodiment, the reference CAR for cross-competition studies against TCR-gamma-delta-targeting -targeting CAR of the invention is a CAR having sequences as shown in SEQ ID NO: 1155 **(Table 19).**

In an embodiment, the reference scFvs for cross-competition studies against CDH6-targeting CARs of the invention (e.g., SEQ ID NOs: 1016-1017) are scFvs having sequences as shown in SEQ ID NOs: 2418-2419 **(Table 11).**

In an embodiment, the reference CARs for cross-competition studies against CDH6-targeting CARs of the invention are CARs having sequences as shown in SEQ ID NOs: 1016-1017 **(Table 19).**

In an embodiment, the reference scFvs for cross-competition studies against CDH19-targeting CARs of the invention (e.g., SEQ ID NOs: 1019 and 1180) are scFvs having sequences as shown in SEQ ID NOs: 2421 and 2558 **(Table 11).**

In an embodiment, the reference CARs for cross-competition studies against CDH19-targeting CARs of the invention are CARs having sequences as shown in SEQ ID NOs: 1019 and 1180 **(Table 19).**

In an embodiment, the reference scFvs for cross-competition studies against CD324-targeting CARs of the invention (e.g., SEQ ID NOs: 1014-1015) are scFvs having sequences as shown in SEQ ID NOs: 2416-2417 **(Table 11).**

In an embodiment, the reference CARs for cross-competition studies against CD324-targeting CARs of the invention are CARs having sequences as shown in SEQ ID NOs: 1014-1015 **(Table 19).**

In an embodiment, the reference scFv for cross-competition studies against a CD276-targeting CAR (e.g., SEQ ID NO: 2578) is a scFv having sequence as shown in SEQ ID NO: 2415 **(Table 11).**

In an embodiment, the reference CAR for cross-competition studies against a CD276-targeting CARs of the invention is a CAR having sequences as shown in SEQ ID NO: 2578 **(Table 19).**

In an embodiment, the reference scFvs for cross-competition studies against GFRa4-targeting CARs of the invention (e.g., SEQ ID NOs: 1066-1067) are scFvs having sequences as shown in SEQ ID NOs: 2461-2462 **(Table 11).**

In an embodiment, the reference CARs for cross-competition studies against GFRa4-targeting CARs of the invention are CARs having sequences as shown in SEQ ID NOs: 1066-1067 **(Table 19).**

In an embodiment, the reference scFvs for cross-competition studies against CSF2RA-targeting CARs of the invention (e.g., SEQ ID NOs: 1040-1041) are scFvs having sequences as shown in SEQ ID NOs: 2441-2442 **(Table 11).**

In an embodiment, the reference CARs for cross-competition studies against CSF2RA-targeting CARs of the invention are CARs having sequences as shown in SEQ ID NOs: 1040-1041 **(Table 19).**

In an embodiment, the reference scFvs for cross-competition studies against B7H4-targeting CARs of the invention (e.g., SEQ ID NOs: 2929-2930) are scFvs having sequences as shown in SEQ ID NOs: 2562-2563 **(Table 11).**

In an embodiment, the reference CARs for cross-competition studies against B7H4-targeting CARs of the invention are CARs having sequences as shown in SEQ ID NOs: 2929-2930 **(Table 19).**

In an embodiment, the reference scFv for cross-competition studies against a NY-BR1-targeting CAR (e.g., SEQ ID NO: 1131) is a scFv having sequence as shown in SEQ ID NO: 2512 **(Table 11).**

In an embodiment, the reference CAR for cross-competition studies against a NY-BR1-targeting CARs of the invention is a CAR having sequences as shown in SEQ ID NO: 1131 **(Table 19).**

In an embodiment, the reference scFv for cross-competition studies against a CD200R-targeting CAR (e.g., SEQ ID NO: 1190) is a scFv having sequence as shown in SEQ ID NO: 2568 **(Table 11).**

In an embodiment, the reference CAR for cross-competition studies against a CD200R-targeting CARs of the invention is a CAR having sequences as shown in SEQ ID NO: 1190 **(Table 19).**

In an embodiment, the reference scFv for cross-competition studies against a HIV1-envelop glycoprotein gp120-targeting CAR (e.g., SEQ ID NO: 945) is a scFv having sequence as shown in SEQ ID NO: 2512 **(Table 11).**

In an embodiment, the reference CAR for cross-competition studies against a HIV1-envelop glycoprotein gp120-targeting CAR of the invention is a CAR having sequences as shown in SEQ ID NO: 945 **(Table 19).**

In an embodiment, the reference ligand for cross-competition studies against a TSHR-targeting CAR (e.g., SEQ ID NOs: 1167) is a ligand having sequences as shown in SEQ ID NO: **(Table 11).**

In an embodiment, the reference scFvs for cross-competition studies against TSHR-targeting CARs of the invention (e.g., SEQ ID NOs: 1168-1170) are scFvs having sequences as shown in SEQ ID NOs: 2333 **(Table 10).**

In an embodiment, the reference CARs for cross-competition studies against TSHR-targeting CARs of the invention are CARs having sequences as shown in SEQ ID NOs: 1167-1170 **(Table 19).**

In some embodiment, the CARs targeting gp100, MART, Tyrosinase, hTERT, MUC1, CMV-pp65, HTLV1-Tax, HIV1-gag, NY-ESO, WT1, AFP, HPV-16-E7, PR1 bind to target peptides shown in Table 23 in complex with MHC class I (HLA-A2). ***Linkers***

In some embodiments, two or more functional domains of the CARs as described herein, are separated by one or more linkers. Linkers are oligo- or polypeptides region from about 1 to 100 amino acids in length, that link together any of the domains/regions of the CAR of the invention. In some embodiments, the linkers may be for example, 5-12 amino acids in length, 5-15 amino acids in length or 5 to 20 amino acids in length. Linkers may be composed of flexible residues like glycine and serine so that the adjacent protein domains are free to move relative to one another. Longer linkers, for example those longer than 100 amino acids, may be used in connection with alternate embodiments of the invention, and may be selected to, for example, ensure that two adjacent domains do not sterically interfere with one another. In exemplary embodiments, linkers are described in SEQ ID NOs: 250-258 **(Tables 5).**

### Hinge Region

In some embodiments, the CARs (which form part of the backbones) described herein comprise a hinge region between the antigen specific domain and the transmembrane domain. In some embodiments, the hinge region comprises any one or more of human CD8α or an Fc fragment of an antibody or a functional equivalent, fragment or derivative thereof, a hinge region of human CD8α or an antibody or a functional equivalent, fragment or derivative thereof, a CH2 region of an antibody, a CH3 region of an antibody, an artificial spacer sequence and combinations thereof. In exemplary embodiments, the hinge region comprises any one or more of (i) a hinge, CH2 and CH3 region of IgG4, (ii) a hinge region of IgG4, (iii) a hinge and CH2 region of IgG4, (iv) a hinge region of CD8α, (v) a hinge, CH2 and CH3 region of IgG1, (vi) a hinge region of IgG1, (vi) a hinge and CH2 region of IgG1, or (vii) combinations thereof.

### Transmembrane Domain

As described herein, the CARs (which form part of the backbones) described herein comprise a transmembrane domain. The transmembrane domain may comprise the transmembrane sequence from any protein which has a transmembrane domain, including any of the type I, type II or type III transmembrane proteins. The transmembrane domain of the CAR of the invention may also comprise an artificial hydrophobic sequence. The transmembrane domains of the CARs described herein may be selected so that the transmembrane domain do not dimerize. In some embodiments, the TMD encoded CAR comprising any of the backbones described herein comprises a transmembrane domain selected from the transmembrane domain of an alpha, beta or zeta chain of a T-cell receptor, CD28, CD3 epsilon, CD45, CD4, CD5, CD8, CD9, CD16, CD22, CD33, CD37, CD64, CD80, CD86, CD134, CD137, CD154, KIRDS2, OX40, CD2, CD27, LFA-1 (CDl la, CD18), ICOS (CD278), 4-1BB (CD137), GITR, CD40, BAFFR, HVEM (LIGHTR), SLAMF7, NKp80 (KLRFl), CD160, CD19, IL2R beta, IL2R gamma, IL7R a, ITGA1, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGB1, CD29, ITGB2, CD18, LFA-1, ITGB7, TNFR2, DNAM1(CD226), SLAMF4 (CD244, 2B4), CD84, CD96 (Tactile), CEACAM1, CRT AM, Ly9 (CD229), CD160 (BY55), PSGL1, CDIOO (SEMA4D), SLAMF6 (NTB-A, Lyl08), SLAM (SLAMF1, CD150, IPO-3), BLAME (SLAMF8), SELPLG (CD162), LTBR, PAG/Cbp, NKp44, NKp30, NKp46, NKG2D, and/or NKG2C

A transmembrane domain can include one or more additional amino acids adjacent to the transmembrane region, e.g., one or more amino acid associated with the extracellular region of the protein from which the transmembrane was derived (e.g., 1, 2, 3, 4, 5, 6, 7, 8, 9, 10 up to 15 amino acids of the extracellular region) and/or one or more additional amino acids associated with the intracellular region of the protein from which the transmembrane protein is derived (e.g., 1, 2, 3, 4, 5, 6, 7, 8, 9, 10 up to 15 amino acids of the intracellular region). In one aspect, the transmembrane domain is contiguous with one of the other domains of the CAR. In one embodiment, the transmembrane domain may be from the same protein that the signaling domain, costimulatory domain or the hinge domain is derived from. In another aspect, the transmembrane domain is not derived from the same protein that any other domain of the CAR is derived from.

### Intracellular signaling domain of chimeric antigen receptors

As described herein, the CARs (which form part of the backbones) described herein comprise an intracellular signaling domain. This domain may be cytoplasmic and may transduce the effector function signal and direct the cell to perform its specialized function. Examples of intracellular signaling domains include, but are not limited to, ζ chain of the T-cell receptor or any of its homologs (*e.g.,* η chain, FcεR1γ and β chains, MB1 (Igα) chain, B29 (Igβ) chain, etc.), CD3 polypeptides (Δ, δ and ε), syk family tyrosine kinases (Syk, ZAP 70, etc.), src family tyrosine kinases (Lck, Fyn, Lyn, etc.) and other molecules involved in T-cell transduction, such as CD2, CD5 and CD28. Specifically, the intracellular signaling domain may be human CD3 zeta chain, FcγRIII, FcεRI, cytoplasmic tails of Fc receptors, immunoreceptor tyrosine-based activation motif (ITAM) bearing cytoplasmic receptors or combinations thereof. Additional intracellular signaling domains will be apparent to those of skill in the art and may be used in connection with alternate embodiments of the invention. In some embodiments, the intracellular signaling domain comprises a signaling domain of one or more of a human CD3 zeta chain, FcgRIII, FceRI, a cytoplasmic tail of a Fc receptor, an immunoreceptor tyrosine-based activation motif (ITAM) bearing cytoplasmic receptors, and combinations thereof.

### Co-stimulatory Domain

As described herein, the CARs (which form part of the backbones) described herein comprise a co-stimulatory domain. In exemplary embodiments, the co-stimulatory domain comprises a signaling domain from any one or more of CD28, CD137 (4-1BB), CD134 (OX40), Dap10, CD27, CD2, CD5, ICAM-1, LFA-1, Lck, TNFR-I, TNFR-II, Fas, CD30, CD40 and combinations thereof.

### Cleavable Linkers

Cleavable linkers as described herein include 2A linkers (for example T2A), 2A-like linkers or functional equivalents thereof and combinations thereof. In some embodiments, the linkers include the picornaviral 2A-like linker, CHYSEL sequences of porcine teschovirus (P2A), *Thosea asigna* virus (T2A) or combinations, variants and functional equivalents thereof. In other embodiments, the linker sequences may comprise Asp-Val/Ile-Glu-X-Asn-Pro-Gly^{(2A)-}Pro^{(2B)} motif, which results in cleavage between the 2A glycine and the 2B proline. The nucleic sequences of several exemplary cleavable linkers are provided in SEQ ID NO: 831 to SEQ ID NO: 836 and amino acid sequences of several exemplary linkers are provided in SEQ ID NO: 2598 to SEQ ID NO: 2602. Other linkers will be apparent to those of skill in the art and may be used in connection with alternate embodiments of the invention. In an embodiment, a Ser-Gly-Ser-Gly (SGSG) motif (SEQ ID NOs: 837-838 and SEQ ID NO: 2603) is also added upstream of the cleavable linker sequences to enhance the efficiency of cleavage. A potential drawback of the cleavable linkers is the possibility that the small 2A tag left at the end of the N-terminal protein may affect protein function or contribute to the antigenicity of the proteins. To overcome this limitation, in some embodiments, a furine cleavage site (RAKR) (SEQ ID NO: 839-841 and SEQ ID NO: 2604) is added upstream of the SGSG motifs to facilitate cleavage of the residual 2A peptide following translation. In an embodiment, cleavable linkers are placed between the polypeptide encoding the CAR and the polypeptide encoding the accessory modules. The cleavage at the site of cleavable linker results in separation of the two polypeptides.

### Accessory Modules

"Accessory modules" as used herein refer to agents that enhance, reduce or modify the activity of T cells expressing the CARs or reduce toxicity associated with CARs so that the therapeutic response of the CARs is enhanced.

In some embodiments, vectors comprising polynucleotides encoding CARs further comprise polynucleotides encoding viral and cellular signaling proteins which (i) extend the life span of T cells expressing the CARs, (ii) stimulate T cell proliferation and/or (iii) protect T cells expressing the CARs from apoptosis. In exemplary embodiments, such proteins include but are not limited to vFLIP-K13 from Kaposi's sarcoma associated herpes virus (SEQ ID NO: 866), MC159 from molluscum contagiosum virus (SEQ ID NO: 867), cFLIP-L/MRITα (SEQ ID NO: 873), cFLIP-p22 (SEQ ID NO: 874) and Tax from human T cell leukemia/lymphoma virus (HTLV-1 and HTLV-2) (SEQ ID NOs: 875 and SEQ ID NO: 876) and Tax-RS mutant from HTLV-2 (SEQ ID NO: 877).

In one embodiment, vectors encoding CARs further encode vFLIP-K13. In another embodiment, vectors encoding CARs further encode MC159. In another embodiment, vectors encoding CARs further encode HTLV1-Tax. In another embodiment, vectors encoding CARs further encode HTLV2-Tax. In another embodiment, vectors encoding CARs further encode cFLIP-L/MRITα.

In some embodiments, the accessory molecules are encoded by vectors that are distinct from the vectors encoding by the CARs described herein. In some embodiments, effector cells comprising vectors encoding CARs also comprise vectors encoding accessory molecules.

In some embodiments, vectors comprising polynucleotides encoding CARs further express accessory molecule vFLIP MC160 from molluscum contagiosum virus, vFLIP E8 from equine herpes virus 2, vFLIP from human herpes virus saimiri, vFLIP from bovine herpes virus (BHV) 4, vFLIP from mecaca herpes virus and/or full length or N-terminal fragment of human cellular FLICE inhibitory protein (cFLIP-L/MRITα or cFLIP-p22). **(Table 16).**

In some embodiments, vectors comprising polynucleotides encoding CARs further comprise polynucleotides encoding siRNA or scFv specific for cytokines. In exemplary embodiments, the cytokines are any one or more of IL-10, IL-6, IFNγ or combinations thereof. In some embodiment, the CARs are co-expressed with a secreted bispecific antibody fragment that binds to IL6 receptor α and human serum albumin. In some embodiment, the CARs are co-expressed with a secreted scFv fragment that binds to IL6. In some embodiments, the CARs are coexpressed with the peptide FX06 so as to mitigate capillary leak associated with CAR therapy.

In further embodiments, vectors comprising polynucleotides encoding CARs further comprise polynucleotides encoding siRNA or a nuclease targeting the endogenous TCR-α, TCR-β, TCR-γ, TCR-delta, CD3gamma, CD3zeta, CD3epsilon, CD3-delta.

In further embodiments, vectors comprising polynucleotides encoding CARs further comprise polynucleotides encoding a selectable marker. In exemplary embodiment, the selectable marker can encode a drug resistance gene, such as gene that confers resistance to puromycin or calcineurin inhibitors (e.g. CNB30; SEQ ID NO: 852). In some embodiment, the selectable marker may encode for extracellular and transmembrane domains of human CD30, CD20, CD19 (SEQ ID NO: 854), BCMA (SEQ ID NO: 855), EGFR (SEQ ID NO: 853), CD34, or any protein or protein fragment that is expressed on cell surface and can be recognized by an antibody that can be used to eliminate cells expressing its target antigen. In an exemplary embodiment, cetuximab, an anti-EGFR monoclonal is used to eliminate CAR-expressing cells of the invention which coexpress a truncated EGFR (SEQ ID NO: 853). The selectable marker(s) can be used to enrich for cells expressing the CAR, to select for cells that express high levels of CAR and/or to reduce the clonal diversity of the cells expressing the CAR. In further embodiments, polynucleotides encoding CARs may encode for epitope tags that are expressed on the extracellular domain of the CARs and can be used to enrich for cells expressing the CAR, to select for cells that express high levels of CAR and/or to reduce the clonal diversity of the cells expressing the CAR. Non-limiting examples of such tags are provided in SEQ ID NOs: 553-563 and 3526-3530 **(Table 12).** Reducing the clonal diversity of allogeneic T cells expressing the CARs will in turn lead to reduced incidence of Graft versus Host Disease (GVHD), thereby allowing the use of allogeneic T cells for CAR-T cell therapy.

### Polynucleotides and Polypeptides

Provided herein are one or more polypeptides encoded by one or more nucleic acid molecules encoding conventional CARs I to III or any one or more of backbones 1-62 described herein **(Table 2).**

Also provided herein are one or more polypeptides encoded by one or more nucleic acid molecules encoding conventional CARs I to III. In some embodiments, the antigen-specific domain of the CARs is specific to one, two, three or more antigens on target cells, such as cancer cells. As described herein, each component of the CAR is contiguous and in the same reading frame with each other components of the CAR. In some embodiments, in the CAR comprising backbone comprises more than one antigen specific domain, each of the antigen specific domains are contiguous and in the same reading frame as the other antigen specific domains in the same CAR.

Also provided herein are one or more polypeptides encoded by one or more nucleic acid molecules encoding backbone-1 comprising conventional CAR I and K13-vFLIP as described herein. In some embodiments, the antigen-specific domain of the CAR comprising backbone-1 is specific to one, two, three or more antigens on target cells, such as cancer cells. As described herein, each component of the CAR is contiguous and in the same reading frame with each other components of the CAR comprising backbone-1. In some embodiments, in the CAR comprising backbone-1 comprises more than one antigen specific domain, each of the antigen specific domains are contiguous and in the same reading frame as the other antigen specific domains in the same CAR.

Also provided herein are one or more polypeptides encoded by one or more nucleic acid molecules encoding backbone-32 which comprises conventional CAR II and K13-vFLIP as described herein. In some embodiments, the antigen-specific domain of the CAR comprising backbone-32 is specific to one, two, three or more antigens on target cells, such as cancer cells. As described herein, each component of the CAR is contiguous and in the same reading frame with each other components of the CAR. In some embodiments, in the CAR comprising backbone-32 comprises more than one antigen specific domain, each of the antigen specific domains are contiguous and in the same reading frame as the other antigen specific domains in the same CAR.

In various embodiments, the polypeptides encoded by the nucleic acid molecules encoding CARs which are part of conventional CARs I to III or part of the backbones described herein, such as backbone-1, backbone-2, backbone-32 or backbone-33, comprise two, three or more antigen specific domains.

In various embodiments, the polypeptides encoded by the nucleic acid molecules encoding CARs which are part of conventional CARs I to III or part of the backbones described herein, such as backbone-1, backbone-2, backbone-32 or backbone-33, comprise two, three or more co-stimulatory domains.

In various embodiments, the polypeptides encoded by the nucleic acid molecules encoding CARs which are part of conventional CARs I to III or part of the backbones described herein, such as backbone-1, backbone-2, backbone-32 or backbone-33, comprise two, three or more intracellular signaling domain.

In various embodiments, the polypeptides encoded by the nucleic acid molecules encoding CARs which are part of the backbones described herein, such as backbone-1, backbone-2, backbone-32 or backbone-33, comprise one, two, three or more viral and/or cellular signaling proteins.

The nucleic acid sequences encoding for the desired components of the CARs described herein can be obtained using recombinant methods known in the art, such as, for example by screening libraries from cells expressing the nucleic acid molecule, by deriving the nucleic acid molecule from a vector known to include the same, or by isolating directly from cells and tissues containing the same, using standard techniques. Alternatively, the nucleic acid of interest can be produced synthetically, rather than cloned.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to target as described in Tables 19-22.

In one embodiment, an antigen specific domain of a CAR against a target antigen is an antigen binding portion, e.g., CDRs, of vL and vH fragments targeting this antigen as shown in Tables 4 and 6, respectively.

In one embodiment, an antigen specific domain of a CAR against a target antigen is an antigen binding portion, e.g., CDRs, of vHH fragments targeting this antigen as shown in Table 7.

In one embodiment, an antigen specific domain of a CAR against a target antigen is an antigen binding portion of a non-immunoglobulin scaffold targeting this antigen as shown in Table 8.

In one embodiment, an antigen specific domain of a CAR against a target antigen is an antigen binding portion of a receptor known to bind this target antigen.

In one embodiment, an antigen binding specific domain of a CAR against a target antigen is an antigen binding portion of a ligand known to bind this target antigen.

In one embodiment, an antigen specific domain of a CAR against a target antigen is an antigen binding portion, e.g., CDRs, of vL and vH fragments of a scFV targeting this antigen as shown in Table 11.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to the targets described in Tables 19-22.

In some embodiments, provided herein are polypeptides encoded by the nucleic acid molecules encoding CARs which are part of the conventional CARs I to III or are part of the backbones described herein, such as backbone-1, backbone-2, backbone-32 or backbone-33, wherein the antigen-specific domain of the CARs is specific to the thrombopoietin receptor, MPL.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CD19.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CD20.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CD22.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CD23

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CD30, .

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CD32.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CD33.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CD123.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CD138.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CD200R.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CD276.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CD324.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to BCMA.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CS1.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to ALK1.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to ROR1.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CDH6

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CDH16.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CDH17.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CDH19.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to EGFRviii.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to Her2.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to Her3.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to Mesothelin.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to Folate Receptor alpha.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to Folate Receptor beta.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CLL-1.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CLEC5A.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to NY-ESO/MHC class I complex.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to WT1/MHC class I complex.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to WT1/MHC class I complex.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to AFP/MHC class I complex.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to HPV16-E7/MHC class I complex.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to gp100/MHC class I complex.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to hTERT/MHC class I complex.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to MART1/MHC class I complex.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to HTLV1-Tax/MHC class I complex.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to PR1/MHC class I complex.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to HIV1-gag/MHC class I complex.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to HIV1-envelop gp120.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to DLL3.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to PTK7.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to TROP2.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to LAMP1.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to Tim1.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to TCR gamma-delta.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to TCR beta1 constant chain.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to TCR beta2 constant chain.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to GCC.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to B7H4.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to LHR.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to TSHR.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to Tn-Muc1.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to TSLPR.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to Tissue Factor.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to SSEA-4.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to SLea.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to Muc1/MHC class I complex.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to Muc16.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to NYBR-1.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to IL13Ra2.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to IL11Ra.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to L1CAM.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to EpCAM1.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to gpNMB.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to GRP78.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to GPC3.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to GRPC5D.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to GFRa4.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to FITC.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to CD79b.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to Lym1.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to Lym2.

In some embodiments, provided herein are polypeptides encoded by the nucleic acid molecules encoding CARs which are part of the conventional CARs I to III or are part of backbones described herein, such as backbone-1, backbone-2, backbone-32 or backbone-33, wherein the antigen-specific domain of the CARs is specific to Fc portion of an antibody (i.e. Ig Fc). An exemplary CAR with the antigen-specific domain specific to Ig Fc is represented by SEQ ID NO: 2708 and contains the extracellular domain of CD16-V158 as the antigen specific domain.

In some embodiments, the nucleic acid molecule encoding the CARs and/or accessory molecules described herein is provided as a messenger RNA (mRNA) transcript. In another embodiment, the nucleic acid molecule encoding the CARs and/or accessory molecules described herein is provided as a DNA construct.

Also provided herein are vectors comprising the polynucleotides described herein. In some embodiments, the vectors are viral vectors. Examples of viral vectors include but are not limited to retrovirus, an adenovirus, an adeno-associated virus, a lentivirus, a pox virus, a herpes virus vector or a sleeping beauty transposon vector. In various embodiments, the present invention includes retroviral and lentiviral vector constructs expressing the CAR and the accessory molecules that can be directly transduced into a cell.

The present invention also includes an RNA construct that can be directly transfected into a cell. A method for generating mRNA for use in transfection involves in vitro transcription (IVT) of a template with specially designed primers, followed by polyA addition, to produce a construct containing 3' and 5' untranslated sequence ("UTR") (e.g., a 3' and/or 5' UTR described herein), a 5' cap (e.g., a 5' cap described herein) and/or Internal Ribosome Entry Site (IRES) (e.g., an IRES described herein), the nucleic acid to be expressed, and a polyA tail, typically 50-2000 bases in length (SEQ ID NO:922). RNA so produced can efficiently transfect different kinds of cells. In one embodiment, the template includes sequences for the CAR. In an embodiment, an RNA CAR vector is transduced into a cell, e.g., a T cell or a NK cell, by electroporation. In another embodiment, an RNA CAR vector is transduced into a cell, e.g., a T cell or a NK cell, by causing transient perturbations in cell membrane using a microfluid device as described in patent application WO 2013/059343 A1 (PCT/US2012/060646). The polynucleotide sequences coding for the desired molecules can be obtained using recombinant methods known in the art, for example by screening libraries from cells expressing the gene, by deriving the gene from a vector known to include the same, or by isolating directly from cells and tissues containing the same, using standard techniques. Alternatively, the gene of interest can be produced synthetically, rather than cloned.

The present invention also provides vectors in which a DNA encoding the CARs of the present invention is inserted. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity. Exemplary lentiviral vectors are provided in SEQ ID NOs: 905-906. A retroviral vector may also be, e.g., a gammaretroviral vector. A gammaretroviral vector may include, e.g., a promoter, a packaging signal (ψ), a primer binding site (PBS), one or more (e.g., two) long terminal repeats (LTR), and a transgene of interest, e.g., a gene encoding a CAR. A gammaretroviral vector may lack viral structural gens such as gag, pol, and env. Exemplary gammaretroviral vectors include Murine Leukemia Virus (MLV), Spleen-Focus Forming Virus (SFFV), and Myeloproliferative Sarcoma Virus (MPSV), and vectors derived therefrom. Other gammaretroviral vectors are described, e.g., in Tobias Maetzig et al., "Gammaretroviral Vectors: Biology, Technology and Application" Viruses. 2011 Jun; 3(6): 677-713. An exemplary retroviral vector is provided in SEQ ID NO: 907. In another embodiment, the vector comprising the nucleic acid encoding the desired CAR of the invention is an adenoviral vector (A5/35).

The expression of natural or synthetic nucleic acids encoding CARs is typically achieved by operably linking a nucleic acid encoding the CAR polypeptide or portions thereof to a promoter, and incorporating the construct into an expression vector. The vectors can be suitable for replication and integration eukaryotes. Typical cloning vectors contain transcription and translation terminators, initiation sequences, and promoters useful for regulation of the expression of the desired nucleic acid sequence. The expression constructs of the present invention may also be used for nucleic acid immunization and gene therapy, using standard gene delivery protocols. Methods for gene delivery are known in the art. See, e.g., U.S. Pat. Nos. 5,399,346, 5,580,859, 5,589,466, incorporated by reference herein in their entireties.

Cloning and expression methods will be apparent to a person of skill in the art and may be as described in WO 2015/142675; Sambrook et al., 2012, MOLECULAR CLONING: A LABORATORY MANUAL, volumes 1 -4, Cold Spring Harbor Press, NY; June et al. 2009 Nature Reviews Immunology 9.10: 704-716; WO 01/96584; WO 01/29058; U.S. Pat. No. 6,326,193, the contents of each of which are herein incorporated by reference in their entirety as though set forth herein.
Physical methods for introducing polynucleotides of into host cells such as calcium phosphate transfection and the like are well known in the art and will be apparent to a person of skill in the art. In exemplary embodiments, such methods are set forth in Sambrook et al., 2012, MOLECULAR CLONING: A LABORATORY MANUAL, volumes 1 -4, Cold Spring Harbor Press, NY); U.S. Pat. Nos. 5,350,674 and 5,585,362, the contents of each of which are herein incorporated by reference in their entirety as though set forth herein. In another embodiment, a CAR vector is transduced into a cell, e.g., a T cell or a NK cell, by causing transient perturbations in cell membrane using a microfluid device as described in patent application WO 2013/059343 A1 (PCT/US2012/060646) and in Ding X et al, Nat. Biomed. Eng. 1, 0039 (2017) the contents of each of which are herein incorporated by reference in their entirety as though set forth herein.

### Genetically Engineered Cells

In various embodiments, the cells for modifications with CARs described herein, including T cells or NK cells may be obtained from a subject desiring therapy. T cells can be obtained from a number of sources, including peripheral blood mononuclear cells, bone marrow, lymph node tissue, cord blood, thymus tissue, tissue from a site of infection, ascites, pleural effusion, spleen tissue, and tumors. T cells could be tissue resident gamma-delta T cells, which can be cultured and expanded in vitro prior to expression of the CAR.

In one aspect, the invention provides a number of chimeric antigen receptors (CAR) comprising an antigen binding domain (e.g., antibody or antibody fragment, TCR or TCR fragment) engineered for specific binding to a disease-associated antigen, e.g., a tumor antigen described herein. In one aspect, the invention provides an immune effector cell (e.g., T cell, NK cell) engineered to express a CAR, wherein the engineered immune effector cell exhibits a therapeutic property. In one aspect, the invention provides an immune effector cell (e.g., T cell, NK cell) engineered to express a CAR, wherein the engineered immune effector cell exhibits an anticancer property In one aspect, a cell is transformed with the CAR and the CAR is expressed on the cell surface. In some embodiments, the cell (e.g., T cell, NK cell) is transduced with a viral vector encoding a CAR. In some embodiments, the viral vector is a retroviral vector. In some embodiments, the viral vector is a lentiviral vector. In some such embodiments, the cell may stably express the CAR. In another embodiment, the cell (e.g., T cell, NK cell) is transfected with a nucleic acid, e.g., mRNA, cDNA, DNA, encoding a CAR. In some such embodiments, the cell may transiently express the CAR.

The present invention provides immune effector cells (e.g., T cells, NK cells) that are engineered to contain one or more CARs that direct the immune effector cells to diseased cells or disease-associated cells, such as cancer cells. This is achieved through an antigen binding domain on the CAR that is specific for a cancer associated antigen. There are two classes of cancer associated antigens (tumor antigens) that can be targeted by the CARs of the instant invention: (1) cancer associated antigens that are expressed on the surface of cancer cells; and (2) cancer associated antigens that itself is intracellular, however, a fragment of such antigen (peptide) is presented on the surface of the cancer cells by MHC (major histocompatibility complex).

Furthermore, the present invention provides CARs and CAR-expressing cells and their use in medicaments or methods for treating, among other diseases, cancer or any malignancy or autoimmune diseases or infectious disease or degenerative disease or allergic disease involving cells or tissues which express a tumor antigen or disease associated antigen as described herein.

In one aspect, the invention provides an immune effector cell (e.g., T cell, NK cell) engineered to express a chimeric antigen receptor (CAR), wherein the engineered immune effector cell exhibits an anti-disease property, such as antitumor property. A preferred antigen is a cancer associated antigen (i.e., tumor antigen) described herein. In one aspect, the antigen binding domain of the CAR comprises a partially humanized antibody fragment. In one aspect, the antigen binding domain of the CAR comprises a partially humanized scFv. Accordingly, the invention provides CARs that comprises a humanized antigen binding domain and is engineered into a cell, e.g., a T cell or a NK cell, and methods of their use for adoptive therapy.

Further provided herein are genetically engineered cells, comprising the polynucleotides and/or the chimeric antigen receptors described herein. In some embodiments, the cell is a T-lymphocyte (T-cell). In some embodiment the cell is a naïve T cells, a central memory T cells, an effector memory T cell, a regulatory T cell (Treg) or a combination thereof. In some embodiments, the cell is a natural killer (NK) cell, a hematopoietic stem cell (HSC), an embryonic stem cell, or a pluripotent stem cell. Genetically engineered cells which may comprise and express the CARs of the invention include, but are not limited to, T-lymphocytes (T-cells), naive T cells (TN), memory T cells (for example, central memory T cells (TCM), effector memory cells (TEM)), natural killer cells, hematopoietic stem cells and/or pluripotent embryonic/induced stem cells capable of giving rise to therapeutically relevant progeny. In an embodiment, the genetically engineered cells are autologous cells. In an embodiment, the genetically engineered cells are allogeneic cells. By way of example, individual T-cells of the invention may be CD4+/CD8-, CD4-/CD8+, CD4-/CD8- or CD4+/CD8+. The T-cells may be a mixed population of CD4+/CD8-and CD4-/CD8+ cells or a population of a single clone. CD4+ T-cells of the invention may produce IL-2, IFNγ, TNFα and other T-cell effector cytokines when co-cultured in vitro with cells expressing the target antigens (for example CD20+ and/or CD19+ tumor cells). CD8+ T-cells of the invention may lyse antigen-specific target cells when co-cultured in vitro with the target cells. In some embodiments, T cells may be any one or more of CD45RA+ CD62L+ naive cells, CD45RO+ CD62L+ central memory cells, CD62L- effector memory cells or a combination thereof (Berger et al., Adoptive transfer of virus-specific and tumor-specific T cell immunity. Curr Opin Immunol 2009 21(2)224-232). Genetically modified cells may be produced by stably transfecting cells with DNA encoding the CAR of the invention.

Various methods produce stable transfectants which express the CARs of the invention. In one embodiment, a method of stably transfecting and re-directing cells is by electroporation using naked DNA. By using naked DNA, the time required to produce redirected cells may be significantly reduced. Additional methods to genetically engineer cells using naked DNA encoding the CAR of the invention include but are not limited to chemical transformation methods (e.g., using calcium phosphate, dendrimers, liposomes and/or cationic polymers), non-chemical transformation methods (e.g., electroporation, optical transformation, gene electrotransfer, transient perturbation in cell membranes and/or hydrodynamic delivery) and/or particle-based methods (e.g., impalefection, using a gene gun and/or magnetofection). The transfected cells demonstrating presence of a single integrated un-rearranged vector and expression of the CAR may be expanded ex vivo. In one embodiment, the cells selected for ex vivo expansion are CD8+ and demonstrates the capacity to specifically recognize and lyse antigen-specific target cells.

Viral transduction methods may also be used to generate redirected cells which express the CAR of the invention. Cell types that may be used to generate genetically modified cells expressing the CAR of the invention include but are not limited to T-lymphocytes (T-cells), natural killer cells, hematopoietic stem cells and/or pluripotent embryonic/induced stem cells capable of giving rise to therapeutically relevant progeny.

Stimulation of the T-cells by an antigen under proper conditions results in proliferation (expansion) of the cells and/or production of IL-2. The cells comprising the CAR of the invention will expand in number in response to the binding of one or more antigens to the antigen-specific targeting regions of the CAR. The invention also provides a method of making and expanding cells expressing a CAR. The method comprises transfecting or transducing the cells with the vector expressing the CAR and stimulating the cells with cells expressing the target antigens, recombinant target antigens, or an antibody to the receptor to cause the cells to proliferate, so as to make and expand T-cells. In an embodiment, the cells may be any one or more of T-lymphocytes (T-cells), natural killer (NK) cells, hematopoietic stem cells (HSCs) or pluripotent embryonic/induced stem cells capable of giving rise to therapeutically relevant progeny.

In some embodiments, genetically engineered cells described herein express the various backbones described herein, wherein the CAR component of the backbone determines target specificity based on the antigen specific domain of the CAR.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to MPL.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to CD19.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to CD20.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to BCMA.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to CD22.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to CD30.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to CD33.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to CD123.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to CD138.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to CLL1.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to TCR-beta1 constant chain.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to TCR-beta2 constant chain.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to ALK.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to PTK7.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to DLL3.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to TROP2.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to Tim1.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to LAMP1.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to CS1.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to Lym1.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to Lym2.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to TSHR.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to NY-ESO/MHC class I complex.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to WT1/ MHC class I complex.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to HIV1 envelop glycoprotein gp120.

In one embodiment, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen-specific domain of the CARs is specific to Fc region of an immunoglobulin.

In various embodiments, the genetically engineered cells comprise nucleic acid molecules encoding conventional CARs I to III or conventional CARs I to III which are part of the backbones described herein, such as backbone-1, backbone-2, backbone-32 or backbone-33, wherein the antigen specific domain targets antigen described in Tables 4, 7, 8, 11, 19, 20, 21 or 22.

Immune effector cells such as T cells and NK cells comprising CARs and/or enhancers as described herein may be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

### Therapeutic Methods

Provided herein are methods for treating a disease associated with expression of a disease-associated antigen or a cancer associated antigen.

In one embodiment, provided herein are methods for treating a disease in a subject in need thereof by administering to the subject a therapeutically effective amount of genetically modified cells described herein (such as T cells, NK cells) that are engineered to express an antigen-specific CAR alone or an antigen specific CAR and an accessory molecule, wherein the antigen is a disease specific antigen as described herein, and wherein the disease causing or disease-associated cells express the said disease-specific antigen.

In one embodiment, provided herein are methods for treating cancer in a subject in need thereof by administering to the subject a therapeutically effective amount of genetically modified cells described herein (such as T cells, NK cells) that are engineered to express an antigen-specific CAR alone or an antigen specific CAR and an accessory molecule, wherein the antigen is a cancer specific antigen as described herein, and wherein the cancer cells express the said tumor antigen.

In one embodiment, the cancer specific antigen is expressed on both normal cells and cancers cells, but is expressed at lower levels on normal cells. In one embodiment, the method further comprises selecting a CAR that binds the cancer specific antigen of interest with an affinity that allows the antigen specific CAR to bind and kill the cancer cells. In some embodiments, the antigen specific CAR kills cancer cells but kills less than 30%, 25%, 20%, 15%, 10%, 5% or less of the normal cells expressing the cancer antigen. In exemplary embodiments, the percentage of cells killed by the antigen specific CARs may be determined using the cell death assays described herein.

In some embodiment, the present invention provides methods of treating cancer in a subject in need thereof comprising administering to the subject a therapeutically effective amount of the genetically modified cells (e.g., T cells, NK cells) that are engineered to express conventional CARs I to III, wherein the ASD of the CARs is specific to the antigen that is expressed on cancer cells (for example, the antigen is expressed at lower levels on normal cells relative to cancer cells).

In some embodiment, the present invention provides methods of treating cancer in a subject in need thereof comprising administering to the subject a therapeutically effective amount of the genetically modified cells (e.g., T cells, NK cells) that are engineered to express backbone-1 comprising the conventional CARs I and the accessory module K13-vFLIP, wherein the ASD of the CARs is specific to the antigen that is expressed on cancer cells (for example, the antigen is expressed at lower levels on normal cells relative to cancer cells).

In some embodiment, the present invention provides methods of treating cancer in a subject in need thereof comprising administering to the subject a therapeutically effective amount of the genetically modified cells (e.g., T cells, NK cells) that are engineered to comprising backbone-2 comprising the conventional CARs I and the accessory module MC159-vFLIP, wherein the ASD of the CARs is specific to the antigen that is expressed on cancer cells (for example, the antigen is expressed at lower levels on normal cells relative to cancer cells).

In some embodiment, the present invention provides methods of treating cancer in a subject in need thereof comprising administering to the subject a therapeutically effective amount of the genetically modified cells (e.g., T cells, NK cells) that are engineered to comprising backbone-32 comprising the conventional CAR II and the accessory module K13-vFLIP, wherein the ASD of the CARs is specific to the antigen that is expressed on cancer cells (for example, the antigen is expressed at lower levels on normal cells relative to cancer cells).

In some embodiment, the present invention provides methods of treating cancer in a subject in need thereof comprising administering to the subject a therapeutically effective amount of the genetically modified cells (e.g., T cells, NK cells) that are engineered to comprising backbone-33 comprising the conventional CAR II and the accessory module MC159-vFLIP, wherein the ASD of the CARs is specific to the antigen that is expressed on cancer cells (for example, the antigen is expressed at lower levels on normal cells relative to cancer cells).

In exemplary embodiments, the antigens that may be targeted for the therapeutic methods described herein include but are not limited to any one, two, three, four or more of: CD19; CD123; CD22; CD23, CD30; CD32, CD99: Tissue Factor; MPL; CD171; CS-1 (also referred to as CD2 subset 1, CRACC, SLAMF7, CD319, and 19A24); C-type lectin-like molecule-1 (CLL-1 or CLECL1); CD33; epidermal growth factor receptor variant III (EGFRvIII); ganglioside G2 (GD2); ganglioside GD3(aNeu5Ac(2-8)aNeu5Ac(2-3)bDGalp(1-4)bDGlcp(1-1)Cer); TNF receptor family member B cell maturation (BCMA); Tn antigen ((Tn Ag) or (GalNAca-Ser/Thr)); prostate-specific membrane antigen (PSMA); Receptor tyrosine kinase-like orphan receptor 1 (ROR1); Fms- Like Tyrosine Kinase 3 (FLT3); Tumor-associated glycoprotein 72 (TAG72); CD38; CD44v6; Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Mesothelin; Interleukin 11 receptor alpha (IL-1 1Ra); prostate stem cell antigen (PSCA); Protease Serine 21 (Testisin or PRSS21); vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha; Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CAFX); Proteasome (Prosome, Macropain) Subunit, Beta Type, 9 (LMP2); glycoprotein 100 (gplOO); oncogene fusion protein consisting of breakpoint cluster region (BCR) and Abelson murine leukemia viral oncogene homolog 1 (Abl) (bcr-abl); tyrosinase; ephrin type-A receptor 2 (EphA2); Fucosyl GM1; sialyl Lewis adhesion molecule (sLe); ganglioside GM3 (aNeu5Ac(2-3)bDGalp(1- 4)bDGlcp(1-1)Cer); transglutaminase 5 (TGSS); high molecular weight-melanoma- associated antigen (HMWMAA); o-acetyl-GD2 ganglioside (OAcGD2); Folate receptor beta; tumor endothelial marker 1 (TEM1/CD248); tumor endothelial marker 7-related (TEM7R); claudin 6 (CLDN6); thyroid stimulating hormone receptor (TSHR); G protein- coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCRl); adrenoceptor beta 3 (ADRB3); pannexin 3 (PANX3); G protein-coupled receptor 20 (GPR20); lymphocyte antigen 6 complex, locus K 9 (LY6K); Olfactory receptor 51 E2 (OR51E2); TCR Gamma Alternate Reading Frame Protein (TARP), Wilms tumor protein (WT1); Cancer/testis antigen 1 (NY-ESO-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen- 1 (MAD-CT-1); melanoma cancer testis antigen-2 (MAD-CT-2); Fos-related antigen 1; tumor protein p53 (p53); p53 mutant; prostein; surviving; telomerase; prostate carcinoma tumor antigen- 1 (PCTA-1 or Galectin 8), melanoma antigen recognized by T cells 1 (MelanA or MARTI); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin Bl; v-myc avian myelocytomatosis viral oncogene neuroblastoma derived homolog (MYCN); Ras Homolog Family Member C (RhoC); Tyrosinase-related protein 2 (TRP-2); Cytochrome P450 1B1 (CVP1B1); CCCTC-Binding Factor (Zinc Finger Protein)-Like (BORIS or Brother of the Regulator of Imprinted Sites), Squamous Cell Carcinoma Antigen Recognized By T Cells 3 (SART3); Paired box protein Pax-5 (PAX5); proacrosin binding protein sp32 (OY-TES1); lymphocyte- specific protein tyrosine kinase (LCK); A kinase anchor protein 4 (AKAP-4); synovial sarcoma, X breakpoint 2 (SSX2); Receptor for Advanced Glycation Endproducts (RAGE-1); renal ubiquitous 1 (RU1); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a, CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin- like hormone receptor- like 2 (EMR2); lymphocyte antigen 75 (LY75); Glypican-3 (GPC3); Fc receptor-like 5 (FCRL5); and immunoglobulin lambda-like polypeptide 1 (IGLLl), PTK7, LAMP1, TROP2, Tim1 and HIV1-envelop glycoprotein gp120.

In exemplary embodiments, the antigens that may be targeted for the therapeutic methods described herein include but are not limited to any one, two, three, four or more of: TSHR. CD171, CS-1, CLL-1, GD3, Tn Ag, FLT3, CD38, CD44v6, B7H3, KIT, IL-13Ra2, IL-11Ra, PSCA, PRSS21, VEGFR2, LewisY, CD24, PDGFR-beta, SSEA-4, MUC1, EGFR, NCAM, CAIX, LMP2, EphA2, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, ETV6-AML, sperm protein 17, XAGE1, Tie 2, MAD-CT-1, MAD-CT-2, Fos-related antigen 1, p53 mutant, hTERT, sarcoma translocation breakpoints, ML-IAP, ERG (TMPRSS2 ETS fusion gene), NA17, PAX3. Androgen receptor, Cyclin Bl, MYCN, RhoC, CYP1B1, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5, and IGLL1.

In exemplary embodiments, the antigens that may be targeted for the therapeutic methods described herein include but are not limited to any one, two, three, four or more of: WT-1, hTERT, PRAME, HMMR/Rhamm, PR1, CG1, CD33, Cyclin-A1, NY-ESO-1, MAGE, HA-1, ACC1, T4A, LB-LY75-IK, BCR-ABL, FLT3-ITD, B-cell receptor idiotype, HTLV-I Tax protein, CD19, Lewis Y, CD22 and ROR1.

In exemplary embodiments, the antigens that may be targeted for the therapeutic methods described herein include but are not limited to any one, two, three, four or more of the targets described in Tables 4, 7, 8, 11, 19, 20, 21 or 22.

In one embodiment, the present invention provides methods of treating cancer by providing to the subject in need thereof immune effector cells (e.g., T cells, NK cells) that are engineered to express conventional CARs I to III or backbones (for example, backbone-1, backbone-2, backbone-32 or backbone-33) specific to MPL, wherein the cancer cells express MPL. In one embodiment, the cancer to be treated is acute myeloid leukemia, chronic myeloid leukemia, and myelodysplastic syndrome. In one embodiment, the antigen specific domain of the MPL specific CAR comprises V_{L} fragments as described in SEQ ID NOs: 180-187, V_{H} fragments as described in SEQ ID NOs: 427-434 and/or scFV as described in SEQ ID NOs: 729-736. In some embodiments, the activity of CAR-T cells may be controlled using Dasatinib, Ponatinib and/or A-770041, concurrently or sequentially with the immune effector cells described herein.

In one embodiment, the present invention provides methods of treating cancer, autoimmune or allergic disease by providing to the subject in need thereof immune effector cells (e.g., T cells, NK cells) that are engineered to express conventional CARs I to III or backbones (for example, backbone-1, backbone-2, backbone-32 or backbone-33) specific to CD19, wherein the cancer cells express CD19. In one embodiment, the cancer to be treated is acute lymphoblastic leukemia, chronic lymphocytic leukemia, , B cell malignancy, non-Hodgkins lymphoma, diffuse large B-cell lymphoma, mantle cell lymphoma, or, multiple myeloma. In one embodiment, the antigen specific domain of the CD19 specific CAR comprises V_{L} fragments as described in SEQ ID NOs: 40-55, V_{H} fragments as described in SEQ ID NOs: 288-303 and/or scFV as described in SEQ ID NOs: 564-577. In some embodiments, the activity of CAR-T cells may be controlled using Dasatinib, Ponatinib and/or A-770041, concurrently or sequentially with the immune effector cells described herein.

In one embodiment, the present invention provides methods of treating cancer by providing to the subject in need thereof immune effector cells (e.g., T cells, NK cells) that are engineered to express backbones (for example, backbone-1, backbone-2, backbone-32 or backbone-33) specific to GRP-78, wherein the cancer cells express GRP-78. In one embodiment, the cancer to be treated is breast cancer. In one embodiment, when the immune effector cells expressing the GRP-78 CAR also expresses an MPL CAR, the cancer to be treated is acute lymphoblastic leukemia, chronic lymphocytic leukemia, chronic myeloid leukemia, diffuse large B-cell lymphoma, mantle cell lymphoma, myelodysplastic syndrome or multiple myeloma. In one embodiment, the antigen specific domain of the GRP-78 specific CAR comprises scFV as described in SEQ ID NO: 703. In one embodiment, the antigen specific domain of the MPL specific CAR comprises V_{L} fragments as described in SEQ ID NOs: 180-187, V_{H} fragments as described in SEQ ID NOs: 427-434 and/or scFV as described in SEQ ID NOs: 729-736. In some embodiments, the activity of CAR-T cells may be controlled using Dasatinib, Ponatinib and/or A-770041, concurrently or sequentially with the immune effector cells described herein.

In one embodiment, the present invention provides methods of treating cancer, autoimmune or allergic disease by providing to the subject in need thereof immune effector cells (e.g., T cells, NK cells) that are engineered to express backbones (for example, backbone-1, backbone-2, backbone-32 or backbone-33) specific to CD79b, wherein the cancer cells express CD79b. In one embodiment, the cancer to be treated is acute lymphoblastic leukemia, chronic lymphocytic leukemia, chronic myeloid leukemia, diffuse large B-cell lymphoma, mantle cell lymphoma, myelodysplastic syndrome or multiple myeloma. In one embodiment, the antigen specific domain of the CD79b specific CAR comprises V_{L} fragments as described in SEQ ID NOs: 92-93, V_{H} fragments as described in SEQ ID NOs: 340-341 and/or scFV as described in SEQ ID NO: 628. In some embodiments, the activity of CAR-T cells may be controlled using Dasatinib, Ponatinib and/or A-770041, concurrently or sequentially with the immune effector cells described herein.

In one embodiment, the present invention provides methods of treating cancer, autoimmune or allergic disease by providing to the subject in need thereof immune effector cells (e.g., T cells, NK cells) that are engineered to express conventional CARs I to III or backbones (for example, backbone-1, backbone-2, backbone-32 or backbone-33) specific to ROR-1 (Receptor tyrosine kinase-like orphan receptor 1), wherein the disease associated or disease causing cells express ROR-1.In some embodiments, cancer to be treated is chronic lymphocytic leukemia or solid organ tumors. In one embodiment, the antigen specific domain of the ROR-1 specific CAR comprises V_{H} fragments as described in SEQ ID NOs: 454-455 and/or scFV as described in SEQ ID NO: 755-756. In some embodiments, the activity of CAR-T cells may be controlled using Dasatinib, Ponatinib and/or A-770041, concurrently or sequentially with the immune effector cells described herein.

In one embodiment, the present invention provides methods of treating cancer, autoimmune or allergic a disease by providing to the subject in need thereof immune effector cells (e.g., T cells, NK cells) that are engineered to express backbones (for example, backbone-1, backbone-2, backbone-32 or backbone-33) specific to Lym-1. In one embodiment, the cancer to be treated or prevented is a B cell malignancy.In one embodiment, the antigen specific domain of the Lym-1 specific CAR comprises V_{L} fragments as described in SEQ ID NO: 175, V_{H} fragments as described in SEQ ID NO: 421 and/or scFV as described in SEQ ID NO: 723. In some embodiments, the activity of CAR-T cells may be controlled using Dasatinib, Ponatinib and/or A-770041, concurrently or sequentially with the immune effector cells described herein.

In one embodiment, the present invention provides methods of treating cancer, autoimmune or allergic disease by providing to the subject in need thereof immune effector cells (e.g., T cells, NK cells) that are engineered to express conventional CAR I to III or backbones (for example, backbone-1, backbone-2, backbone-32 or backbone-33) specific to Lym-2. In some embodiments, the cancer to be treated is blood cancer, ovarian cancer, prostate cancer or pancreatic tumors. In one embodiment, the antigen specific domain of the Lym-2 specific CAR comprises V_{L} fragments as described in SEQ ID NO: 176, V_{H} fragments as described in SEQ ID NO: 422 and/or scFV as described in SEQ ID NO: 724. In some embodiments, the activity of CAR-T cells may be controlled using Dasatinib, Ponatinib and/or A-770041, concurrently or sequentially with the immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express a CD123-specific conventional CAR or backbones 1-62 (for example, backbone-1, backbone-2, backbone-32 or backbone-33), wherein the disease causing or disease associated cells express CD123. In one embodiment, the disease to be treated or prevented is a cancer. In one embodiment, the cancer to be treated or prevented is AML or myelodysplastic syndrome (MDS). In some embodiments, the activity of CAR-T cells may be controlled using Dasatinib. Ponatinib and/or A-770041, concurrently or sequentially with the immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express a CD22-specific conventional CAR or backbones 1-62 (for example, backbone-1, backbone-2, backbone-32 or backbone-33), wherein the disease causing or disease associated cells express CD22. In one embodiment, the disease to be treated or prevented is a cancer. In one embodiment, the cancer to be treated or prevented is B cell malignancies. In one embodiment, the disease to be treated or prevented is an immune disease or allergic disease. In some embodiments, the activity of CAR-T cells may be controlled using Dasatinib, Ponatinib and/or A-770041, concurrently or sequentially with the immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express a CD23-specific conventional CAR or backbones 1-62 (for example, backbone-1, backbone-2, backbone-32 or backbone-33), wherein the disease causing or disease associated cells express CD23. In one embodiment, the disease to be treated or prevented is a cancer. In one embodiment, the cancer to be treated or prevented is B cell malignancies. In one embodiment, the disease to be treated or prevented is an immune disease or allergic disease. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express a CS-1-specific conventional CAR or backbones 1-62 (for example, backbone-1, backbone-2, backbone-32 or backbone-33), wherein the disease causing or disease associated cells express CS-1. In one embodiment, the disease to be treated or prevented is an immune disease or allergic disease. In one embodiment, the disease to be treated or prevented is a cancer. In one embodiment, the cancer to be treated or prevented is a plasma cell malignancy or multiple myeloma or primary effusion lymphoma. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express a BCMA-CAR, wherein the disease causing or disease associated cells express BCMA. In one embodiment, the disease to be treated or prevented is an immune disease or allergic disease. In one embodiment, the disease to be treated or prevented is a cancer. In one embodiment, the cancer to be treated or prevented is a plasma cell malignancy or multiple myeloma or primary effusion lymphoma. In one embodiment, the disease to be treated or prevented is an immune disease. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express a CLL-1-specific conventional CAR or backbones 1-62 (for example, backbone-1, backbone-2, backbone-32 or backbone-33), wherein the disease causing or disease associated cells express CLL-1. In one embodiment, the disease to be treated or prevented is a cancer. In one embodiment, the cancer to be treated or prevented is AML or MDS. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express a CD30-specific conventional CAR or backbones 1-62 (for example, backbone-1, backbone-2, backbone-32 or backbone-33), wherein the disease causing or disease associated cells express CD30. In one embodiment, the disease to be treated or prevented is a cancer. In one embodiment, the cancer to be treated or prevented is lymphoma or leukemia. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express a CD33-specific conventional CAR or backbones 1-62 (for example, backbone-1, backbone-2, backbone-32 or backbone-33), wherein the disease causing or disease associated cells express CD33. In one embodiment, the disease to be treated or prevented is a cancer. In one embodiment, the cancer to be treated or prevented is AML or MDS. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express a IL-13Ra2-specific conventional CAR or backbones 1-62 (for example, backbone-1, backbone-2, backbone-32 or backbone-33), wherein the disease causing or disease associated cells express IL-13Ra2. In one embodiment, the disease to be treated or prevented is a cancer. In one embodiment, the cancer to be treated or prevented is glioblastoma. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express a IL-11Ra-CAR, wherein the disease causing or disease associated cells express IL-11Ra. In one embodiment, the disease to be treated or prevented is a cancer. In one embodiment, the cancer to be treated or prevented is osteosarcoma. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express CD20-CAR, wherein the disease causing or disease associated cells express CD20. In one embodiment, the disease to be treated or prevented is a cancer. In one embodiment, the cancer to be treated or prevented is a B cell malignancy, such as chronic lymphocytic leukemia, acute lymphocytic leukemia, diffuse large cell lymphoma, follicular lymphoma or mantle cell lymphoma. In one embodiment, the disease to be treated or prevented is an immune disease or allergic disease. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express folate receptor alpha-specific conventional CAR or backbones 1-62 (for example, backbone-1, backbone-2, backbone-32 or backbone-33), wherein the disease causing or disease associated cells express folate receptor alpha. In one embodiment, the disease to be treated or prevented is a cancer. In one embodiment, the cancer to be treated or prevented is ovarian cancer, NSCLC, endometrial cancer, renal cancer, or other solid tumors. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express TSHR-specific conventional CAR or backbones 1-62 (for example, backbone-1, backbone-2, backbone-32 or backbone-33), wherein the disease causing or disease associated cells express TSHR (Thyroid Stimulating Hormone Receptor). In one embodiment, the disease to be treated or prevented is cancer. In one embodiment, the cancer to be treated or prevented is thyroid cancer, or multiple myeloma or T cell leukemia or T cell lymphoma. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express GPRC5D-specific conventional CAR or backbones 1-62 (for example, backbone-1, backbone-2, backbone-32 or backbone-33), wherein the disease causing or disease associated cells express GPRC5D. In one embodiment, the disease to be treated or prevented is cancer, immune or allergic disease. In one embodiment, the cancer to be treated or prevented is a plasma cell disorder, multiple myeloma or primary effusion lymphoma. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express ALK-specific conventional CAR or backbones 1-62 (for example, backbone-1, backbone-2, backbone-32 or backbone-33), wherein the disease causing or disease associated cells express ALK. In one embodiment, the disease to be treated or prevented is a cancer. In one embodiment, the cancer to be treated or prevented is a NSCLC, ALCL (anaplastic large cell lymphoma), IMT (inflammatory myofibroblastic tumor), or neuroblastoma. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express a HAVCRl-specific conventional CAR or backbones 1-62 (for example, backbone-1, backbone-2, backbone-32 or backbone-33), wherein the disease causing or disease associated cells express HAVCRl. In one embodiment, the disease to be treated or prevented is a cancer. In one embodiment, the cancer to be treated or prevented is renal cancer. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express a WTl/MHC I complex-specific CAR, wherein the disease causing or disease associated cells express WTl in association with MHC class I complex. In one embodiment, the disease to be treated or prevented is a cancer. In one embodiment, the cancer to be treated or prevented is AML or myeloma. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express a NY-ESO-1/MHC 1 complex-specific conventional CAR or backbones 1-62 (for example, backbone-1, backbone-2, backbone-32 or backbone-33), wherein the disease causing or disease associated cells express NY-ESO-1 in association with MHC class I complex. In one embodiment, the disease to be treated or prevented is a cancer. In one embodiment, the cancer to be treated or prevented is myeloma. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express PTK7-specific conventional CAR or backbones 1-62 (for example, backbone-1, backbone-2, backbone-32 or backbone-33), wherein the disease causing or disease associated cells express PTK7. In one embodiment, the disease to be treated or prevented is a cancer. In one embodiment, the cancer to be treated or prevented is melanoma, lung cancer or ovarian cancer. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express DLL3-specific conventional CAR or backbones 1-62 (for example, backbone-1, backbone-2, backbone-32 or backbone-33), wherein the disease causing or disease associated cells express DLL3. In one embodiment, the disease to be treated or prevented is a cancer. In one embodiment, the cancer to be treated or prevented is melanoma, lung cancer or ovarian cancer. In some embodiments, the activity of CAR-T cells may be controlled using Dasatinib, Ponatinib and/or A-770041, concurrently or sequentially with the administration of immune effector cells described herein

In one aspect, the present invention provides methods of treating or preventing a disease by providing to the subject in need thereof immune effector cells (e.g., T cells, NK cells) or stem cells that can give rise to immune effector cells that are engineered to express GCC-specific conventional CAR or backbones 1-62 (for example, backbone-1, backbone-2, backbone-32 or backbone-33), wherein the disease causing or disease associated cells express GCC. In one embodiment, the disease to be treated or prevented is a cancer. In one embodiment, the cancer to be treated or prevented is gastrointestinal cancer. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express CCR4-specific conventional CAR or backbones 1-62 (for example, backbone-1, backbone-2, backbone-32 or backbone-33), wherein the disease causing or disease associated cells express CCR4. In one embodiment, the disease to be treated or prevented is a cancer. In one embodiment, the cancer to be treated or prevented is T cell leukemia or lymphoma. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express CDH1/CD324-specific conventional CAR or backbones 1-62 (for example, backbone-1, backbone-2, backbone-32 or backbone-33), wherein the disease causing or disease associated cells express CDH1/CD324. In one embodiment, the disease to be treated or prevented is a cancer. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express CD200R-specific conventional CAR or backbones 1-62 (for example, backbone-1, backbone-2, backbone-32 or backbone-33), wherein the disease causing or disease associated cells express CD200R. In one embodiment, the disease to be treated or prevented is a cancer. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) that are engineered to express a CDH19-specific conventional CAR or backbones 1-62 (for example, backbone-1, backbone-2, backbone-32 or backbone-33), wherein the disease causing or disease associated cells express CDH19. In one embodiment, the disease to be treated or prevented is a cancer. In one embodiment, the cancer to be treated or prevented is a solid tumor. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) that are engineered to express a CDH17-specific conventional CAR or backbones 1-62 (for example, backbone-1, backbone-2, backbone-32 or backbone-33), wherein the disease causing or disease associated cells express CDH17. In one embodiment, the disease to be treated or prevented is a cancer. In one embodiment, the cancer to be treated or prevented is a solid tumor. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) that are engineered to express a CDH6-specific conventional CAR or backbones 1-62 (for example, backbone-1, backbone-2, backbone-32 or backbone-33), wherein the disease causing or disease associated cells express CDH6. In one embodiment, the disease to be treated or prevented is a cancer. In one embodiment, the cancer to be treated or prevented is a solid tumor. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) that are engineered to express a GFRa4-specific conventional CAR or backbones 1-62 (for example, backbone-1, backbone-2, backbone-32 or backbone-33), wherein the disease causing or disease associated cells express GFRa4. In one embodiment, the disease to be treated or prevented is a cancer. In one embodiment, the cancer to be treated or prevented is a thyroid medullary cancer. In some embodiments, the activity of CAR-T cells may be controlled using Dasatinib, Ponatinib and/or A-770041, concurrently or sequentially with the administration of immune effector cells described herein.

In one aspect, the present invention provides methods of treating or preventing a disease by providing to the subject in need thereof immune effector cells (e.g., T cells, NK cells) or stem cells that can give rise to immune effector cells that are engineered to express LHR-specific conventional CAR or backbones 1-62 (for example, backbone-1, backbone-2, backbone-32 or backbone-33), wherein the disease causing or disease associated cells express LHR (Leutanizing Hormone Receptor). In one embodiment, the disease to be treated or prevented is a cancer. In one embodiment, the cancer to be treated or prevented is prostate cancer, ovarian cancer or breast cancer.

In one aspect, the present invention provides methods of treating or preventing a disease by providing to the subject in need thereof immune effector cells (e.g., T cells, NK cells) or stem cells that can give rise to immune effector cells that are engineered to express FHR-specific conventional CAR or backbones 1-62 (for example, backbone-1, backbone-2, backbone-32 or backbone-33), wherein the disease causing or disease associated cells express FHR (Follicular Hormone Receptor). In one embodiment, the disease to be treated or prevented is a cancer. In one embodiment, the cancer to be treated or prevented is prostate cancer, ovarian cancer or breast cancer.

In one aspect, the present invention provides methods of treating or preventing a disease by providing to the subject in need thereof immune effector cells (e.g., T cells, NK cells) or stem cells that can give rise to immune effector cells that are engineered to express GR-specific conventional CAR or backbones 1-62 (for example, backbone-1, backbone-2, backbone-32 or backbone-33), wherein the disease causing or disease associated cells express GR (Gonadotropin Receptor). In one embodiment, the disease to be treated or prevented is a cancer. In one embodiment, the cancer to be treated or prevented is prostate cancer, ovarian cancer or breast cancer.

In one aspect, the present invention provides methods of treating or preventing a disease by providing to the subject in need thereof immune effector cells (e.g., T cells, NK cells) or stem cells that can give rise to immune effector cells that are engineered to express CD45-specific conventional CAR or backbones 1-62 (for example, backbone-1, backbone-2, backbone-32 or backbone-33), wherein the disease causing or disease associated cells express CD45. In one embodiment, the disease to be treated or prevented is a cancer. In one embodiment, the cancer to be treated or prevented is a blood cancer.

In one aspect, the present invention provides methods of treating or preventing a disease by providing to the subject in need thereof immune effector cells (e.g., T cells, NK cells) or stem cells that can give rise to immune effector cells that are engineered to express CD32-specific conventional CAR or backbones 1-62 (for example, backbone-1, backbone-2, backbone-32 or backbone-33), wherein the disease causing or disease associated cells express CD32. In one embodiment, the disease to be treated or prevented is a cancer. In one embodiment, the cancer to be treated or prevented is a blood cancer.

In one aspect, the present invention provides methods of treating or preventing a disease by providing to the subject in need thereof immune effector cells (e.g., T cells, NK cells) or stem cells that can give rise to immune effector cells that are engineered to express TROP2-specific conventional CAR or backbones 1-62 (for example, backbone-1, backbone-2, backbone-32 or backbone-33), wherein the disease causing or disease associated cells express TROP2. In one embodiment, the disease to be treated or prevented is a cancer. In one embodiment, the cancer to be treated or prevented is breast or prostate cancer.

In one aspect, the present invention provides methods of treating or preventing a disease by providing to the subject in need thereof immune effector cells (e.g., T cells, NK cells) or stem cells that can give rise to immune effector cells that are engineered to express LAMP-specific conventional CAR or backbones 1-62 (for example, backbone-1, backbone-2, backbone-32 or backbone-33), wherein the disease causing or disease associated cells express LAMP1. In one embodiment, the disease to be treated or prevented is a cancer. In one embodiment, the cancer to be treated or prevented is a leukemia, lymphoma or myeloma.

In one aspect, the present invention provides methods of treating or preventing a disease by providing to the subject in need thereof immune effector cells (e.g., T cells, NK cells) or stem cells that can give rise to immune effector cells that are engineered to express CD32-specific conventional CAR or backbones 1-62 (for example, backbone-1, backbone-2, backbone-32 or backbone-33), wherein the disease causing or disease associated cells express CD32. In one embodiment, the disease to be treated or prevented is a cancer.

In one aspect, the present invention provides methods of treating or preventing a disease by providing to the subject in need thereof immune effector cells (e.g., T cells, NK cells) or stem cells that can give rise to immune effector cells that are engineered to express TCRB1-specific conventional CAR or backbones 1-62 (for example, backbone-1, backbone-2, backbone-32 or backbone-33), wherein the disease causing or disease associated cells express TCRB1 (T cell receptor Beta1 constant chain). In one embodiment, the disease to be treated or prevented is a cancer or immune disease. In one embodiment, the cancer to be treated or prevented is T cell leukemia or T cell lymphoma. In one embodiment, the immune disorder to be treated or prevented is multiple sclerosis, rheumatoid arthritis, ankylosing spondylitis, inflammatory Bowel Disease, Diabetes Mellitus, Graft vs host disease or autoimmune Thyroiditis.

In one aspect, the present invention provides methods of treating or preventing a disease by providing to the subject in need thereof immune effector cells (e.g., T cells, NK cells) or stem cells that can give rise to immune effector cells that are engineered to express TCRB2-specific conventional CAR or backbones 1-62 (for example, backbone-1, backbone-2, backbone-32 or backbone-33), wherein the disease causing or disease associated cells express TCRB2 (T cell receptor Beta2 constant chain). In one embodiment, the disease to be treated or prevented is a cancer or immune disorder. In one embodiment, the cancer to be treated or prevented is T cell leukemia or T cell lymphoma. In one embodiment, the immune disorder to be treated or prevented is multiple sclerosis, rheumatoid arthritis, ankylosing spondylitis, inflammatory Bowel Disease, Diabetes Mellitus, Graft vs host disease or autoimmune Thyroiditis.

In one aspect, the present invention provides methods of treating or preventing a disease by providing to the subject in need thereof immune effector cells (e.g., T cells, NK cells) or stem cells that can give rise to immune effector cells that are engineered to express T cell receptor gamma-delta-specific conventional CAR or backbones 1-62 (for example, backbone-1, backbone-2, backbone-32 or backbone-33), wherein the disease causing or disease associated cells express T cell receptor gamma-delta. In one embodiment, the disease to be treated or prevented is a cancer or immune disorder. In one embodiment, the cancer to be treated or prevented is T cell leukemia or T cell lymphoma. In one embodiment, the immune disorder to be treated or prevented is multiple sclerosis, rheumatoid arthritis, ankylosing spondylitis, inflammatory bowel disease, diabetes mellitus, Graft vs host disease or autoimmune Thyroiditis.

In one aspect, the present invention provides methods of treating or preventing a disease by providing to the subject in need thereof immune effector cells (e.g., T cells, NK cells) or stem cells that can give rise to immune effector cells that are engineered to express HLA-A2-specific conventional CAR or backbones 1-62 (for example, backbone-1, backbone-2, backbone-32 or backbone-33). In one embodiment, the disease to be treated or prevented is Graft vs Host disease.

In one aspect, the present invention provides methods of treating or preventing a disease by providing to the subject in need thereof immune effector cells (e.g., T cells, NK cells) or stem cells that can give rise to immune effector cells that are engineered to express HIV1-CAR on backbones 1-62 (for example, backbone-1, backbone-2, backbone-32 or backbone-33) directed against the HIV envelop glycoprotein gp120, wherein the disease causing or disease associated cells express the HIV1 envelop glycoprotein gp120. In one embodiment, the disease to be treated or prevented is HIV1/AIDS.

In one aspect, the present invention provides methods of treating or preventing CMV (Cytomegalovirus) infection or associated disease by providing to the subject in need thereof immune effector cells (e.g., T cells, NK cells) or stem cells that can give rise to immune effector cells that are engineered to express CMV-specific CAR or backbones 1-62 (for example, backbone-1, backbone-2, backbone-32 or backbone-33), wherein the disease causing or disease associated cells are infected with CMV. In one aspect the CMV CAR and backbones are directed against the CMV pp65 protein. In one aspect, the CMV-associated disease is pneumonia, colitis or meningoencephalitis.

In one aspect, the present invention provides methods of treating or preventing EBB infection or diseases associated with EBB infection by providing to the subject in need thereof immune effector cells (e.g., T cells, NK cells) or stem cells that can give rise to immune effector cells that are engineered to express an EBB-specific conventional CAR or backbones 1-62 (for example, backbone-1, backbone-2, backbone-32 or backbone-33), wherein the disease causing or disease associated cells are infected with EBB. In one aspect the EBB-specific CARs and backbones are directed against the EBB EBNA3c protein. In one aspect, the EBB-specific CARs and backbones comprise a broadly neutralizing antibody against EBB. In one aspect, the EBB-associated disease is lymphoma.

In one aspect, the present invention provides methods of treating or preventing KSHV infection or diseases associated with KSHV infection by providing to the subject in need thereof immune effector cells (e.g., T cells, NK cells) or stem cells that can give rise to immune effector cells that are engineered to express an KSHV-specific conventional CAR or backbones 1-62 (for example, backbone-1, backbone-2, backbone-32 or backbone-33), wherein the disease causing or disease associated cells are infected with KSHV. In one aspect, the KSHV associated disease is Kaposi's sarcoma or primary effusion lymphoma or multicentric castleman's disease.

In one aspect, the present invention provides methods of treating or preventing HTLV1 infection or diseases associated with HTLV1 infection by providing to the subject in need thereof immune effector cells (e.g., T cells, NK cells) or stem cells that can give rise to immune effector cells that are engineered to express HTLV1-specific conventional CAR or backbones 1-62 (for example, backbone-1, backbone-2, backbone-32 or backbone-33), wherein the disease causing or disease associated cells are infected with HTLV1. In one aspect, the HTLV1-specific conventional CAR or backbones target HTLV1 Tax protein/MHC class I complex. In one aspect, the HTLV1 disease is human T cell leukemia or lymphoma.

In one aspect, the present invention provides methods of treating or preventing Influenza A infection or diseases associated with Influenza A infection by providing to the subject in need thereof immune effector cells (e.g., T cells, NK cells) or stem cells that can give rise to immune effector cells that are engineered to express InfluenzaA-specific conventional CAR or backbones 1-62 (for example, backbone-1, backbone-2, backbone-32 or backbone-33), wherein the disease causing or disease associated cells are infected with Influenza A. In one aspect, the InfluenzaA-specific conventional CAR or backbones comprise antigen binding domains derived from a broadly neutralizing antibody against Influenza A hemagglutinin (HA), such as MEDI8852.

In one aspect, the present invention provides methods of treating or preventing a cancer, infection, autoimmune or allergic diseases by providing to the subject in need thereof immune effector cells (e.g., T cells, NK cells) or stem cells that can give rise to immune effector cells that are engineered to express a universal conventional CAR or backbones 1-62 (for example, backbone-1, backbone-2, backbone-32 or backbone-33) encoding CD16V158 or a deletion- or point-mutant fragment thereof along with an antibody or an antibody fragment that binds to the CD16 domain of the CAR through its Fc region and on an antigen expressed on the disease associated cells through its variable (vL and/or vH) regions. In one aspect the disease associated cell is a cancer cell, an infected cell, or a plasma cell or a B cell or a T cell.

In one aspect, the present invention provides methods of treating or preventing a cancer, infection, autoimmune or allergic diseases by providing to the subject in need thereof immune effector cells (e.g., T cells, NK cells) or stem cells that can give rise to immune effector cells that are engineered to express a FITC-specific conventional CAR or backbones 1-62 (for example, backbone-1, backbone-2, backbone-32 or backbone-33) along with a FITC-labeled antibody or an antibody fragment that binds to an antigen expressed on the disease associated cells. In one aspect the disease associated cell is a cancer cell, an infected cell, or a plasma cell or a B cell or a T cell.

Exemplary cancers whose growth can be inhibited include cancers typically responsive to immunotherapy. Non-limiting examples of cancers for treatment include melanoma (e.g., metastatic malignant melanoma), renal cancer (e.g. clear cell carcinoma), prostate cancer (e.g. hormone refractory prostate adenocarcinoma), breast cancer, colon cancer and lung cancer (e.g. non-small cell lung cancer). Additionally, refractory or recurrent malignancies can be treated using the molecules described herein.

In exemplary embodiments, cancers treated by the methods described herein include solid tumors such as sarcomas, adenocarcinomas, and carcinomas, of the various organ systems, such as those affecting liver, lung, breast, lymphoid, gastrointestinal (e.g., colon). genitourinary tract (e.g , renal, urothelial cells), prostate and pharynx. Adenocarcinomas include malignancies such as most colon cancers, rectal cancer, renal-cell carcinoma, liver cancer, non-small cell carcinoma of the lung, cancer of the small intestine and cancer of the esophagus. In one embodiment, the cancer is a melanoma, e.g., an advanced stage melanoma. Metastatic lesions of the aforementioned cancers can also be treated or prevented using the methods and compositions of the invention.

Examples of other cancers that can be treated include bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin Disease, non-Hodgkin lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, and combinations of said cancers. Treatment of metastatic cancers, e.g., metastatic cancers that express PD-L1 (Iwai et al. (2005) Int. Immunol. 17:133-144) can be effected using the antibody molecules described herein. Further a disease associated with a cancer associate antigen as described herein expression include, but not limited to, e.g., atypical and/or non-classical cancers, malignancies, precancerous conditions or proliferative diseases associated with expression of a cancer associate antigen as described herein. In some embodiments, a CAR-expressing T cell or NK cell as described herein reduces the quantity, number, amount or percentage of cells and/or cancer cells by at least 25%, at least 30%, at least 40%, at least 50%, at least 65%, at least 75%, at least 85%, at least 95%, or at least 99% in a subject with hematological cancer or another cancer associated with a cancer associated antigen as described herein, expressing cells relative to a negative control. In one embodiment, the subject is a human.

In one embodiment, the present invention provides methods of treating hematological cancer by providing to the subject in need thereof genetically modified cells (e.g., T cells, NK cells) that are engineered to express the conventional CARs or backbones comprising antigen specific CARs specific to thrombopoietin receptor (MPL). In some embodiments, the hematological cancers include chronic myelogenous leukemia (CML), Acute Myeloid Leukemia and Myelodysplastic syndrome. In some embodiments, a cancer that can be treated using the compositions and methods of the present invention is multiple myeloma. Multiple myeloma is a cancer of the blood, characterized by accumulation of a plasma cell clone in the bone marrow. Current therapies for multiple myeloma include, but are not limited to, treatment with lenalidomide, which is an analog of thalidomide. Lenalidomide has activities which include anti-tumor activity, angiogenesis inhibition, and immunomodulation. Generally, myeloma cells are thought to be negative for a cancer associate antigen as described herein expression by flow cytometry. Thus, in some embodiments, a BCMA CAR (SEQ ID Nos: 951-957), may be used to target myeloma cells In some embodiments, the CARs of the present invention therapy can be used in combination with one or more additional therapies, e.g., lenalidomide treatment.

In one aspect, the invention pertains to a method of inhibiting growth of a disease (e.g., cancer, autoimmune disease, infectious disease or allergic disease or a degenerative disease), comprising contacting the disease causing or disease associated cell with a genetically modified cell of the present invention expressing a conventional CAR or a CAR with accessory modules (i.e., backbones 1-62) such that the CAR-T is activated in response to the antigen and targets the disease causing or disease associated cell, wherein the growth of the disease causing or disease associated cell is inhibited In one aspect, the invention pertains to a method of preventing a disease, comprising administering to a patient at risk of disease a CAR-expressing cell or a cell that is capable of generating a CAR-expressing cell of the present invention such that the CAR-T is activated in response to the antigen and targets the disease causing or disease associated cell, wherein the growth of the disease causing or disease associated cell is prevented. In one aspect the disease is a cancer, an infectious disease, an immune disease, an allergic disease, or a degenerative disease.

Embodiments of the instant invention includes a type of cellular therapy where effector cells (such as T cells and NK cells) or stem cells that can give rise to effector cells are genetically modified to express a CAR as described herein and the CAR-expressing T cell or NK cell is infused to a recipient in need thereof. The infused cell is able to kill tumor cells in the recipient. In various aspects, the immune effector cells (e.g., T cells, NK cells) administered to the patient, or their progeny, persist in the patient for at least four months, five months, six months, seven months, eight months, nine months, ten months, eleven months, twelve months, thirteen months, fourteen month, fifteen months, sixteen months, seventeen months, eighteen months, nineteen months, twenty months, twenty-one months, twenty-two months, twenty- three months, two years, three years, four years, or five years after administration of the T cell or NK cell to the patient.

The invention also includes a type of cellular therapy where immune effector cells (e.g., T cells, NK cells) are modified, e.g., by in vitro transcribed RNA, to transiently express a conventional CAR or a conventional CAR with accessory modules (e.g., backbones 1-62). T cells or NK cells are infused to a recipient in need thereof The infused cells are able to kill disease associated cells (e.g., tumor cells or virally infected cells) in the recipient. Thus, in various aspects, the CAR-expressing immune effector cells (e.g., T cells, NK cells) persist for less than one month, e.g., three weeks, two weeks, one week, after administration of the T cell or NK cell to the patient.

The invention also includes a type of cellular therapy where stem cells (e.g., hematopoietic stem cell or lymphoid stem cells or embryonic stem cells, or induced pluripotent stem cells) that are capable of giving rise to immune effector cells (e.g., T cells or NK cells) are modified to express a conventional CAR or a conventional CAR with accessory modules (e.g., backbones 1-62) and are administered to a recipient in need thereof. The administered stem cells give rise to immune effector cells (e.g., T cells or NK cells) after transplantation into the recipient, which (i.e. the immune effector cells) are able to kill disease associated cells in the recipient. Thus, in various aspects, the immune effector cells (e.g., T cells, NK cells) that are produced in the patient after administration of CAR-expressing stem cells persist in the patient for at least one week, 2 weeks, 3 weeks, one month, two months, three months, four months, five months, six months, seven months, eight months, nine months, ten months, eleven months, twelve months, thirteen months, fourteen month, fifteen months, sixteen months, seventeen months, eighteen months, nineteen months, twenty months, twenty-one months, twenty-two months, twenty three months, two years, three years, four years, five years, ten years or twenty years after administration of the genetically modified stem cells to the patient. The invention also includes a type of cellular therapy where stem cells that are capable of giving rise to immune effector cells (e.g., T cells or NK cells) are modified to express a conventional CAR or a conventional CAR with accessory modules (e.g. backbones 1-62) and are differentiated in vitro to generate immune effector cells that are infused to a recipient in need thereof. The infused immune effector cells (e.g., T cells or NK cells) after infusion into the recipient are able to kill disease associated cells in the recipient. Thus, in various aspects, the immune effector cells (e.g., T cells, NK cells) that are administered to the patient persist in the patient for at least 1 day, 2 days, 3 days, 4 days, 5 days, 6 days, one week, 2 weeks, 3 weeks, one month, two months, three months, four months, five months, six months, seven months, eight months, nine months, ten months, eleven months, twelve months, thirteen months, fourteen month, fifteen months, sixteen months, seventeen months, eighteen months, nineteen months, twenty months, twenty-one months, twenty-two months, twenty three months, two years, three years, four years, five years, ten years or twenty years.

The invention also includes a type of cellular therapy where regulatory immune effector cells (e.g., TREG, or CD25+ T Cells) are modified to express a conventional CAR or a conventional CAR with accessory modules (e.g., backbones 1-62) targeting a specific antigen. Such CAR-TREG are administered to a patient to suppress immune response against the specific antigen. The CAR-TREG can be used to prevent and treat autoimmune diseases and to enhance immune tolerance.

The anti-tumor immunity response elicited by the CAR-modified immune effector cells (e.g., T cells, NK cells) may be an active or a passive immune response, or alternatively may be due to a direct vs indirect immune response. In one aspect, the CAR-transduced immune effector cells (e.g., T cells, NK cells) exhibit specific pro-inflammatory cytokine secretion and potent cytolytic activity in response to human diseased cells (e.g., cancer or infected cells) expressing the disease associate antigen as described herein, resist soluble disease associate antigen as described herein, mediate bystander killing and mediate regression of an established human disease, including cancer.

The invention also includes a type of cellular therapy where immune effector cells (e.g, T cells and NK cells) or stem cells that are capable of giving rise to immune effector cells (e.g., T cells or NK cells) are modified to express a conventional CAR or a conventional CAR with accessory modules (e g., backbones 1-62) and are used ex vivo to purge the bone marrow or peripheral blood hematopoietic stem cells of disease-associated cells (e.g. cancer cells). As an example, T cells expressing a CD19-specific CAR are cocultured with bone marrow or peripheral blood stem cell sample taken from a patient with acute lymphocytic leukemia or non-Hodgkin lymphoma so as to kill off any leukemia or lymphoma cells present in the bone marrow or peripheral blood stem cell preparation After a suitable duration of culture in vitro (ex vivo), which may range from a 6 hours to several days, the purged bone marrow and peripheral blood sample is used for autologous transplant in the patient.

Ex vivo expansion of hematopoietic stem and progenitor cells has been described in U.S. Pat. No. 5,199,942, and is incorporated herein by reference, and can be applied to the cells of the present invention. However, the present invention is not limited to any particular method of ex vivo expansion of the cells and other suitable methods known in the art can be utilized. Briefly, ex vivo culture and expansion of hematopoietic stem cells comprises: (1) collecting CD34+ hematopoietic stem and progenitor cells from a mammal from peripheral blood harvest or bone marrow explants; and (2) expanding such cells ex vivo. In addition to the cellular growth factors described in U.S. Pat. No. 5,199,942, other factors such as flt3-L, IL-1, IL-3 and c-kit ligand, can be used for culturing and expansion of the cells.

In addition to using a cell-based vaccine in terms of ex vivo immunization, the present invention also provides compositions and methods for in vivo immunization to elicit an immune response directed against an antigen in a patient.

In some embodiments, the fully-human CAR-modified genetically modified cells (such as T cells, NK cells) of the invention may be a type of vaccine for ex vivo immunization and/or in vivo therapy in a mammal (for example, human). With respect to ex vivo immunization, at least one of the following occurs in vitro prior to administering the cell into a mammal: i) expansion of the cells, ii) introducing a nucleic acid encoding a CAR to the cells or iii) cryopreservation of the cells. Ex vivo procedures are well known in the art, for example, as described in U.S. Pat. No. 5,199,942, incorporated herein by reference.

In addition to using a cell-based vaccine in terms of ex vivo immunization, the present invention also provides compositions and methods for in vivo immunization to elicit an immune response directed against an antigen in a patient.

Further described herein are methods for controlling the activity of CAR-T cells and Bispecific T cell Engager (BiTE) (such as Blincyto or Blinatumomab) when administered to the patients. In some embodiment these methods can be used to control the side effects of CAR-T cells and BiTE, such as cytokine release syndrome, capillary leak syndrome and neurological complications. In majority of patients who respond to engineered CAR T cells and Blinatumomab, excessive release of proinflammatory cytokines causes symptoms that include fevers, hypotension, hypoxemia, cardiac dysfunction, kidney failure and electrolyte abnormalities, collectively termed as "Cytokine release syndrome' (CRS). A number of these patients require admission to intensive care units for pressure support and artificial ventilation. In significant number of cases, CAR T cells and Blinatumomab therapy can lead to neurologic symptoms including tremor, seizures and can be fatal. Although a number of treatment approaches, including steroids and an IL6 receptor antibody, are used for the treatment of these disorders, a number of patients are refractory to such therapies. The instant invention provides a novel method for the treatment and prevention of complications, such as cytokine release syndrome and neurological complications, of adoptive cellular therapies, including but not limited to CAR-T cell and Bispecific T cell engagers (BiTE). In some embodiment the method involves administration of inhibitors of tyrosine kinases, particularly Scr family kinase, and in particular Lck kinase. In one embodiment, the method involves administration of Dasatinib, an oral small molecule inhibitor of Abl and Src family tyrosine kinases (SFK), including p56Lck (Lck) (Lee KC et al, Leukemia (2010) 24, 896-900). Dasatinib has been previously shown to block antigen-specific effector T cell functions (Weichsel R et al, Clin Cancer Res 2008;14(8), 2484). However, it has never been tested for the ability to block functions of CAR-T cells and Bispecific T cell engagers. CAR-expressing T cells have been detected in the cerebrospinal fluid (CSF) of patients suffering from neurological complications (Hu, Y et al, Journal of Hematology & Oncology20169:70). A particularly important advantage of Dasatinib for the treatment and prevention of cytokine release syndrome and neurological complications caused by CAR-T cells and BiTE is its ability to cross the blood-brain barrier and achieve effective concentration in the brain when administered at clinically acceptable doses (Porkka K et al., Blood. 2008 Aug 15;112(4):1005-12). Therefore, Dasatinib is expected to block the effector functions, including cytokine release, of CAR-T cells that have crossed the blood brain barrier.

In one embodiment, the Src kinase inhibitor is administered to the patient after the administration of CAR-expressing cells to control or terminate the activity of CAR-expressing cells. In one embodiment, an Lck inhibitor is administered to the patient after the administration of CAR-expressing cells to control or terminate the activity of CAR-expressing cells. In one embodiment, Lck inhibitor is A-770041.

In one embodiment, Dasatinib is administered to the patient after the administration of CAR-expressing cells to control or terminate the activity of CAR-expressing cells. In one embodiment, dasatinib is administered orally at a dose of at least 10 mg/day, 20 mg/day, 40mg/day, 60mg/day, 70mg/day, 90 mg/day, 100mg/day, 140mg/day, 180mg/day, 210mg/day, 250mg/day or 280mg/day.

In one embodiment, Ponatinib is administered to the patient after the administration of CAR-expressing cells to control or terminate the activity of CAR-expressing cells. In one embodiment, ponatinib is administered orally at a dose of at least 15 mg/day, 30 mg/day, 45mg/day, 60mg/day.

T lymphocytes have a limited replicative life span until they reach the terminally differentiated state and then enter into a replicative senescence phase due to progressive loss of telomeres with age. Human T lymphocytes display a limited life-span of about 30-50 population doublings when cultured in vitro.

Further contemplated herein are methods for promoting the survival and proliferation of peripheral blood mononuclear cells and T cells and preventing their replicative senescence to extend the life span of immune cells (e.g., lymphocytes and NK cells) for the purpose of adoptive cell therapy. The method entails ectopic expression of viral and cellular proteins that promote survival and proliferation and block activation induced cell death. The exemplary viral proteins suitable for this purpose include viral FLICE Inhibitory Protein (vFLIP) K13 encoded by the Kaposi's sarcoma associated herpesvirus (also known as human herpesvirus 8), MC159-vFLIP and MC160 vFLIP from the molluscum contagiosum virus (MCV), E8-vFLIP from the equine herpesvirus 2 (EHV2), bovine herpesvirus 4 encoded vFLIP (BHV-vFLIP), vFLIP encoded by herpesvirus mecaca, vFLIP encoded by herpesvirus saimiri and HTLV1-encoded Tax, HTLV2-encoded Tax and HTLV2-encoded Tax-RS mutant. Exemplary cellular proteins suitable for this purpose include cFLIP-L isoform or MRIT-α, cFLIP-p22 deletion mutant or MRIT-p22 (Bertin et al., Proc Natl Acad Sci U S A 94:1172-6, 1997; Hu et al., J Biol Chem 272:9621-4, 1997; Thome et al., Nature 386:517-21, 1997). In some embodiments, the above viral and cellular proteins are expressed in the immune cells (e.g., T cells and NK cells) in their native state or carrying small epitope tags and are functionally active in a constitutive manner. In other embodiments, the above viral and cellular proteins are expressed in the immune cells (e.g., T cells and NK cells) in fusion with one or more copies of a switch domain (or a dimerization domain), such as FKBP (SEQ ID NOs: 2620 and 2621) and FKBPx2 (SEQ ID NO: 2622). In other embodiment, the FKBP or the FKBP-x2 domain may additionally carry an N-terminal myrisotylation (Myr) sequence to anchor the fusion proteins to the cell membrane. The sequence of an exemplary FKBPx2-K13 fusion protein carrying a Myr sequence is presented in SEQ ID NO: 2623. The fusion proteins carrying the switch domains are functionally inactive in their basal state but are activated upon addition of a dimerizer agent, such as AP20187.

In an embodiment, an immune effector cell, e.g., a T cell, ectopically expresses a viral or cellular signaling protein selected from the group of K13-vFLIP (SEQ ID NO: 2629), MC159-vFLIP (SEQ ID NO: 2630), MC160-vFLIP (SEQ ID NO: 2631), E8-vFLIP (SEQ ID NO: 2632), BHV-vFLIP (SEQ ID NO: 2635), mecaca vFLIP (SEQ ID NO: 2634), herpesvirus saimiri vFLIP (SEQ ID NO: 2633), cFLIP-L/MRITα (SEQ ID NO: 2636), cFLIP-p22 (SEQ ID NO: 2637), HTLV1-Tax (SEQ ID NO: 2638), HTLV2-Tax (SEQ ID NO: 2639), HTLV2-Tax-RS mutants (SEQ ID NO: 2640) or proteins with 70-99% identity to amino acid sequences of the above proteins (SEQ ID NOs: 2629 to 2640).

In an embodiment, an immune effector cell, e.g., a T cell, ectopically expresses a fusion protein containing one or more switch domains, e.g., FKBP (SEQ ID NO: 2620-2621), FKBPx2 (SEQ ID NO: 2622) or Myr-FKBP, and one or more viral or cellular signaling protein selected from the group of K13-vFLIP (SEQ ID NO: 2629), MC159-vFLIP (SEQ ID NO: 2630), MC160-vFLIP (SEQ ID NO: 2631), E8-vFLIP (SEQ ID NO: 2632), BHV-vFLIP (SEQ ID NO: 2635), mecaca vFLIP (SEQ ID NO: 2634), herpesvirus saimiri vFLIP (SEQ ID NO: 2633), cFLIP-L/MRITα (SEQ ID NO: 2636), cFLIP-p22 (SEQ ID NO: 2637), HTLV1-Tax (SEQ ID NO: 2638), HTLV2-Tax (SEQ ID NO: 2639), HTLV2-Tax-RS mutants (SEQ ID NO: 2640) or proteins with 70-99% identity to amino acid sequences of SEQ ID NOs: 2629 to 2640. Exemplary fusion proteins containing one or more switch domains and viral signaling proteins are represented by SEQ ID NOs: 2641-2645.

In some aspects, this disclosure provides a method of producing an immune effector cell suitable for adoptive cellular therapy, comprising contacting the cell with a nucleic acid encoding one or more of a viral or cellular signaling protein selected from the group of K13-vFLIP (SEQ ID NO: 2629), MC159-vFLIP (SEQ ID NO: 2630), MC160-vFLIP (SEQ ID NO: 2631), E8-vFLIP (SEQ ID NO: 2632), BHV-vFLIP (SEQ ID NO: 2635), mecaca vFLIP (SEQ ID NO: 2634), herpesvirus saimiri vFLIP (SEQ ID NO: 2633), cFLIP-L/MRITα (SEQ ID NO: 2636), cFLIP-p22 (SEQ ID NO: 2637), HTLV1-Tax (SEQ ID NO: 2638), HTLV2-Tax (SEQ ID NO: 2639), HTLV2-Tax-RS mutants (SEQ ID NO: 2640) or proteins with 70-99% identity to amino acid sequences of SEQ ID NOs: 2629 to 2640.

In some aspects, this disclosure provides a method of producing an immune effector cell suitable for adoptive cellular therapy, comprising contacting the cell with a nucleic acid encoding expresses a fusion protein containing one or more switch domains, e.g., FKBP (SEQ ID NO: 2620-2621), FKBPx2 (SEQ ID NO: 2622) or Myr-FKBP, and one or more viral or cellular signaling protein selected from the group of K13-vFLIP (SEQ ID NO: 2629), MC159-vFLIP (SEQ ID NO: 2630), MC160-vFLIP (SEQ ID NO: 2631), E8-vFLIP (SEQ ID NO: 2632), BHV-vFLIP (SEQ ID NO: 2635), mecaca vFLIP (SEQ ID NO: 2634), herpesvirus saimiri vFLIP (SEQ ID NO: 2633), cFLIP-L/MRITα (SEQ ID NO: 2636), cFLIP-p22 (SEQ ID NO: 2637), HTLV1-Tax (SEQ ID NO: 2638), HTLV2-Tax (SEQ ID NO: 2639), HTLV2-Tax-RS mutants (SEQ ID NO: 2640) or proteins with 70-99% identity to amino acid sequences of SEQ ID NOs: 2629 to 2640.

In an embodiment, the cell suitable for adoptive cell therapy expresses a natural or synthetic immune receptor. Exemplary such immune receptors include a chimeric antigen receptor (CAR), a T cell receptor (TCR), a chimeric T cell receptor (cTCR), a synthetic T cell receptor and a synthetic notch receptor. In an embodiment, the cell may be contacted with the nucleic acid encoding the viral and cellular signaling proteins before, simultaneous with, or after being contacted with a construct encoding a natural or synthetic immune receptor. In an embodiment, the cell may be contacted with the nucleic acid encoding the viral and cellular signaling proteins containing a switch or dimerization domain before, simultaneous with, or after being contacted with a construct encoding a natural or synthetic immune receptor.

In one aspect, the disclosure features a method of making a population of immune effector cells (e.g., T cells, NK cells). In an embodiment, the method comprises: providing a population of immune effector cells (e.g., T cells or NK cells), contacting the population of immune effector cells with a nucleic acid encoding an immune receptor (e.g., CAR, TCR, synthetic TCR) and contacting the population of immune effector cells with a nucleic acid encoding a viral or cellular signaling protein of SEQ ID NOs: 2629-2645, under conditions that allow for immune receptor and viral or cellular signaling protein co-expression.

In an embodiment, the nucleic acid encoding the viral or cellular signaling protein of SEQ ID NOs: 2629-2645 is DNA. In an embodiment, the nucleic acid encoding the viral or cellular signaling protein of SEQ ID NOs: 2629-2645 contains promoter capable of driving expression of the viral and cellular signaling proteins. In an embodiment, the nucleic acid encoding the viral or cellular signaling protein of SEQ ID NOs: 2629-2645 and the nucleic acid encoding the immune receptor is expressed from the same vector. In an embodiment, the nucleic acid encoding the viral or cellular signaling protein of SEQ ID NOs: 2629-2645 and the nucleic acid encoding the immune receptor is expressed from separate vectors. In an embodiment, the nucleic acid encoding the viral or cellular signaling protein of SEQ ID NOs: 2629-2645 (subunit 1) and the nucleic acid encoding the immune receptor (subunit 2) is expressed from the same polynucleotide fragment containing an internal ribosomal entry site (IRES) that allows the translation of the second subunit. In an embodiment, the nucleic acid encoding the viral or cellular signaling protein of SEQ ID NOs: 2629-2645 (subunit 1) and the nucleic acid encoding the immune receptor (subunit 2) is expressed from a single polynucleotide fragment that encodes and the different subunits are separated by a cleavable linker (e.g., SEQ ID NOs: 831-836).

In an embodiment, the nucleic acid encoding the viral or cellular signaling protein of SEQ ID NOs: 2629-2645 is an in vitro transcribed RNA. In an embodiment, the viral or cellular signaling protein of SEQ ID NOs: 2629-2645 (subunit 1) and the immune receptor (subunit 2) is expressed from the same RNA containing an internal ribosomal entry site (IRES) that allows the translation of the second subunit. In an embodiment, the viral or cellular signaling protein of SEQ ID NOs: 2629-2645 (subunit 1) and the immune receptor (subunit 2) is expressed from a single RNA and the different subunits are separated by cleavable linkers (e.g., SEQ ID NOs: 831-836).

### Combination therapies

Therapeutic methods described herein comprise using compositions comprising genetically modified cells comprising nucleic acids encoding CARs described herein. In various embodiments, the therapeutic methods described herein may be combined with existing therapies and agents. The therapeutic compositions described herein, comprising genetically modified cells comprising nucleic acids encoding the CARs described herein, are administered to the subject with at least one additional known therapy or therapeutic agent. In some embodiments, the compositions described herein and the additional therapy or therapeutic agents are administered sequentially. In some embodiments, the compositions described herein and the additional therapy or therapeutic agents are administered simultaneously. The optimum order of administering the compositions described herein and the existing therapies will be apparent to a person of skill in the art, such as a physician.

A CAR-expressing cell described herein and the at least one additional therapeutic agent can be administered simultaneously, in the same or in separate compositions, or sequentially. For sequential administration, the CAR-expressing cell described herein can be administered first, and the additional agent can be administered second, or the order of administration can be reversed.

Combinations therapies may be administered to the subject over the duration of the disease. Duration of the disease includes from diagnosis until conclusion of treatment, wherein the treatment results in reduction of symptoms and/or elimination of symptoms. In various embodiments, the effect of the two treatments can be partially additive, wholly additive, or greater than additive. The delivery can be such that an effect of the first treatment delivered is still detectable when the second is delivered.

The CAR therapy and/or other therapeutic agents, procedures or modalities can be administered during periods of active disorder, or during a period of remission or less active disease. The CAR therapy can be administered before the other treatment, concurrently with the treatment, post-treatment, or during remission of the disorder.

When administered in combination, the CAR therapy and the additional agent (e.g., second or third agent), or all, can be administered in an amount or dose that is higher, lower or the same than the amount or dosage of each agent used individually, e.g., as a monotherapy. In certain embodiments, the administered amount or dosage of the CAR therapy, the additional agent (e.g., second or third agent), or all, is lower (e.g., at least 20%, at least 30%, at least 40%, or at least 50%) than the amount or dosage of each agent used individually, e.g., as a monotherapy. In other embodiments, the amount or dosage of the CAR therapy, the additional agent (e.g., second or third agent), or all, that results in a desired effect (e.g., treatment of cancer) is lower (e.g., at least 20%, at least 30%, at least 40%, or at least 50% lower) than the amount or dosage of each agent used individually, e.g., as a monotherapy, required to achieve the same therapeutic effect.

Further method aspects relate administering to the subject an effective amount of a cell, e.g., an immune effector cell, or a population thereof, each cell comprising a CAR molecule, optionally in combination with an agent that increases the efficacy and/or safety of the immune cell. In further aspects, the agent that increases the efficacy and/or safety of the immune cell is one or more of: (i) a protein phosphatase inhibitor; (ii) a kinase inhibitor; (iii) a cytokine; (iv) an inhibitor of an immune inhibitory molecule; or (v) an agent that decreases the level or activity of a TREG cell; vi) an agent that increase the proliferation and/or persistence of CAR-modified cells vii) a chemokine viii) an agent that increases the expression of CAR ix) an agent that allows regulation of the expression or activity of CAR x) an agent that allows control over the survival and/or persistence of CAR-modified cells xi) an agent that controls the side effects of CAR-modified cells xii) a Brd4 inhibitor xiii) an agent that delivers a therapeutic (e.g. sHVEM) or prophylactic agent to the site of the disease xiv) an agent that increases the expression of the target antigen against which CAR is directed; xv) an adenosine A2a receptor antagonist

In some embodiments, a the genetically modified cells described herein may be used in a treatment regimen in combination with surgery, chemotherapy, radiation, immunosuppressive agents, such as cyclosporin, azathioprine, methotrexate, mycophenolate, and FK506, antibodies, or other immunoablative agents such as CAMPATH, anti-CD3 antibodies or other antibody therapies, cytoxin, fludarabine, cyclosporin, FK506, rapamycin, mycophenolic acid, steroids, FR901228, cytokines, and irradiation, peptide vaccine, such as that described in Izumoto et al. 2008 J Neurosurg 108:963-971. In one embodiment, a CAR-expressing cell described herein can be used in combination with a chemotherapeutic agent Exemplary chemotherapeutic agents include an anthracycline (e.g., doxorubicin (e.g., liposomal doxorubicin)), a vinca alkaloid (e.g., vinblastine, vincristine, vindesine, vinorelbine), an alkylating agent (e.g, cyclophosphamide, decarbazine, melphalan, ifosfamide, temozolomide), an immune cell antibody (e.g., alemtuzamab, gemtuzumab, rituximab, ofatumumab, tositumomab, brentuximab), an antimetabolite (including, e.g., folic acid antagonists, pyrimidine analogs, purine analogs and adenosine deaminase inhibitors (e.g., fludarabine)), an mTOR inhibitor, a TNFR glucocorticoid induced TNFR related protein (GITR) agonist, a proteasome inhibitor (e.g., aclacinomycin A, gliotoxin or bortezomib), an immunomodulator such as thalidomide or a thalidomide derivative (e.g., lenalidomide).

In embodiments, a CAR-expressing cell described herein is administered to a subject in combination with cyclophosphamide and fludarabine.

In embodiments, a CAR-expressing cell described herein is administered to a subject in combination with bendamustine and rituximab. In embodiments, the subject has CLL.

In embodiments, a CAR-expressing cell described herein is administered to a subject in combination with rituximab, cyclophosphamide, doxorubicin, vincristine, and/or a corticosteroid (e.g., prednisone). In embodiments, a CAR-expressing cell described herein is administered to a subject in combination with rituximab, cyclophosphamide, doxorubicin, vincristine, and prednisone (R-CHOP). In embodiments, the subject has diffuse large B-celllymphoma (DLBCL).

In embodiments, a CAR-expressing cell described herein is administered to a subject in combination with etoposide, prednisone, vincristine, cyclophosphamide, doxorubicin, and/or rituximab. In embodiments, a CAR-expressing cell described herein is administered to a subject in combination with etoposide, prednisone, vincristine, cyclophosphamide, doxorubicin, and rituximab (EPOCH-R). In embodiments, a CAR-expressing cell described herein is administered to a subject in combination with dose adjusted EPOCH-R (DA-EPOCH-R). In embodiments, the subject has a B cell lymphoma, e.g., a Myc-rearranged aggressive B cell lymphoma.

In embodiments, a CAR-expressing cell described herein is administered to a subject in combination with brentuximab. Brentuximab is an antibody-drug conjugate of anti-CD30 antibody and monomethyl auristatin E. In embodiments, the subject has Hodgkin's lymphoma (HL), e.g., relapsed or refractory HL. In embodiments, the subject comprises CD30+ HL. In embodiments, the subject has undergone an autologous stem cell transplant (ASCT).

In some embodiments, a CAR-expressing cell described herein is administered to a subject in combination with a CD20 inhibitor, e.g., an anti-CD20 antibody (e.g., an anti-CD20 mono- or bispecific antibody) or a fragment thereof.

In one embodiment, a CAR expressing cell described herein is administered to a subject in combination with an mTOR inhibitor, e.g., an mTOR inhibitor described herein, e.g., a rapalog such as everolimus. In one embodiment, the mTOR inhibitor is administered prior to the CAR-expressing cell. For example, in one embodiment, the mTOR inhibitor can be administered prior to apheresis of the cells. In one embodiment, the subject has CLL.

In one embodiment, a CAR-expressing cell described herein can be used in combination with a kinase inhibitor. In one embodiment, the kinase inhibitor is a CDK4 inhibitor, e.g., a CDK4 inhibitor described herein, e.g., a CD4/6 inhibitor, such as, e.g., 6-Acetyl-8-cyclopentyl-5-methyl-2-(5-piperazin-1-yl-pyridin-2-ylamino )-8H-pyrido[2,3-d]pyrimidin-7-one, hydrochloride (also referred to as palbociclib or PD0332991). In one embodiment, the kinase inhibitor is a BTK inhibitor, e.g., a BTK inhibitor described herein, such as, e.g., ibrutinib. In some embodiments, ibrutinib is administered at a dosage of about 300-600 mg/day (e.g., about 300-350, 350-400, 400-450, 450-500, 500-550, or 550-600 mg/day, e.g., about 420 mg/day or about 560 mg/day), e.g., orally. In embodiments, the ibrutinib is administered at a dose of about 250 mg, 300 mg, 350 mg, 400 mg, 420 mg, 440 mg, 460 mg, 480 mg, 500 mg, 520 mg, 540 mg, 560 mg, 580 mg, 600 mg (e.g., 250 mg, 420 mg or 560 mg) daily for a period of time, e.g., daily for 21 day cycle, or daily for 28 day cycle. In one embodiment, 1, 2, 3, 4, 5, 6, 7, 8, 9, 10, 11, 12 or more cycles of ibrutinib are administered. Without being bound by theory, it is thought that the addition of ibrutinib enhances the T cell proliferative response and may shift T cells from a T-helper-2 (Th2) to T-helper-1 (Th1) phenotype. Th1 and Th2 are phenotypes of helper T cells, with Th1 versus Th2 directing different immune response pathways. A Th1 phenotype is associated with proinflammatory responses, e.g., for killing cells, such as intracellular pathogens/viruses or cancerous cells, or perpetuating autoimmune responses. A Th2 phenotype is associated with eosinophil accumulation and anti-inflammatory responses. In one embodiment, the kinase inhibitor is an mTOR inhibitor, e.g., an mTOR inhibitor described herein, such as, e.g., rapamycin, a rapamycin analog, OSI- 027. The mTOR inhibitor can be, e.g., an mTORC1 inhibitor and/or an mTORC2 inhibitor, e.g., an mTORC1 inhibitor and/or mTORC2 inhibitor described herein. In one embodiment, the kinase inhibitor is a MNK inhibitor, e.g., a MNK inhibitor described herein, such as, e.g., 4-amino-5-(4-fluoroanilino)-pyrazolo [3,4-d] pyrimidine. The MNK inhibitor can be, e.g., a MNK1a, MNK1b, MNK2a and/or MNK2b inhibitor. In one embodiment, the kinase inhibitor is a dual PI3K/mTOR inhibitor described herein, such as, e.g., PF-04695102. In one embodiment, the kinase inhibitor is a Src kinase inhibitor. In one embodiment, the kinase inhibitor is Dasatinib. In one embodiment, the Src kinase inhibitor is administered to the patient after the administration of CAR expressing cells to control or terminate the activity of CAR-expressing cells. In one embodiment, Dasatinib is administered to the patient after the administration of CAR expressing cells to control or terminate the activity of CAR-expressing cells. In one embodiment, dasatinib is administered orally at a dose of at least 10 mg/day, 20 mg/day, 40mg/day, 60mg/day, 70 mg/day, 90 mg/day, 100mg/day, 140 mg/day, 180 mg/day or 210 mg/day.

In embodiments, a CAR-expressing cell described herein is administered to a subject in combination with an anaplastic lymphoma kinase (ALK) inhibitor. Exemplary ALK kinases include but are not limited to crizotinib (Pfizer), ceritinib (Novartis), alectinib (Chugai), brigatinib (also called AP26113; Ariad), entrectinib (Ignyta), PF-06463922 (Pfizer), TSR-011 (Tesaro) (see, e.g., Clinical Trial Identifier No. NCT02048488), CEP-37440 (Teva), and X-396 (Xcovery). In some embodiments, the subject has a solid cancer, e.g., a solid cancer described herein, e.g., lung cancer.

Drugs that inhibit either the calcium dependent phosphatase calcineurin ( cyclosporine and FK506) or inhibit the p70S6 kinase that is important for growth factor induced signaling (rapamycin). (Liu et al., Cell66:807-815, 1991; Henderson et al., Immun. 73:316-321, 1991; Bierer et al., Curr. Opin. Immun. 5:763-773, 1993) can also be used. In a further aspect, the cell compositions of the present invention may be administered to a patient in conjunction with (e.g., before, simultaneously or following) bone marrow transplantation, T cell ablative therapy using chemotherapy agents such as, fludarabine, external-beam radiation therapy (XRT), cyclophosphamide, and/or antibodies such as OKT3 or CAMP ATH. In one aspect, the cell compositions of the present invention are administered following B-cell ablative therapy such as agents that react with CD20, e.g., Rituxan. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

In embodiments, a CAR-expressing cell described herein is administered to a subject in combination with an autologous stem cell transplant, an allogeneic stem cell transplant, an autologous bone marrow transplant or an allogeneic bone marrow transplant.

In embodiments, a CAR-expressing cell described herein is administered to a subject in combination with microtransplant or HLA mismatched allogeneic cellular therapy (Guo M et al, J Clin Oncol. 2012 Nov 20;30(33):4084-90).

In embodiments, a CAR-expressing cell described herein is administered to a subject in combination with an indoleamine 2,3-dioxygenase (IDO) inhibitor.

In embodiments, a CAR-expressing cell described herein is administered to a subject in combination with a modulator of myeloid-derived suppressor cells (MDSCs). MDSCs accumulate in the periphery and at the tumor site of many solid tumors. These cells suppress T cell responses, thereby hindering the efficacy of CAR-expressing cell therapy. Without being bound by theory, it is thought that administration of a MDSC modulator enhances the efficacy of a CAR-expressing cell described herein. In an embodiment, the subject has a solid tumor, e.g., a solid tumor described herein, e.g., glioblastoma. Exemplary modulators of MDSCs include but are not limited to MCSllO and BLZ945. MCSllO is a monoclonal antibody (mAb) against macrophage colony-stimulating factor (M-CSF). See, e.g., Clinical Trial Identifier No. NCT00757757. BLZ945 is a small molecule inhibitor of colony stimulating factor 1 receptor (CSF1R). See, e.g., Pyonteck et al. Nat. Med. 19(2013):1264-72.

In embodiments, a CAR-expressing cell described herein is administered to a subject in combination with a Brd4 or BET (bromodomain and extra-terminal motif) inhibitor. BET protein BRD4 directly regulated expression of the transcription factor BATF in CD8+ T cells, which was associated with differentiation of T cells into an effector memory phenotype. JQ1, an inhibitor of bromodomain and extra-terminal motif (BET) proteins, maintained CD8+ T cells with functional properties of stem cell-like and central memory T cells. Exemplary Brd4 inhibitors that can be administered in combination with CAR-expressing cells include but are not limited to JQ1, MS417, OTXO15, LY 303511 and Brd4 inhibitor as described in US 20140256706 A1 and any analogs thereof.

In some embodiments, a CAR-expressing cell described herein is administered to a subject in combination with a interleukin-15 (IL-15) polypeptide, a interleukin-15 receptor alpha (IL-15Ra) polypeptide, or a combination of both a IL-15 polypeptide and a IL-15Ra polypeptide e.g., hetiL-15 (Admune Therapeutics, LLC). hetiL- 15 is a heterodimeric noncovalent complex of IL-15 and IL-15Ra. hetiL-15 is described in, e.g., U.S. 8,124,084, U.S. 2012/0177598, U.S. 2009/0082299, U.S. 2012/0141413, and U.S. 201110081311, incorporated herein by reference. In embodiments, het-IL-15 is administered subcutaneously. In embodiments, the subject has a cancer, e.g., solid cancer, e.g., melanoma or colon cancer. In embodiments, the subject has a metastatic cancer.

In one embodiment, the subject can be administered an agent which reduces or ameliorates a side effect associated with the administration of a CAR-expressing cell. Side effects associated with the administration of a CAR-expressing cell include, but are not limited to CRS, and hemophagocytic lymphohistiocytosis (HLH), also termed Macrophage Activation Syndrome (MAS). Symptoms of CRS include high fevers, nausea, transient hypotension, hypoxia, and the like. CRS may include clinical constitutional signs and symptoms such as fever, fatigue, anorexia, myalgias, arthalgias, nausea, vomiting, and headache. CRS may include clinical skin signs and symptoms such as rash. CRS may include clinical gastrointestinal signs and symptoms such as nausea, vomiting and diarrhea. CRS may include clinical respiratory signs and symptoms such as tachypnea and hypoxemia. CRS may include clinical cardiovascular signs and symptoms such as tachycardia, widened pulse pressure, hypotension, increased cardiac output (early) and potentially diminished cardiac output (late). CRS may include clinical coagulation signs and symptoms such as elevated d-dimer, hypofibrinogenemia with or without bleeding. CRS may include clinical renal signs and symptoms such as azotemia. CRS may include clinical hepatic signs and symptoms such as transaminitis and hyperbilirubinemia. CRS may include clinical neurologic signs and symptoms such as headache, mental status changes, confusion, delirium, word finding difficulty or frank aphasia, hallucinations, tremor, dymetria, altered gait, and seizures.

Accordingly, the methods described herein can comprise administering a CAR-expressing cell described herein to a subject and further administering one or more agents to manage elevated levels of a soluble factor resulting from treatment with a CAR-expressing cell. In one embodiment, the soluble factor elevated in the subject is one or more of IFN-y, TNFa, IL-2 and IL-6. In an embodiment, the factor elevated in the subject is one or more of IL-1, GM-CSF, IL-10, IL-8, IL-5 and fraktalkine. Therefore, an agent administered to treat this side effect can be an agent that neutralizes one or more of these soluble factors. In one embodiment, the agent that neutralizes one or more of these soluble forms is an antibody or antigen binding fragment thereof. Examples of such agents include, but are not limited to a steroid (e.g., corticosteroid), Src inhibitors (e.g., Dasatinib) an inhibitor of TNFa, and an inhibitor of IL-6. An example of a TNFa inhibitor is an anti-TNFa antibody molecule such as, infliximab, adalimumab, certolizumab pegol, and golimumab. Another example of a TNFa inhibitor is a fusion protein such as entanercept. An example of an IL-6 inhibitor is an anti-IL-6 antibody molecule or an anti-IL-6 receptor antibody molecule such as tocilizumab (toe), sarilumab, elsilimomab, CNTO 328, ALD518/BMS-945429, CNTO 136, CPSI-2364, CDP6038, VX30, ARGX-109, FE301, and FM101. In one embodiment, the anti-IL-6 receptor antibody molecule is tocilizumab. In one embodiment, the IL-6 inhibitor is a camelid bispecific antibody that binds to IL6R and human serum albumin (e.g., IL6R-304-Alb8) (SEQ ID NO: 2649). An example of an IL-IR based inhibitor is anakinra. In one embodiment, an agent administered to treat the side effects of CAR-expressing cells is a Src inhibitor (e.g., Dasatinib). In one embodiment, an agent administered to treat the side effects of CAR-expressing cells is the Src inhibitor Dasatinib. In embodiments, Dasatinib is administered at a dose of about 10 mg/day to 240 mg/day (e.g., 10mg/day, 20mg/day, 40mg/day, 50mg/day, 70 mg/day, 80mg/day, 100mg/day, 110mg/day, 120mg/day, 140mg/day, 180mg/day, 210 mg/day, 240mg/day or 300mg/day).

In one embodiment, the subject can be administered an agent which enhances the activity of a CAR-expressing cell. For example, in one embodiment, the agent can be an agent which inhibits an inhibitory molecule. Inhibitory molecules, e.g., Programmed Death 1 (PD-1), can, in some embodiments, decrease the ability of a CAR-expressing cell to mount an immune effector response. Examples of inhibitory molecules include PD-1, PDL1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGFR beta. Inhibition of an inhibitory molecule, e.g., by inhibition at the DNA, RNA or protein level, can optimize a CAR-expressing cell performance. In embodiments, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used to inhibit expression of an inhibitory molecule in the CAR-expressing cell. In an embodiment the inhibitor is an shRNA. In an embodiment, the inhibitory molecule is inhibited within a CAR-expressing cell. In these embodiments, a dsRNA molecule that inhibits expression of the inhibitory molecule is linked to the nucleic acid that encodes a component, e.g., all of the components, of the CAR. In one embodiment, the inhibitor of an inhibitory signal can be, e.g., an antibody or antibody fragment that binds to an inhibitory molecule. For example, the agent can be an antibody or antibody fragment that binds to PD-1, PD-L1, PD-L2 or CTLA4 (e.g., ipilimumab (also referred to as MDX-010 and MDX- 101, and marketed as Yervoy^{®}; Bristol-Myers Squibb; Tremelimumab (IgG2 monoclonal antibody available from Pfizer, formerly known as ticilimumab, CP-675,206).). In an embodiment, the agent is an antibody or antibody fragment that binds to TIM3. In an embodiment, the agent is an antibody or antibody fragment that binds to CEACAM (CEACAM-1, CEACAM-3, and/or CEACAM-5). In an embodiment, the agent is an antibody or antibody fragment that binds to LAG3.

PD-1 is an inhibitory member of the CD28 family of receptors that also includes CD28, CTLA-4, ICOS, and BTLA. PD-1 is expressed on activated B cells, T cells and myeloid cells. Two ligands for PD-1, PD-L1 and PD-L2 have been shown to down regulate T cell activation upon binding to PD-1 . PD-L1 is abundant in human cancers. Immune suppression can be reversed by inhibiting the local interaction of PD-1 with PD-L1. Antibodies, antibody fragments, and other inhibitors of PD-1, PD-L1 and PD-L2 are available in the art and may be used combination with a CAR of the present invention described herein. For example, nivolumab (also referred to as BMS-936558 or MDX1106; Bristol-Myers Squibb) is a fully human IgG4 monoclonal antibody which specifically blocks PD-1. Nivolumab (clone 5C4) and other human monoclonal antibodies that specifically bind to PD-1 are disclosed in US 8,008,449 and WO2006/121168. Pidilizumab (CT-011; Cure Tech) is a humanized IgG1k monoclonal antibody that binds to PD-1. Pidilizumab and other humanized anti-PD-1 monoclonal antibodies are disclosed in WO2009/101611. Pembrolizumab (formerly known as lambrolizumab, and also referred to as MK03475; Merck) is a humanized IgG4 monoclonal antibody that binds to PD-1. Pembrolizumab and other humanized anti-PD-1 antibodies are disclosed in US 8,354,509 and WO2009/114335. MEDI4736 (Medimmune) is a human monoclonal antibody that binds to PDL1, and inhibits interaction of the ligand with PD1. MDPL3280A (Genentech I Roche) is a human Fe optimized IgG1 monoclonal antibody that binds to PD-L1. MDPL3280A and other human monoclonal antibodies to PD-L1 are disclosed in U.S. Patent No.: 7,943,743 and U.S Publication No.: 20120039906. In other embodiments, the agent that enhances the activity of a CAR-expressing cell is a CEACAM inhibitor (e.g., CEACAM-1, CEACAM-3, and/or CEACAM-5 inhibitor).

In one embodiment, the agent which enhances activity of a CAR-described herein is another agent that increases the expression of the target antigen against which the CAR is directed. The agents that can be administered to the subject receiving a CAR-expressing cell described herein include: Arsenic trioxide, ATRA (all-trans-retinoic acid), compounds 27, 40, 49 of (Du et al, Blood; Prepublished online October 12, 2016), IDH2 inhibitors (e.g., AG-221) or a combination thereof. In an embodiment, the agents are administered prior to, concurrently or after administration of CAR-expressing cells. In preferred embodiments these agents are administered prior to administration of CAR-expressing cells. In preferred embodiment, the CAR expressing cells that are administered with the above agents target a B cell antigen (e.g., CD19, CD20, or CD22 etc.).

In one embodiment, the agent which enhances activity of a CAR described herein is a soluble receptor. Soluble receptor that can be administered to the subject receiving a CAR-expressing cell described herein include: sHVEM (SEQ ID NO: 2664), sHVEM-Alb8-vHH fusion protein (SEQ ID NO: 2665), or a combination thereof. The soluble receptor can be administered once a day or more than once a day, e.g., twice a day, three times a day, or four times a day. The soluble receptor can be administered for more than one day, e.g. the soluble receptor is administered for 2 days, 3 days, 4 days, 5 days, 6 days, 1 week, 2 weeks, 3 weeks, or 4 weeks. For example, the soluble receptor is administered once a day for 7 days.

In one embodiment, the subject can be administered an agent which protects against the toxicity of a CAR-expressing cell on normal tissues. One of the limitations of CAR-T cell therapy could be toxicity on normal tissue. For example, CAR targeting CD19 could lead to long-term depletion of normal B cells, which also express CD19 antigen. In one embodiment, CD19 CAR-T cell therapy can be combined with knock-out or mutation of endogenous CD19 in normal hematopoietic stem cells. In one embodiment, the knock out or mutation of the endogenous CD19 is achieved using CRIPS/Cas9, Talons or other suitable gene editing methods which are known in the art. The epitope of CD19 bound by the CD19 CAR-T cells in current clinical use has been mapped to exon2-4. In one embodiment, missense or nonsense mutations are generated in exon 2 (or other suitable exons/regions that are recognized by CD19 targeted CAR T-cells) of autologous or allogeneic hematopoietic stem cells using CRISP/Cas9, Zn finger nucleases, Talons or other methods known in the art. In one embodiment, the subject is given CD19 CART cells infusion to control his/her disease and an autologous or allogeneic stem cell transplant using CD19 deleted/mutated hematopoietic stem cells. As the B cells that will originate from the modified stem cells will not be targeted by CD19-CAR-T cells, the patient will escape B cell aplasia which is a common side effect of CD19 CAR-T cells. In another embodiment, MPL CAR-T cell therapy is combined with knock-out or mutation of endogenous MPL in normal hematopoietic stem cells. In another embodiment, CD123 CAR-T cell therapy is combined with knock-out or mutation of endogenous CD123 in normal hematopoietic stem cells. In another embodiment, CD33 CAR-T cell therapy is combined with knock-out or mutation of endogenous CD33 in normal hematopoietic stem cells. In another embodiment, CD20 CAR-T cell therapy is combined with knock-out or mutation of endogenous CD20 in normal hematopoietic stem cells. In another embodiment, CD22 CAR-T cell therapy is combined with knock-out or mutation of endogenous CD22 in normal hematopoietic stem cells. In another embodiment, CS1 CAR-T cell therapy is combined with knock-out or mutation of endogenous CS1 in normal hematopoietic stem cells. In another embodiment, BCMA CAR-T cell therapy is combined with knock-out or mutation of endogenous BCMA in normal hematopoietic stem cells. In another embodiment, CD45 CAR-T cell therapy is combined with knock-out or mutation of endogenous CD45 in normal hematopoietic stem cells or immune effector cells (e.g., T cells or NK cells). Essentially, a similar approach could be used to mitigate the toxicity of CAR-T cells against normal tissue where the antigen targeted by the CAR is also expressed on normal hematopoietic stem cells or one of its progenies.

In another embodiment, CAR-T cell therapy is combined with knock-out or mutation of endogenous gene or protein targeted by the CAR in the immune effector cell (e.g., T cells or NK cells) or stem cells that give rise to immune effector cells. For example, since CD45 is expressed on all hematopoietic cells, CAR-T cells targeting CD45 would be difficult to generate as they would be killed off by neighboring CD45-CART cells. However, such cells can be generated if expression of CD45 CAR in T cells is combined with knockdown or deletion of endogenous CD45 in the T cells in which CD45 CAR is being expressed. Essentially a similar approach can be used to generate CAR targeting other antigens that are expressed on immune effector cells. Exemplary such antigens include, but are not limited to, CD5, TCRα, TCRβ1, TCRβ2, TCRγ, TCRδ, preTCRα and various receptors expressed on NK cells.

Cytokines that can be administered to the subject receiving a CAR-expressing cell described herein include: IL-2, IL-4, IL-7, IL-9, IL-15, IL- 18, LIGHT, and IL-21, or a combination thereof. In preferred embodiments, the cytokine administered is IL-7, IL-15, or IL-21, IL12F, or a combination thereof. The cytokine can be administered once a day or more than once a day, e.g., twice a day, three times a day, or four times a day. The cytokine can be administered for more than one day, e.g. the cytokine is administered for 2 days, 3 days, 4 days, 5 days, 6 days, 1 week, 2 weeks, 3 weeks, or 4 weeks. For example, the cytokine is administered once a day for 7 days. Administration of the cytokine to the subject that has sub-optimal response to the CAR-expressing cell therapy improves CAR-expressing cell efficacy or anti-cancer activity. In a preferred embodiment, the cytokine administered after administration of CAR-expressing cells is IL-7.

In one embodiment, the agent which enhances activity of a CAR-expressing cell described herein is a Brd4 inhibitor or an siRNA or an shRNA targeting BRD4 as described in (Tolani, B et al., Oncogene, 29; 33(22):2928-37. PMID: 23792448)(Tolani, Gopalakrishnan, Punj, Matta, & Chaudhary, 2014).

### Pharmaceutical Compositions

Also provided herein are pharmaceutical compositions comprising any one or more of the chimeric antigen receptors, the polynucleotides, the polypeptides, the vectors, the viruses, and/or the genetically engineered cells and/or chemical compounds described herein and a pharmaceutically acceptable carrier. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are in one aspect formulated for intravenous administration.

Pharmaceutical compositions of the present invention may be administered in a manner appropriate to the disease to be treated. The quantity and frequency of administration will be determined by such factors as the condition of the patient, and the type and severity of the patient's disease, although appropriate dosages may be determined by clinical trials.

When a "therapeutically effective amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the genetically modified cells (T cells, NK cells) described herein may be administered at a dosage of 10⁴ to 10⁴ cells/kg body weight, in some instances 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng J. of Med. 319:1676, 1988).

In some embodiments, it may be desired to administer activated genetically modified cells (T cells, NK cells) to a subject and then subsequently redraw blood (or have an apheresis performed), activate the genetically modified cells therefrom and reinfuse the patient with these activated and expanded genetically modified cells. This process can be carried out multiple times every few weeks. In certain aspects, immune effector cells (e.g., T cells, NK cells) can be activated from blood draws of from 10cc to 400cc. In certain aspects, immune effector cells (e.g., T cells, NK cells) are activated from blood draws of 20cc, 30cc. 40cc, 50cc, 60cc, 70cc, 80cc, 90cc, or 100cc.

"Pharmaceutically acceptable excipient" means an excipient that is useful in preparing a pharmaceutical composition that is generally safe, non-toxic, and desirable, and includes excipients that are acceptable for veterinary use as well as for human pharmaceutical use. Such excipients may be solid, liquid, semisolid, or, in the case of an aerosol composition, gaseous.

In various embodiments, the pharmaceutical compositions according to the invention may be formulated for delivery via any route of administration. "Route of administration" may refer to any administration pathway known in the art, including but not limited to aerosol, nasal, oral, intravenous, intramuscular, intraperitoneal, inhalation, transmucosal, transdermal, parenteral, implantable pump, continuous infusion, topical application, capsules and/or injections.

The pharmaceutical compositions according to the invention can also contain any pharmaceutically acceptable carrier. "Pharmaceutically acceptable carrier" as used herein refers to a pharmaceutically acceptable material, composition, or vehicle that is involved in carrying or transporting a compound of interest from one tissue, organ, or portion of the body to another tissue, organ, or portion of the body. For example, the carrier may be a liquid or solid filler, diluent, excipient, solvent, or encapsulating material, or a combination thereof. Each component of the carrier must be "pharmaceutically acceptable" in that it must be compatible with the other ingredients of the formulation. It must also be suitable for use in contact with any tissues or organs with which it may come in contact, meaning that it must not carry a risk of toxicity, irritation, allergic response, immunogenicity, or any other complication that excessively outweighs its therapeutic benefits.

The pharmaceutical compositions according to the invention can also be encapsulated, tableted or prepared in an emulsion or syrup for oral administration. Pharmaceutically acceptable solid or liquid carriers may be added to enhance or stabilize the composition, or to facilitate preparation of the composition. Liquid carriers include syrup, peanut oil, olive oil, glycerin, saline, alcohols and water. Solid carriers include starch, lactose, calcium sulfate, dihydrate, terra alba, magnesium stearate or stearic acid, talc, pectin, acacia, agar or gelatin. The carrier may also include a sustained release material such as glyceryl monostearate or glyceryl distearate, alone or with a wax

The pharmaceutical preparations are made following the conventional techniques of pharmacy involving milling, mixing, granulation, and compressing, when necessary, for tablet forms; or milling, mixing and filling for hard gelatin capsule forms. When a liquid carrier is used, the preparation will be in the form of syrup, elixir, emulsion or an aqueous or non-aqueous suspension. Such a liquid formulation may be administered directly p.o. or filled into a soft gelatin capsule.

The pharmaceutical compositions according to the invention may be delivered in a therapeutically effective amount. The precise therapeutically effective amount is that amount of the composition that will yield the most effective results in terms of efficacy of treatment in a given subject. This amount will vary depending upon a variety of factors, including but not limited to the characteristics of the therapeutic compound (including activity, pharmacokinetics, pharmacodynamics, and bioavailability), the physiological condition of the subject (including age, sex, disease type and stage, general physical condition, responsiveness to a given dosage, and type of medication), the nature of the pharmaceutically acceptable carrier or carriers in the formulation, and the route of administration. One skilled in the clinical and pharmacological arts will be able to determine a therapeutically effective amount through routine experimentation, for instance, by monitoring a subject's response to administration of a compound and adjusting the dosage accordingly. For additional guidance, see Remington: The Science and Practice of Pharmacy (Gennaro ed. 20th edition, Williams & Wilkins PA, USA) (2000).

The administration of the subject compositions may be carried out in any convenient manner, including by aerosol inhalation, injection, transfusion, implantation or transplantation. The compositions described herein may be administered to a patient trans-arterially, subcutaneously, intradermally, intratumorally, intranodally, intramedullary, intramuscularly, by intravenous (i.v.) injection, or intraperitoneally. In one aspect, the T cell compositions of the present invention are administered to a patient by intradermal or subcutaneous injection. In one aspect, the T cell compositions of the present invention are administered by i. v. injection. The compositions of immune effector cells (e.g., T cells, NK cells) may be injected directly into a tumor, lymph node, or site of infection.

In a particular exemplary aspect, subjects may undergo leukapheresis, wherein leukocytes are collected, enriched, or depleted ex vivo to select and/or isolate the cells of interest, e.g., T cells. These T cell isolates may be expanded by methods known in the art and treated such that one or more CAR constructs of the invention may be introduced, thereby creating a CAR T cell of the invention. Subjects in need thereof may subsequently undergo standard treatment with high dose chemotherapy followed by peripheral blood stem cell transplantation. In certain aspects, following or concurrent with the transplant, subjects receive an infusion of the expanded CAR T cells of the present invention. In an additional aspect, expanded cells are administered before or following surgery.

In one embodiment, the CAR is introduced into immune effector cells (e.g., T cells, NK cells), e.g., using in vitro transcription, and the subject (e.g., human) receives an initial administration of CAR immune effector cells (e.g., T cells, NK cells) of the invention, and one or more subsequent administrations of the CAR immune effector cells (e.g., T cells, NK cells) of the invention, wherein the one or more subsequent administrations are administered less than 15 days, e.g., 14, 13, 12, 11, 10, 9, 8, 7, 6, 5, 4, 3, or 2 days after the previous administration. In one embodiment, more than one administration of the CAR immune effector cells (e.g., T cells, NK cells) of the invention are administered to the subject (e.g., human) per week, e.g., 2, 3, or 4 administrations of the CAR immune effector cells (e.g., T cells, NK cells) of the invention are administered per week. In one embodiment, the subject (e.g., human subject) receives more than one administration of the CAR immune effector cells (e.g., T cells, NK cells) per week (e.g., 2, 3 or 4 administrations per week) (also referred to herein as a cycle), followed by a week of no CAR immune effector cells (e.g., T cells, NK cells) administrations, and then one or more additional administration of the CAR immune effector cells (e.g., T cells, NK cells) (e.g., more than one administration of the CAR immune effector cells (e.g., T cells, NK cells) per week) is administered to the subject. In another embodiment, the subject (e.g., human subject) receives more than one cycle of CAR immune effector cells (e.g., T cells, NK cells), and the time between each cycle is less than 10, 9, 8, 7, 6, 5, 4, or 3 days. In one embodiment, the CAR immune effector cells (e.g., T cells, NK cells) are administered every other day for 3 administrations per week. In one embodiment, the CAR immune effector cells (e.g., T cells, NK cells) of the invention are administered for at least two, three, four, five, six, seven, eight or more weeks.

A potential issue that can arise in patients being treated using transiently expressing CAR immune effector cells (e.g., T cells, NK cells) (particularly with murine scFv bearing CAR-Ts) is anaphylaxis after multiple treatments.

Without being bound by this theory, it is believed that such an anaphylactic response might be caused by a patient developing humoral anti -CAR response, i.e., anti-CAR antibodies having an anti-IgE isotype. It is thought that a patient's antibody producing cells undergo a class switch from IgG isotype (that does not cause anaphylaxis) to IgE isotype when there is a ten to fourteen day break in exposure to antigen.

If a patient is at high risk of developing an anaphylactic response to CAR therapy or generating an IgE type CAR antibody response during the course of CAR therapy, then omalizumab (Xolair) can be administered before or during the CAR therapy.

If a patient is at high risk of generating an anti-CAR antibody response during the course of transient CAR therapy (such as those generated by RNA transductions), CART infusion breaks should not last more than ten to fourteen days.

### Kits

Kits to practice the invention are also provided. For example, kits for treating a cancer in a subject, or making a CAR T cell that expresses one or more of the CARs disclosed herein. The kits may include a nucleic acid molecule or a polypeptide molecule encoding a CAR or a vector encoding a CAR along with a method to introduce the nucleic acid into the immune effector cells. The kit may include a virus comprising a nucleic acid encoding a CAR and chemicals, such as polybrene, to enhance the virus transduction. The kit may contain components for isolation of T cells for expressing a CAR. Alternatively, the kit may contain immune effector cells (e.g., T cells or NK cells) or stem cells expressing a CAR. More than one of the disclosed CAR can be included in the kit. The kit can include a container and a label or package insert on or associated with the container.

Suitable containers include, for example, bottles, vials, syringes, etc. The containers may be formed from a variety of materials such as glass or plastic. The container typically holds a composition including one or more of the nucleic acid molecules, viruses, vectors, T cells expressing a CAR. In several embodiments the container may have a sterile access port (for example the container may be an intravenous solution bag or a vial having a stopper pierceable by a hypodermic injection needle). A label or package insert indicates that the composition is used for treating the particular condition. The label or package insert typically will further include instructions for use of a disclosed nucleic acid molecules, CARs or T cells expressing a CAR, for example, in a method of treating or preventing a tumor or of making a CAR T cell. The package insert typically includes instructions customarily included in commercial packages of therapeutic products that contain information about the indications, usage, dosage, administration, contraindications and/or warnings concerning the use of such therapeutic products. The instructional materials may be written, in an electronic form (such as a computer diskette or compact disk) or may be visual (such as video files). The kits may also include additional components to facilitate the particular application for which the kit is designed. Thus, for example, the kit may additionally contain means for measuring the expression of CAR on T cells or of determining the number or percentage of T cells that express the CAR or of determining the functionality of CART cells. The kits may additionally include buffers and other reagents routinely used for the practice of a particular method. Such kits and appropriate contents are well known to those of skill in the art.

**Table 3: Exemplary signal peptides**

| **NAME** | **SEQ ID (DNA)** | **SEQ ID (PRT)** |
|---|---|---|
| CD8_Signal_Peptide | 1 | 1781 |
| CD8_Signal_Peptide | 2 | 1781 |
| CD8_Signal_Peptide | 3 | 1781 |
| CD8-SIGNAL-PEPTIDE | 4 | 1781 |
| IgH_Signal_Peptide | 5 | 1782 |
| IgH_Signal_Peptide | 6 | 1782 |
| IgH-Signal-Peptide | 7 | 1782 |
| IgH_Signal_Peptide | 8 | 1782 |
| Amyloid-158-158-Signal-peptide | 9 | 1783 |
| hCD19 Signal Peptide | 10 | 1784 |

**Table 4: Exemplary VL fragments**

| **Target** | **SEQ ID (DNA)** | **SEQ ID (PRT)** | **NAME** |
|---|---|---|---|
| AFP/MHC class I complex | 11 | 1785 | AFP-61-vL |
| AFP/MHC class I complex | 12 | 1786 | AFP-76-vL |
| AFP/MHC class I complex | 13 | 1787 | AFP-79-vL |
| ALK | 14 | 1788 | Alk-48-vL |
| ALK | 15 | 1789 | Alk-58-vL |
| Amyloid | 16 | 1790 | Amyloid-158-vL |
| BCMA | 17 | 1791 | BCMA-ET-40-vL |
| BCMA | 18 | 1792 | BCMA-ET-54-vL |
| BCMA | 19 | 1793 | BCMA-huC12A3-vL |
| BCMA | 20 | 1794 | BCMA-J6M0-vL |
| BCMA | 21 | 1795 | BCMA-ET-03-vL |
| BCMA | 22 | 1796 | BCMA-huC11.D5.3L1H3-vL |
| BCMA | 23 | 1797 | BCMA-huC13-F12-vL |
| CCR4 | 24 | 1798 | CCR4-humAb1567-vL |
| CD123 | 25 | 1799 | CD123-CSL362-vL |
| CD123 | 26 | 1800 | CD123-1172-vL |
| CD123 | 27 | 1801 | CD123-DART-1-vL |
| CD123 | 28 | 1802 | CD123-DART-2-vL |
| CD123 | 29 | 1803 | CD123-13RB18-vL |
| CD123 | 30 | 1804 | CD123-hu3E3-vL |
| CD123 | 31 | 1805 | CD123-9F6-vL |
| CD123 | 32 | 1806 | CD123-I3RB2-vL |
| CD123 | 33 | 1807 | CD123-1176-vL |
| CD123 | 34 | 1808 | CD123-8B11-vL |
| CD123 | 35 | 1809 | CD123-2B8-vL |
| CD123 | 36 | 1810 | CD123-9D7-vL |
| CD123 | 37 | 1811 | CD123-3B10-vL |
| CD138 | 38 | 1812 | CD138-vL |
| CD179b | 39 | 1813 | CD179b-vL |
| CD19 | 40 | 1814 | CD19-4G7-vL |
| CD19 | 41 | 1815 | CD19Bu12-vL |
| CD19 | 42 | 1816 | CD19MM-vL |
| CD19 | 43 | 1817 | FMC63-vL |
| CD19 | 44 | 1818 | FMC63-[2]-vL |
| CD19 | 45 | 1819 | FMC63-[3]-vL |
| CD19 | 46 | 1820 | huFMC63-11-vL |
| CD19 | 47 | 1821 | CD19-MEDI-3649-vL |
| CD19 | 48 | 1822 | CD19-Medrex-24D1-vL |
| CD19 | 49 | 1823 | CD19-MOR0028-vL |
| CD19 | 50 | 1824 | CD19-HD37-H2L1-vL |
| CD19 | 51 | 1825 | CD19-huBly3-vL |
| CD19 | 52 | 1826 | CD19-huSJ25C1-vL |
| CD19 | 53 | 1827 | CD19-hB4-vL |
| CD19 | 54 | 1828 | CD19-hu-mROO5-1-vL |
| CD19 | 55 | 1829 | CD19-hA19-vL |
| CD20 | 56 | 1830 | CD20-2F2-vL |
| CD20 | 57 | 1831 | CD20-GA101-vL |
| CD20 | 58 | 1832 | CD20-Leu16-vL |
| CD20 | 59 | 1833 | CD20-11B8-vL |
| CD20 | 60 | 1834 | CD20-2C6-vL |
| CD20 | 61 | 1835 | CD20-2H7-vL |
| CD20 | 62 | 1836 | CD20-hA20-vL |
| CD20 | 63 | 1837 | CD20-BM-CA-1925-v4-vL |
| CD20 | 64 | 1838 | CD20-Ubli-v4-vL |
| CD20 | 65 | 1839 | CD20-2H7-vL |
| CD20 | 66 | 1840 | CD20-h1F5-vL |
| CD20 | 67 | 1841 | CD20-7D8-vL |
| CD20 | 68 | 1842 | CD20-AME-33-vL |
| CD22 | 69 | 1843 | CD22-h1OF4-vL |
| CD22 | 70 | 1844 | CD22-H22Rhov2ACDRKA-vL |
| CD22 | 71 | 1845 | CD22m971-vL |
| CD276 | 72 | 1846 | CD276-17-vL |
| CD30 | 73 | 1847 | CD30-5F11-vL |
| CD30 | 74 | 1848 | CD30-Ac10-vL |
| CD32 | 75 | 1849 | CD32-Med9-vL |
| CD324 | 76 | 1850 | CD324-hSC10-17-vL |
| CD324 | 77 | 1851 | CD324-SC10-6-vL |
| CD33 | 78 | 1852 | CD33-huMyc9-vL |
| CD33 | 79 | 1853 | CD33-AF5-vL |
| CD33 | 80 | 1854 | CD33-Boehr2800308-vL |
| CD33 | 81 | 1855 | CD33-Him3-4-vL |
| CD33 | 82 | 1856 | CD33-SGNh2H12-vL |
| CD33 | 83 | 1857 | CD33-15G15-33-vL |
| CD33 | 84 | 1858 | CD33-33H4-vL |
| CD33 | 85 | 1859 | CD33-9C3-2-vL |
| CD34 | 86 | 1860 | CD34-hu4C7-[2]-vL |
| CD34 | 87 | 1861 | CD34-hu4C7-vL |
| CD44v6 | 88 | 1862 | CD44v6-Biwa8-vL |
| CD5 | 89 | 1863 | CD5-18-vL |
| CD5 | 90 | 1864 | CD5-9-vL |
| CD70 | 91 | 1865 | CD70-h1F6-vL |
| CD79b | 92 | 1866 | CD79b-2F2-vL |
| CD79b | 93 | 1867 | huMA79bv28-vL |
| CD99 | 94 | 1868 | CD99-hu12E7-vL |
| CDH17 | 95 | 1869 | CDH17-PTA001A4-vL |
| CDH19 | 96 | 1870 | CDH19-16A4-vL |
| CDH6 | 97 | 1871 | CDH6-NOV710-vL |
| CDH6 | 98 | 1872 | CDH6-NOV712-vL |
| CLEC5A | 99 | 1873 | CLEC5A-3E12A2-vL |
| CLEC5A | 100 | 1874 | CLEC5A-8H8F5-vL |
| CLL1 | 101 | 1875 | CLL1-M26-vL |
| CLL1 | 102 | 1876 | CLL1-M32-vL |
| CLL1 | 103 | 1877 | CLL1-21C9-L2H3-vL |
| CLL1 | 104 | 1878 | CLL1-6E7L4H1e-vL |
| CLL1 | 105 | 1879 | CLL1-hu1075-v1-vL |
| CLL1 | 106 | 1880 | CLL1-hu1075-v2-vL |
| CMVpp65/MHC class I complex | 107 | 1881 | CMVpp65-F5-vL |
| CS1 | 108 | 1882 | huLuc63-vL |
| CS1 | 109 | 1883 | HuLuc64-[2]-vL |
| CS1 | 110 | 1884 | HuLuc64-vL |
| CS1 | 111 | 1885 | Luc90-vL |
| CS1 | 112 | 1886 | CS1-PDL241-vL |
| CS1 | 113 | 1887 | CS1-Hu27A-vL |
| CS1 | 114 | 1888 | CS1-ScHu34C3-vL |
| CS1 | 115 | 1889 | CS1-Hu31-D2-vL |
| CS1 | 116 | 1890 | CS1-Luc34-vL |
| CS1 | 117 | 1891 | CS1-LucX2-vL |
| CSF2RA | 118 | 1892 | CSF2RA-Abl-vL |
| CSF2RA | 119 | 1893 | CSF2RA-Ab6-vL |
| DLL3 | 120 | 1894 | DLL3-hSC16-13-vL |
| DLL3 | 121 | 1895 | DLL3-hSC16-56-vL |
| EBNA3c//MHC class I complex | 122 | 1896 | EBNA3c-315-vL |
| EGFR | 123 | 1897 | Cetuximab-vL |
| EGFR | 124 | 1898 | Nimotuzumab-vL |
| EGFRviii | 125 | 1899 | EGFRviii-139-vL |
| EGFRviii | 126 | 1900 | EGFRviii-2173-vL |
| EpCam1 | 127 | 1901 | EpCam1-D5K5-vL |
| EpCam1 | 128 | 1902 | Epcam1-MM1-vL |
| FITC | 129 | 1903 | FITC-vL |
| FITC | 130 | 1904 | FITC-4M-53-vL |
| FITC | 131 | 1905 | FITC-E2-vL |
| FLT3 | 132 | 1906 | FLT3-NC7-vL |
| HIV1-envelop glycoprotein | 133 | 1907 | HIV1-N6-vL |
| FR1 (Folate Receptor alpha) | 134 | 1908 | FR1-huMov19-vL |
| GAD65/MHC class I complex | 135 | 1909 | GAD-G3H8-vL |
| GD2 | 136 | 1910 | GD2-hu14-18-vL |
| GD2 | 137 | 1911 | GD2-hu3F8-vL |
| GD3 | 138 | 1912 | GD3-KM-641-vL |
| GFRa4 | 139 | 1913 | GFRa4-P4-10-2-vL |
| GFRa4 | 140 | 1914 | GFRa4-P4-10-vL |
| GFRa4 | 141 | 1915 | GFRAlpha4-P4-6-vL |
| GM1 | 142 | 1916 | GM1-5B2-vL |
| GM1 | 143 | 1917 | GM1-7E5-vL |
| gp100//MHC class I complex | 144 | 1918 | gp100-G2D12-vL |
| gp100//MHC class I complex | 145 | 1919 | gp100-vL |
| GPC3 | 146 | 1920 | GPC3-4E5-vL |
| gpNMB | 147 | 1921 | gpNMB-115-vL |
| GPRC5D | 148 | 1922 | GPRC5D-ET150-18-vL |
| GPRC5D | 149 | 1923 | GPRC5D-ET150-5-vL |
| GPRC5D | 150 | 1924 | GPRC5D-ET150-1-vL |
| GPRC5D | 151 | 1925 | GPRC5D-ET150-2-vL |
| Her2 | 152 | 1926 | Her2-Hu4D5-vL |
| HIV 1-envelop glycoprotein | 153 | 1927 | HIV1-3BNC117-vL |
| HIV1-gag (77-85)/MHC class I complex | 154 | 1928 | HIV1-E5-vL |
| HIV 1-envelop glycoprotein | 155 | 1929 | HIV1-PGT-128-vL |
| HIV 1-envelop glycoprotein | 156 | 1930 | HIV1-VR-C01-vL |
| HIV 1-envelop glycoprotein | 157 | 1931 | HIV1-X5-vL |
| HLA-A2 | 158 | 1932 | HLA-A2-3PB2-vL |
| HMW-MAA | 159 | 1933 | HMW-MAA-hIND-vL |
| HPV16-E7/MHC class I complex | 160 | 1934 | HPV16-7-8-vL |
| HPV16-E7/MHC class I complex | 161 | 1935 | HPV16-2-vL |
| HTLV1-TAX/MHC class I complex | 162 | 1936 | TAX-T3E3-vL |
| HTLV1-TAX/MHC class I complex | 163 | 1937 | TAX-T3F2-vL |
| IL11Ra | 164 | 1938 | IL11Ra-8E2-vL |
| IL13Ra2 | 165 | 1939 | IL13Ra2-hu107-vL |
| IL13Ra2 | 166 | 1940 | IL13Ra2-Hu108-vL |
| IL6R | 167 | 1941 | IL6R-M83-vL |
| Influenza A HA | 168 | 1942 | FLU-MEDI-8852-vL |
| KSHV-gH | 169 | 1943 | YC15-vL |
| KSHV-K8.1 | 170 | 1944 | 4C3-vL |
| L1CAM | 171 | 1945 | L1CAM-9-3-HU3-vL |
| LAMP1 | 172 | 1946 | LAMP1-humab1-2-vL |
| LAMP1 | 173 | 1947 | LAMP1-Mb4-vL |
| LewisY | 174 | 1948 | LewisY-huS193-vL |
| Lym1 | 175 | 1949 | Lym1-vL |
| Lym2 | 176 | 1950 | Lym2-vL |
| MART1/MHC class I complex | 177 | 1951 | NART1-CAG10-vL |
| MART1/MHC class I complex | 178 | 1952 | MART1-CLA12-vL |
| Mesothelin | 179 | 1953 | Mesothelin-m912-vL |
| MPL | 180 | 1954 | MPL-111-vL |
| MPL | 181 | 1955 | MPL-161-HL-vL |
| MPL | 182 | 1956 | MPL-161-vL |
| MPL | 183 | 1957 | MPL-175-vL |
| MPL | 184 | 1958 | MPL-178-vL |
| MPL | 185 | 1959 | MPL-huVB22Bw5-vL |
| MPL | 186 | 1960 | MPL-12E10-vL |
| MPL | 187 | 1961 | MPL-AB317-vL |
| Mucl/MHC class I complex | 188 | 1962 | MUC1-D6-M3A1-vL |
| Mucl/MHC class I complex | 189 | 1963 | Muc1-D6-M3B8-vL |
| Mucl6 | 190 | 1964 | Mucl6-4H11-vL |
| NKG2D | 191 | 1965 | NKG2D-MS-vL |
| NYBR1 | 192 | 1966 | NYBR1-vL |
| NY-ESO/MHC class I complex | 193 | 1967 | NY-ESO-T1-vL |
| PD1 | 194 | 1968 | PD1-4H1-vL |
| PD1 | 195 | 1969 | PD1-5C4-vL |
| PDL1 | 196 | 1970 | PDL1-10A5-vL |
| PDL1 | 197 | 1971 | PDL1-Atezoli-vL |
| PDL1 | 198 | 1972 | PDL1-SP142-vL |
| PR1/MHC class I complex | 199 | 1973 | PR1-vL |
| PSCA | 200 | 1974 | PSCA-Ha14-117-vL |
| PSCA | 201 | 1975 | PSCA-Ha14-121-vL |
| PSMA | 202 | 1976 | PSMA-006-vL |
| PSMA | 203 | 1977 | PSMA-J591-vL |
| PTK7 | 204 | 1978 | PTK7-hSC6-23-vL |
| PTK7 | 205 | 1979 | PTK7-SC6-10-2-vL |
| ROR1 | 206 | 1980 | ROR1-4A5-vL |
| ROR1 | 207 | 1981 | ROR1-4C10-vL |
| SLea | 208 | 1982 | SLea-5B1-vL |
| SLea | 209 | 1983 | SLea-7E3-vL |
| SSEA4 | 210 | 1984 | SSEA4-vL |
| TCRB1 | 211 | 1985 | TCRB1-E09-vL |
| TCRB1 | 212 | 1986 | TCRB1-Jovi1-vL |
| TCRB2 | 213 | 1987 | TCRB2-CP01-D05-vL |
| TCRB2 | 214 | 1988 | TCRB2-CP01-E05-vL |
| TCR gamma-delta (TCRgd) | 215 | 1989 | TCRgd-G5-4-vL |
| TERT/MHC class I complex | 216 | 1990 | TERT-3G3-T865-vL |
| TERT/MHC class I complex | 217 | 1991 | TERT-4A9-T540-vL |
| TF1 | 218 | 1992 | TF1-98-vL |
| TGFBR2 | 219 | 1993 | TGFBR2-Ab1-vL |
| TIM1 | 220 | 1994 | **TIM**1-HVCR1-270-2-vL |
| TIM1 | 221 | 1995 | Tim1HVCR1-ARD5-vL |
| TnAg | 222 | 1996 | TnAg-vL |
| Tn-Muc1 | 223 | 1997 | Tn-Muc1-hu5E5-vL |
| TROP2 | 224 | 1998 | TROP2-ARA47-HV3KV3-vL |
| TROP2 | 225 | 1999 | TROP2-h7E6-SVG-vL |
| TSHR | 226 | 2000 | TSHR-5C9-vL |
| TSHR | 227 | 2001 | TSHR-K1-70-vL |
| TSHR | 228 | 2002 | TSHR-KB1-vL |
| TSLPR | 229 | 2003 | TSLPR-vL |
| Tyrosinase/MHC class I complex | 230 | 2004 | Tvro-B2-vL |
| Tyrosinase/MHC class I complex | 231 | 2005 | Tvro-Mcl-vL |
| Tyrosinase/MHC class I complex | 232 | 2006 | TA2-vL |
| VEGFR3 | 233 | 2007 | VEGFR3-Ab1-vL |
| WT1/MHC class I complex | 234 | 2008 | WTl-Abl3-vL |
| WT1/MHC class I complex | 235 | 2009 | WT1-Ab15-vL |
| WTI/MHC class I complex | 236 | 2010 | WT1-Ab1-vL |
| WT1/MHC class I complex | 237 | 2011 | WT1-Ab5-vL |
| EBV-gp350 | 238 | 2012 | EBV-gp350-vL |
| CDH19 | 239 | 2013 | CDH19-4B10-vL |
| Folate Receptor beta (FRbeta) | 240 | 2014 | FRbeta-m923-vL |
| LHR | 241 | 2015 | LHR-8B7-vL |
| LHR | 242 | 2016 | LHR-5F4-21-vL |
| B7H4 | 243 | 2017 | B7H4-hu22C10-vL |
| B7H4 | 244 | 2018 | B7H4-hu1D11-vL |
| IgE | 245 | 2019 | IgE-omalizumab-vL |
| CD23 | 246 | 2020 | CD23-p5E8-vL |
| GCC | 247 | 2021 | GCC-5F9-vL |
| GCC | 248 | 2022 | GCC-Ab229-vL |
| CD200R | 249 | 2023 | CD200R-huDx182-vL |

**Table 5: Exemplary Linkers**

| **NAME** | **SEQ ID (DNA)** | **SEQ ID (PRT)** |
|---|---|---|
| (GGGGS)x3 LINKER | 250 | 2024 |
| (GGGGS)x3 Linker | 251 | 2024 |
| (Gly4Ser)x3 Linker | 252 | 2024 |
| (Glv4Ser)x3 Linker | 253 | 2024 |
| (GGGGS)x3-Linker | 254 | 2024 |
| (GGGGS)x3-Linker | 255 | 2024 |
| (GGSG)7 Linker | 256 | 2025 |
| (GGSG)7 Linker 2 | 257 | 2026 |
| DDAKK linker | 258 | 2027 |

**Table 6: Exemplary V_{H} fragments**

| **Target** | **SEQ ID (DNA)** | **SEQ ID (PRT)** | **CONSTRUCT NAME** |
|---|---|---|---|
| AFP/MHC class I complex | 259 | 2028 | AFP-61-vH |
| AFP/MHC class I complex | 260 | 2029 | AFP-76-vH |
| AFP/MHC class I complex | 261 | 2030 | AFP-79-vH |
| ALK | 262 | 2031 | Alk-48-vH |
| ALK | 263 | 2032 | Alk-58-vH |
| Amyloid | 264 | 2033 | Amyloid-158-vH |
| BCMA | 265 | 2034 | BCMA-ET-40-vH |
| BCMA | 266 | 2035 | BCMA-ET-54-vH |
| BCMA | 267 | 2036 | BCMA-huC12A3-vH |
| BCMA | 268 | 2037 | BCMA-J6M0-vH |
| BCMA | 269 | 2038 | BCMA-ET-03-vH |
| BCMA | 270 | 2039 | BCMA-huCl1.D5.3LlH3-vH |
| BCMA | 271 | 2040 | BCMA-huC13-F12-vH |
| CCR4 | 272 | 2041 | CCR4-humAb1567-vH |
| CD123 | 273 | 2042 | CD123-CSL362-vH |
| CD123 | 274 | 2043 | CD123-1172-vH |
| CD123 | 275 | 2044 | CD123-DART-1-vH |
| CD123 | 276 | 2045 | CD123-DART-2-vH |
| CD123 | 277 | 2046 | CD123-13RB18-vH |
| CD123 | 278 | 2047 | CD123-hu3E3-vH |
| CD123 | 279 | 2048 | CD123-9F6-vH |
| CD123 | 280 | 2049 | CD123-13RB2-vH |
| CD123 | 281 | 2050 | CD123-1176-vH |
| CD123 | 282 | 2051 | CD123-8B11-vH |
| CD123 | 283 | 2052 | CD123-2B8-vH |
| CD123 | 284 | 2053 | CD123-9D7-vH |
| CD123 | 285 | 2054 | CD123-3B10-vH |
| CD138 | 286 | 2055 | CD138-vH |
| CD179b | 287 | 2056 | CD179b-vH |
| CD19 | 288 | 2057 | CD19-4G7-vH |
| CD19 | 289 | 2058 | CD19Bu12-vH |
| CD19 | 290 | 2059 | CD19Bu12-[2]-vH |
| CD19 | 291 | 2060 | CD19MM-vH |
| CD19 | 292 | 2061 | FMC63-vH |
| CD19 | 293 | 2062 | FMC-63-vH |
| CD19 | 294 | 2063 | huFMC63-11-vH |
| CD19 | 295 | 2064 | CD19-MEDI-3649-vH |
| CD19 | 296 | 2065 | CD19-Medrex-24D1-vH |
| CD19 | 297 | 2066 | CD19-MOR0028-vH |
| CD19 | 298 | 2067 | CD19-HD37-H2L1-vH |
| CD19 | 299 | 2068 | CD19-huBly3-vH |
| CD19 | 300 | 2069 | CD19-huSJ25C1-vH |
| CD19 | 301 | 2070 | CD19-hB4-vH |
| CD19 | 302 | 2071 | CD19-hu-mROO5-1-vH |
| CD19 | 303 | 2072 | CD19-hA19-vH |
| CD20 | 304 | 2073 | CD20-2F2-vH |
| CD20 | 305 | 2074 | CD20-GA101-vH |
| CD20 | 306 | 2075 | CD20-Leu16-vH |
| CD20 | 307 | 2076 | CD20-11B8-vH |
| CD20 | 308 | 2077 | CD20-2C6-vH |
| CD20 | 309 | 2078 | CD20-2H7-vH |
| CD20 | 310 | 2079 | CD20-hA20-vH |
| CD20 | 311 | 2080 | CD20-BM-CA-1925-v4-vH |
| CD20 | 312 | 2081 | CD20-Ubli-v4-vH |
| CD20 | 313 | 2082 | CD20-2H7-vH |
| CD20 | 314 | 2083 | CD20-h1F5-vH |
| CD20 | 315 | 2084 | CD20-7D8-vH |
| CD20 | 316 | 2085 | CD20-AME-33-vH |
| CD22 | 317 | 2086 | CD22-h10F4-vH |
| CD22 | 318 | 2087 | CD22-H22Rhov2ACDRKA-vH |
| CD22 | 319 | 2088 | CD22m971-vH |
| CD276 | 320 | 2089 | CD276-17-vH |
| CD30 | 321 | 2090 | CD30-5F11-vH |
| CD30 | 322 | 2091 | CD30-Acl0-vH |
| CD32 | 323 | 2092 | CD32-Med9-vH |
| CD324 | 324 | 2093 | CD324-hSC10-17-vH |
| CD324 | 325 | 2094 | CD324-SC10-6-vH |
| CD33 | 326 | 2095 | CD33-huMyc9-vH |
| CD33 | 327 | 2096 | CD33-AF5-vH |
| CD33 | 328 | 2097 | CD33-Boehr2800308-vH |
| CD33 | 329 | 2098 | CD33-Him3-4-vH |
| CD33 | 330 | 2099 | CD33-SGNh2H12-vH |
| CD33 | 331 | 2100 | CD33-15G15-33-vH |
| CD33 | 332 | 2101 | CD33-33H4-vH |
| CD33 | 333 | 2102 | CD33-33H4-vH |
| CD33 | 334 | 2103 | CD33-9C3-2-vH |
| CD34 | 335 | 2104 | CD34-hu4C7-vH |
| CD44v6 | 336 | 2105 | CD44v6-Biwa8-vH |
| CD5 | 337 | 2106 | CD5-18-vH |
| CD5 | 338 | 2107 | CD5-9-vH |
| CD70 | 339 | 2108 | CD70-h1F6-vH |
| CD79b | 340 | 2109 | CD79b-2F2-vH |
| CD79b | 341 | 2110 | huMA79bv28-vH |
| CD99 | 342 | 2111 | CD99-hu12E7-vH |
| CDH17 | 343 | 2112 | CDH17-PTA001A4-vH |
| CDH19 | 344 | 2113 | CDH19-16A4-vH |
| CDH6 | 345 | 2114 | CDH6-NOV710-vH |
| CDH6 | 346 | 2115 | CDH6-NOV712-vH |
| CLEC5A | 347 | 2116 | CLEC5A-3E12A2-vH |
| CLEC5A | 348 | 2117 | CLEC5A-8H8F5-vH |
| CLL1 | 349 | 2118 | CLL1-M26-vH |
| CLL1 | 350 | 2119 | CLL1-M32-vH |
| CLL1 | 351 | 2120 | CLL1-21C9-L2H3-vH |
| CLL1 | 352 | 2121 | CLL1-6E7L4H1e-vH |
| CLL1 | 353 | 2122 | CLL1-hu1075-v1-vH |
| CLL1 | 354 | 2123 | CLL1-hu1075-v2-vH |
| CMVpp65/MHC class I complex | 355 | 2124 | CMVpp65-F5-vH |
| CS1 | 356 | 2125 | huLuc63-vH |
| CS1 | 357 | 2126 | HuLuc64-vH |
| CS1 | 358 | 2127 | Luc90-vH |
| CS1 | 359 | 2128 | CS1-PDL241-vH |
| CS1 | 360 | 2129 | CS1-Hu27A-vH |
| CS1 | 361 | 2130 | CS1-ScHu34C3-vH |
| CS1 | 362 | 2131 | CS1-Hu31-D2-vH |
| CS1 | 363 | 2132 | CS1-Luc34-vH |
| CS1 | 364 | 2133 | CS1-LucX2-vH |
| CSF2RA | 365 | 2134 | CSF2RA-Abl-vH |
| CSF2RA | 366 | 2135 | CSF2RA-Ab6-vH |
| DLL3 | 367 | 2136 | DLL3-hSC16-13-vH |
| DLL3 | 368 | 2137 | DLL3-hSC16-56-vH |
| EBNA3c/MHC class I complex | 369 | 2138 | EBNA3c-315-vH |
| EGFR | 370 | 2139 | Cetuximab-vH |
| EGFR | 371 | 2140 | Nimotuzumab-vH |
| EGFRviii | 372 | 2141 | EGFRviii-139-vH |
| EGFRviii | 373 | 2142 | EGFRviii-2173-vH |
| EpCam1 | 374 | 2143 | EpCam1-D5K5-vH |
| EpCam1 | 375 | 2144 | Epcam1-MM1-vH |
| FITC | 376 | 2145 | FITC-vH |
| FITC | 377 | 2146 | FITC-4M-53-vH |
| FITC | 378 | 2147 | FITC-E2-vH |
| FLT3 | 379 | 2148 | FLT3-NC7-vH |
| HIV 1-envelop glycoprotein | 380 | 2149 | HIV1-N6-vH |
| FR1 (Folate Receptor alpha) | 381 | 2150 | FR1-huMov19-vH |
| GAD65/MHC class I complex | 382 | 2151 | GAD-G3H8-vH |
| GD2 | 383 | 2152 | GD2-hu14-18-vH |
| GD2 | 384 | 2153 | GD2-hu3F8-vH |
| GD3 | 385 | 2154 | GD3-KM-641-vH |
| GFRa4 | 386 | 2155 | GFRa4-P4-10-vH |
| GFRa4 | 387 | 2156 | GFRAlpha4-P4-6-vH |
| GM1 | 388 | 2157 | GM1-5B2-vH |
| GM1 | 389 | 2158 | GM1-7E5-vH |
| gp100/MHC class I complex | 390 | 2159 | gp100-G2D12-vH |
| gp100/MHC class I complex | 391 | 2160 | gp100-vH |
| GPC3 | 392 | 2161 | GPC3-4E5-vH |
| gpNMB | 393 | 2162 | gpNMB-115-vH |
| GPRC5D | 394 | 2163 | GPRC5D-ET150-18-vH |
| GPRC5D | 395 | 2164 | GPRC5D-ET150-5-vH |
| GPRC5D | 396 | 2165 | GPRC5D-ET150-1-vH |
| GPRC5D | 397 | 2166 | GPRC5D-ET150-2-vH |
| Her2 | 398 | 2167 | Her2-Hu4D5-vH |
| HIV 1-envelop glycoprotein | 399 | 2168 | HIV1-3BNC117-vH |
| HIV1-gag (77-85) MHC class I complex HIV1 -envelop glycoprotein | 400 | 2169 | HIV1-E5-vH |
| HIV1-envelop glycoprotein | 401 | 2170 | HIV1-PGT-128-vH |
| HIV1-envelop glycoprotein | 402 | 2171 | HIV1-VR-C01-vH |
| HIV1-envelop glycoprotein | 403 | 2172 | HIV1-X5-vH |
| HLA-A2 | 404 | 2173 | HLA-A2-3PB2-vH |
| HMW-MAA | 405 | 2174 | HMW-MAA-hIND-vH |
| HPV16-E7/MHC class I | 406 | 2175 | HPV16-7-8-vH |
| complex | | | |
| HPV16-E7/MHC class I complex | 407 | 2176 | HPV16-2-vH |
| HTLV1-TAX/MHC class I | 408 | 2177 | TAX-T3E3-vH |
| HTLV1-TAX/MHC class I | 409 | 2178 | TAX-T3F2-vH |
| II11Ra | 410 | 2179 | IL11Ra-8E2-vH |
| IL13Ra2 | 411 | 2180 | IL13Ra2-hu107-vH |
| IL13Ra2 | 412 | 2181 | IL13Ra2-Hu108-vH |
| IL6R | 413 | 2182 | IL6R-M83-vH |
| Influenza A HA | 414 | 2183 | FLU-MEDI-8852-vH |
| KSHV-gH | 415 | 2184 | YC15-vH |
| KSHV-K8.1 | 416 | 2185 | 4C3-vH |
| L1CAM | 417 | 2186 | L1CAM-9-3-HU3-vH |
| LAMP1 | 418 | 2187 | LAMP1-humab1-2-vH |
| LAMP1 | 419 | 2188 | LAMP1-Mb4-vH |
| LewisY | 420 | 2189 | LewisY-huS193-vH |
| Lym1 | 421 | 2190 | Lym1-vH |
| Lym2 | 422 | 2191 | Lym2-vH |
| MART1/MHC class I complex | 423 | 2192 | MART1-CAG10-vH |
| MART1/MHC class I complex | 424 | 2193 | MART1-CLA12-vH |
| Mesothelin | 425 | 2194 | Mesothelin-m912-[2]-vH |
| Mesothelin | 426 | 2195 | Mesothelin-m912-vH |
| MPL | 427 | 2196 | MPL-111-vH |
| MPL | 428 | 2197 | MPL-161-HL-vH |
| MPL | 429 | 2198 | MPL-161-vH |
| MPL | 430 | 2199 | MPL-175-vH |
| MPL | 431 | 2200 | MPL-178-vH |
| MPL | 432 | 2201 | MPL-huVB22Bw5-vH |
| MPL | 433 | 2202 | MPL-12E10-vH |
| MPL | 434 | 2203 | MPL-AB317-vH |
| Mucl/MHC class I complex | 435 | 2204 | MUC1-D6-M3A1-vH |
| Muc1/MHC class I complex | 436 | 2205 | Muc1-D6-M3B8-vH |
| Mucl6 | 437 | 2206 | Muc16-4H11-vH |
| NKG2D | 438 | 2207 | NKG2D-MS-vH |
| NYBR1 | 439 | 2208 | NYBR1-vH |
| NY-ESO/MHC class I complex | 440 | 2209 | NY-ESO-T1-vH |
| NY-ESO/MHC class I complex | 441 | 2210 | NY-ESO-T2-vH |
| PD1 | 442 | 2211 | PD1-4H1-vH |
| PD1 | 443 | 2212 | PD1-5C4-vH |
| PDL1 | 444 | 2213 | PDL1-Atezoli-vH |
| PDL1 | 445 | 2214 | PDL1-SP142-vH |
| PR1 | 446 | 2215 | PR1-vH |
| PSCA | 447 | 2216 | PSCA-Ha14-117-vH |
| PSCA | 448 | 2217 | PSCA-Ha14-121-vH |
| PSMA | 449 | 2218 | PSMA-006-vH |
| PSMA | 450 | 2219 | PSMA-J591-vH |
| PTK7 | 451 | 2220 | PTK7-hSC6-23-vH |
| PTK7 | 452 | 2221 | PTK7-SC6-10-2-vH |
| ROR1 | 453 | 2222 | ROR1-4A5-vH |
| ROR1 | 454 | 2223 | ROR1-4C10-vH |
| SLea | 455 | 2224 | SLea-5Bl-vH |
| SLea | 456 | 2225 | SLea-7E3-vH |
| SSEA4 | 457 | 2226 | SSEA4-vH |
| TCRB1 (TCR-betal constant chain) | 458 | 2227 | TCRB1-E09-vH |
| TCRB1 | 459 | 2228 | TCRB1-Jovi1-vH |
| TCRB2 (TCR-beta2 constant chain) | 460 | 2229 | TCRB2-CP01-D05-vH |
| TCRB2 | 461 | 2230 | TCRB2-CP01-E05-vH |
| TCR gamma-delta (TCRgd) | 462 | 2231 | TCRgd-G5-4-vH |
| TERT/MHC class I complex | 463 | 2232 | TERT-3G3-T865-vH |
| TERT/MHC class I complex | 464 | 2233 | TERT-4A9-T540-vH |
| TF1 (Tissue Factor 1) | 465 | 2234 | TF1-98-vH |
| TGFBR2 | 466 | 2235 | TGFBR2-Abl-vH |
| TIM1 | 467 | 2236 | TIM1-HVCR1-270-2-vH |
| TIM1 | 468 | 2237 | Tim1HVCR1-ARD5-vH |
| TnAg | 469 | 2238 | TnAg-vH |
| Tn-Muc1 | 470 | 2239 | Tn-Muc1-hu5E5-vH |
| TROP2 | 471 | 2240 | TROP2-ARA47-HV3KV3-vH |
| TROP2 | 472 | 2241 | TROP2-h7E6-SVG-vH |
| TSHR (Thyrotropin receptor) | 473 | 2242 | TSHR-5C9-vH |
| TSHR | 474 | 2243 | TSHR-K1-70-vH |
| TSHR | 475 | 2244 | TSHR-KB1-vH |
| TSLPR (Thymic stromal lymphopoietin receptor) | 476 | 2245 | TSLPR-vH |
| Tyrosinase/MHC class I complex | 477 | 2246 | Tyro-B2-vH |
| Tyrosinase/MHC class 1 complex | 478 | 2247 | Tyro-Mcl-vH |
| Tyrosinase/MHC class 1 complex | 479 | 2248 | TA2-vH |
| VEGFR3 | 480 | 2249 | VEGFR3-Abl-vH |
| WTl/MHC class I complex | 481 | 2250 | WT1-Ab13-vH |
| WT1/MHC class I complex | 482 | 2251 | WT1-Ab15-vH |
| WT1/MHC class I complex | 483 | 2252 | WT1-Ab1-vH |
| WT1/MHC class I complex | 484 | 2253 | WT1-Ab5-[2]-vH |
| WT1/MHC class I complex | 485 | 2254 | WT1-Ab5-vH |
| EBV-gp350 | 486 | 2255 | EBV-gp350-vH |
| CDH19 | 487 | 2256 | CDH19-4B10-vH |
| Folate Receptor beta (FRbeta) | 488 | 2257 | FRbeta-m923-vH |
| LHR (Luteinizing hormone Receptor) | 489 | 2258 | LHR-8B7-vH |
| LHR | 490 | 2259 | LHR-5F4-21-vH |
| B7H4 | 491 | 2260 | B7H4-hu22Cl0-vH |
| B7H4 | 492 | 2261 | B7H4-hu1D11-vH |
| IgE | 493 | 2262 | IgE-omalizumab-vH |
| CD23 | 494 | 2263 | CD23-p5E8-vH |
| GCC (Guanylyl cyclase C) | 495 | 2264 | GCC-5F9-vH |
| GCC (Guanylyl cyclase C) | 496 | 2265 | GCC-Ab229-vH |
| CD200R | 497 | 2266 | CD200R-huDx182-vH |
| PDL1 | 498 | 2268 | PDL1-10A5-vH |

**Table 7: Exemplary V_{HH} fragments (Nanobodies)**

| **Target** | **SEQ ID (DNA)** | **SEQ ID (PRT)** | **NAME** |
|---|---|---|---|
| Her2 | 499 | 2269 | Her2-2D3-vHH |
| Her2 | 500 | 2270 | Her2-5F7-vHH |
| Her2 | 501 | 2271 | Her2-47D5-vHH |
| Her3 | 502 | 2272 | Her3-17B05So-vHH |
| Her3 | 503 | 2273 | Her3-21F06-vHH |
| CEA | 504 | 2274 | CEA1-vHH |
| CEA | 505 | 2275 | CEA5-vHH |
| EGFR | 506 | 2276 | EGFR1-vHH |
| EGFR | 507 | 2277 | EGFR33-vHH |
| cMet | 508 | 2278 | cMET-171-vHH |
| CXCR4 | 509 | 2279 | CXCR4-2-vHH |
| CXCR4 | 510 | 2280 | CXCR4-1-vHH |
| Mesothelin | 511 | 2281 | SD1-vHH |
| Mesothelin | 512 | 2282 | SD2-vHH |
| Albumin | 513 | 2283 | Alb8-vHH |
| CD123 | 514 | 2284 | CD123-1-vHH |
| CD123 | 515 | 2285 | CD123-2-vHH |
| IL6R | 516 | 2286 | IL6R-304-vHH |
| EGFR & CEA | 517 | 2287 | EGFR1-vHH-Gly-Ser-Linker-CEA5-vHH |
| EGFR & CEA | 518 | 2288 | EGFR33-vHH-Gly-Ser-Linker-CEA5-vHH |
| Her2 | 519 | 2289 | Her2-5F7-vHH-Gly-Ser-Linker-Her2-47D5-vHH |
| Her2 | 520 | 2290 | Her2-Hu4D5-vL-Gly-Ser-Linker-Her2-Hu4D5-vH |
| Her3 & Her2 | 521 | 2291 | Her3-17B05So-vHH-Gly-Ser-Linker-Her2-2D3-vHH |
| cMet & Her3 | 522 | 2292 | cMET-171-vHH-Gly-Ser-Linker-Her3-21F06-vHH |
| Mesothelin | 523 | 2293 | SD1-vHH-Gly-Ser-Linker-SD2-vHH |

**Table 8: Exemplary non-immunoglobulin antigen binding scaffolds (affibodies, darpins).**

| **Target** | **SEQ ID (DNA)** | **SEQ ID (PRT)** | **NAME** |
|---|---|---|---|
| Her2 | 524 | 2294 | Her2-DARPIN-1 |
| Her2 | 525 | 2295 | Her2-DARPIN-2 |
| Her3 | 526 | 2296 | Her3-affi |
| Her2 | 527 | 2297 | Her2-affi |
| EGFR | 528 | 2298 | EGFR-affi |

**Table 9: Exemplary receptor extracellular domain**

| **SEQ ID (DNA)** | **SEQ ID (PRT)** | **NAME** |
|---|---|---|
| 529 | 2299 | CD19-Extracellular-Domain-minus-signal-peptide(61-867) |
| 530 | 2300 | MPL-Extracellular-Domain-with-signal-ptepide |
| 531 | 2301 | CD8-SP-PD1-opt-ECD |
| 532 | 2302 | PD1-opt-ECD-minus-signal-peptide |
| 533 | 2303 | PD1-ECD-with-native-Signal-Peptide |
| 534 | 2304 | CTLA4-opt-ECD with signal peptide |
| 535 | 2305 | CD138-SDC1-ECD |
| 536 | 2306 | Synth-CD123-ECD |
| 537 | 2307 | CDH1-ECD |
| 538 | 2308 | CD200RIL-ECD |
| 539 | 2309 | GPNMB-ECD |
| 540 | 2310 | PTK7-ECD |
| 541 | 2311 | CD33-ECD |
| 542 | 2312 | CD34-ECD |
| 543 | 2313 | EpCAM-ECD |
| 544 | 2314 | CLEC12A-ECD |
| 545 | 2315 | CD20-ECx2-ECD |
| 546 | 2316 | CD20-ECx1-ECD |
| 547 | 2317 | CD22-v5-ECD |
| 548 | 2318 | Thyroid Stimulating Hormone Receptor (TSHR)-ECD |
| 549 | 2319 | EGFRviii-ECD |
| 550 | 2320 | BCMA-ECD |
| 551 | 2321 | SLAMF7-CS1-ECD |
| 552 | 2322 | NKG2D-ECD-minus-signal-peptide |

**Table 10: Exemplary ligands**

| **SEQ ID (DNA)** | **SEQ ID (PRT)** | **NAME** |
|---|---|---|
| 553 | 2323 | hTPO (1-187) |
| 554 | 2324 | mTPO(1-187) |
| 555 | 2325 | CGH-alpha-minus-Signal-Peptide |
| 556 | 2326 | CGH-beta-with-Signal-Peptide |
| 557 | 2327 | FSH-beta-minus-Signal-Peptide |
| 558 | 2328 | LH-beta-with-Signal-Peptide |
| 559 | 2329 | TSH-beta-with-Signal-Peptide |
| 560 | 2330 | SP-CGHb-Gly-Ser-Linker-CGHa |
| 561 | 2331 | CD8SP-FSHb-Gly-Ser-Linker-CGHa |
| 562 | 2332 | SP-LHb-Gly-Ser-Linker-CGHa |
| 563 | 2333 | SP-TSHb-Gly-Ser-Linker-CGHa |

**Table 11: Exemplary scFv fragments**

| **Target** | **SEQ ID** | **SEQ ID** | **CONSTRUCT NAME** |
|---|---|---|---|
| CD19 | 564 | 2334 | FMC63-(vL-vH) |
| CD19 | 565 | 2335 | huFMC63-11-(vL-vH) |
| CD19 | 566 | 2336 | CD19Bu12-(vL-vH) |
| CD19 | 567 | 2337 | CD19MM-(vL-vH) |
| CD19 | 568 | 2338 | CD19-4G7-(vL-vH) |
| CD19 | 569 | 2339 | CD19-MEDI-3649-(vL-vH) |
| CD19 | 570 | 2340 | CD19-Medrex-24D1-(vL-vH) |
| CD19 | 571 | 2341 | CD8SP-Ritx-CD19-MOR0028-(vL-vH) |
| CD19 | 572 | 2342 | CD19-HD37-H2L1-(vL-vH) |
| CD19 | 573 | 2343 | CD19-huBly3-(vL-vH) |
| CD19 | 574 | 2344 | CD19-huSJ25C1-(vL-vH) |
| CD19 | 575 | 2345 | CD8SP-Ritx-CD19-hB4-(vL-vH) |
| CD19 | 576 | 2346 | CD19-hu-mR005-1-(vL-vH) |
| CD19 | 577 | 2347 | CD19-hA19-(vL-vH) |
| AFP/MHC class I complex | 578 | 2348 | AFP-61-(vL-vH) |
| AFP/MHC class I complex | 579 | 2349 | AFP-76-(vL-vH) |
| AFP/MHC class I complex | 580 | 2350 | AFP-79-(vL-vH) |
| HIV1-env | 581 | 2351 | HIV1-N6-(vL-vH) |
| ALK | 582 | 2352 | Alk-48-(vL-vH) |
| ALK | 583 | 2353 | Alk-58-(vL-vH) |
| Amyloid | 584 | 2354 | Amyloid-158-(vL-vH) |
| CD45 | 585 | 2355 | BC8-CD45-(vL-vH) |
| BCMA | 586 | 2356 | BCMA-J6M0-(vL-vH) |
| BCMA | 587 | 2357 | BCMA-huC12A3-L3H3-(vL-vH) |
| BCMA | 588 | 2358 | BCMA-ET-40-(vL-vH) |
| BCMA | 589 | 2359 | BCMA-ET-54-(vL-vH) |
| BCMA | 590 | 2360 | BCMA-ET-03-(vL-vH) |
| BCMA | 591 | 2361 | BCMA-huC11.D5.3L1H3-(vL-vH) |
| BCMA | 592 | 2362 | BCMA-huC13-F12-(vL-vH) |
| CCR4 | 593 | 2363 | CCR4-humAb1567-(vL-vH) |
| CD5 | 594 | 2364 | CD5-9-(vL-vH) |
| CD5 | 595 | 2365 | CD5-18-(vL-vH) |
| CD20 | 596 | 2366 | CD20-2F2-(vL-vH) |
| CD20 | 597 | 2367 | CD20-GA101-(vL-vH) |
| CD22 | 598 | 2368 | CD22-h10F4v2-(vL-vH) |
| CD20 | 599 | 2369 | CD20-Leu16-(vL-vH) |
| CD20 | 600 | 2370 | CD20-11B8-(vL-vH) |
| CD20 | 601 | 2371 | CD20-2C6-(vL-vH) |
| CD20 | 602 | 2372 | CD20-2H7-(vL-vH) |
| CD20 | 603 | 2373 | CD20-hA20-(vL-vH) |
| CD20 | 604 | 2374 | CD20-BM-CA-1925-v4-(vL-vH) |
| CD20 | 605 | 2375 | CD20-Ubli-v4-(vL-vH) |
| CD20 | 606 | 2376 | CD20-2H7-(vL-vH) |
| CD20 | 607 | 2377 | CD20-h1F5-(vL-vH) |
| CD20 | 608 | 2378 | CD20-7D8-(vL-vH) |
| CD20 | 609 | 2379 | CD20-7D8-(vL-GA-tag-vH) |
| CD20 | 610 | 2380 | CD20-AME-33-(vL-vH) |
| CD22 | 611 | 2381 | CD22-H22Rhov2ACDRKA-(vL-vH) |
| CD22 | 612 | 2382 | CD22-m971-(vL-vH) |
| CD22 | 613 | 2383 | CD22-m971-HL-(vH-vL) |
| CD30 | 614 | 2384 | CD30-5F11-(vL-vH) |
| CD30 | 615 | 2385 | CD30-Ac10-(vL-vH) |
| CD32 | 616 | 2386 | CD32-Med9-(vL-vH) |
| CD33 | 617 | 2387 | CD33-AF5-(vL-vH) |
| CD33 | 618 | 2388 | CD33-huMyc9-(vL-vH) |
| CD33 | 619 | 2389 | CD8SP-Ritx2-BC33-Boehr2800308- |
| CD33 | 620 | 2390 | CD8SP-Ritx2-CD33-Him3-4-(vL-vH) |
| CD33 | 621 | 2391 | CD33-SGNh2H12-(vL-vH) |
| CD33 | 622 | 2392 | CD33-15G15-33-(vL-vH) |
| CD33 | 623 | 2393 | CD33-33H4-(vL-vH) |
| CD33 | 624 | 2394 | CD33-9C3-2-(vL-vH) |
| CD34 | 625 | 2395 | CD34-hu4C7-(vL-vH) |
| CD44v6 | 626 | 2396 | CD44v6-Biwa8-(vL-vH) |
| CD70 | 627 | 2397 | CD70-h1F6-(vL-vH) |
| CD79b | 628 | 2398 | CD79b-2F2-(vL-vH) |
| CD99 | 629 | 2399 | CD99-hu12E7-(vL-vH) |
| CD123 | 630 | 2400 | CD123-CSL362-(vL-vH) |
| CD123 | 631 | 2401 | CD123-1172-(vL-vH) |
| CD123 | 632 | 2402 | CD123-DART1-1-(vL-vH) |
| CD123 | 633 | 2403 | CD123-DART1-2-(vL-vH) |
| CD123 | 634 | 2404 | CD123-13RB18-(vL-vH) |
| CD123 | 635 | 2405 | CD123-hu3E3-(vL-vH) |
| CD123 | 636 | 2406 | CD123-9F6-(vL-vH) |
| CD123 | 637 | 2407 | CD123-I3RB2-(vL-vH) |
| CD123 | 638 | 2408 | CD123-1176-(vL-vH) |
| CD123 | 639 | 2409 | CD8SP-Ritx2-CD123-8B11-(vL-vH) |
| CD123 | 640 | 2410 | CD123-2B8-(vL-vH) |
| CD123 | 641 | 2411 | CD123-9D7-(vL-vH) |
| CD123 | 642 | 2412 | CD123-3B10-(vL-vH) |
| CD138 | 643 | 2413 | CD138-(vL-vH) |
| CD179b | 644 | 2414 | CD179b-(vL-vH) |
| CD276 | 645 | 2415 | CD276-17-(vL-vH) |
| CD324 | 646 | 2416 | CD324-SC10-6-(vL-vH) |
| CD324 | 647 | 2417 | CD324-hSC10-17-(vL-vH) |
| CDH6 | 648 | 2418 | CDH6-NOV710-(vL-vH) |
| CDH6 | 649 | 2419 | CDH6-NOV712-(vL-vH) |
| CDH17 | 650 | 2420 | CDH17-PTA001A4-(vL-vH) |
| CDH19 | 651 | 2421 | CDH19-16A4-(vL-vH) |
| EGFR | 652 | 2422 | Cetuximab-(vL-vH) |
| CLEC5A | 653 | 2423 | CLEC5A-8H8F5-(vL-vH) |
| CLEC5A | 654 | 2424 | CLEC5A-3E12A2-(vL-vH) |
| CLL1 | 655 | 2425 | CLL1-M26-(vL-vH) |
| CLL1 | 656 | 2426 | CLL1-M32-(vL-vH) |
| CLL1 | 657 | 2427 | CLL1-21C9-L2H3-(vL-vH) |
| CLL1 | 658 | 2428 | CLL1-6E7L4Hle-(vL-vH) |
| CLL1 | 659 | 2429 | CLL1-hu1075-v1-(vL-vH) |
| CLL1 | 660 | 2430 | CLL1-hu1075-v2-(vL-vH) |
| CMVpp65/MHC class I | 661 | 2431 | CMVpp65-F5-(vL-vH) |
| CS1 | 662 | 2432 | CS1-huLuc63-(vL-vH) |
| CS1 | 663 | 2433 | CS1-HuLuc64-(vL-vH) |
| CS1 | 664 | 2434 | CS1-Luc90-(vL-vH) |
| CS1 | 665 | 2435 | CS1-PDL241-(vL-vH) |
| CS1 | 666 | 2436 | CS1-Hu27A-(vL-vH) |
| CS1 | 667 | 2437 | CS1-ScHu34C3-(vL-vH) |
| CS1 | 668 | 2438 | CS1-Hu31-D2-(vL-vH) |
| CS1 | 669 | 2439 | CS1-Luc34-(vL-vH) |
| CS1 | 670 | 2440 | CS1-LucX2-(vL-vH) |
| CSF2RA | 671 | 2441 | CSF2RA-Ab6-(vL-vH) |
| CSF2RA | 672 | 2442 | CSF2RA-Abl-(vL-vH) |
| DLL3 | 673 | 2443 | DLL3-hSC16-13-(vL-vH) |
| DLL3 | 674 | 2444 | DLL3-hSC16-56-(vL-vH) |
| EBNA3c/MHC class I | 675 | 2445 | EBNA3c-315-(vL-vH) |
| EBV-gp350 | 676 | 2446 | EBV-gp350-(vL-vH) |
| EGFRvIII | 677 | 2447 | EGFRvIII-139-(vL-vH) |
| EGFRvIII | 678 | 2448 | EGFRvIII-2173-(vH-vL) |
| EpCam1 | 679 | 2449 | Epcam1-MM1-(vL-vH) |
| EpCam1 | 680 | 2450 | Epcam1-D5K5-(vL-vH) |
| FLT3 | 681 | 2451 | FLT3-NC7-(vL-vH) |
| FITC | 682 | 2452 | FITC-(vL-vH) |
| FITC | 683 | 2453 | FITC-4M-53-(vL-vH) |
| FITC | 684 | 2454 | FITC-E2-HL-(vH-vL) |
| Influenza A HA | 685 | 2455 | FLU-MEDI-8852-(vL-vH) |
| FR1 (Folate Receptor a) | 686 | 2456 | FR1-huMov19-(vL-vH) |
| GAD | 687 | 2457 | GAD-G3H8-(vL-vH) |
| GD2 | 688 | 2458 | GD2-hu 14-18-(vL-vH) |
| GD2 | 689 | 2459 | GD2-hu3F8-(vL-vH) |
| GD3 | 690 | 2460 | GD3-KM-641-(vL-vH) |
| GFRa4 | 691 | 2461 | GFRAlpha4-P4-6-(vL-vH) |
| GFRa4 | 692 | 2462 | GFRa4-P4-10-(vL-vH) |
| GM1 | 693 | 2463 | GM1-5B2-(vL-vH) |
| GM1 | 694 | 2464 | GM 1-7E5-(vL-vH) |
| GPRC5D | 695 | 2465 | GPRC5D-ET150-5-(vL-vH) |
| GPRC5D | 696 | 2466 | GPRC5D-ET150-18-(vL-vH) |
| GPRC5D | 697 | 2467 | GPRC5D-ET150-1-(vL-vH) |
| GPRC5D | 698 | 2468 | GPRC5D-ET150-2-(vL-vH) |
| gp 100/MHC class I | 699 | 2469 | gp100-(vL-vH) |
| gp 100/MHC class I | 700 | 2470 | gp100-G2D12-(vL-vH) |
| GPC3 | 701 | 2471 | GPC3-4E5-(vL-vH) |
| gpNMB | 702 | 2472 | gpNMB-115-(vL-vH) |
| GRP78 | 703 | 2473 | GRP78-GC18-(vL-vH) |
| HIV-gag/MHC class I | 704 | 2474 | HIV1-E5-(vL-vH) |
| HIV1-env | 705 | 2475 | HIV1-3BNC117-(vL-vH) |
| HIV1-env | 706 | 2476 | HIV1-PGT-128-(vL-vH) |
| HIV1-env | 707 | 2477 | HIV1-VR-C01-(vL-vH) |
| HIV1-env | 708 | 2478 | HIV1-X5-(vL-vH) |
| HLA-A2 | 709 | 2479 | HLA-A2-3PB2-(vL-vH) |
| HMW-MAA | 710 | 2480 | HMW-MAA-hIND-(vL-vH) |
| HPV16 | 711 | 2481 | HPV16-7-8-(vL-vH) |
| HPV16 | 712 | 2482 | HPV16-2-(vL-vH) |
| HTLV1-TAX/MHC class I | 713 | 2483 | HTLV-TAX-T3F2-(vL-vH) |
| HTLV1-TAX/MHC class I | 714 | 2484 | HTLV-TAX-T3E3-(vL-vH) |
| IL11Ra | 715 | 2485 | IL11Ra-8E2-Ts107-(vL-vH) |
| IL13Ra2 | 716 | 2486 | IL13Ra2-hu107-(vL-vH) |
| IL13Ra2 | 717 | 2487 | IL13Ra2-Hu108-(vL-vH) |
| KSHV-K8.1 | 718 | 2488 | KSHV-4C3-(vL-vH) |
| LAMP1 | 719 | 2489 | LAMP1-humab1-2-(vL-vH) |
| LAMP1 | 720 | 2490 | LAMP1-Mb4-(vL-vH) |
| LewisY | 721 | 2491 | LewisY-huS193-(vL-vH) |
| L1CAM | 722 | 2492 | L1CAM-9-3-HU3-(vL-vH) |
| Lym1 | 723 | 2493 | Lym1-(vL-vH) |
| Lym2 | 724 | 2494 | Lym2-(vL-vH) |
| CD79b | 725 | 2495 | huMA79bv28-(vL-vH) |
| MART/MHC class I | 726 | 2496 | MART1-CAG10-(vL-vH) |
| MART/MHC class I | 727 | 2497 | MART1-CLA12-(vL-vH) |
| Mesothelin | 728 | 2498 | Mesothelin-m912-(vL-vH) |
| MPL | 729 | 2499 | MPL-175-(vL-vH) |
| MPL | 730 | 2500 | MPL-161-(vL-vH) |
| MPL | 731 | 2501 | MPL-161-HL-(vH-vL) |
| MPL | 732 | 2502 | MPL-111-(vL-vH) |
| MPL | 733 | 2503 | MPL-178-(vL-vH) |
| MPL | 734 | 2504 | MPL-AB317-(vL-vH) |
| MPL | 735 | 2505 | MPL-12E10-(vL-vH) |
| MPL | 736 | 2506 | MPL-huVB22Bw5-(vL-vH) |
| Muc1/MHC class I complex | 737 | 2507 | Muc1-D6-M3B8-(vL-vH) |
| Muc1/MHC class I complex | 738 | 2508 | MUC1-D6-M3A1-(vL-vH) |
| Mucl6 | 739 | 2509 | Muc16-4H11-(vL-vH) |
| EGFR | 740 | 2510 | Nimotuzumab-(vL-vH) |
| NKG2D | 741 | 2511 | NKG2D-MS-(vL-vH) |
| NYBR1 | 742 | 2512 | NYBR1-(vL-vH) |
| NY-ESO/MHC class I | 743 | 2513 | NYESO-T1-(vL-vH) |
| NY-ESO/MHC class I | 744 | 2514 | NYESO-T2-(vL-vH) |
| PDL1 | 745 | 2515 | PDL1-Atezoli-(vL-vH) |
| PDL1 | 746 | 2516 | PDL1-SP142-(vL-vH) |
| PDL1 | 747 | 2517 | PDL1-10A5-(vL-vH) |
| PSCA | 748 | 2518 | PSCA-Ha14-121-(vL-vH) |
| PSCA | 749 | 2519 | PSCA-Ha14-117-(vL-vH) |
| PR1/MHC class I complex | 750 | 2520 | PR1-(vL-vH) |
| PSMA | 751 | 2521 | PSMA-006-(vL-vH) |
| PSMA | 752 | 2522 | PSMA-J591-(vL-vH) |
| PTK7 | 753 | 2523 | PTK7-hSC6-23-(vL-vH) |
| PTK7 | 754 | 2524 | PTK7-SC6-10-2-(vL-vH) |
| ROR1 | 755 | 2525 | ROR1-4A5-(vL-vH) |
| ROR1 | 756 | 2526 | ROR1-4C10-(vL-vH) |
| Mesothelin | 757 | 2527 | SD1-vHH-Gly-Ser-Linker-SD2-vHH |
| SLea | 758 | 2528 | SLea-7E3-(vL-vH) |
| SLea | 759 | 2529 | SLea-5B1-(vL-vH) |
| SSEA4 | 760 | 2530 | SSEA4-(vL-vH) |
| TCRB1 | 761 | 2531 | TCRB1-CP01-E09-(vL-vH) |
| TCRB1 | 762 | 2532 | TCRB1-Jovi1-(vL-vH) |
| TCRB2 | 763 | 2533 | TCRB2-CP01-D05-(vL-vH) |
| TCRB2 | 764 | 2534 | TCRB2-CP01-E05-(vL-vH) |
| TCRgd | 765 | 2535 | TCRgd-G5-4-(vL-vH) |
| hTERT | 766 | 2536 | TERT-4A9-T540-(vL-vH) |
| hTERT | 767 | 2537 | TERT-3G3-T865-(vL-vH) |
| Tissue Factor-1 | 768 | 2538 | TF1-98-(vL-vH) |
| TGFBR2 | 769 | 2539 | TGFBR2-Abl-(vL-vH) |
| TIM1 | 770 | 2540 | TIM1-HVCR1-270-2-(vL-vH) |
| TIM1 | 771 | 2541 | TIM1-HVCR1-ARD5-(vL-vH) |
| TnAg | 772 | 2542 | TnAg-(vL-vH) |
| Tn-Muc 1 | 773 | 2543 | TnMuc1-hu5E5-RHA8-RKA-2-(vL-vH) |
| TROP2 | 774 | 2544 | TROP2-ARA47-HV3KV3-(vL-vH) |
| TROP2 | 775 | 2545 | TROP2-h7E6-SVG-(vL-vH) |
| TSHR | 776 | 2546 | TSHR-K1-70-(vL-vH) |
| TSHR | 777 | 2547 | TSHR-KB 1-(vL-vH) |
| TSHR | 778 | 2548 | TSHR-5C9-(vL-vH) |
| TSLPR | 779 | 2549 | TSLPR-(vL-vH) |
| Tyrosinase/MHC class I | 780 | 2550 | Tyros-B2-(vL-vH) |
| Tyrosinase/MHC class I | 781 | 2551 | Tyros-MC1-(vL-vH) |
| Tyrosinase/MHC class I | 782 | 2552 | Tyros-TA2-(vL-vH) |
| VEGFR3 | 783 | 2553 | VEGFR3-Ab 1-(vL-vH) |
| WT1/MHC class I complex | 784 | 2554 | WT1-Ab1-(vL-vH) |
| WT1/MHC class I complex | 785 | 2555 | WTI1-Ab5-(vL-vH) |
| WT1/MHC class I complex | 786 | 2556 | WT1-Ab13-(vL-vH) |
| WT1/MHC class I complex | 787 | 2557 | WT1-Ab15-(vL-vH) |
| CDH 19 | 788 | 2558 | CDH 19-4B 10-(vL-vH) |
| Folate Receptor beta | 789 | 2559 | FRbeta-m923-(vL-vH) |
| LHR | 790 | 2560 | LHR-8B7-(vL-vH) |
| LHR | 791 | 2561 | LHR-5F4-21-(vL-vH) |
| B7H4 | 792 | 2562 | B7H4-hu22C10-(vL-vH) |
| B7H4 | 793 | 2563 | B7H4-hu1D11-(vL-vH) |
| IgE | 794 | 2564 | IgE-omalizumab-(vL-vH) |
| CD23 | 795 | 2565 | CD23-p5E8-(vL-vH) |
| GCC | 796 | 2566 | GCC-5F9-(vL-vH) |
| GCC | 797 | 2567 | GCC-Ab229-(vL-vH) |
| CD200R | 798 | 2568 | CD200R-huDx182-(vL-vH) |

**Table 12: Epitope Tags**

| **SEQ ID (DNA)** | **SEQ ID (PRT)** | **NAME** |
|---|---|---|
| 553 | 2569 | Myc-TAG |
| 554 | 2570 | FLAG-TAG |
| 555 | 2571 | AcV5 |
| 556 | 2572 | V5-TAG |
| 557 | 2573 | HA-TAG |
| 558 | 2574 | HIS-TAG |
| 559 | 2575 | AVI-TAG-delta-GSG |
| 560 | 2576 | StrepTagII |
| 561 | 2577 | 4XFLAG-2xSTREP-8xHIS |
| 562 | 2578 | 3xFLAG |
| 563 | 2579 | 4xHA-Strep-8xHis |
| 3526 | 3530 | RITX-TAG |
| 3527 | 3531 | RITX2-TAG |
| 3528 | 3532 | RITX4-TAG |
| 3529 | 3533 | GA-TAG |

**Table 13: Exemplary reporter fragments**

| **SEQ ID (DNA)** | **SEQ ID (PRT)** | **REPORTER NAME** |
|---|---|---|
| 813 | 2580 | GLuc (Gaussia princeps Luc) Minus Secretory Signal |
| 814 | 2581 | NLuc (NanoLuc) |
| 815 | 2582 | TLuc (TurboLuc16) Minus Secretory Signal |
| 816 | 2583 | MLuc7-(Metrida longa) Luc M43L/M110L Variant Minus Secretory Signal |
| 817 | 2584 | LoLuc (Lucicutia ovaliformis Luc) Minus Secretory Signal |
| 818 | 2585 | HtLuc (H.tanneri Luc) Minus Secretory Signal |
| 819 | 2586 | PaLuc1 (Pleuromamma abdominalis Luc) minus Secretory Signal |
| 820 | 2587 | PaLuc2 (Pleuromamma abdominalis Luc2)-minus Secretory Signal |
| 821 | 2588 | MpLuc1[Metridia pacifica] minus secretory signal |
| 822 | 2589 | McLuc1 [Metridia curticaudal minus secretory signal |
| 823 | 2590 | MaLuc1 [Metridia asymmetrical minus secretory signal |
| 824 | 2591 | MoLuc1 [Metridia okhotensis] minus secretory signal |
| 825 | 2592 | MoLuc2 [Metridia okhotensisl minus secretory signal |
| 826 | 2593 | MLuc39 [Metridia longa] minus secretory signal |
| 827 | 2594 | PsLuc1 [Pleuromamma scutullatal minus secretory signal |
| 828 | 2595 | LoLuc 1-3 [Lucicutia ovaliformis] minus secretory signal |
| 829 | 2596 | HtLuc2 [Heterorhabdus tanneril minus secretory signal |
| 830 | 2597 | Renilla Luc |

**Table 14: Exemplary cleavable linkers and furine cleavage site**

| **NAME** | **SEQ ID (DNA)** | **SEQ ID (PRT)** |
|---|---|---|
| F2A | 831 | 2598 |
| T2A | 832 | 2599 |
| T2A | 833 | 2599 |
| P2A | 834 | 2600 |
| P2a-variant | 835 | 2601 |
| E2A | 836 | 2602 |
| SGSG | 837 | 2603 |
| SGSG | 838 | 2603 |
| FURINE CLEAVAGE SITE | 839 | 2604 |
| FURINE CLEAVAGE SITE | 840 | 2604 |
| FURINE Cleavage Site | 841 | 2604 |

**Table 15: Exemplary CAR components**

| **SEQ ID (DNA)** | **SEQ ID (Prt)** | **NAME** |
|---|---|---|
| 842 | 2605 | hCD8-Hinge-TM |
| 843 | 2606 | hCD8-Hinge-TM-BBz |
| 844 | 2607 | hCD8TM-Hinge-BB |
| 845 | 2608 | 4-1 BB-cvtosolic-domain |
| 846 | 2609 | CD3z-cytosolic-domain |
| 847 | 2610 | CD3z-cytosolic-domain |
| 848 | 2611 | CD28-Hinge-TM-cytosolic-domain |
| 849 | 2612 | LAILR1-TM-CP |

**Table 16: Exemplary Therapeutic Controls, Accessory modules and their components**

| **SEQ ID (DNA)** | **SEQ ID (PRT)** | **NAME** |
|---|---|---|
| 850 | 2613 | PuroR Variant-(PAC) |
| 851 | 2614 | BlastR |
| 852 | 2615 | CNB30 |
| 853 | 2616 | GMCSF-SP-tEGFR |
| 854 | 2617 | tEGFRviii |
| 855 | 2618 | tCD 19 |
| 856 | 2619 | tBCMA |
| 857 | 2620 | FKBP |
| 858 | 2621 | FKBP |
| 859 | 2622 | FKBPx2 |
| 860 | 2623 | Myr-FKBPx2-K13 |
| 861 | 2624 | MYR |
| 862 | 2625 | Myr-MYD88-CD40-Fv'-Fv |
| 863 | 2626 | IL12F |
| 864 | 2627 | 41BB-L |
| 865 | 2628 | CD40L |
| 866 | 2629 | K13-vFLIP |
| 867 | 2630 | MC159-vFLIP |
| 868 | 2631 | MC160-vFLIP |
| 869 | 2632 | E8-vFLIP |
| 870 | 2633 | Herpesvirus-saimiri-vFLIP |
| 871 | 2634 | Mecaca-vFLIP |
| 872 | 2635 | BHV-vFLIP |
| 873 | 2636 | cFLIP-L/MRIT-alpha |
| 874 | 2637 | cFLIP-p22 |
| 875 | 2638 | HTLV1-TAX |
| 876 | 2639 | HTLV2-TAX |
| 877 | 2640 | HTLV2-TAX-RS |
| 878 | 2641 | FKBP-K13 |
| 879 | 2642 | FKBPX2-K13 |
| 880 | 2643 | Myr-FKBPx2-K13 |
| 881 | 2644 | FKBPx2-HTLV2-Tax-RS |
| 882 | 2645 | FKBPx2-Flag-HTLV2-Tax-RS |
| 883 | 2646 | icaspase-9 |
| 884 | 2647 | IL6R-304-vHH |
| 885 | 2648 | Alb8-vHH |
| 886 | 2649 | IGHSP2-IL6R-304-VHH-ALB8-VHH |
| 887 | 2650 | IL6-19A-vL |
| 888 | 2651 | 1L6-19A-vH |
| 889 | 2652 | CD8SP2-PD1-4H1-scFv |
| 890 | 2653 | CD8SP2-PD 1-5C4-scFv |
| 891 | 2654 | CD8SP2-CTLA4-Ipilimumab-scFv |
| 892 | 2655 | CD8SP2-PD 1-4H1-Alb8-vHH |
| 893 | 2656 | CD8SP2-PD1-5C4-Alb8-vHH |
| 894 | 2657 | CD8SP2-CTLA4-Ipilimumab-Alb8-vHH |
| 895 | 2658 | IL6-19A-vL |
| 896 | 2659 | 1L6-19A-vH |
| 897 | 2660 | IL6R-M83-vL |
| 898 | 2661 | IL6R-M83-vH |
| 899 | 2662 | IgSP-IL6-19A-scFV |
| 900 | 2663 | IgSP-Fx06 |
| 901 | 2664 | CD8SP2-sHVEM |
| 902 | 2665 | CD8SP2-sHVEM-Alb8-vHH |
| 903 | 2666 | hTERT |
| 904 | 2667 | Heparinase |

**Table 17: Exemplary vectors, components and polyA sequence**

| **SEQ ID (DNA)** | **NAME** |
|---|---|
| 905 | pLenti-EF1a |
| 906 | pLenti-EF1a-DWPRE |
| 907 | MSCV-Bgl2-AvrII-Bam-EcoR1-Xho-BstB1-Mlu-Sal-ClaI.I03 |
| 908 | pSBbi-Pur |
| 909 | EFlalpha (EF1a) Promoter Variant |
| 910 | T7 |
| 911 | T3 |
| 912 | MSCVhygro-GLuc-HA-G02 |
| 913 | MSCVpac-GLUC-R03 |
| 914 | pLENTI-NLuc-AcV5-Blasticidin-Pa08 |
| 915 | pLENTI-TurboLuc-16-X3Fag-C04, |
| 916 | pLenti-EF1a-Pac-T2A-Gluc-B07 |
| 917 | MSCV-hygro-NLuc-AcV5-D07 |
| 918 | pLENTI-Gluc-Flag-blast-B07 |
| 919 | H1-shRNA-BRD4-4 |
| 920 | pLenti-EF1a-shRNA-BRD4-DWPRE |
| 921 | pLenti-EF1a-CD8SP-FMC63-(vL-vH)-Myc-z-P2A-K13- Flag-T2A-PAC-H1-prom-shRNA-BRD4-DWPRE |
| 922 | PolyA |

**Table 18: : Exemplary GGS-Luc reporter proteins**

| **SEQ ID (DNA)** | **SEQ ID (PRT)** | **NAME** |
|---|---|---|
| 923 | 2668 | CD8SP-FMC63(vL-vH)-GGSG-NLuc-AcV5 |
| 924 | 2669 | CD8SP-161-(vL-vH)-GGSG-NLuc-AcV5 |
| 925 | 2670 | MPL-ECD-GGSG-Nluc-AcV5 |
| 926 | 2671 | FLAG-CD19-ECD-GGSG-NLuc-AcV5 |

**Table 19: Exemplary CAR constructs containing CD3z activation domain and coexpressing K13.**

| **Target** | **Clone ID** | **SEQ ID (DNA)** | **SEQ ID (PRT)** | **CONSTRUCT NAME** |
|---|---|---|---|---|
| CD19 | 111614-B05 | 927 | 2672 | CD8SP-FMC63-(vL-vH)-Myc-z-P2A- K13-Flag-T2A-PAC |
| CD19 | | 928 | 2673 | CD8SP-huFMC63-11-(vL-vH)-Myc-z- P2A-K13-Flag-T2A-PAC |
| CD19 | | 929 | 2674 | CD8SP-huFMC63-11-N203Q-(vL-vH)- Myc-z-P2A-K13-Flag-T2A-PAC |
| CD19 | | 930 | 2675 | CD8SP-CD19Bu12-(vL-vH)-Myc-z- P2A-K13-Flag-T2A-PAC |
| CD19 | | 931 | 2676 | CD8SP-2-CD19MM-(vL-vH)-Myc-z- P2A-K13-Flag-T2A-PAC |
| CD19 | | 932 | 2677 | CD8SP-CD19-4G7-(vL-vH)-Myc-z- P2A-K13-Flag-T2A-PAC |
| CD19 | | 933 | 2678 | CD8SP-CD19-MEDI-3649-(vL-vH)- Myc-z-P2A-K13-Flag-T2A-PAC |
| CD19 | | 934 | 2679 | CD8SP-CD19-Medrex-24D1-(vL-vH)- Myc-z-P2A-K13-Flag-T2A-PAC |
| CD19 | | 935 | 2680 | CD8SP-Ritx-CD19-MOR0028-(vL- vH)-Myc-z-P2A-K13-Flag-T2A-PAC |
| CD19 | | 936 | 2681 | CD8SP-CD19-HD37-H2L1-(vL-vH)- Myc-z-P2A-K13-Flag-T2A-PAC |
| CD19 | | 937 | 2682 | CD8SP-CD19-huBly3-(vL-vH)-Myc-z- P2A-K13-Flag-T2A-PAC |
| CD19 | | 938 | 2683 | CD8SP-CD19-huSJ25C1-(vL-vH)- Myc-z-P2A-K13-Flag-T2A-PAC |
| CD19 | | 939 | 2684 | CD8SP-Ritx-CD19-hB4-(vL-vH)-Myc- z-P2A-K13-Flag-T2A-PAC |
| CD19 | | 940 | 2685 | CD8SP-CD19-hu-mROO5-1-(vL-vH)- Myc-z-P2A-K13-Flag-T2A-PAC |
| CD19 | | 941 | 2686 | CD8SP-CD19-hA19-(vL-vH)-Myc-z- P2A-K13-Flag-T2A-PAC |
| AFP/MHC class I complex | | 942 | 2687 | CD8SP-AFP-61-(vL-vH)-Myc-z-P2A- K13-Flag-T2A-PAC |
| AFP/MHC class I complex | | 943 | 2688 | CD8SP-AFP-76-(vL-vH)-Myc-z-P2A- K13-Flag-T2A-PAC |
| AFP/MHC class I complex | | 944 | 2689 | CD8SP-AFP-79-(vL-vH)-Myc-z-P2A- K13-Flag-T2A-PAC |
| HIV1-envelop glycoprotein | | 945 | 2690 | CD8SP-HIV1-N6-(vL-vH)-Myc-z- P2A-K13-Flag-T2A-PAC |
| ALK (Anaplastic Lymphoma Kinase) | 051916-D04 | 946 | 2691 | CD8SP-Alk-48-(vL-vH)-Myc-z-P2A- K13-Flag-T2A-PAC |
| ALK (Anaplastic Lymphoma Kinase) | | 947 | 2692 | CD8SP-Alk-58-(vL-vH)-Myc-z-P2A- K13-Flag-T2A-PAC |
| Amyloid | | 948 | 2693 | SP-Amyloid-158-(vL-vH)-Myc-z-P2A- K13-Flag-T2A-PAC |
| Biotin | | 949 | 2694 | CD8SP-dc-Avidin-Myc-z-P2A-K13- Flag-T2A-PAC |
| CD45 | | 950 | 2695 | CD8SP-BC8-CD45-(vL-vH)-Myc-z- P2A-K13-Flag-T2A-PAC |
| BCMA | | 951 | 2696 | CD8SP-BCMA-J6M0-(vL-vH)-Myc-z- P2A-K13-Flag-T2A-PAC |
| BCMA | | 952 | 2697 | CD8SP-BCMA-huC12A3-L3H3-(vL- vH)-Myc-z-P2A-K13-Flag-T2A-PAC |
| BCMA | | 953 | 2698 | CD8SP-BCMA-ET-40-(vL-vH)-Myc-z- P2A-K13-Flag-T2A-PAC |
| BCMA | | 954 | 2699 | CD8SP-BCMA-ET-54-(vL-vH)-Myc-z- P2A-K13-Flag-T2A-PAC |
| BCMA | | 955 | 2700 | CD8SP-BCMA-ET-03-(vL-vH)-Myc-z- P2A-K13-Flag-T2A-PAC |
| BCMA | | 956 | 2701 | CD8SP-BCMA-huC11.D5.3L1H3-(vL- vH)-Myc-z-P2A-K13-Flag-T2A-PAC |
| BCMA | | 957 | 2702 | CD8SP-BCMA-huC13-F12-(vL-vH)- Myc-z-P2A-K13-Flag-T2A-PAC |
| CCR4 | | 958 | 2703 | CD8SP-CCR4-humAb1567-(vL-vH)- Myc-z-P2A-K13-Flag-T2A-PAC |
| HIV1-envelop glycoprotein | 052615-G02 | 959 | 2704 | CD8SP-CD4-ECD-Gly-Ser-Linker-DC- SIGN-Myc-z-P2A-K13-Flag-T2A-PAC |
| CD5 | | 960 | 2705 | CD8SP-CD5-9-(vL-vH)-Myc-z-P2A- K13-Flag-T2A-PAC |
| CD5 | | 961 | 2706 | CD8SP-CD5-18-(vL-vH)-Myc-z-P2A- K13-Flag-T2A-PAC |
| Ig Fc | | 962 | 2707 | CD8SP-CD16A-V158-ECD-v2-Myc-z- |
| | | | | P2A-K13-Flag-T2A-PAC |
| Ig Fc | 091015-Z07 | 963 | 2708 | CD8SP-CD16A-V158-ECD-v1-Myc-z- P2A-K13-Flag-T2A-PAC |
| CD20 | | 964 | 2709 | CD8SP-CD20-2F2-(vL-vH)-Myc-z- P2A-K13-Flag-T2A-PAC |
| CD20 | | 965 | 2710 | CD8SP-CD20-GA101-(vL-vH)-Myc-z- P2A-K13-Flag-T2A-PAC |
| CD20 | | 966 | 2711 | CD8SP-CD20-Leu16-(vL-vH)-Myc-z- P2A-K13-Flag-T2A-PAC |
| CD20 | | 967 | 2712 | CD8SP-CD20-11B8-(vL-vH)-Myc-z- P2A-K13-Flag-T2A-PAC |
| CD20 | | 968 | 2713 | CD8SP-CD20-2C6-(vL-vH)-Myc-z- P2A-K13-Flag-T2A-PAC |
| CD20 | | 969 | 2714 | CD8SP-CD20-2H7-(vL-vH)-Myc-z- P2A-K13-Flag-T2A-PAC |
| CD20 | | 970 | 2715 | CD8SP-CD20-hA20-(vL-vH)-Myc-z- P2A-K13-Flag-T2A-PAC |
| CD20 | | 971 | 2716 | CD8SP-CD20-BM-CA-1925-v4-(vL- vH)-Myc-z-P2A-K13-Flag-T2A-PAC |
| CD20 | | 972 | 2717 | CD8SP-CD20-Ubli-v4-(vL-vH)-Myc-z- P2A-K13-Flag-T2A-PAC |
| CD20 | | 973 | 2718 | CD8SP-CD20-2H7-(vL-vH)-Myc-z- P2A-K13-Flag-T2A-PAC |
| CD20 | | 974 | 2719 | CD8SP-CD20-h1F5-(vL-vH)-Myc-z- P2A-K13-Flag-T2A-PAC |
| CD20 | | 975 | 2720 | CD8SP-CD20-7D8-(vL-vH)-Myc-z- P2A-K13-Flag-T2A-PAC |
| CD20 | | 976 | 2721 | CD8SP-CD20-AME-33-(vL-vH)-Myc- z-P2A-K13-Flag-T2A-PAC |
| CD22 | | 977 | 2722 | CD8SP-CD22-h10F4v2-(vL-vH)-Myc- z-P2A-K13-Flag-T2A-PAC |
| CD22 | 050516-H05 | 978 | 2723 | CD8SP-CD22-H22Rhov2ACDRKA- (vL-vH)-Myc-z-P2A-K13-Flag-T2A- PAC |
| CD22 | | 979 | 2724 | CD8SP-CD22-m971-(vL-vH)-Myc-z- P2A-K13-Flag-T2A-PAC |
| CD22 | | 980 | 2725 | CD8SP-CD22-m971-HL-(vH-vL)-Myc- z-P2A-K13-Flag-T2A-PAC |
| CD30 | 060616-H05 | 981 | 2726 | CD8SP-CD30-5F11-(vL-vH)-Myc-z- P2A-K13-Flag-T2A-PAC |
| CD30 | | 982 | 2727 | CD8SP-CD30-Ac10-(vL-vH)-Myc-z- P2A-K13-Flag-T2A-PAC |
| CD32 | 060916-A02 | 983 | 2728 | CD8SP-CD32-Med9-(vL-vH)-Myc-z- P2A-K13-Flag-T2A-PAC |
| CD33 | | 984 | 2729 | CD8SP-CD33-AF5-(vL-vH)-Myc-z- P2A-K13-Flag-T2A-PAC |
| CD33 | | 985 | 2730 | CD8SP-CD33-huMyc9-(vL-vH)-Myc- z-P2A-K13-Flag-T2A-PAC |
| CD33 | | 986 | 2731 | CD8SP-CD33-Boehr2800308-(vL-vH)- |
| | | | | Myc-z-P2A-K13-Flag-T2A-PAC |
| CD33 | | 987 | 2732 | CD8SP-CD33-Him3-4-(vL-vH)-Myc-z- P2A-K13-Flag-T2A-PAC |
| CD33 | | 988 | 2733 | CD8SP-CD33-SGNh2H12-(vL-vH)- Myc-z-P2A-K13-Flag-T2A-PAC |
| CD33 | | 989 | 2734 | CD8SP-CD33-15G15-33-(vL-vH)- Myc-z-P2A-K13-Flag-T2A-PAC |
| CD33 | | 990 | 2735 | CD8SP-CD33-33H4-(vL-vH)-Myc-z- P2A-K13-Flag-T2A-PAC |
| CD33 | | 991 | 2736 | CD8SP-CD33-9C3-2-(vL-vH)-Myc-z- P2A-K13-Flag-T2A-PAC |
| CD34 | 051616-102 | 992 | 2737 | CD8SP-CD34-hu4C7-(vL-vH)-Myc-z- P2A-K13-Flag-T2A-PAC |
| CD44v6 | | 993 | 2738 | CD8SP-CD44v6-Biwa8-(vL-vH)-Myc- z-P2A-K13-Flag-T2A-PAC |
| CD70 | | 994 | 2739 | CD8SP-CD70-h1F6-(vL-vH)-Myc-z- P2A-K13-Flag-T2A-PAC |
| CD79b | | 995 | 2740 | CD8SP-CD79b-2F2-(vL-vH)-Myc-z- P2A-K13-Flag-T2A-PAC |
| CD79b | | 996 | 2741 | CD8SP-huMA79bv28-(vL-vH)-Myc-z- P2A-K13-Flag-T2A-PAC |
| CD99 | | 997 | 2742 | CD8SP-CD99-hu12E7-(vL-vH)-Myc-z- P2A-K13-Flag-T2A-PAC |
| CD 123 | | 998 | 2743 | CD8SP-CD123-CSL362-(vL-vH)-Myc- z-P2A-K13-Flag-T2A-PAC |
| CD 123 | | 999 | 2744 | CD8SP-CD123-1172-(vL-vH)-Myc-z- P2A-K13-Flag-T2A-PAC |
| CD123 | | 1000 | 2745 | CD8SP-CD123-DART-1-(vL-vH)- Myc-z-P2A-K13-Flag-T2A-PAC |
| CD123 | | 1001 | 2746 | CD8SP-CD123-DART-2-(vL-vH)- Myc-z-P2A-K13-Flag-T2A-PAC |
| CD123 | | 1002 | 2747 | CD8SP-CD123-I3RB18-(vL-vH)-Myc- z-P2A-K13-Flag-T2A-PAC |
| CD123 | | 1003 | 2748 | CD8SP-CD123-hu3E3-(vL-vH)-Myc-z- P2A-K13-Flag-T2A-PAC |
| CD 123 | | 1004 | 2749 | CD8SP-CD123-9F6-(vL-vH)-Myc-z- P2A-K13-Flag-T2A-PAC |
| CD 123 | | 1005 | 2750 | CD8SP-CD123-I3RB2-(vL-vH)-Myc-z- P2A-K13-Flag-T2A-PAC |
| CD 123 | | 1006 | 2751 | CD8SP-CD123-1176-(vL-vH)-Myc-z- P2A-K13-Flag-T2A-PAC |
| CD123 | | 1007 | 2752 | CD8SP-Ritx2-CD123-8B11-(vL-vH)- Myc-z-P2A-K13-Flag-T2A-PAC |
| CD123 | | 1008 | 2753 | CD8SP-CD123-2B8-(vL-vH)-Myc-z- P2A-K13-Flag-T2A-PAC |
| CD123 | | 1009 | 2754 | CD8SP-CD123-9D7-(vL-vH)-Myc-z- P2A-K13-Flag-T2A-PAC |
| CD123 | | 1010 | 2755 | CD8SP-CD123-3B10-(vL-vH)-Myc-z- P2A-K13-Flag-T2A-PAC |
| CD138 | | 1011 | 2756 | CD8SP-CD138-(vL-vH)-Myc-z-P2A- K13-Flag-T2A-PAC |
| CD 179b | 051716-N05 | 1012 | 2757 | CD8SP-CD179b-(vL-vH)-Myc-z-P2A- K13-Flag-T2A-PAC |
| CD276 | | 1013 | 2758 | CD8SP-CD276-17-(vL-vH)-Myc-z- P2A-K13-Flag-T2A-PAC |
| CD324 | | 1014 | 2759 | CD8SP-CD32410-6-(vL-vH)-Myc-z- P2A-K13-Flag-T2A-PAC |
| CD324 | | 1015 | 2760 | CD8SP-CD324-hSC10-17-(vL-vH)- Myc-z-P2A-K13-Flag-T2A-PAC |
| CDH6 | 051716-105 | 1016 | 2761 | CD8SP-CDH6-NOV710-(vL-vH)-Myc- z-P2A-K13-Flag-T2A-PAC |
| CDH6 | 051716-J05 | 1017 | 2762 | CD8SP-CDH6-NOV712-(vL-vH)-Myc- z-P2A-K13-Flag-T2A-PAC |
| CDH17 | 051716-M06 | 1018 | 2763 | CD8SP-CDH17-PTA001A4-(vL-vH)- Myc-z-P2A-K13-Flag-T2A-PAC |
| CDH 19 | 051716-V04 | 1019 | 2764 | CD8SP-CDH19-16A4-(vL-vH)-Myc-z- P2A-K13-Flag-T2A-PAC |
| EGFR | | 1020 | 2765 | CD8SP-Cetuximab-(vL-vH)-Myc-z- P2A-K13-Flag-T2A-PAC |
| CLEC5A | 050516-E03 | 1021 | 2766 | CD8SP-CLEC5A-8H8F5-(vL-vH)- Myc-z-P2A-K13-Flag-T2A-PAC |
| CLEC5A | 051016-F06 | 1022 | 2767 | CD8SP-CLEC5A-3E12A2-(vL-vH)- Myc-z-P2A-K13-Flag-T2A-PAC |
| GR/LHR (Gonadotropin Receptor) | | 1023 | 2768 | SP-CGHb-Gly-Ser-Linker-CGHa-Myc- z-P2A-K13-Flag-T2A-PAC |
| CLLI | | 1024 | 2769 | CD8SP-CLL1-M26-(vL-vH)-Myc-z- P2A-K13-Flag-T2A-PAC |
| CLLI | | 1025 | 2770 | CD8SP-CLL1-M32-(vL-vH)-Myc-z- P2A-K13-Flag-T2A-PAC |
| CLLI | | 1026 | 2771 | CD8SP-CLL1-21C9-L2H3-(vL-vH)- Myc-z-P2A-K13-Flag-T2A-PAC |
| CLLI | | 1027 | 2772 | CD8SP-CLL1-6E7L4H1e-(vL-vH)- Myc-z-P2A-K13-Flag-T2A-PAC |
| CLLI | | 1028 | 2773 | CD8SP-CLL1-hu1075-v1-(vL-vH)- Myc-z-P2A-K13-Flag-T2A-PAC |
| CLLI | | 1029 | 2774 | CD8SP-CLL1-hu1075-v2-(vL-vH)- Myc-z-P2A-K13-Flag-T2A-PAC |
| CMVpp65/MHC class I complex | | 1030 | 2775 | CD8SP-CMVpp65-F5-(vL-vH)-Myc-z- P2A-K13-Flag-T2A-PAC |
| CS1 (SLAMF7) | | 1031 | 2776 | CD8SP-CS1-huLuc63-(vL-vH)-Myc-z- P2A-K13-Flag-T2A-PAC |
| CS1 (SLAMF7) | | 1032 | 2777 | CD8SP-CS1-HuLuc64-(vL-vH)-Myc-z- P2A-K13-Flag-T2A-PAC |
| CS1 (SLAMF7) | | 1033 | 2778 | CD8SP-CS1-huLuc90-(vL-vH)-Myc-z- P2A-K13-Flag-T2A-PAC |
| CS1 (SLAMF7) | | 1034 | 2779 | CD8SP-CS1-PDL241-(vL-vH)-Myc-z- |
| | | | | P2A-K13-Flag-T2A-PAC |
| CS1 (SLAMF7) | | 1035 | 2780 | CD8SP-CS1-Hu27A-(vL-vH)-Myc-z- P2A-K13-Flag-T2A-PAC |
| CS1 (SLAMF7) | | 1036 | 2781 | CD8SP-CS1Hu34C3-(vL-vH)-Myc-z- P2A-K13-Flag-T2A-PAC |
| CS1 (SLAMF7) | | 1037 | 2782 | CD8SP-CS1-Hu31-D2-(vL-vH)-Myc-z- P2A-K13-Flag-T2A-PAC |
| CS1(SLAMF7) | | 1038 | 2783 | CD8SP-CS1-Luc34-(vL-vH)-Myc-z- P2A-K13-Flag-T2A-PAC |
| CS1 (SLAMF7) | | 1039 | 2784 | CD8SP-CS1-LucX2-(vL-vH)-Myc-z- P2A-K13-Flag-T2A-PAC |
| CSF2RA | 051616-H05 | 1040 | 2785 | CD8SP-CSF2RA-Ab6-(vL-vH)-Myc-z- P2A-K13-Flag-T2A-PAC |
| CSF2RA | 051616-G01 | 1041 | 2786 | CD8SP-CSF2RA-Ab1-(vL-vH)-Myc-z- P2A-K13-Flag-T2A-PAC |
| CXCR4 and CD123 | | 1042 | 2787 | CD8SP-CXCR4-1-vHH-Gly-Ser- Linker-CD123-1-vHH-Myc-z-P2A- K13-Flag-T2A-PAC |
| CXCR4 and CD123 | | 1043 | 2788 | CD8SP-CXCR4-2-VHH-Gly-Ser- Linker-CD123-2-VHH-Myc-z-P2A- K13-Flag-T2A-PAC |
| DLL3 (Delta Like Ligand 3) | | 1044 | 2789 | CD8SP-DLL3-hSC16-13-(vL-vH)- Myc-z-P2A-K13-Flag-T2A-PAC |
| DLL3 (Delta Like Ligand 3) | | 1045 | 2790 | CD8SP-DLL3-hSC16-56-(vL-vH)- Myc-z-P2A-K13-Flag-T2A-PAC |
| EBNA3c/MHC class I complex | | 1046 | 2791 | CD8SP-EBNA3c-315-(vL-vH)-Myc-z- P2A-K13-Flag-T2A-PAC |
| EBV-gp350 | | 1047 | 2792 | CD8SP-EBV-gp350-(vL-vH)-Myc-z- P2A-K13-Flag-T2A-PAC |
| EGFR | | 1048 | 2793 | CD8SP-EGFR1-vHH-Myc-z-P2A-K13- Flag-T2A-PAC |
| EGFR & CEA | 040716-008 | 1049 | 2794 | CD8SP-EGFR1-vHH-Gly-Ser-Linker- CEA1-vHH-Myc-z-P2A-K13-Flag- T2A-PAC |
| EGFR & CEA | 040716-P01 | 1050 | 2795 | CD8SP-EGFR33-vHH-Gly-Ser-Linker- CEA5-vHH-Myc-z-P2A-K13-Flag- T2A-PAC |
| EGFRvIII | | 1051 | 2796 | CD8SP-EGFRvIII-139-(vL-vH)-Myc-z- P2A-K13-Flag-T2A-PAC |
| EGFRvIII | | 1052 | 2797 | CD8SP-EGFRvIII-2173-(vL-vH)-Myc- z-P2A-K13-Flag-T2A-PAC |
| EpCam1 | | 1053 | 2798 | CD8SP-Epcam1-MM1-(vL-vH)-Myc-z- P2A-K13-Flag-T2A-PAC |
| EpCam1 | | 1054 | 2799 | CD8SP-Epcam1-D5K5-(vL-vH)-Myc- z-P2A-K13-Flag-T2A-PAC |
| FLT3 | | 1055 | 2800 | CD8SP-FLT3-NC7-(vL-vH)-Myc-z- P2A-K13-Flag-T2A-PAC |
| FITC | 051016-E04 | 1056 | 2801 | CD8SP-FITC-(vL-vH)-Myc-z-P2A- |
| | | | | K13-Flag-T2A-PAC |
| FITC | | 1057 | 2802 | CD8SP-FITC-4M-53-(vL-vH)-Myc-z- P2A-K13-Flag-T2A-PAC |
| FITC | | 1058 | 2803 | CD8SP-FITC-E2-HL-(vH-vL)-Myc-z- P2A-K13-Flag-T2A-PAC |
| Influenza A HA | | 1059 | 2804 | CD8SP-FLU-MEDI-8852-(vL-vH)- Myc-z-P2A-K13-Flag-T2A-PAC |
| FR1 (Folate Receptor alpha) | | 1060 | 2805 | CD8SP-FR1-huMov19-(vL-vH)-Myc- z-P2A-K13-Flag-T2A-PAC |
| FSHR (Follicle Stimulating Hormone Receptor) | | 1061 | 2806 | CD8SP-FSHb-Gly-Ser-Linker-CGHa- Myc-z-P2A-K13-Flag-T2A-PAC |
| GAD (Glutamic Acid Decarboxylase)/M HC class I complex | | 1062 | 2807 | CD8SP-GAD-G3H8-(vL-vH)-Myc-z- P2A-K13-Flag-T2A-PAC |
| GD2 | | 1063 | 2808 | CD8SP-GD2-hu14-18-(vL-vH)-Myc-z- P2A-K13-Flag-T2A-PAC |
| GD2 | | 1064 | 2809 | CD8SP-GD2-hu3F8-(vL-vH)-Myc-z- P2A-K13-Flag-T2A-PAC |
| GD3 | 051016-D04 | 1065 | 2810 | CD8SP-GD3-KM-641-(vL-vH)-Myc-z- P2A-K13-Flag-T2A-PAC |
| GFRa4 (GDNF Family Receptor Alpha 4) | 051716-K05 | 1066 | 2811 | CD8SP-GFRAlpha4-P4-6-(vL-vH)- Myc-z-P2A-K13-Flag-T2A-PAC |
| GFRa4 (GDNF Family Receptor Alpha 4) | 051316-L02 | 1067 | 2812 | CD8SP-GFRa4-P4-10-(vL-vH)-Myc-z- P2A-K13-Flag-T2A-PAC |
| GM1 | | 1068 | 2813 | CD8SP-GM1-5B2-(vL-vH)-Myc-z- P2A-K13-Flag-T2A-PAC |
| GM1 | | 1069 | 2814 | CD8SP-GM1-7E5-(vL-vH)-Myc-z- P2A-K13-Flag-T2A-PAC |
| GPRC5D (G-protein coupled receptor family C group 5 member D) | | 1070 | 2815 | CD8SP-GPRC5D-ET150-5-(vL-vH)- Myc-z-P2A-K13-Flag-T2A-PAC |
| GPRC5D | | 1071 | 2816 | CD8SP-GPRC5D-ET150-18-(vL-vH)- Myc-z-P2A-K13-Flag-T2A-PAC |
| GPRC5D | | 1072 | 2817 | CD8SP-GPRC5D-ET150-1-(vL-vH)- Myc-z-P2A-K13-Flag-T2A-PAC |
| GPRC5D | | 1073 | 2818 | CD8SP-GPRC5D-ET150-2-(vL-vH)- Myc-z-P2A-K13-Flag-T2A-PAC |
| gp100/MHC class I complex | | 1074 | 2819 | CD8SP-gp100-(vL-vH)-Myc-z-P2A- K13-Flag-T2A-PAC |
| gp100/MHC class I complex | | 1075 | 2820 | CD8SP-gp100-G2D12-(vL-vH)-Myc-z- P2A-K13-Flag-T2A-PAC |
| GPC3 (Glypican 3) | | 1076 | 2821 | CD8SP-GPC3-4E5-(vL-vH)-Myc-z- P2A-K13-Flag-T2A-PAC |
| gpNMB (Glycoprotein Nmb) | 060616-105 | 1077 | 2822 | CD8SP-gpNMB-115-(vL-vH)-Myc-z- P2A-K13-Flag-T2A-PAC |
| GRP78 | | 1078 | 2823 | CD8SP-GRP78-GC18-(vL-vH)-Myc-z- P2A-K13-Flag-T2A-PAC |
| Her2 | | 1079 | 2824 | CD8SP-Her2-5F7-vHH-Myc-z-P2A- K13-Flag-T2A-PAC |
| Her2 | | 1080 | 2825 | IgHSP-Her2-Affi-Myc-z-P2A-K13- Flag-T2A-PAC |
| Her2 | | 1081 | 2826 | CD8SP-Her2-1-Darpin-Myc-z-P2A- K13-Flag-T2A-PAC |
| Her2 | | 1082 | 2827 | IgHSP-Her2-2-Darpin-Myc-z-P2A- K13-Flag-T2A-PAC |
| Her2 | | 1083 | 2828 | CD8SP-Her2-5F7-vHH-Gly-Ser- Linker-Her2-47D5-vHH-Myc-z-P2A- K13-Flag-T2A-PAC |
| Her2 | 050516-103 | 1084 | 2829 | CD8SP-Her2-Hu4D5-(vL-vH)-Myc-z- P2A-K13-Flag-T2A-PAC |
| Her3 | | 1085 | 2830 | CD8SP-Her3-17B05So-vHH-Myc-z- P2A-K13-Flag-T2A-PAC |
| Her3 | | 1086 | 2831 | CD8SP-Her3-Affi-Myc-z-P2A-K13- Flag-T2A-PAC |
| Her2 and Her3 | | 1087 | 2832 | CD8SP-Her3-17B05So-vHH-Gly-Ser- Linker-Her2-2D3-vHH-Myc-z-P2A- K13-Flag-T2A-PAC |
| HIV1-gag/MHC class I complex | | 1088 | 2833 | CD8SP-HIV1-E5-(vL-vH)-Myc-z-P2A- K13-Flag-T2A-PAC |
| HIV 1-envelop glycoprotein | | 1089 | 2834 | CD8SP-HIV1-3BNC117-(vL-vH)-Myc- z-P2A-K13-Flag-T2A-PAC |
| HIV 1-envelop glycoprotein | | 1090 | 2835 | CD8SP-HIV1-PGT-128-(vL-vH)-Myc- z-P2A-K13-Flag-T2A-PAC |
| HIV1-envelop glycoprotein | | 1091 | 2836 | CD8SP-HIV1-VR-C01-(vL-vH)-Myc- z-P2A-K13-Flag-T2A-PAC |
| HIV1-envelop glycoprotein | | 1092 | 2837 | CD8SP-HIV1-X5-(vL-vH)-Myc-z- P2A-K13-Flag-T2A-PAC |
| HLA-A2 | | 1093 | 2838 | CD8SP-HLA-A2-3PB2-(vL-vH)-Myc- z-P2A-K13-Flag-T2A-PAC |
| HMW-MAA | 060116-C03 | 1094 | 2839 | CD8SP-HMW-MAA-hIND-(vL-vH)- Myc-z-P2A-K13-Flag-T2A-PAC |
| HPV 16-E7/MHC class I complex | | 1095 | 2840 | CD8SP-HPV16-7-8-(vL-vH)-Myc-z- P2A-K13-Flag-T2A-PAC |
| HPV 16-E7/MHC class I complex | | 1096 | 2841 | CD8SP-HPV16-2-(vL-vH)-Myc-z- P2A-K13-Flag-T2A-PAC |
| HTLV1-TAX/MHC class I complex | | 1097 | 2842 | CD8SP-HTLV-TAX-T3F2-(vL-vH)- |
| | | | | Myc-z-P2A-K13-Flag-T2A-PAC |
| HTLV1-TAX/MHC class I complex | | 1098 | 2843 | CD8SP-HTLV-TAX-T3E3-(vL-vH)- Myc-z-P2A-K13-Flag-T2A-PAC |
| IL11Ra | 050516-D01 | 1099 | 2844 | CD8SP-IL11Ra-8E2-Ts107-(vL-vH)- Myc-z-P2A-K13-Flag-T2A-PAC |
| IL6Ra | | 1100 | 2845 | IgHSP-IL6R-304-vHH-Myc-z-P2A- K13-Flag-T2A-PAC |
| IL13Ra2 | | 1101 | 2846 | CD8SP-IL13Ra2-hu107-(vL-vH)-Myc- z-P2A-K13-Flag-T2A-PAC |
| IL13Ra2 | 050516-F04 | 1102 | 2847 | CD8SP-IL13Ra2-Hu108-(vL-vH)-Myc- z-P2A-K13-Flag-T2A-PAC |
| KSHV-K8.1 | 070315-G04 | 1103 | 2848 | CD8SP-KSHV-4C3-(vL-vH)-Myc-z- P2A-K13-Flag-T2A-PAC |
| LAMP1 (Lysosomal-associated membrane protein 1) | 051716-T05 | 1104 | 2849 | CD8SP-LAMP1-humab1-2-(vL-vH)- Myc-z-P2A-K13-Flag-T2A-PAC |
| LAMP1 (Lysosomal-associated membrane protein 1) | 051916-B07 | 1105 | 2850 | CD8SP-LAMP1-Mb4-(vL-vH)-Myc-z- P2A-K13-Flag-T2A-PAC |
| LewisY | | 1106 | 2851 | CD8SP-LewisY-huS193-(vL-vH)-Myc- z-P2A-K13-Flag-T2A-PAC |
| LICAM | | 1107 | 2852 | CD8SP-L1CAM-9-3-HU3-(vL-vH)- Myc-z-P2A-K13-Flag-T2A-PAC |
| LHR | | 1108 | 2853 | SP-LHb-Gly-Ser-Linker-CGHa-Myc-z- P2A-K13-Flag-T2A-PAC |
| Lym1 | | 1109 | 2854 | CD8SP-Lym1-(vL-vH)-Myc-z-P2A- K13-Flag-T2A-PAC |
| Lym2 | | 1110 | 2855 | CD8SP-Lym2-(vL-vH)-Myc-z-P2A- K13-Flag-T2A-PAC |
| CD79b | | 1111 | 2856 | CD8SP-huMA79bv28-(vL-vH)-Myc-z- P2A-K13-Flag-T2A-PAC |
| MART1/MHC class I complex | | 1112 | 2857 | CD8SP-MART1-CAG10-(vL-vH)- Myc-z-P2A-K13-Flag-T2A-PAC |
| MART1/MHC class I complex | | 1113 | 2858 | CD8SP-MART1-CLA12-(vL-vH)- Myc-z-P2A-K13-Flag-T2A-PAC |
| Mesothelin | | 1114 | 2859 | CD8SP-Mesothelin-m912-(vL-vH)- Myc-z-P2A-K13-Flag-T2A-PAC |
| cMet | | 1115 | 2860 | CD8SP-cMet-171-vHH-Myc-z-P2A- K13-Flag-T2A-PAC |
| cMet and Her3 | 041116-B05 | 1116 | 2861 | CD8SP-cMET-171-vHH-Gly-Ser- Linker-Her3-21F06-vHH-Myc-z-P2A- K13-Flag-T2A-PAC |
| MPL (Thrombopoietin receptor) | | 1117 | 2862 | CD8SP-MPL-175-(vL-vH)-Myc-z- P2A-K13-Flag-T2A-PAC |
| MPL (Thrombopoietin receptor) | 111516-N05 | 1118 | 2863 | CD8SP-MPL-161-(vL-vH)-Myc-z- P2A-K13-Flag-T2A-PAC |
| MPL | | 1119 | 2864 | CD8SP-MPL-161-HL-(vH-vL)-Myc-z- P2A-K13-Flag-T2A-PAC |
| MPL | 040716-M02 | 1120 | 2865 | CD8SP-2-MPL-111-(vL-vH)-Myc-z- P2A-K13-Flag-T2A-PAC |
| MPL | | 1121 | 2866 | CD8SP-MPL-178-(vL-vH)-Myc-z- P2A-K13-Flag-T2A-PAC |
| MPL | | 1122 | 2867 | CD8SP-MPL-AB317-(vL-vH)-Myc-z- P2A-K13-Flag-T2A-PAC |
| MPL | | 1123 | 2868 | CD8SP-MPL-12E10-(vL-vH)-Myc-z- P2A-K13-Flag-T2A-PAC |
| MPL | | 1124 | 2869 | CD8SP-MPL-huVB22Bw5-(vL-vH)- Myc-z-P2A-K13-Flag-T2A-PAC |
| Muc1/MHC class I complex | | 1125 | 2870 | CD8SP-Muc1-D6-M3B8-(vL-vH)- Myc-z-P2A-K13-Flag-T2A-PAC |
| Muc1/MHC class I complex | | 1126 | 2871 | CD8SP-MUC1-D6-M3A1-(vL-vH)- Myc-z-P2A-K13-Flag-T2A-PAC |
| Muc16 | | 1127 | 2872 | CD8SP-Muc16-4H11-(vL-vH)-Myc-z- P2A-K13-Flag-T2A-PAC |
| EGFR | | 1128 | 2873 | CD8SP-Nimotuzumab-(vL-vH)-Myc-z- P2A-K13-Flag-T2A-PAC |
| NKG2D Ligand | 050516-J04 | 1129 | 2874 | CD8SP-NKG2D-(GGGGS-GGGGD)- Myc-z-P2A-K13-Flag-T2A-PAC |
| NKG2D | | 1130 | 2875 | CD8SP-NKG2D-MS-(vL-vH)-Myc-z- P2A-K13-Flag-T2A-PAC |
| NY-BR1 | 051716-Q05 | 1131 | 2876 | CD8SP-NYBR1-(vL-vH)-Myc-z-P2A- K13-Flag-T2A-PAC |
| NY-ESO/MHC class I complex | | 1132 | 2877 | CD8SP-NYESO-T1-(vL-vH)-Myc-z- P2A-K13-Flag-T2A-PAC |
| NY-ESO/MHC class I complex | | 1133 | 2878 | CD8SP-NYESO-T2-(vL-vH)-Myc-z- P2A-K13-Flag-T2A-PAC |
| PD1 ligand (e.g., PDL1) | | 1134 | 2879 | CD8SP-PD1-ECD-Myc-z-P2A-K13- Flag-T2A-PAC |
| PDL1 | | 1135 | 2880 | CD8SP-PDL1-Atezoli-(vL-vH)-Myc-z- P2A-K13-Flag-T2A-PAC |
| PDL1 | | 1136 | 2881 | CD8SP-PDL1-SP142-(vL-vH)-Myc-z- P2A-K13-Flag-T2A-PAC |
| PDL1 | | 1137 | 2882 | CD8SP-PDL1-10A5-(vL-vH)-Myc-z- P2A-K13-Flag-T2A-PAC |
| PSCA (Prostate stem cell antigen) | | 1138 | 2883 | CD8SP-PSCA-Ha14-121-(vL-vH)- Myc-z-P2A-K13-Flag-T2A-PAC |
| PSCA (Prostate stem cell antigen) | | 1139 | 2884 | CD8SP-PSCA-Ha14-117-(vL-vH)- Myc-z-P2A-K13-Flag-T2A-PAC |
| PR1/MHC class I complex | | 1140 | 2885 | CD8SP-PR1-(vL-vH)-Myc-z-P2A-K13- Flag-T2A-PAC |
| PSMA (Prostate Specific Membrane Antigen) | | 1141 | 2886 | CD8SP-PSMA-006-(vL-vH)-Myc-z- P2A-K13-Flag-T2A-PAC |
| PSMA (Prostate Specific Membrane Antigen) | | 1142 | 2887 | CD8SP-PSMA-J591-(vL-vH)-Myc-z- P2A-K13-Flag-T2A-PAC |
| PTK7 (Tyrosine-protein kinase-like 7) | | 1143 | 2888 | CD8SP-PTK7-hSC6-23-(vL-vH)-Myc- z-P2A-K13-Flag-T2A-PAC |
| PTK7 (Tyrosine-protein kinase-like 7) | | 1144 | 2889 | CD8SP-PTK76-10-2-(vL-vH)-Myc-z- P2A-K13-Flag-T2A-PAC |
| ROR1 | | 1145 | 2890 | CD8SP-ROR1-4A5-(vL-vH)-Myc-z- P2A-K13-Flag-T2A-PAC |
| ROR1 | | 1146 | 2891 | CD8SP-ROR1-4C10-(vL-vH)-Myc-z- P2A-K13-Flag-T2A-PAC |
| Mesothelin | | 1147 | 2892 | CD8SP-SD1-vHH-Gly-Ser-Linker- SD2-vHH-Myc-z-P2A-K13-Flag-T2A- PAC |
| SLea | | 1148 | 2893 | CD8SP-SLea-7E3-(vL-vH)-Myc-z- P2A-K13-Flag-T2A-PAC |
| SLea | 051716-X05 | 1149 | 2894 | CD8SP-SLea-5B1-(vL-vH)-Myc-z- P2A-K13-Flag-T2A-PAC |
| SSEA4 (stage-specific embryonic antigen 4) | | 1150 | 2895 | CD8SP-SSEA4-(vL-vH)-Myc-z-P2A- K13-Flag-T2A-PAC |
| TCRB1 (TCR beta 1 constant chain) | | 1151 | 2896 | CD8SP-TCRB1-CP01-E09-(vL-vH)- Myc-z-P2A-K13-Flag-T2A-PAC |
| TCRB1 (TCR beta 1 constant chain) | | 1152 | 2897 | CD8SP-TCRB1-Jovi1-(vL-vH)-Myc-z- P2A-K13-Flag-T2A-PAC |
| TCRB2 (TCR beta 2 constant chain) | | 1153 | 2898 | CD8SP-TCRB2-CP01-D05-(vL-vH)- Myc-z-P2A-K13-Flag-T2A-PAC |
| TCRB2 (TCR beta 2 constant chain) | | 1154 | 2899 | CD8SP-TCRB2-CP01-E05-(vL-vH)- Myc-z-P2A-K13-Flag-T2A-PAC |
| TCRgd (TCR gamma/delta) | 051716-H05 | 1155 | 2900 | CD8SP-TCRgd-G5-4-(vL-vH)-Myc-z- P2A-K13-Flag-T2A-PAC |
| hTERT/MHC class I complex | | 1156 | 2901 | CD8SP-TERT-4A9-T540-(vL-vH)- Myc-z-P2A-K13-Flag-T2A-PAC |
| hTERT/MHC class I complex | | 1157 | 2902 | CD8SP-TERT-3G3-T865-(vL-vH)- Myc-z-P2A-K13-Flag-T2A-PAC |
| Tissue Factor-1 | 051716-005 | 1158 | 2903 | CD8SP-TGFBR2-Ab1-(vL-vH)-Myc-z- P2A-K13-Flag-T2A-PAC |
| TGFBR2 | | 1159 | 2904 | CD8SP-TF1-98-(vL-vH)-Myc-z-P2A- K13-Flag-T2A-PAC |
| TIM1/HAVCR | 051716-S05 | 1160 | 2905 | CD8SP-TIM1-HVCR1-270-2-(vL-vH)- Myc-z-P2A-K13-Flag-T2A-PAC |
| TIM1/HAVCR | 051716-P05 | 1161 | 2906 | CD8SP-TIM1-HVCR1-ARD5-(vL- vH)-Myc-z-P2A-K13-Flag-T2A-PAC |
| TnAg | 051716-H05 | 1162 | 2907 | CD8SP-TnAg-(vL-vH)-Myc-z-P2A- K13-Flag-T2A-PAC |
| Tn-Muc1 | | 1163 | 2908 | CD8SP-TnMuc1-hu5E5-RHA8-RKA- 2-(vL-vH)-Myc-z-P2A-K13-Flag-T2A- PAC |
| MPL (Thrombopoietin receptor) | | 1164 | 2909 | CD8SP-hTPO-Myc-z-P2A-K13-Flag- T2A-PAC |
| TROP2 (Trophoblast cell-surface antigen-2) | 051716-G05 | 1165 | 2910 | CD8SP-TROP2-ARA47-HV3KV3-(vL- vH)-Myc-z-P2A-K13-Flag-T2A-PAC |
| TROP2 (Trophoblast cell-surface antigen-2) | 052616-D04 | 1166 | 2911 | CD8SP-TROP2-h7E6-SVG-(vL-vH)- Myc-z-P2A-K13-Flag-T2A-PAC |
| TSHR (Thyrotropin receptor) | | 1167 | 2912 | SP-TSHb-Gly-Ser-Linker-CGHa-Myc- z-P2A-K13-Flag-T2A-PAC |
| TSHR (Thyrotropin receptor) | | 1168 | 2913 | CD8SP-TSHR-K1-70-(vL-vH)-Myc-z- P2A-K13-Flag-T2A-PAC |
| TSHR (Thyrotropin receptor) | 051616-D04 | 1169 | 2914 | CD8SP-TSHR-KB1-(vL-vH)-Myc-z- P2A-K13-Flag-T2A-PAC |
| TSHR (Thyrotropin receptor) | | 1170 | 2915 | CD8SP-TSHR-5C9-(vL-vH)-Myc-z- P2A-K13-Flag-T2A-PAC |
| TSLPR (Thymic stromal lymphopoietin receptor) | | 1171 | 2916 | CD8SP-TSLPR-(vL-vH)-Myc-z-P2A- K13-Flag-T2A-PAC |
| Tyrosinase/MHC class I complex | | 1172 | 2917 | CD8SP-Tyros-B2-(vL-vH)-Myc-z- P2A-K13-Flag-T2A-PAC |
| Tyrosinase/MHC class I complex | | 1173 | 2918 | CD8SP-Tyros-MC1-(vL-vH)-Myc-z- P2A-K13-Flag-T2A-PAC |
| Tyrosinase/MHC class I complex | | 1174 | 2919 | CD8SP-Tyros-TA2-(vL-vH)-Myc-z- P2A-K13-Flag-T2A-PAC |
| VEGFR3 | 060616-P04 | 1175 | 2920 | CD8SP-VEGFR3-Ab1-(vL-vH)-Myc-z- P2A-K13-Flag-T2A-PAC |
| WT1/MHC class I complex | | 1176 | 2921 | CD8SP-WT1-Ab1-(vL-vH)-Myc-z- P2A-K13-Flag-T2A-PAC |
| WT1/MHC class I complex | | 1177 | 2922 | CD8SP-WT1-Ab5-(vL-vH)-Myc-z- |
| | | | | P2A-K13-Flag-T2A-PAC |
| WT1/MHC class I complex | | 1178 | 2923 | CD8SP-MYC3-WT1-Ab13-(vL-vH)- Myc-z-P2A-K13-Flag-T2A-PAC |
| WT1/MHC class I complex | | 1179 | 2924 | CD8SP-MYC3-WT1-Ab15-(vL-vH)- Myc-z-P2A-K13-Flag-T2A-PAC |
| CDH19 | 051716-U05 | 1180 | 2925 | CD8SP-CDH19-4B10-(vL-vH)-Myc-z- P2A-K13-Flag-T2A-PAC |
| Folate Receptor beta | | 1181 | 2926 | CD8SP-FRbeta-m923-(vL-vH)-Myc-z- P2A-K13-Flag-T2A-PAC |
| LHR (Luteinizing hormone Receptor) | | 1182 | 2927 | CD8SP-LHR-8B7-(vL-vH)-Myc-z- P2A-K13-Flag-T2A-PAC |
| LHR (Luteinizing hormone Receptor) | | 1183 | 2928 | CD8SP-LHR-5F4-21-(vL-vH)-Myc-z- P2A-K13-Flag-T2A-PAC |
| B7H4 | | 1184 | 2929 | CD8SP-B7H4-hu22Cl0-(vL-vH)-Myc- z-P2A-K13-Flag-T2A-PAC |
| B7H4 | | 1185 | 2930 | CD8SP-B7H4-hu1D11-(vL-vH)-Myc- z-P2A-K13-Flag-T2A-PAC |
| IgE | | 1186 | 2931 | CD8SP-IgE-omalizumab-(vL-vH)- Myc-z-P2A-K13-Flag-T2A-PAC |
| CD23 | | 1187 | 2932 | CD8SP-CD23-p5E8-(vL-vH)-Myc-z- P2A-K13-Flag-T2A-PAC |
| GCC (Guanylyl cyclase C) | | 1188 | 2933 | CD8SP-GCC-5F9-(vL-vH)-Myc-z- P2A-K13-Flag-T2A-PAC |
| GCC (Guanylyl cyclase C) | | 1189 | 2934 | CD8SP-GCC-Ab229-(vL-vH)-Myc-z- P2A-K13-Flag-T2A-PAC |
| CD200R | | 1190 | 2935 | CD8SP-CD200R-huDx182-(vL-vH)- Myc-z-P2A-K13-Flag-T2A-PAC |
| Tn-Muc1 | | 1191 | 2936 | CD8SP-Tn-Muc1-5E5-HL-(vH-vL)- Myc-z-P2A-K13-Flag-T2A-PAC |
| CD22 | | 1192 | 2937 | CD8SP-CD22-5-HL-(vH-vL)-Myc-z- P2A-K13-Flag-T2A-PAC |
| CD22 | | 1193 | 2938 | CD8SP-CD22-10-HL-(vH-vL)-Myc-z- P2A-K13-Flag-T2A-PAC |
| CD22 | | 1194 | 2939 | CD8SP-CD22-31-HL-(vH-vL)-Myc-z- P2A-K13-Flag-T2A-PAC |
| CD22 | | 1195 | 2940 | CD8SP-CD22-53-HL-(vH-vL)-Myc-z- P2A-K13-Flag-T2A-PAC |
| CD22 | | 1196 | 2941 | CD8SP-CD22-65-HL-(vH-vL)-Myc-z- P2A-K13-Flag-T2A-PAC |

**Table 20: Exemplary CAR constructs containing 41BB costimulatory domain, CD3z activation domain and coexpressing K13**

| **Target** | **Clone ID** | **SEQ ID (DNA)** | **SEQ ID (PRT)** | **CONSTRUCT NAME** |
|---|---|---|---|---|
| CD19 | 111014-Y11 | 1197 | 2942 | CD8SP-FMC63-(vL-vH)-Myc-BBz-P2A- K13-Flag-T2A-PAC |
| CD19 | | 1198 | 2943 | CD8SP-huFMC63-11-(vL-vH)-Myc-BBz- P2A-K13-Flag-T2A-PAC |
| CD19 | | 1199 | 2944 | CD8SP-huFMC63-11-N203Q-(vL-vH)- Myc-BBz-P2A-K13-Flag-T2A-PAC |
| CD19 | | 1200 | 2945 | CD8SP-CD19Bu12-(vL-vH)-Myc-BBz- P2A-K13-Flag-T2A-PAC |
| CD19 | | 1201 | 2946 | CD8SP-2-CD19MM-(vL-vH)-Myc-BBz- P2A-K13-Flag-T2A-PAC |
| CD19 | | 1202 | 2947 | CD8SP-CD19-4G7-(vL-vH)-Myc-BBz- P2A-K13-Flag-T2A-PAC |
| CD19 | | 1203 | 2948 | CD8SP-CD19-MEDI-3649-(vL-vH)-Myc- BBz-P2A-K13-Flag-T2A-PAC |
| CD19 | | 1204 | 2949 | CD8SP-CD19-Medrex-24D1-(vL-vH)- Myc-BBz-P2A-K13-Flag-T2A-PAC |
| CD19 | | 1205 | 2950 | CD8SP-Ritx-CD19-MOR0028-(vL-vH)- Myc-BBz-P2A-K13-Flag-T2A-PAC |
| CD19 | | 1206 | 2951 | CD8SP-CD19-HD37-H2L1-(vL-vH)-Myc- BBz-P2A-K13-Flag-T2A-PAC |
| CD19 | | 1207 | 2952 | CD8SP-CD19-huBly3-(vL-vH)-Myc-BBz- P2A-K13-Flag-T2A-PAC |
| CD19 | | 1208 | 2953 | CD8SP-CD19-huSJ25C1-(vL-vH)-Myc- BBz-P2A-K13-Flag-T2A-PAC |
| CD19 | | 1209 | 2954 | CD8SP-Ritx-CD19-hB4-(vL-vH)-Myc- BBz-P2A-K13-Flag-T2A-PAC |
| CD19 | | 1210 | 2955 | CD8SP-CD19-hu-mROO5-1-(vL-vH)- Myc-BBz-P2A-K13-Flag-T2A-PAC |
| CD19 | | 1211 | 2956 | CD8SP-CD19-hA19-(vL-vH)-Myc-BBz- P2A-K13-Flag-T2A-PAC |
| AFP/MHC class I complex | | 1212 | 2957 | CD8SP-AFP-61-(vL-vH)-Myc-BBz-P2A- K13-Flag-T2A-PAC |
| AFP/MHC class I complex | | 1213 | 2958 | CD8SP-AFP-76-(vL-vH)-Myc-BBz-P2A- K13-Flag-T2A-PAC |
| AFP/MHC class I complex | | 1214 | 2959 | CD8SP-AFP-79-(vL-vH)-Myc-BBz-P2A- K13-Flag-T2A-PAC |
| HIV1 -envelop glycoprotein | | 1215 | 2960 | CD8SP-HIV1-N6-(vL-vH)-Myc-BBz- P2A-K13-Flag-T2A-PAC |
| ALK (Anaplastic Lymphoma Kinase) | | 1216 | 2961 | CD8SP-Alk-48-(vL-vH)-Myc-BBz-P2A- K13-Flag-T2A-PAC |
| ALK (Anaplastic Lymphoma Kinase) | | 1217 | 2962 | CD8SP-Alk-58-(vL-vH)-Myc-BBz-P2A- K13-Flag-T2A-PAC |
| Amyloid | | 1218 | 2963 | SP-Amyloid-158-(vL-vH)-Myc-BBz-P2A- |
| | | | | K13-Flag-T2A-PAC |
| Biotin | | 1219 | 2964 | CD8SP-dc-Avidin-Myc-BBz-P2A-K13- Flag-T2A-PAC |
| CD45 | | 1220 | 2965 | CD8SP-BC8-CD45-(vL-vH)-Myc-BBz- P2A-K13-Flag-T2A-PAC |
| BCMA | | 1221 | 2966 | CD8SP-BCMA-J6M0-(vL-vH)-Myc-BBz- P2A-K13-Flag-T2A-PAC |
| BCMA | | 1222 | 2967 | CD8SP-BCMA-huC12A3-L3H3-(vL-vH)- Myc-BBz-P2A-K13-Flag-T2A-PAC |
| BCMA | | 1223 | 2968 | CD8SP-BCMA-ET-40-(vL-vH)-Myc- BBz-P2A-K13-Flag-T2A-PAC |
| BCMA | | 1224 | 2969 | CD8SP-BCMA-ET-54-(vL-vH)-Myc- BBz-P2A-K13-Flag-T2A-PAC |
| BCMA | | 1225 | 2970 | CD8SP-BCMA-ET-03-(vL-vH)-Myc- BBz-P2A-K13-Flag-T2A-PAC |
| BCMA | | 1226 | 2971 | CD8SP-BCMA-huC11.D5.3L1H3-(vL- vH)-Myc-BBz-P2A-K13-Flag-T2A-PAC |
| BCMA | | 1227 | 2972 | CD8SP-BCMA-huC13-F12-(vL-vH)-Myc- BBz-P2A-K13-Flag-T2A-PAC |
| CCR4 | | 1228 | 2973 | CD8SP-CCR4-humAb1567-(vL-vH)-Myc- BBz-P2A-K13-Flag-T2A-PAC |
| HIV1-envelop glycoprotein | | 1229 | 2974 | CD8SP-CD4-ECD-Gly-Ser-Linker-DC- SIGN-Myc-BBz-P2A-K13-Flag-T2A-PAC |
| CD5 | | 1230 | 2975 | CD8SP-CD5-9-(vL-vH)-Myc-BBz-P2A- K13-Flag-T2A-PAC |
| CD5 | | 1231 | 2976 | CD8SP-CD5-18-(vL-vH)-Myc-BBz-P2A- K13-Flag-T2A-PAC |
| Ig Fc | | 1232 | 2977 | CD8SP-CD16A-V158-ECD-v2-Myc-BBz- P2A-K13-Flag-T2A-PAC |
| Ig Fc | | 1233 | 2978 | CD8SP-CD16A-V158-ECD-v1-Myc-BBz- P2A-K13-Flag-T2A-PAC |
| CD20 | | 1234 | 2979 | CD8SP-CD20-2F2-(vL-vH)-Myc-BBz- P2A-K13-Flag-T2A-PAC |
| CD20 | | 1235 | 2980 | CD8SP-CD20-GA101-(vL-vH)-Myc-BBz- P2A-K13-Flag-T2A-PAC |
| CD20 | | 1236 | 2981 | CD8SP-CD20-Leu16-(vL-vH)-Myc-BBz- P2A-K13-Flag-T2A-PAC |
| CD20 | | 1237 | 2982 | CD8SP-CD20-11B8-(vL-vH)-Myc-BBz- P2A-K13-Flag-T2A-PAC |
| CD20 | | 1238 | 2983 | CD8SP-CD20-2C6-(vL-vH)-Myc-BBz- P2A-K13-Flag-T2A-PAC |
| CD20 | | 1239 | 2984 | CD8SP-CD20-2H7-(vL-vH)-Myc-BBz- P2A-K13-Flag-T2A-PAC |
| CD20 | | 1240 | 2985 | CD8SP-CD20-hA20-(vL-vH)-Myc-BBz- P2A-K13-Flag-T2A-PAC |
| CD20 | | 1241 | 2986 | CD8SP-CD20-BM-CA-1925-v4-(vL-vH)- Myc-BBz-P2A-K13-Flag-T2A-PAC |
| CD20 | | 1242 | 2987 | CD8SP-CD20-Ubli-v4-(vL-vH)-Myc- |
| | | | | BBz-P2A-K13-Flag-T2A-PAC |
| CD20 | | 1243 | 2988 | CD8SP-CD20-2H7-(vL-vH)-Myc-BBz- P2A-K13-Flag-T2A-PAC |
| CD20 | | 1244 | 2989 | CD8SP-CD20-h1F5-(vL-vH)-Myc-BBz- P2A-K13-Flag-T2A-PAC |
| CD20 | | 1245 | 2990 | CD8SP-CD20-7D8-(vL-vH)-Myc-BBz- P2A-K13-Flag-T2A-PAC |
| CD20 | | 1246 | 2991 | CD8SP-CD20-AME-33-(vL-vH)-Myc- BBz-P2A-K13-Flag-T2A-PAC |
| CD22 | | 1247 | 2992 | CD8SP-CD22-h10F4v2-(vL-vH)-Myc- BBz-P2A-K13-Flag-T2A-PAC |
| CD22 | | 1248 | 2993 | CD8SP-CD22-H22Rhov2ACDRKA-(vL- vH)-Myc-BBz-P2A-K13-Flag-T2A-PAC |
| CD22 | | 1249 | 2994 | CD8SP-CD22-m971-(vL-vH)-Myc-BBz- P2A-K13-Flag-T2A-PAC |
| CD22 | | 1250 | 2995 | CD8SP-CD22-m971-HL-(vH-vL)-Myc- BBz-P2A-K13-Flag-T2A-PAC |
| CD30 | | 1251 | 2996 | CD8SP-CD30-5F11-(vL-vH)-Myc-BBz- P2A-K13-Flag-T2A-PAC |
| CD30 | | 1252 | 2997 | CD8SP-CD30-Ac10-(vL-vH)-Myc-BBz- P2A-K13-Flag-T2A-PAC |
| CD32 | | 1253 | 2998 | CD8SP-CD32-Med9-(vL-vH)-Myc-BBz- P2A-K13-Flag-T2A-PAC |
| CD33 | | 1254 | 2999 | CD8SP-CD33-AF5-(vL-vH)-Myc-BBz- P2A-K13-Flag-T2A-PAC |
| CD33 | | 1255 | 3000 | CD8SP-CD33-huMyc9-(vL-vH)-Myc- BBz-P2A-K13-Flag-T2A-PAC |
| CD33 | | 1256 | 3001 | CD8SP-CD33-Boehr2800308-(vL-vH)- Myc-BBz-P2A-K13-Flag-T2A-PAC |
| CD33 | | 1257 | 3002 | CD8SP-CD33-Him3-4-(vL-vH)-Myc- BBz-P2A-K13-Flag-T2A-PAC |
| CD33 | | 1258 | 3003 | CD8SP-CD33-SGNh2H12-(vL-vH)-Myc- BBz-P2A-K13-Flag-T2A-PAC |
| CD33 | | 1259 | 3004 | CD8SP-CD33-15G15-33-(vL-vH)-Myc- BBz-P2A-K13-Flag-T2A-PAC |
| CD33 | | 1260 | 3005 | CD8SP-CD33-33H4-(vL-vH)-Myc-BBz- P2A-K13-Flag-T2A-PAC |
| CD33 | | 1261 | 3006 | CD8SP-CD33-9C3-2-(vL-vH)-Myc-BBz- P2A-K13-Flag-T2A-PAC |
| CD34 | | 1262 | 3007 | CD8SP-CD34-hu4C7-(vL-vH)-Myc-BBz- P2A-K13-Flag-T2A-PAC |
| CD44v6 | | 1263 | 3008 | CD8SP-CD44v6-Biwa8-(vL-vH)-Myc- BBz-P2A-K13-Flag-T2A-PAC |
| CD70 | | 1264 | 3009 | CD8SP-CD70-h1F6-(vL-vH)-Myc-BBz- P2A-K13-Flag-T2A-PAC |
| CD79b | | 1265 | 3010 | CD8SP-CD79b-2F2-(vL-vH)-Myc-BBz- P2A-K13-Flag-T2A-PAC |
| CD79b | | 1266 | 3011 | CD8SP-huMA79bv28-(vL-vH)-Myc-BBz- P2A-K13-Flag-T2A-PAC |
| CD99 | | 1267 | 3012 | CD8SP-CD99-hu12E7-(vL-vH)-Myc- BBz-P2A-K13-Flag-T2A-PAC |
| CD 123 | | 1268 | 3013 | CD8SP-CD123-CSL362-(vL-vH)-Myc- BBz-P2A-K13-Flag-T2A-PAC |
| CD 123 | | 1269 | 3014 | CD8SP-CD123-1172-(vL-vH)-Myc-BBz- P2A-K13-Flag-T2A-PAC |
| CD 123 | | 1270 | 3015 | CD8SP-CD123-DART-1-(vL-vH)-Myc- BBz-P2A-K13-Flag-T2A-PAC |
| CD 123 | | 1271 | 3016 | CD8SP-CD123-DART-2-(vL-vH)-Myc- BBz-P2A-K13-Flag-T2A-PAC |
| CD123 | | 1272 | 3017 | CD8SP-CD123-I3RB18-(vL-vH)-Myc- BBz-P2A-K13-Flag-T2A-PAC |
| CD123 | | 1273 | 3018 | CD8SP-CD123-hu3E3-(vL-vH)-Myc- BBz-P2A-K13-Flag-T2A-PAC |
| CD123 | | 1274 | 3019 | CD8SP-CD123-9F6-(vL-vH)-Myc-BBz- P2A-K13-Flag-T2A-PAC |
| CD123 | | 1275 | 3020 | CD8SP-CD123-I3RB2-(vL-vH)-Myc- BBz-P2A-K13-Flag-T2A-PAC |
| CD123 | | 1276 | 3021 | CD8SP-CD123-1176-(vL-vH)-Myc-BBz- P2A-K13-Flag-T2A-PAC |
| CD 123 | | 1277 | 3022 | CD8SP-Ritx2-CD123-8B11-(vL-vH)- Myc-BBz-P2A-K13-Flag-T2A-PAC |
| CD 123 | | 1278 | 3023 | CD8SP-CD123-2B8-(vL-vH)-Myc-BBz- P2A-K13-Flag-T2A-PAC |
| CD 123 | | 1279 | 3024 | CD8SP-CD123-9D7-(vL-vH)-Myc-BBz- P2A-K13-Flag-T2A-PAC |
| CD123 | | 1280 | 3025 | CD8SP-CD123-3B10-(vL-vH)-Myc-BBz- P2A-K13-Flag-T2A-PAC |
| CD138 | | 1281 | 3026 | CD8SP-CD138-(vL-vH)-Myc-BBz-P2A- K13-Flag-T2A-PAC |
| CD179b | | 1282 | 3027 | CD8SP-CD179b-(vL-vH)-Myc-BBz-P2A- K13-Flag-T2A-PAC |
| CD276 | | 1283 | 3028 | CD8SP-CD276-17-(vL-vH)-Myc-BBz- P2A-K13-Flag-T2A-PAC |
| CD324 | | 1284 | 3029 | CD8SP-CD324-SC10-6-(vL-vH)-Myc- BBz-P2A-K13-Flag-T2A-PAC |
| CD324 | | 1285 | 3030 | CD8SP-CD324-hSC10-17-(vL-vH)-Myc- BBz-P2A-K13-Flag-T2A-PAC |
| CDH6 | | 1286 | 3031 | CD8SP-CDH6-NOV710-(vL-vH)-Myc- BBz-P2A-K13-Flag-T2A-PAC |
| CDH6 | | 1287 | 3032 | CD8SP-CDH6-NOV712-(vL-vH)-Myc- BBz-P2A-K13-Flag-T2A-PAC |
| CDH17 | | 1288 | 3033 | CD8SP-CDH17-PTA001A4-(vL-vH)- Myc-BBz-P2A-K13-Flag-T2A-PAC |
| CDH 19 | | 1289 | 3034 | CD8SP-CDH19-16A4-(vL-vH)-Myc-BBz- P2A-K13-Flag-T2A-PAC |
| EGFR | | 1290 | 3035 | CD8SP-Cetuximab-(vL-vH)-Myc-BBz- P2A-K13-Flag-T2A-PAC |
| CLEC5A | | 1291 | 3036 | CD8SP-CLEC5A-8H8F5-(vL-vH)-Myc- |
| | | | | BBz-P2A-K13-Flag-T2A-PAC |
| CLEC5A | | 1292 | 3037 | CD8SP-CLEC5A-3E12A2-(vL-vH)-Myc- BBz-P2A-K13-Flag-T2A-PAC |
| GR/LHR (Gonadotropin Receptor) | | 1293 | 3038 | SP-CGHb-Gly-Ser-Linker-CGHa-Myc- BBz-P2A-K13-Flag-T2A-PAC |
| CLLI | | 1294 | 3039 | CD8SP-CLL1-M26-(vL-vH)-Myc-BBz- P2A-K13-Flag-T2A-PAC |
| CLLI | | 1295 | 3040 | CD8SP-CLL1-M32-(vL-vH)-Myc-BBz- P2A-K13-Flag-T2A-PAC |
| CLLI | | 1296 | 3041 | CD8SP-CLL1-21C9-L2H3-(vL-vH)-Myc- BBz-P2A-K13-Flag-T2A-PAC |
| CLLI | | 1297 | 3042 | CD8SP-CLL1-6E7L4H1e-(vL-vH)-Myc- BBz-P2A-K13-Flag-T2A-PAC |
| CLLI | | 1298 | 3043 | CD8SP-CLL1-hu1075-v1-(vL-vH)-Myc- BBz-P2A-K13-Flag-T2A-PAC |
| CLLI | | 1299 | 3044 | CD8SP-CLL1-hu1075-v2-(vL-vH)-Myc- BBz-P2A-K13-Flag-T2A-PAC |
| CMVpp65/MHC class I complex | | 1300 | 3045 | CD8SP-CMVpp65-F5-(vL-vH)-Myc-BBz- P2A-K13-Flag-T2A-PAC |
| CS1 (SLAMF7) | | 1301 | 3046 | CD8SP-CS1-huLuc63-(vL-vH)-Myc-BBz- P2A-K13-Flag-T2A-PAC |
| CS1 (SLAMF7) | | 1302 | 3047 | CD8SP-CS1-HuLuc64-(vL-vH)-Myc- BBz-P2A-K13-Flag-T2A-PAC |
| CS1 (SLAMF7) | | 1303 | 3048 | CD8SP-CS1-huLuc90-(vL-vH)-Myc-BBz- P2A-K13-Flag-T2A-PAC |
| CS1 (SLAMF7) | | 1304 | 3049 | CD8SP-CS1-PDL241-(vL-vH)-Myc-BBz- P2A-K13-Flag-T2A-PAC |
| CS1 (SLAMF7) | | 1305 | 3050 | CD8SP-CS1-Hu27A-(vL-vH)-Myc-BBz- P2A-K13-Flag-T2A-PAC |
| CS1 (SLAMF7) | | 1306 | 3051 | CD8SP-CS1-ScHu34C3-(vL-vH)-Myc- BBz-P2A-K13-Flag-T2A-PAC |
| CS1 (SLAMF7) | | 1307 | 3052 | CD8SP-CS1-Hu31-D2-(vL-vH)-Myc- BBz-P2A-K13-Flag-T2A-PAC |
| CS1(SLAMF7) | | 1308 | 3053 | CD8SP-CS1-Luc34-(vL-vH)-Myc-BBz- P2A-K13-Flag-T2A-PAC |
| CS1 (SLAMF7) | | 1309 | 3054 | CD8SP-CS1-LucX2-(vL-vH)-Myc-BBz- P2A-K13-Flag-T2A-PAC |
| CSF2RA | | 1310 | 3055 | CD8SP-CSF2RA-Ab6-(vL-vH)-Myc-BBz- P2A-K13-Flag-T2A-PAC |
| CSF2RA | | 1311 | 3056 | CD8SP-CSF2RA-Ab1-(vL-vH)-Myc-BBz- P2A-K13-Flag-T2A-PAC |
| CXCR4 and CD123 | | 1312 | 3057 | CD8SP-CXCR4-1-vHH-Gly-Ser-Linker- CD123-1-vHH-Myc-BBz-P2A-K13-Flag- T2A-PAC |
| CXCR4 and CD123 | | 1313 | 3058 | CD8SP-CXCR4-2-VHH-Gly-Ser-Linker- CD123-2-VHH-Myc-BBz-P2A-K13-Flag- T2A-PAC |
| DLL3 (Delta Like Ligand 3) | | 1314 | 3059 | CD8SP-DLL3-hSC16-13-(vL-vH)-Myc- BBz-P2A-K13-Flag-T2A-PAC |
| DLL3 (Delta Like Ligand 3) | | 1315 | 3060 | CD8SP-DLL3-hSC16-56-(vL-vH)-Myc- BBz-P2A-K13-Flag-T2A-PAC |
| EBNA3c/MHC class I complex | | 1316 | 3061 | CD8SP-EBNA3c-315-(vL-vH)-Myc-BBz- P2A-K13-Flag-T2A-PAC |
| EBV-gp350 | | 1317 | 3062 | CD8SP-EBV-gp350-(vL-vH)-Myc-BBz- P2A-K13-Flag-T2A-PAC |
| EGFR | | 1318 | 3063 | CD8SP-EGFR1-vHH-Myc-BBz-P2A- K13-Flag-T2A-PAC |
| EGFR & CEA | | 1319 | 3064 | CD8SP-EGFR1-vHH-Gly-Ser-Linker- CEA1-vHH-Myc-BBz-P2A-K13-Flag- T2A-PAC |
| EGFR & CEA | | 1320 | 3065 | CD8SP-EGFR33-vHH-Gly-Ser-Linker- CEA5-vHH-Myc-BBz-P2A-K13-Flag- T2A-PAC |
| EGFRvIII | | 1321 | 3066 | CD8SP-EGFRvIII-139-(vL-vH)-Myc- BBz-P2A-K13-Flag-T2A-PAC |
| EGFRvIII | | 1322 | 3067 | CD8SP-EGFRvIII-2173-(vL-vH)-Myc- BBz-P2A-K13-Flag-T2A-PAC |
| EpCam1 | | 1323 | 3068 | CD8SP-Epcam1-MM1-(vL-vH)-Myc- BBz-P2A-K13-Flag-T2A-PAC |
| EpCam1 | | 1324 | 3069 | CD8SP-Epcam1-D5K5-(vL-vH)-Myc- BBz-P2A-K13-Flag-T2A-PAC |
| FLT3 | | 1325 | 3070 | CD8SP-FLT3-NC7-(vL-vH)-Myc-BBz- P2A-K13-Flag-T2A-PAC |
| FITC | | 1326 | 3071 | CD8SP-FITC-(vL-vH)-Myc-BBz-P2A- K13-Flag-T2A-PAC |
| FITC | | 1327 | 3072 | CD8SP-FITC-4M-53-(vL-vH)-Myc-BBz- P2A-K13-Flag-T2A-PAC |
| FITC | | 1328 | 3073 | CD8SP-FITC-E2-HL-(vH-vL)-Myc-BBz- P2A-K13-Flag-T2A-PAC |
| Influenza A HA | | 1329 | 3074 | CD8SP-FLU-MEDI-8852-(vL-vH)-Myc- BBz-P2A-K13-Flag-T2A-PAC |
| FR1 (Folate Receptor alpha) | | 1330 | 3075 | CD8SP-FR1-huMov19-(vL-vH)-Myc- BBz-P2A-K13-Flag-T2A-PAC |
| FSHR (Follicle Stimulating Hormone Receptor) | | 1331 | 3076 | CD8SP-FSHb-Gly-Ser-Linker-CGHa- Myc-BBz-P2A-K13-Flag-T2A-PAC |
| GAD (Glutamic Acid Decarboxylase)/MH C class I complex | | 1332 | 3077 | CD8SP-GAD-G3H8-(vL-vH)-Myc-BBz- P2A-K13-Flag-T2A-PAC |
| GD2 | | 1333 | 3078 | CD8SP-GD2-hu14-18-(vL-vH)-Myc-BBz- P2A-K13-Flag-T2A-PAC |
| GD2 | | 1334 | 3079 | CD8SP-GD2-hu3F8-(vL-vH)-Myc-BBz- P2A-K13-Flag-T2A-PAC |
| GD3 | | 1335 | 3080 | CD8SP-GD3-KM-641-(vL-vH)-Myc-BBz- P2A-K13-Flag-T2A-PAC |
| GFRa4 (GDNF Family Receptor Alpha 4) | | 1336 | 3081 | CD8SP-GFRAlpha4-P4-6-(vL-vH)-Myc- BBz-P2A-K13-Flag-T2A-PAC |
| GFRa4 (GDNF Family Receptor Alpha 4) | | 1337 | 3082 | CD8SP-GFRa4-P4-10-(vL-vH)-Myc-BBz- P2A-K13-Flag-T2A-PAC |
| GM1 | | 1338 | 3083 | CD8SP-GM1-5B2-(vL-vH)-Myc-BBz- P2A-K13-Flag-T2A-PAC |
| GM1 | | 1339 | 3084 | CD8SP-GM1-7E5-(vL-vH)-Myc-BBz- P2A-K13-Flag-T2A-PAC |
| GPRC5D (G-protein coupled receptor family C group 5 member D) | | 1340 | 3085 | CD8SP-GPRC5D-ET150-5-(vL-vH)-Myc- BBz-P2A-K13-Flag-T2A-PAC |
| GPRC5D | | 1341 | 3086 | CD8SP-GPRC5D-ET150-18-(vL-vH)- Myc-BBz-P2A-K13-Flag-T2A-PAC |
| GPRC5D | | 1342 | 3087 | CD8SP-GPRC5D-ET150-1-(vL-vH)-Myc- BBz-P2A-K13-Flag-T2A-PAC |
| GPRC5D | | 1343 | 3088 | CD8SP-GPRC5D-ET150-2-(vL-vH)-Myc- BBz-P2A-K13-Flag-T2A-PAC |
| gp 100/MHC class I complex | | 1344 | 3089 | CD8SP-gp100-(vL-vH)-Myc-BBz-P2A- K13-Flag-T2A-PAC |
| gp100/MHC class I complex | | 1345 | 3090 | CD8SP-gp100-G2D12-(vL-vH)-Myc-BBz- P2A-K13-Flag-T2A-PAC |
| GPC3 (Glypican 3) | | 1346 | 3091 | CD8SP-GPC3-4E5-(vL-vH)-Myc-BBz- P2A-K13-Flag-T2A-PAC |
| gpNMB (Glycoprotein Nmb) | | 1347 | 3092 | CD8SP-gpNMB-115-(vL-vH)-Myc-BBz- P2A-K13-Flag-T2A-PAC |
| GRP78 | | 1348 | 3093 | CD8SP-GRP78-GC18-(vL-vH)-Myc-BBz- P2A-K13-Flag-T2A-PAC |
| Her2 | | 1349 | 3094 | CD8SP-Her2-5F7-vHH-Myc-BBz-P2A- K13-Flag-T2A-PAC |
| Her2 | | 1350 | 3095 | IgHSP-Her2-Affi-Myc-BBz-P2A-K13- Flag-T2A-PAC |
| Her2 | | 1351 | 3096 | CD8SP-Her2-1-Darpin-Myc-BBz-P2A- K13-Flag-T2A-PAC |
| Her2 | | 1352 | 3097 | IgHSP-Her2-2-Darpin-Myc-BBz-P2A- K13-Flag-T2A-PAC |
| Her2 | | 1353 | 3098 | CD8SP-Her2-5F7-vHH-Gly-Ser-Linker- Her2-47D5-vHH-Myc-BBz-P2A-K13- Flag-T2A-PAC |
| Her2 | | 1354 | 3099 | CD8SP-Her2-Hu4D5-(vL-vH)-Myc-BBz- P2A-K13-Flag-T2A-PAC |
| Her3 | | 1355 | 3100 | CD8SP-Her3-17B05So-vHH-Myc-BBz- P2A-K13-Flag-T2A-PAC |
| Her3 | | 1356 | 3101 | CD8SP-Her3-Affi-Myc-BBz-P2A-K13- Flag-T2A-PAC |
| Her2 and Her3 | | 1357 | 3102 | CD8SP-Her3-17B05So-vHH-Gly-Ser- Linker-Her2-2D3-vHH-Myc-BBz-P2A- K13-Flag-T2A-PAC |
| HIV1-gag/MHC class I complex | | 1358 | 3103 | CD8SP-HIV1-E5-(vL-vH)-Myc-BBz- P2A-K13-Flag-T2A-PAC |
| HIV1-envelop glycoprotein | | 1359 | 3104 | CD8SP-HIV1-3BNC117-(vL-vH)-Myc- BBz-P2A-K13-Flag-T2A-PAC |
| HIV1-envelop glycoprotein | | 1360 | 3105 | CD8SP-HIV1-PGT-128-(vL-vH)-Myc- BBz-P2A-K13-Flag-T2A-PAC |
| HIV 1-envelop glycoprotein | | 1361 | 3106 | CD8SP-HIV1-VR-C01-(vL-vH)-Myc- BBz-P2A-K13-Flag-T2A-PAC |
| HIV1-envelop glycoprotein | | 1362 | 3107 | CD8SP-HIV1-X5-(vL-vH)-Myc-BBz- P2A-K13-Flag-T2A-PAC |
| HLA-A2 | | 1363 | 3108 | CD8SP-HLA-A2-3PB2-(vL-vH)-Myc- BBz-P2A-K13-Flag-T2A-PAC |
| HMW-MAA | | 1364 | 3109 | CD8SP-HMW-MAA-hIND-(vL-vH)-Myc- BBz-P2A-K13-Flag-T2A-PAC |
| HPV16-E7/MHC class I complex | | 1365 | 3110 | CD8SP-HPV16-7-8-(vL-vH)-Myc-BBz- P2A-K13-Flag-T2A-PAC |
| HPV 16-E7/MHC class I complex | | 1366 | 3111 | CD8SP-HPV16-2-(vL-vH)-Myc-BBz- P2A-K13-Flag-T2A-PAC |
| HTLV1-TAX/MHC class I complex | | 1367 | 3112 | CD8SP-HTLV-TAX-T3F2-(vL-vH)-Myc- BBz-P2A-K13-Flag-T2A-PAC |
| HTLV1-TAX/MHC class I complex | | 1368 | 3113 | CD8SP-HTLV-TAX-T3E3-(vL-vH)-Myc- BBz-P2A-K13-Flag-T2A-PAC |
| IL11Ra | | 1369 | 3114 | CD8SP-IL11Ra-8E2-Ts107-(vL-vH)-Myc- BBz-P2A-K13-Flag-T2A-PAC |
| IL6Ra | | 1370 | 3115 | IgHSP-IL6R-304-vHH-Myc-BBz-P2A- K13-Flag-T2A-PAC |
| IL13Ra2 | | 1371 | 3116 | CD8SP-IL13Ra2-hu107-(vL-vH)-Myc- BBz-P2A-K13-Flag-T2A-PAC |
| IL13Ra2 | | 1372 | 3117 | CD8SP-IL13Ra2-Hu108-(vL-vH)-Myc- BBz-P2A-K13-Flag-T2A-PAC |
| KSHV-K8.1 | | 1373 | 3118 | CD8SP-KSHV-4C3-(vL-vH)-Myc-BBz- P2A-K13-Flag-T2A-PAC |
| LAMP1 (Lysosomal-associated membrane protein 1) | | 1374 | 3119 | CD8SP-LAMP1-humab1-2-(vL-vH)-Myc- BBz-P2A-K13-Flag-T2A-PAC |
| LAMP1 (Lysosomal-associated membrane protein 1) | | 1375 | 3120 | CD8SP-LAMP1-Mb4-(vL-vH)-Myc-BBz- P2A-K13-Flag-T2A-PAC |
| LewisY | | 1376 | 3121 | CD8SP-LewisY-huS193-(vL-vH)-Myc- BBz-P2A-K13-Flag-T2A-PAC |
| LICAM | | 1377 | 3122 | CD8SP-L1CAM-9-3-HU3-(vL-vH)-Myc- BBz-P2A-K13-Flag-T2A-PAC |
| LHR | | 1378 | 3123 | SP-LHb-Gly-Ser-Linker-CGHa-Myc-BBz- P2A-K13-Flag-T2A-PAC |
| Lym1 | | 1379 | 3124 | CD8SP-Lym1-(vL-vH)-Myc-BBz-P2A- K13-Flag-T2A-PAC |
| Lym2 | | 1380 | 3125 | CD8SP-Lym2-(vL-vH)-Myc-BBz-P2A- K13-Flag-T2A-PAC |
| CD79b | | 1381 | 3126 | CD8SP-huMA79bv28-(vL-vH)-Myc-BBz- P2A-K13-Flag-T2A-PAC |
| MART1/MHC class I complex | | 1382 | 3127 | CD8SP-MART1-CAG10-(vL-vH)-Myc- BBz-P2A-K13-Flag-T2A-PAC |
| MART1/MHC class I complex | | 1383 | 3128 | CD8SP-MART1-CLA12-(vL-vH)-Myc- BBz-P2A-K13-Flag-T2A-PAC |
| Mesothelin | | 1384 | 3129 | CD8SP-Mesothelin-m912-(vL-vH)-Myc- BBz-P2A-K13-Flag-T2A-PAC |
| cMet | | 1385 | 3130 | CD8SP-cMet-171-vHH-Myc-BBz-P2A- K13-Flag-T2A-PAC |
| cMet and Her3 | | 1386 | 3131 | CD8SP-cMET-171-vHH-Gly-Ser-Linker- Her3-21F06-vHH-Myc-BBz-P2A-K13- Flag-T2A-PAC |
| MPL (Thrombopoietin receptor) | | 1387 | 3132 | CD8SP-MPL-175-(vL-vH)-Myc-BBz- P2A-K13-Flag-T2A-PAC |
| MPL (Thrombopoietin receptor) | | 1388 | 3133 | CD8SP-MPL-161-(vL-vH)-Myc-BBz- P2A-K13-Flag-T2A-PAC |
| MPL (Thrombopoietin receptor) | | 1389 | 3134 | CD8SP-MPL-161-HL-(vH-vL)-Myc-BBz- P2A-K13-Flag-T2A-PAC |
| MPL (Thrombopoietin receptor) | | 1390 | 3135 | CD8SP-2-MPL-111-(vL-vH)-Myc-BBz- P2A-K13-Flag-T2A-PAC |
| MPL (Thrombopoietin receptor) | | 1391 | 3136 | CD8SP-MPL-178-(vL-vH)-Myc-BBz- P2A-K13-Flag-T2A-PAC |
| MPL (Thrombopoietin receptor) | | 1392 | 3137 | CD8SP-MPL-AB317-(vL-vH)-Myc-BBz- P2A-K13-Flag-T2A-PAC |
| MPL (Thrombopoietin receptor) | | 1393 | 3138 | CD8SP-MPL-12E10-(vL-vH)-Myc-BBz- P2A-K13-Flag-T2A-PAC |
| MPL (Thrombopoietin receptor) | | 1394 | 3139 | CD8SP-MPL-huVB22Bw5-(vL-vH)-Myc- |
| | | | | BBz-P2A-K13-Flag-T2A-PAC |
| Muc1/MHC class I complex | | 1395 | 3140 | CD8SP-Muc1-D6-M3B8-(vL-vH)-Myc- BBz-P2A-K13-Flag-T2A-PAC |
| Muc1/MHC class I complex | | 1396 | 3141 | CD8SP-MUC1-D6-M3A1-(vL-vH)-Myc- BBz-P2A-K13-Flag-T2A-PAC |
| Muc16 | | 1397 | 3142 | CD8SP-Muc16-4H11-(vL-vH)-Myc-BBz- P2A-K13-Flag-T2A-PAC |
| EGFR | | 1398 | 3143 | CD8SP-Nimotuzumab-(vL-vH)-Myc-BBz- P2A-K13-Flag-T2A-PAC |
| NKG2D Ligand | | 1399 | 3144 | CD8SP-NKG2D-(GGGGS-GGGGD)- Myc-BBz-P2A-K13-Flag-T2A-PAC |
| NKG2D | | 1400 | 3145 | CD8SP-NKG2D-MS-(vL-vH)-Myc-BBz- P2A-K13-Flag-T2A-PAC |
| NY-BR1 | | 1401 | 3146 | CD8SP-NYBR1-(vL-vH)-Myc-BBz-P2A- K13-Flag-T2A-PAC |
| NY-ESO/MHC class I complex | | 1402 | 3147 | CD8SP-NYESO-T1-(vL-vH)-Myc-BBz- P2A-K13-Flag-T2A-PAC |
| NY-ESO/MHC class I complex | | 1403 | 3148 | CD8SP-NYESO-T2-(vL-vH)-Myc-BBz- P2A-K13-Flag-T2A-PAC |
| PD1 ligand (e.g., PDL1) | | 1404 | 3149 | CD8SP-PD1-ECD-Myc-BBz-P2A-K13- Flag-T2A-PAC |
| PDL1 | | 1405 | 3150 | CD8SP-PDL1-Atezoli-(vL-vH)-Myc-BBz- P2A-K13-Flag-T2A-PAC |
| PDL1 | | 1406 | 3151 | CD8SP-PDL1-SP142-(vL-vH)-Myc-BBz- P2A-K13-Flag-T2A-PAC |
| PDL1 | | 1407 | 3152 | CD8SP-PDL1-10A5-(vL-vH)-Myc-BBz- P2A-K13-Flag-T2A-PAC |
| PSCA (Prostate stem cell antigen) | | 1408 | 3153 | CD8SP-PSCA-Ha14-121-(vL-vH)-Myc- BBz-P2A-K13-Flag-T2A-PAC |
| PSCA (Prostate stem cell antigen) | | 1409 | 3154 | CD8SP-PSCA-Ha14-117-(vL-vH)-Myc- BBz-P2A-K13-Flag-T2A-PAC |
| PR1/MHC class I complex | | 1410 | 3155 | CD8SP-PR1-(vL-vH)-Myc-BBz-P2A- K13-Flag-T2A-PAC |
| PSMA (Prostate Specific Membrane Antigen) | | 1411 | 3156 | CD8SP-PSMA-006-(vL-vH)-Myc-BBz- P2A-K13-Flag-T2A-PAC |
| PSMA (Prostate Specific Membrane Antigen) | | 1412 | 3157 | CD8SP-PSMA-J591-(vL-vH)-Myc-BBz- P2A-K13-Flag-T2A-PAC |
| PTK7 (Tyrosine-protein kinase-like 7) | | 1413 | 3158 | CD8SP-PTK7-hSC6-23-(vL-vH)-Myc- BBz-P2A-K13-Flag-T2A-PAC |
| PTK7 (Tyrosine-protein kinase-like 7) | | 1414 | 3159 | CD8SP-PTK7-SC6-10-2-(vL-vH)-Myc- BBz-P2A-K13-Flag-T2A-PAC |
| ROR1 | | 1415 | 3160 | CD8SP-ROR1-4A5-(vL-vH)-Myc-BBz- |
| | | | | P2A-K13-Flag-T2A-PAC |
| ROR1 | | 1416 | 3161 | CD8SP-ROR1-4C10-(vL-vH)-Myc-BBz- P2A-K13-Flag-T2A-PAC |
| Mesothelin | | 1417 | 3162 | CD8SP-SD1-vHH-Gly-Ser-Linker-SD2- vHH-Myc-BBz-P2A-K13-Flag-T2A-PAC |
| SLea | | 1418 | 3163 | CD8SP-SLea-7E3-(vL-vH)-Myc-BBz- P2A-K13-Flag-T2A-PAC |
| SLea | | 1419 | 3164 | CD8SP-SLea-5B1-(vL-vH)-Myc-BBz- P2A-K13-Flag-T2A-PAC |
| SSEA4 (stage-specific embryonic antigen 4) | | 1420 | 3165 | CD8SP-SSEA4-(vL-vH)-Myc-BBz-P2A- K13-Flag-T2A-PAC |
| TCRB1 (TCR beta 1 constant chain) | | 1421 | 3166 | CD8SP-TCRB1-CP01-E09-(vL-vH)-Myc- BBz-P2A-K13-Flag-T2A-PAC |
| TCRB1 (TCR beta 1 constant chain) | | 1422 | 3167 | CD8SP-TCRB1-Jovi1-(vL-vH)-Myc-BBz- P2A-K13-Flag-T2A-PAC |
| TCRB2 (TCR beta 2 constant chain) | | 1423 | 3168 | CD8SP-TCRB2-CP01-D05-(vL-vH)-Myc- BBz-P2A-K13-Flag-T2A-PAC |
| TCRB2 (TCR beta 2 constant chain) | | 1424 | 3169 | CD8SP-TCRB2-CP01-E05-(vL-vH)-Myc- BBz-P2A-K13-Flag-T2A-PAC |
| TCRgd (TCR gamma/delta) | | 1425 | 3170 | CD8SP-TCRgd-G5-4-(vL-vH)-Myc-BBz- P2A-K13-Flag-T2A-PAC |
| hTERT/MHC class I complex | | 1426 | 3171 | CD8SP-TERT-4A9-T540-(vL-vH)-Myc- BBz-P2A-K13-Flag-T2A-PAC |
| hTERT/MHC class I complex | | 1427 | 3172 | CD8SP-TERT-3G3-T865-(vL-vH)-Myc- BBz-P2A-K13-Flag-T2A-PAC |
| Tissue Factor-1 | | 1428 | 3173 | CD8SP-TGFBR2-Ab1-(vL-vH)-Myc-BBz- P2A-K13-Flag-T2A-PAC |
| TGFBR2 | | 1429 | 3174 | CD8SP-TF1-98-(vL-vH)-Myc-BBz-P2A- K13-Flag-T2A-PAC |
| TIM1/HAVCR | | 1430 | 3175 | CD8SP-TIM1-HVCR1-270-2-(vL-vH)- Myc-BBz-P2A-K13-Flag-T2A-PAC |
| TIM1/HAVCR | | 1431 | 3176 | CD8SP-TIM1-HVCR1-ARD5-(vL-vH)- Myc-BBz-P2A-K13-Flag-T2A-PAC |
| TnAg | | 1432 | 3177 | CD8SP-TnAg-(vL-vH)-Myc-BBz-P2A- K13-Flag-T2A-PAC |
| Tn-Muc 1 | | 1433 | 3178 | CD8SP-TnMuc1-hu5E5-RHA8-RKA-2- (vL-vH)-Myc-BBz-P2A-K13-Flag-T2A- PAC |
| MPL (Thrombopoietin receptor) | | 1434 | 3179 | CD8SP-hTPO-Myc-BBz-P2A-K13-Flag- T2A-PAC |
| TROP2 (Trophoblast cell-surface antigen-2) | | 1435 | 3180 | CD8SP-TROP2-ARA47-HV3KV3-(vL- vH)-Myc-BBz-P2A-K13-Flag-T2A-PAC |
| TROP2 | | 1436 | 3181 | CD8SP-TROP2-h7E6-SVG-(vL-vH)-Myc- BBz-P2A-K13-Flag-T2A-PAC |
| TSHR (Thyrotropin receptor) | | 1437 | 3182 | SP-TSHb-Gly-Ser-Linker-CGHa-Myc- BBz-P2A-K13-Flag-T2A-PAC |
| TSHR | | 1438 | 3183 | CD8SP-TSHR-K1-70-(vL-vH)-Myc-BBz- P2A-K13-Flag-T2A-PAC |
| TSHR | | 1439 | 3184 | CD8SP-TSHR-KB1-(vL-vH)-Myc-BBz- P2A-K13-Flag-T2A-PAC |
| TSHR | | 1440 | 3185 | CD8SP-TSHR-5C9-(vL-vH)-Myc-BBz- P2A-K13-Flag-T2A-PAC |
| TSLPR (Thymic stromal lymphopoietin receptor) | | 1441 | 3186 | CD8SP-TSLPR-(vL-vH)-Myc-BBz-P2A- K13-Flag-T2A-PAC |
| Tyrosinase/MHC class I complex | | 1442 | 3187 | CD8SP-Tyros-B2-(vL-vH)-Myc-BBz- P2A-K13-Flag-T2A-PAC |
| Tyrosinase/MHC class I complex | | 1443 | 3188 | CD8SP-Tyros-MC1-(vL-vH)-Myc-BBz- P2A-K13-Flag-T2A-PAC |
| Tyrosinase/MHC class I complex | | 1444 | 3189 | CD8SP-Tyros-TA2-(vL-vH)-Myc-BBz- P2A-K13-Flag-T2A-PAC |
| VEGFR3 | | 1445 | 3190 | CD8SP-VEGFR3-Ab1-(vL-vH)-Myc- BBz-P2A-K13-Flag-T2A-PAC |
| WT1/MHC class I complex | | 1446 | 3191 | CD8SP-WT1-Ab1-(vL-vH)-Myc-BBz- P2A-K13-Flag-T2A-PAC |
| WT1/MHC class I complex | | 1447 | 3192 | CD8SP-WT1-Ab5-(vL-vH)-Myc-BBz- P2A-K13-Flag-T2A-PAC |
| WT1/MHC class I complex | | 1448 | 3193 | CD8SP-MYC3-WT1-Ab13-(vL-vH)-Myc- BBz-P2A-K13-Flag-T2A-PAC |
| WT1/MHC class I complex | | 1449 | 3194 | CD8SP-MYC3-WT1-Ab15-(vL-vH)-Myc- BBz-P2A-K13-Flag-T2A-PAC |
| CDH 19 | | 1450 | 3195 | CD8SP-CDH19-4B10-(vL-vH)-Myc-BBz- P2A-K13-Flag-T2A-PAC |
| Folate Receptor beta | | 1451 | 3196 | CD8SP-FRbeta-m923-(vL-vH)-Myc-BBz- P2A-K13-Flag-T2A-PAC |
| LHR (Luteinizing hormone Receptor) | | 1452 | 3197 | CD8SP-LHR-8B7-(vL-vH)-Myc-BBz- P2A-K13-Flag-T2A-PAC |
| LHR (Luteinizing hormone Receptor) | | 1453 | 3198 | CD8SP-LHR-5F4-21-(vL-vH)-Myc-BBz- P2A-K13-Flag-T2A-PAC |
| B7H4 | | 1454 | 3199 | CD8SP-B7H4-hu22Cl0-(vL-vH)-Myc- BBz-P2A-K13-Flag-T2A-PAC |
| B7H4 | | 1455 | 3200 | CD8SP-B7H4-hu1D11-(vL-vH)-Myc- BBz-P2A-K13-Flag-T2A-PAC |
| IgE | | 1456 | 3201 | CD8SP-IgE-omalizumab-(vL-vH)-Myc- BBz-P2A-K13-Flag-T2A-PAC |
| CD23 | | 1457 | 3202 | CD8SP-CD23-p5E8-(vL-vH)-Myc-BBz- |
| | | | | P2A-K13-Flag-T2A-PAC |
| GCC (Guanylyl cyclase C) | | 1458 | 3203 | CD8SP-GCC-5F9-(vL-vH)-Myc-BBz- P2A-K13-Flag-T2A-PAC |
| GCC (Guanylyl cyclase C) | | 1459 | 3204 | CD8SP-GCC-Ab229-(vL-vH)-Myc-BBz- P2A-K13-Flag-T2A-PAC |
| CD200R | | 1460 | 3205 | CD8SP-CD200R-huDx182-(vL-vH)-Myc- BBz-P2A-K13-Flag-T2A-PAC |
| Tn-Muc 1 | | 1461 | 3206 | CD8SP-Tn-Muc1-5E5-HL-(vH-vL)-Myc- BBz-P2A-K13-Flag-T2A-PAC |
| CD22 | | 1462 | 3207 | CD8SP-CD22-5-HL-(vH-vL)-Myc-BBz- P2A-K13-Flag-T2A-PAC |
| CD22 | | 1463 | 3208 | CD8SP-CD22-10-HL-(vH-vL)-Myc-BBz- P2A-K13-Flag-T2A-PAC |
| CD22 | | 1464 | 3209 | CD8SP-CD22-31-HL-(vH-vL)-Myc-BBz- P2A-K13-Flag-T2A-PAC |
| CD22 | | 1465 | 3210 | CD8SP-CD22-53-HL-(vH-vL)-Myc-BBz- P2A-K13-Flag-T2A-PAC |
| CD22 | | 1466 | 3211 | CD8SP-CD22-65-HL-(vH-vL)-Myc-BBz- P2A-K13-Flag-T2A-PAC |

**Table 21: Exemplary CAR constructs containing 41BB costimulatory domain, CD3z activation domain**

| **Target** | **Clone ID** | **SEQ ID (DNA)** | **SEQ ID (PRT)** | **CONSTRUCT NAME** |
|---|---|---|---|---|
| CD19 | 112014-A13 | 1467 | 3212 | CD8SP-FMC63-(vL-vH)-Myc- BBz-T2A-PAC |
| CD19 | 052616-T03 | 1468 | 3213 | CD8SP-huFMC63-11-(vL-vH)- Myc-BBz-T2A-PAC |
| CD19 | | 1469 | 3214 | CD8SP-huFMC63-11-N203Q-(vL- vH)-Myc-BBz-T2A-PAC |
| CD19 | 082815-P08 | 1470 | 3215 | CD8SP-CD19Bu12-(vL-vH)-Myc- BBz-T2A-PAC |
| CD19 | 062915-D03 | 1471 | 3216 | CD8SP-2-CD19MM-(vL-vH)-Myc- BBz-T2A-PAC |
| CD19 | | 1472 | 3217 | CD8SP-CD19-4G7-(vL-vH)-Myc- BBz-T2A-PAC |
| CD19 | | 1473 | 3218 | CD8SP-CD19-MEDI-3649-(vL- vH)-Myc-BBz-T2A-PAC |
| CD19 | | 1474 | 3219 | CD8SP-CD19-Medrex-24D1-(vL- vH)-Myc-BBz-T2A-PAC |
| CD19 | | 1475 | 3220 | CD8SP-Ritx-CD19-MOR0028-(vL- vH)-Myc-BBz-T2A-PAC |
| CD19 | | 1476 | 3221 | CD8SP-CD19-HD37-H2L1-(vL- vH)-Myc-BBz-T2A-PAC |
| CD19 | | 1477 | 3222 | CD8SP-CD19-huBly3-(vL-vH)- |
| | | | | Myc-BBz-T2A-PAC |
| CD19 | | 1478 | 3223 | CD8SP-CD19-huSJ25C1-(vL-vH)- Myc-BBz-T2A-PAC |
| CD19 | | 1479 | 3224 | CD8SP-Ritx-CD19-hB4-(vL-vH)- Myc-BBz-T2A-PAC |
| CD19 | | 1480 | 3225 | CD8SP-CD19-hu-mROO5-1-(vL- vH)-Myc-BBz-T2A-PAC |
| CD19 | | 1481 | 3226 | CD8SP-CD19-hA19-(vL-vH)-Myc- BBz-T2A-PAC |
| AFP/MHC class I complex | | 1482 | 3227 | CD8SP-AFP-61-(vL-vH)-Myc- BBz-T2A-PAC |
| AFP/MHC class I complex | | 1483 | 3228 | CD8SP-AFP-76-(vL-vH)-Myc- BBz-T2A-PAC |
| AFP/MHC class I complex | | 1484 | 3229 | CD8SP-AFP-79-(vL-vH)-Myc- BBz-T2A-PAC |
| HIV 1-envelop glycoprotein | | 1485 | 3230 | CD8SP-HIV1-N6-(vL-vH)-Myc- BBz-T2A-PAC |
| ALK (Anaplastic Lymphoma Kinase) | 052616-C05 | 1486 | 3231 | CD8SP-Alk-48-(vL-vH)-Myc-BBz- T2A-PAC |
| ALK (Anaplastic Lymphoma Kinase) | | 1487 | 3232 | CD8SP-Alk-58-(vL-vH)-Myc-BBz- T2A-PAC |
| Amyloid | | 1488 | 3233 | SP-Amyloid-158-(vL-vH)-Myc- BBz-T2A-PAC |
| Biotin | 052616-105 | 1489 | 3234 | CD8SP-dc-Avidin-Myc-BBz-T2A- PAC |
| CD45 | | 1490 | 3235 | CD8SP-BC8-CD45-(vL-vH)-Myc- BBz-T2A-PAC |
| BCMA | | 1491 | 3236 | CD8SP-BCMA-J6M0-(vL-vH)- Myc-BBz-T2A-PAC |
| BCMA | 123015-J03 | 1492 | 3237 | CD8SP-BCMA-huC12A3-L3H3- (vL-vH)-Myc-BBz-T2A-PAC |
| BCMA | | 1493 | 3238 | CD8SP-BCMA-ET-40-(vL-vH)- Myc-BBz-T2A-PAC |
| BCMA | | 1494 | 3239 | CD8SP-BCMA-ET-54-(vL-vH)- Myc-BBz-T2A-PAC |
| BCMA | | 1495 | 3240 | CD8SP-BCMA-ET-03-(vL-vH)- Myc-BBz-T2A-PAC |
| BCMA | | 1496 | 3241 | CD8SP-BCMA-huC11.D5.3L1H3- (vL-vH)-Myc-BBz-T2A-PAC |
| BCMA | | 1497 | 3242 | CD8SP-BCMA-huC13-F12-(vL- vH)-Myc-BBz-T2A-PAC |
| CCR4 | | 1498 | 3243 | CD8SP-CCR4-humAb1567-(vL- vH)-Myc-BBz-T2A-PAC |
| HIV 1-envelop glycoprotein | | 1499 | 3244 | CD8SP-CD4-ECD-Gly-Ser-Linker- DC-SIGN-Myc-BBz-T2A-PAC |
| CD5 | 031416-B03 | 1500 | 3245 | CD8SP-CD5-9-(vL-vH)-Myc-BBz- T2A-PAC |
| CD5 | 031416-A04 | 1501 | 3246 | CD8SP-CD5-18-(vL-vH)-Myc- BBz-T2A-PAC |
| Ig Fc | | 1502 | 3247 | CD8SP-CD16A-V158-ECD-v2- Myc-BBz-T2A-PAC |
| Ig Fc | | 1503 | 3248 | CD8SP-CD16A-V158-ECD-v1- Myc-BBz-T2A-PAC |
| CD20 | 081115-Y02 and 081115-B01 | 1504 | 3249 | CD8SP-CD20-2F2-(vL-vH)-Myc- BBz-T2A-PAC |
| CD20 | 111815-A03 | 1505 | 3250 | CD8SP-CD20-GA101-(vL-vH)- Myc-BBz-T2A-PAC |
| CD20 | | 1506 | 3251 | CD8SP-CD20-Leu16-(vL-vH)- Myc-BBz-T2A-PAC |
| CD20 | | 1507 | 3252 | CD8SP-CD20-11B8-(vL-vH)-Myc- BBz-T2A-PAC |
| CD20 | | 1508 | 3253 | CD8SP-CD20-2C6-(vL-vH)-Myc- BBz-T2A-PAC |
| CD20 | | 1509 | 3254 | CD8SP-CD20-2H7-(vL-vH)-Myc- BBz-T2A-PAC |
| CD20 | | 1510 | 3255 | CD8SP-CD20-hA20-(vL-vH)-Myc- BBz-T2A-PAC |
| CD20 | | 1511 | 3256 | CD8SP-CD20-BM-CA-1925-v4- (vL-vH)-Myc-BBz-T2A-PAC |
| CD20 | | 1512 | 3257 | CD8SP-CD20-Ubli-v4-(vL-vH)- Myc-BBz-T2A-PAC |
| CD20 | | 1513 | 3258 | CD8SP-CD20-2H7-(vL-vH)-Myc- BBz-T2A-PAC |
| CD20 | | 1514 | 3259 | CD8SP-CD20-h1F5-(vL-vH)-Myc- BBz-T2A-PAC |
| CD20 | | 1515 | 3260 | CD8SP-CD20-7D8-(vL-vH)-Myc- BBz-T2A-PAC |
| CD20 | | 1516 | 3261 | CD8SP-CD20-AME-33-(vL-vH)- Myc-BBz-T2A-PAC |
| CD22 | 052616-H04 & 052516-I07 | 1517 | 3262 | CD8SP-CD22-h10F4v2-(vL-vH)- Myc-BBz-T2A-PAC |
| CD22 | 052316-H07 | 1518 | 3263 | CD8SP-CD22- H22Rhov2ACDRKA-(vL-vH)- Myc-BBz-T2A-PAC |
| CD22 | 091515-A02 | 1519 | 3264 | CD8SP-CD22-m971-(vL-vH)-Myc- BBz-T2A-PAC |
| CD22 | | 1520 | 3265 | CD8SP-CD22-m971-HL-(vH-vL)- Myc-BBz-T2A-PAC |
| CD30 | | 1521 | 3266 | CD8SP-CD30-5F11-(vL-vH)-Myc- BBz-T2A-PAC |
| CD30 | 012216-H04 | 1522 | 3267 | CD8SP-CD30-Ac10-(vL-vH)-Myc- BBz-T2A-PAC |
| CD32 | 060916-A02 | 1523 | 3268 | CD8SP-CD32-Med9-(vL-vH)-Myc- |
| | | | | BBz-T2A-PAC |
| CD33 | | 1524 | 3269 | CD8SP-CD33-AF5-(vL-vH)-Myc- BBz-T2A-PAC |
| CD33 | 111815-C07 | 1525 | 3270 | CD8SP-CD33-huMyc9-(vL-vH)- Myc-BBz-T2A-PAC |
| CD33 | | 1526 | 3271 | CD8SP-CD33-Boehr2800308-(vL- vH)-Myc-BBz-T2A-PAC |
| CD33 | | 1527 | 3272 | CD8SP-CD33-Him3-4-(vL-vH)- Myc-BBz-T2A-PAC |
| CD33 | | 1528 | 3273 | CD8SP-CD33-SGNh2H12-(vL- vH)-Myc-BBz-T2A-PAC |
| CD33 | | 1529 | 3274 | CD8SP-CD33-15G15-33-(vL-vH)- Myc-BBz-T2A-PAC |
| CD33 | | 1530 | 3275 | CD8SP-CD33-33H4-(vL-vH)-Myc- BBz-T2A-PAC |
| CD33 | | 1531 | 3276 | CD8SP-CD33-9C3-2-(vL-vH)- Myc-BBz-T2A-PAC |
| CD34 | 052316-103 & 052616-H06 | 1532 | 3277 | CD8SP-CD34-hu4C7-(vL-vH)- Myc-BBz-T2A-PAC |
| CD44v6 | | 1533 | 3278 | CD8SP-CD44v6-Biwa8-(vL-vH)- Myc-BBz-T2A-PAC |
| CD70 | | 1534 | 3279 | CD8SP-CD70-h1F6-(vL-vH)-Myc- BBz-T2A-PAC |
| CD79b | 111815-B04 | 1535 | 3280 | CD8SP-CD79b-2F2-(vL-vH)-Myc- BBz-T2A-PAC |
| CD79b | | 1536 | 3281 | CD8SP-huMA79bv28-(vL-vH)- Myc-BBz-T2A-PAC |
| CD99 | | 1537 | 3282 | CD8SP-CD99-hu12E7-(vL-vH)- Myc-BBz-T2A-PAC |
| CD123 | 081115-X01 | 1538 | 3283 | CD8SP-CD123-CSL362-(vL-vH)- Myc-BBz-T2A-PAC |
| CD123 | | 1539 | 3284 | CD8SP-CD123-1172-(vL-vH)- Myc-BBz-T2A-PAC |
| CD123 | | 1540 | 3285 | CD8SP-CD123-DART-1-(vL-vH)- Myc-BBz-T2A-PAC |
| CD 123 | | 1541 | 3286 | CD8SP-CD123-DART-2-(vL-vH)- Myc-BBz-T2A-PAC |
| CD 123 | | 1542 | 3287 | CD8SP-CD123-I3RB18-(vL-vH)- Myc-BBz-T2A-PAC |
| CD 123 | | 1543 | 3288 | CD8SP-CD123-hu3E3-(vL-vH)- Myc-BBz-T2A-PAC |
| CD123 | | 1544 | 3289 | CD8SP-CD123-9F6-(vL-vH)-Myc- BBz-T2A-PAC |
| CD123 | | 1545 | 3290 | CD8SP-CD123-I3RB2-(vL-vH)- Myc-BBz-T2A-PAC |
| CD123 | | 1546 | 3291 | CD8SP-CD123-1176-(vL-vH)- Myc-BBz-T2A-PAC |
| CD123 | | 1547 | 3292 | CD8SP-Ritx2-CD123-8B11-(vL- |
| | | | | vH)-Myc-BBz-T2A-PAC |
| CD123 | | 1548 | 3293 | CD8SP-CD123-2B8-(vL-vH)-Myc- BBz-T2A-PAC |
| CD123 | | 1549 | 3294 | CD8SP-CD123-9D7-(vL-vH)-Myc- BBz-T2A-PAC |
| CD123 | | 1550 | 3295 | CD8SP-CD123-3B10-(vL-vH)- Myc-BBz-T2A-PAC |
| CD138 | 041715-D08 | 1551 | 3296 | CD8SP-CD138-(vL-vH)-Myc-BBz- T2A-PAC |
| CD179b | | 1552 | 3297 | CD8SP-CD179b-(vL-vH)-Myc- BBz-T2A-PAC |
| CD276 | 052316-C05 | 1553 | 3298 | CD8SP-CD276-17-(vL-vH)-Myc- BBz-T2A-PAC |
| CD324 | 052516-B02 | 1554 | 3299 | CD8SP-CD32410-6-(vL-vH)-Myc- BBz-T2A-PAC |
| CD324 | 052516-A07 | 1555 | 3300 | CD8SP-CD324-hSC10-17-(vL- vH)-Myc-BBz-T2A-PAC |
| CDH6 | | 1556 | 3301 | CD8SP-CDH6-NOV710-(vL-vH)- Myc-BBz-T2A-PAC |
| CDH6 | | 1557 | 3302 | CD8SP-CDH6-NOV712-(vL-vH)- Myc-BBz-T2A-PAC |
| CDH17 | | 1558 | 3303 | CD8SP-CDH17-PTA001A4-(vL- vH)-Myc-BBz-T2A-PAC |
| CDH19 | | 1559 | 3304 | CD8SP-CDH19-16A4-(vL-vH)- Myc-BBz-T2A-PAC |
| EGFR | | 1560 | 3305 | CD8SP-Cetuximab-(vL-vH)-Myc- BBz-T2A-PAC |
| CLEC5A | | 1561 | 3306 | CD8SP-CLEC5A-8H8F5-(vL-vH)- Myc-BBz-T2A-PAC |
| CLEC5A | 052316-G04 | 1562 | 3307 | CD8SP-CLEC5A-3E12A2-(vL- vH)-Myc-BBz-T2A-PAC |
| GR/LHR (Gonadotropin Receptor) | | 1563 | 3308 | SP-CGHb-Gly-Ser-Linker-CGHa- Myc-BBz-T2A-PAC |
| CLLI | 110215-H06 | 1564 | 3309 | CD8SP-CLL1-M26-(vL-vH)-Myc- BBz-T2A-PAC |
| CLLI | 110215-I08 | 1565 | 3310 | CD8SP-CLL1-M32-(vL-vH)-Myc- BBz-T2A-PAC |
| CLLI | | 1566 | 3311 | CD8SP-CLL1-21C9-L2H3-(vL- vH)-Myc-BBz-T2A-PAC |
| CLLI | | 1567 | 3312 | CD8SP-CLL1-6E7L4H1c-(vL-vH)- Myc-BBz-T2A-PAC |
| CLLI | | 1568 | 3313 | CD8SP-CLL1-hu1075-v1-(vL-vH)- Myc-BBz-T2A-PAC |
| CLLI | | 1569 | 3314 | CD8SP-CLL1-hu1075-v2-(vL-vH)- Myc-BBz-T2A-PAC |
| CMVpp65/MHC class I complex | | 1570 | 3315 | CD8SP-CMVpp65-F5-(vL-vH)- Myc-BBz-T2A-PAC |
| CS1 (SLAMF7) | | 1571 | 3316 | CD8SP-CS1-huLuc63-(vL-vH)- |
| | | | | Myc-BBz-T2A-PAC |
| CS1 (SLAMF7) | 052616-U05 | 1572 | 3317 | CD8SP-CS1-HuLuc64-(vL-vH)- Myc-BBz-T2A-PAC |
| CS1 (SLAMF7) | | 1573 | 3318 | CD8SP-CS1-Luc90-(vL-vH)-Myc- BBz-T2A-PAC |
| CS1 (SLAMF7) | | 1574 | 3319 | CD8SP-CS1-PDL241-(vL-vH)- Myc-BBz-T2A-PAC |
| CS1 (SLAMF7) | | 1575 | 3320 | CD8SP-CS1-Hu27A-(vL-vH)-Myc- BBz-T2A-PAC |
| CS1 (SLAMF7) | | 1576 | 3321 | CD8SP-CS1Hu34C3-(vL-vH)- Myc-BBz-T2A-PAC |
| CS1 (SLAMF7) | | 1577 | 3322 | CD8SP-CS1-Hu31-D2-(vL-vH)- Myc-BBz-T2A-PAC |
| CS1(SLAMF7) | | 1578 | 3323 | CD8SP-CS1-Luc34-(vL-vH)-Myc- BBz-T2A-PAC |
| CS1 (SLAMF7) | | 1579 | 3324 | CD8SP-CS1-LucX2-(vL-vH)-Myc- BBz-T2A-PAC |
| CSF2RA | 052616-G03 | 1580 | 3325 | CD8SP-CSF2RA-Ab6-(vL-vH)- Myc-BBz-T2A-PAC |
| CSF2RA | | 1581 | 3326 | CD8SP-CSF2RA-Ab1-(vL-vH)- Myc-BBz-T2A-PAC |
| CXCR4 and CD123 | | 1582 | 3327 | CD8SP-CXCR4-1-vHH-Gly-Ser- Linker-CD123-1-vHH-Myc-BBz- T2A-PAC |
| CXCR4 and CD123 | | 1583 | 3328 | CD8SP-CXCR4-2-VHH-Gly-Ser- Linker-CD123-2-VHH-Myc-BBz- T2A-PAC |
| DLL3 (Delta Like Ligand 3) | 052516-D07 | 1584 | 3329 | CD8SP-DLL3-hSC16-13-(vL-vH)- Myc-BBz-T2A-PAC |
| DLL3 (Delta Like Ligand 3) | 052516-C07 | 1585 | 3330 | CD8SP-DLL3-hSC16-56-(vL-vH)- Myc-BBz-T2A-PAC |
| EBNA3c/MHC class I complex | | 1586 | 3331 | CD8SP-EBNA3c-315-(vL-vH)- Myc-BBz-T2A-PAC |
| EBV-gp350 | | 1587 | 3332 | CD8SP-EBV-gp350-(vL-vH)-Myc- BBz-T2A-PAC |
| EGFR | | 1588 | 3333 | CD8SP-EGFR1-vHH-Myc-BBz- T2A-PAC |
| EGFR & CEA | | 1589 | 3334 | CD8SP-EGFR1-vHH-Gly-Ser- Linker-CEA1-vHH-Myc-BBz-T2A- PAC |
| EGFR & CEA | | 1590 | 3335 | CD8SP-EGFR33-vHH-Gly-Ser- Linker-CEA5-vHH-Myc-BBz-T2A- PAC |
| EGFRvIII | 081115-A02 | 1591 | 3336 | CD8SP-EGFRvIII-139-(vL-vH)- Myc-BBz-T2A-PAC |
| EGFRvIII | 081115-E01 | 1592 | 3337 | CD8SP-EGFRvIII-2173-(vL-vH)- Myc-BBz-T2A-PAC |
| EpCam1 | | 1593 | 3338 | CD8SP-Epcam1-MM1-(vL-vH)- |
| | | | | Myc-BBz-T2A-PAC |
| EpCam1 | 111915-E05 & 112015-C04 | 1594 | 3339 | CD8SP-Epcam1-D5K5-(vL-vH)- Myc-BBz-T2A-PAC |
| FLT3 | | 1595 | 3340 | CD8SP-FLT3-NC7-(vL-vH)-Myc- BBz-T2A-PAC |
| FITC | 052316-B01 | 1596 | 3341 | CD8SP-FITC-(vL-vH)-Myc-BBz- T2A-PAC |
| FITC | | 1597 | 3342 | CD8SP-FITC-4M-53-(vL-vH)- Myc-BBz-T2A-PAC |
| FITC | | 1598 | 3343 | CD8SP-FITC-E2-HL-(vH-vL)- Myc-BBz-T2A-PAC |
| Influenza A HA | | 1599 | 3344 | CD8SP-FLU-MEDI-8852-(vL-vH)- Myc-BBz-T2A-PAC |
| FR1 (Folate Receptor alpha) | 101215-A01 | 1600 | 3345 | CD8SP-FR1-huMov19-(vL-vH)- Myc-BBz-T2A-PAC |
| FSHR (Follicle Stimulating Hormone Receptor) | | 1601 | 3346 | CD8SP-FSHb-Gly-Ser-Linker- CGHa-Myc-BBz-T2A-PAC |
| GAD (Glutamic Acid Decarboxylase)/MHC class I complex | | 1602 | 3347 | CD8SP-GAD-G3H8-(vL-vH)-Myc- BBz-T2A-PAC |
| GD2 | 100515 F01 | 1603 | 3348 | CD8SP-GD2-hu14-18-(vL-vH)- Myc-BBz-T2A-PAC |
| GD2 | 052516-O08 | 1604 | 3349 | CD8SP-GD2-hu3F8-(vL-vH)-Myc- BBz-T2A-PAC |
| GD3 | 052316-A06 | 1605 | 3350 | CD8SP-GD3-KM-641-(vL-vH)- Myc-BBz-T2A-PAC |
| GFRa4 (GDNF Family Receptor Alpha 4) | | 1606 | 3351 | CD8SP-GFRAlpha4-P4-6-(vL-vH)- Myc-BBz-T2A-PAC |
| GFRa4 (GDNF Family Receptor Alpha 4) | | 1607 | 3352 | CD8SP-GFRa4-P4-10-(vL-vH)- Myc-BBz-T2A-PAC |
| GM1 | | 1608 | 3353 | CD8SP-GM1-5B2-(vL-vH)-Myc- BBz-T2A-PAC |
| GM1 | | 1609 | 3354 | CD8SP-GM1-7E5-(vL-vH)-Myc- BBz-T2A-PAC |
| GPRC5D (G-protein coupled receptor family C group 5 member D) | | 1610 | 3355 | CD8SP-GPRC5D-ET150-5-(vL- vH)-Myc-BBz-T2A-PAC |
| GPRC5D | | 1611 | 3356 | CD8SP-GPRC5D-ET150-18-(vL- vH)-Myc-BBz-T2A-PAC |
| GPRC5D | | 1612 | 3357 | CD8SP-GPRC5D-ET150-1-(vL- vH)-Myc-BBz-T2A-PAC |
| GPRC5D | | 1613 | 3358 | CD8SP-GPRC5D-ET150-2-(vL- vH)-Myc-BBz-T2A-PAC |
| gp100/MHC class I complex | | 1614 | 3359 | CD8SP-gp100-(vL-vH)-Myc-BBz- T2A-PAC |
| gp100/MHC class I complex | | 1615 | 3360 | CD8SP-gp100-G2D12-(vL-vH)- Myc-BBz-T2A-PAC |
| GPC3 (Glypican 3) | | 1616 | 3361 | CD8SP-GPC3-4E5-(vL-vH)-Myc- BBz-T2A-PAC |
| gpNMB (Glycoprotein Nmb) | | 1617 | 3362 | CD8SP-gpNMB-115-(vL-vH)- Myc-BBz-T2A-PAC |
| GRP78 | | 1618 | 3363 | CD8SP-GRP78-GC18-(vL-vH)- Myc-BBz-T2A-PAC |
| Her2 | | 1619 | 3364 | CD8SP-Her2-5F7-vHH-Myc-BBz- T2A-PAC |
| Her2 | | 1620 | 3365 | IgHSP-Her2-Affi-Myc-BBz-T2A- PAC |
| Her2 | | 1621 | 3366 | CD8SP-Her2-1-Darpin-Myc-BBz- T2A-PAC |
| Her2 | | 1622 | 3367 | IgHSP-Her2-2-Darpin-Myc-BBz- T2A-PAC |
| Her2 | | 1623 | 3368 | CD8SP-Her2-5F7-vHH-Gly-Ser- Linker-Her2-47D5-vHH-Myc-BBz- T2A-PAC |
| Her2 | | 1624 | 3369 | CD8SP-Her2-Hu4D5-(vL-vH)- Myc-BBz-T2A-PAC |
| Her3 | | 1625 | 3370 | CD8SP-Her3-17B05So-vHH-Myc- BBz-T2A-PAC |
| Her3 | | 1626 | 3371 | CD8SP-Her3-Affi-Myc-BBz-T2A- PAC |
| Her2 and Her3 | 041316-B06 | 1627 | 3372 | CD8SP-Her3-17B05So-vHH-Gly- Ser-Linker-Her2-2D3-vHH-Myc- BBz-T2A-PAC |
| HIV1-gag/MHC class I complex | | 1628 | 3373 | CD8SP-HIV1-E5-(vL-vH)-Myc- BBz-T2A-PAC |
| HIV 1-envelop glycoprotein | | 1629 | 3374 | CD8SP-HIV1-3BNC117-(vL-vH)- Myc-BBz-T2A-PAC |
| HIV1envelop glycoprotein | | 1630 | 3375 | CD8SP-HIV1-PGT-128-(vL-vH)- Myc-BBz-T2A-PAC |
| HIV1-envelop glycoprotein | | 1631 | 3376 | CD8SP-HIV1-VR-C01-(vL-vH)- Myc-BBz-T2A-PAC |
| HIV1-envelop glycoprotein | | 1632 | 3377 | CD8SP-HIV1-X5-(vL-vH)-Myc- BBz-T2A-PAC |
| HLA-A2 | | 1633 | 3378 | CD8SP-HLA-A2-3PB2-(vL-vH)- Myc-BBz-T2A-PAC |
| HMW-MAA | 060116-B02 | 1634 | 3379 | CD8SP-HMW-MAA-hIND-(vL- vH)-Myc-BBz-T2A-PAC |
| HPV 16-E7/MHC class I complex | | 1635 | 3380 | CD8SP-HPV16-7-8-(vL-vH)-Myc- BBz-T2A-PAC |
| HPV 16-E7/MHC class I complex | | 1636 | 3381 | CD8SP-HPV16-2-(vL-vH)-Myc- BBz-T2A-PAC |
| HTLV1-TAX/MHC class I complex | | 1637 | 3382 | CD8SP-HTLV-TAX-T3F2-(vL- vH)-Myc-BBz-T2A-PAC |
| HTLV1-TAX/MHC class I complex | | 1638 | 3383 | CD8SP-HTLV-TAX-T3E3-(vL- vH)-Myc-BBz-T2A-PAC |
| IL11Ra | 052316-D02 | 1639 | 3384 | CD8SP-IL11Ra-8E2-Ts107-(vL- vH)-Myc-BBz-T2A-PAC |
| IL6Ra | | 1640 | 3385 | IgHSP-IL6R-304-vHH-Myc-BBz- T2A-PAC |
| IL13Ra2 | 052616-B03 | 1641 | 3386 | CD8SP-IL13Ra2-hu107-(vL-vH)- Myc-BBz-T2A-PAC |
| IL13Ra2 | | 1642 | 3387 | CD8SP-IL13Ra2-Hu108-(vL-vH)- Myc-BBz-T2A-PAC |
| KSHV-K8.1 | 042315-N01 | 1643 | 3388 | CD8SP-KSHV-4C3-(vL-vH)-Myc- BBz-T2A-PAC |
| LAMP1 (Lysosomal-associated membrane protein 1) | | 1644 | 3389 | CD8SP-LAMP1-humab1-2-(vL- vH)-Myc-BBz-T2A-PAC |
| LAMP1 (Lysosomal-associated membrane protein 1) | | 1645 | 3390 | CD8SP-LAMP1-Mb4-(vL-vH)- Myc-BBz-T2A-PAC |
| LewisY | | 1646 | 3391 | CD8SP-LewisY-huS193-(vL-vH)- Myc-BBz-T2A-PAC |
| L1CAM | 052516-Q07 | 1647 | 3392 | CD8SP-L1CAM-9-3-HU3-(vL- vH)-Myc-BBz-T2A-PAC |
| LHR | | 1648 | 3393 | SP-LHb-Gly-Ser-Linker-CGHa- Myc-BBz-T2A-PAC |
| Lym1 | | 1649 | 3394 | CD8SP-Lym1-(vL-vH)-Myc-BBz- T2A-PAC |
| Lym2 | | 1650 | 3395 | CD8SP-Lym2-(vL-vH)-Myc-BBz- T2A-PAC |
| CD79b | | 1651 | 3396 | CD8SP-huMA79bv28-(vL-vH)- Myc-BBz-T2A-PAC |
| MART1/MHC class I complex | | 1652 | 3397 | CD8SP-MART1-CAG10-(vL-vH)- Myc-BBz-T2A-PAC |
| MART1/MHC class I complex | | 1653 | 3398 | CD8SP-MART1-CLA12-(vL-vH)- Myc-BBz-T2A-PAC |
| Mesothelin | 052516-P07 | 1654 | 3399 | CD8SP-Mesothelin-m912-(vL-vH)- Myc-BBz-T2A-PAC |
| cMet | | 1655 | 3400 | CD8SP-cMet-171-vHH-Myc-BBz- T2A-PAC |
| cMet and Her3 | 041316-F06 | 1656 | 3401 | CD8SP-cMET-171-vHH-Gly-Ser- Linker-Her3-21F06-vHH-Myc- BBz-T2A-PAC |
| MPL (Thrombopoietin receptor) | 022415-Q05 | 1657 | 3402 | CD8SP-MPL-175-(vL-vH)-Myc- |
| | | | | BBz-T2A-PAC |
| MPL (Thrombopoietin receptor) | | 1658 | 3403 | CD8SP-MPL-161-(vL-vH)-Myc- BBz-T2A-PAC |
| MPL (Thrombopoietin receptor) | | 1659 | 3404 | CD8SP-MPL-161-HL-(vH-vL)- Myc-BBz-T2A-PAC |
| MPL (Thrombopoietin receptor) | 041316-A03 | 1660 | 3405 | CD8SP-2-MPL-111-(vL-vH)-Myc- BBz-T2A-PAC |
| MPL (Thrombopoietin receptor) | | 1661 | 3406 | CD8SP-MPL-178-(vL-vH)-Myc- BBz-T2A-PAC |
| MPL (Thrombopoietin receptor) | | 1662 | 3407 | CD8SP-MPL-AB317-(vL-vH)- Myc-BBz-T2A-PAC |
| MPL (Thrombopoietin receptor) | | 1663 | 3408 | CD8SP-MPL-12E10-(vL-vH)-Myc- BBz-T2A-PAC |
| MPL (Thrombopoietin receptor) | | 1664 | 3409 | CD8SP-MPL-huVB22Bw5-(vL- vH)-Myc-BBz-T2A-PAC |
| Muc1/MHC class I complex | 052316 A02 | 1665 | 3410 | CD8SP-Muc1-D6-M3B8-(vL-vH)- Myc-BBz-T2A-PAC |
| Muc1/MHC class I complex | | 1666 | 3411 | CD8SP-MUC1-D6-M3A1-(vL- vH)-Myc-BBz-T2A-PAC |
| Muc16 | 111915-F04 | 1667 | 3412 | CD8SP-Muc16-4H11-(vL-vH)- Myc-BBz-T2A-PAC |
| EGFR | | 1668 | 3413 | CD8SP-Nimotuzumab-(vL-vH)- Myc-BBz-T2A-PAC |
| NKG2D Ligand | | 1669 | 3414 | CD8SP-NKG2D-(GGGGS- GGGGD)-Myc-BBz-T2A-PAC |
| NKG2D | | 1670 | 3415 | CD8SP-NKG2D-MS-(vL-vH)- Myc-BBz-T2A-PAC |
| NY-BR1 | | 1671 | 3416 | CD8SP-NYBR1-(vL-vH)-Myc- BBz-T2A-PAC |
| NY-ESO/MHC class I complex | | 1672 | 3417 | CD8SP-NYESO-T1-(vL-vH)-Myc- BBz-T2A-PAC |
| NY-ESO/MHC class I complex | | 1673 | 3418 | CD8SP-NYESO-T2-(vL-vH)-Myc- BBz-T2A-PAC |
| PD1 ligand (e.g., PDL1) | | 1674 | 3419 | CD8SP-PD1-ECD-Myc-BBz-T2A- PAC |
| PDL1 | 100516-J03 | 1675 | 3420 | CD8SP-PDL1-Atezoli-(vL-vH)- Myc-BBz-T2A-PAC |
| PDL1 | 100516-K03 | 1676 | 3421 | CD8SP-PDL1-SP142-(vL-vH)- Myc-BBz-T2A-PAC |
| PDL1 | | 1677 | 3422 | CD8SP-PDL1-10A5-(vL-vH)-Myc- BBz-T2A-PAC |
| PSCA (Prostate stem cell antigen) | | 1678 | 3423 | CD8SP-PSCA-Ha14-121-(vL-vH)- Myc-BBz-T2A-PAC |
| PSCA (Prostate stem cell antigen) | | 1679 | 3424 | CD8SP-PSCA-Ha14-117-(vL-vH)- Myc-BBz-T2A-PAC |
| PR1/MHC class I complex | 052516-K07 | 1680 | 3425 | CD8SP-PR1-(vL-vH)-Myc-BBz- T2A-PAC |
| PSMA (Prostate Specific Membrane Antigen) | 052516-N07 | 1681 | 3426 | CD8SP-PSMA-006-(vL-vH)-Myc- BBz-T2A-PAC |
| PSMA (Prostate Specific Membrane Antigen) | 111815-E06 | 1682 | 3427 | CD8SP-PSMA-J591-(vL-vH)-Myc- BBz-T2A-PAC |
| PTK7 (Tyrosine-protein kinase-like 7) | 052516-F07 | 1683 | 3428 | CD8SP-PTK7-hSC6-23-(vL-vH)- Myc-BBz-T2A-PAC |
| PTK7 (Tyrosine-protein kinase-like 7) | 052516-E07 | 1684 | 3429 | CD8SP-PTK76-10-2-(vL-vH)-Myc- BBz-T2A-PAC |
| ROR1 | 082815-M05 | 1685 | 3430 | CD8SP-ROR1-4A5-(vL-vH)-Myc- BBz-T2A-PAC |
| ROR1 | 082815-N06 | 1686 | 3431 | CD8SP-ROR1-4C10-(vL-vH)-Myc- BBz-T2A-PAC |
| Mesothelin | | 1687 | 3432 | CD8SP-SD1-vHH-Gly-Ser-Linker- SD2-vHH-Myc-BBz-T2A-PAC |
| SLea | | 1688 | 3433 | CD8SP-SLea-7E3-(vL-vH)-Myc- BBz-T2A-PAC |
| SLea | | 1689 | 3434 | CD8SP-SLea-5B1-(vL-vH)-Myc- BBz-T2A-PAC |
| SSEA4 (stage-specific embryonic antigen 4) | | 1690 | 3435 | CD8SP-SSEA4-(vL-vH)-Myc-BBz- T2A-PAC |
| TCRB1 (TCR beta 1 constant chain) | | 1691 | 3436 | CD8SP-TCRB1-CP01-E09-(vL- vH)-Myc-BBz-T2A-PAC |
| TCRB1 (TCR beta 1 constant chain) | 050516-A07 | 1692 | 3437 | CD8SP-TCRB1-Jovi1-(vL-vH)- Myc-BBz-T2A-PAC |
| TCRB2 (TCR beta 2 constant chain) | 050516-C07 | 1693 | 3438 | CD8SP-TCRB2-CP01-D05-(vL- vH)-Myc-BBz-T2A-PAC |
| TCRB2 (TCR beta 2 constant chain) | 050516-B05 | 1694 | 3439 | CD8SP-TCRB2-CP01-E05-(vL- vH)-Myc-BBz-T2A-PAC |
| TCRgd (TCR gamma/delta) | | 1695 | 3440 | CD8SP-TCRgd-G5-4-(vL-vH)- Myc-BBz-T2A-PAC |
| hTERT/MHC class I complex | 051816-H02 | 1696 | 3441 | CD8SP-TERT-4A9-T540-(vL-vH)- Myc-BBz-T2A-PAC |
| hTERT/MHC class I complex | | 1697 | 3442 | CD8SP-TERT-3G3-T865-(vL-vH)- Myc-BBz-T2A-PAC |
| Tissue Factor-1 | | 1698 | 3443 | CD8SP-TGFBR2-Ab1-(vL-vH)- Myc-BBz-T2A-PAC |
| TGFBR2 | | 1699 | 3444 | CD8SP-TF1-98-(vL-vH)-Myc- BBz-T2A-PAC |
| TIM1/HAVCR | | 1700 | 3445 | CD8SP-TIM1-HVCR1-270-2-(vL- vH)-Myc-BBz-T2A-PAC |
| TIM1/HAVCR | | 1701 | 3446 | CD8SP-TIM1-HVCR1-ARD5-(vL- vH)-Myc-BBz-T2A-PAC |
| TnAg | 052616-F06 | 1702 | 3447 | CD8SP-TnAg-(vL-vH)-Myc-BBz- |
| | | | | T2A-PAC |
| Tn-Muc 1 | | 1703 | 3448 | CD8SP-TnMuc1-hu5E5-RHA8- RKA-2-(vL-vH)-Myc-BBz-T2A- PAC |
| MPL (Thrombopoietin receptor) | | 1704 | 3449 | CD8SP-hTPO-Myc-BBz-T2A-PAC |
| TROP2 (Trophoblast cell-surface antigen-2) | | 1705 | 3450 | CD8SP-TROP2-ARA47-HV3KV3- (vL-vH)-Myc-BBz-T2A-PAC |
| TROP2 (Trophoblast cell-surface antigen-2) | | 1706 | 3451 | CD8SP-TROP2-h7E6-SVG-(vL- vH)-Myc-BBz-T2A-PAC |
| TSHR (Thyrotropin receptor) | | 1707 | 3452 | SP-TSHb-Gly-Ser-Linker-CGHa- Myc-BBz-T2A-PAC |
| TSHR (Thyrotropin receptor) | | 1708 | 3453 | CD8SP-TSHR-K1-70-(vL-vH)- Myc-BBz-T2A-PAC |
| TSHR (Thyrotropin receptor) | 052616-E05 | 1709 | 3454 | CD8SP-TSHR-KB1-(vL-vH)-Myc- BBz-T2A-PAC |
| TSHR (Thyrotropin receptor) | | 1710 | 3455 | CD8SP-TSHR-5C9-(vL-vH)-Myc- BBz-T2A-PAC |
| TSLPR (thymic stromal lymphopoietin receptor) | 052516-L07 | 1711 | 3456 | CD8SP-TSLPR-(vL-vH)-Myc- BBz-T2A-PAC |
| Tyrosinase/MHC class I complex | 052516-D06 | 1712 | 3457 | CD8SP-Tyros-B2-(vL-vH)-Myc- BBz-T2A-PAC |
| Tyrosinase/MHC class I complex | | 1713 | 3458 | CD8SP-Tyros-MC1-(vL-vH)-Myc- BBz-T2A-PAC |
| Tyrosinase/MHC class I complex | | 1714 | 3459 | CD8SP-Tyros-TA2-(vL-vH)-Myc- BBz-T2A-PAC |
| VEGFR3 | | 1715 | 3460 | CD8SP-VEGFR3-Ab1-(vL-vH)- Myc-BBz-T2A-PAC |
| WT1/MHC class I complex | 101415-P01 | 1716 | 3461 | CD8SP-WT1-Ab1-(vL-vH)-Myc- BBz-T2A-PAC |
| WT1/MHC class I complex | 052516-M07 | 1717 | 3462 | CD8SP-WT1-Ab5-(vL-vH)-Myc- BBz-T2A-PAC |
| WT1/MHC class I complex | 052516-R04 | 1718 | 3463 | CD8SP-MYC3-WT1-Ab13-(vL- vH)-Myc-BBz-T2A-PAC |
| WT1/MHC class I complex | 052616-S02 | 1719 | 3464 | CD8SP-MYC3-WT1-Ab15-(vL- vH)-Myc-BBz-T2A-PAC |
| CDH19 | | 1720 | 3465 | CD8SP-CDH19-4B10-(vL-vH)- Myc-BBz-T2A-PAC |
| Folate Receptor beta | | 1721 | 3466 | CD8SP-FRbeta-m923-(vL-vH)- Myc-BBz-T2A-PAC |
| LHR (Luteinizing hormone Receptor) | | 1722 | 3467 | CD8SP-LHR-8B7-(vL-vH)-Myc- BBz-T2A-PAC |
| LHR (Luteinizing hormone Receptor) | | 1723 | 3468 | CD8SP-LHR-5F4-21-(vL-vH)- Myc-BBz-T2A-PAC |
| B7H4 | | 1724 | 3469 | CD8SP-B7H4-hu22Cl0-(vL-vH)- Myc-BBz-T2A-PAC |
| B7H4 | | 1725 | 3470 | CD8SP-B7H4-hu1D11-(vL-vH)- Myc-BBz-T2A-PAC |
| IgE | | 1726 | 3471 | CD8SP-IgE-omalizumab-(vL-vH)- Myc-BBz-T2A-PAC |
| CD23 | | 1727 | 3472 | CD8SP-CD23-p5E8-(vL-vH)-Myc- BBz-T2A-PAC |
| GCC (Guanylyl cyclase C) | | 1728 | 3473 | CD8SP-GCC-5F9-(vL-vH)-Myc- BBz-T2A-PAC |
| GCC (Guanylyl cyclase C) | | 1729 | 3474 | CD8SP-GCC-Ab229-(vL-vH)- Myc-BBz-T2A-PAC |
| CD200R | | 1730 | 3475 | CD8SP-CD200R-huDx182-(vL- vH)-Myc-BBz-T2A-PAC |

**Table 22: Exemplary CAR constructs targeting different antigens**

| **Target** | **CLONE ID** | **SEQ ID (DNA)** | **SEQ ID (PRT)** | **CONSTRUCT NAME** |
|---|---|---|---|---|
| MPL | 021715-Z07 | 1731 | 3476 | CD8SP-161-(vL-vH)-Myc-CD28z-T2A-PAC |
| MPL | 090814-C03 | 1732 | 3477 | CD8SP-161-(vL-vH)-Myc-CD8TM-BBz-T2A-EGFP |
| MPL | 031615-Q04 | 1733 | 3478 | CD8SP-175-(vL-vH)-Myc-CD28z-T2A-PAC |
| MPL | 031615-R04 | 1734 | 3479 | CD8SP-178-(vL-vH)-Myc-CD28z-T2A-PAC |
| MPL | 031615-T04 | 1735 | 3480 | CD8SP-AB317-(vL-vH)-Myc-CD28z-T2A-PAC |
| MPL | 031615-S03 | 1736 | 3481 | CD8SP-12E10-(vL-vH)-Myc-CD28z-T2A-PAC |
| MPL | 032415-M03 | 1737 | 3482 | CD8SP-huVB22Bw5-(vL-vH)-Myc-CD28z-T2A-PAC |
| MPL | 031915-U04 | 1738 | 3483 | hTPO-(1-187)-Myc-CD28z-T2A-PAC |
| MPL | 031915-V03 | 1739 | 3484 | mTPO-(1-187)-Myc-CD28z-T2A-PAC |
| MPL | 021216-D01 | 1740 | 3485 | CD8SP-161-(vL-vH)-BBz-P2A-IgHSP-IL6R-304- vHH-Alb8-vHH-Flag-T2A-PAC |
| MPL | 112916-U06 | 1741 | 3486 | CD8SP-161-(vL-vH)-Myc-z-P2A-FKBP-K13-FLAG- T2A-PAC |
| MPL | 112916-V01 | 1742 | 3487 | CD8SP-161-(vL-vH)-Myc-z-P2A-FLAG-HTLV2- TAX-RS-T2A-PAC |
| MPL | 112916-W06 | 1743 | 3488 | CD8SP-161-(vL-vH)-Myc-z-P2A-HTLV2-TAX-RS- T2A-PAC |
| MPL | 112614-E05 | 1744 | 3489 | CD8SP-161-(vL-vH)-Myc-BBz-P2A-FKBP-K13-Flag- T2A-EGFP |
| MPL | 011315-F02 | 1745 | 3490 | CD8SP-161(vL-vH)-BBz-P2A-IgHSP-IL6-19A(vL- vH)-Flag-T2A-EGFP |
| MPL | 012015-I29 | 1746 | 3491 | CD8SP-161(vL-vH)-BBz-P2A-IgHSP-IL6R-M83(vL- vH)-Flag-T2A-EGFP |
| MPL | 022415-T02 | 1747 | 3492 | CD8SP-161-(vL-vH)-Myc-CD28z-P2A-K13-Flag- T2A-EGFP |
| MPL | 012115-L04 | 1748 | 3493 | CD8SP-161-(vL-vH)-BBz-P2A-IgHSP-Fx06-Flag- T2A-EGFP |
| MPL | 070315-H03 | 1749 | 3494 | CD8SP-161-(vL-vH)-Myc-BBZ-P2A-MC159-Flag- T2A-Pac |
| CD19 | 112614-C06 | 1750 | 3495 | CD8SP-FMC63(vL-vH)-Myc-z-P2A-MC159-Flag- T2A-PAC |
| CD19 | 111014-Z13 | 1751 | 3496 | CD8SP-FMC63(vL-vH)-Myc-BBz-P2A-MC159-Flag- T2A-PAC |
| CD19 | 010100-N01 | 1752 | 3497 | CD8SP-FMC63(vL-vH)-Myc-BBz-P2A-Flag-HTLV2- TAX-RS-T2A-PAC |
| CD19 | 100215-B07 | 1753 | 3498 | CD8SP-FMC63(vL-vH)-Myc-BBz-P2A-cFLIP-p22- Flag-T2A-PAC |
| CD19 | 092215-A06 | 1754 | 3499 | CD8SP-FMC63(vL-vH)-Myc-BBz-P2A-cFLIP-L- Flag-T2A-PAC |
| CD19 | 112614-B06 | 1755 | 3500 | CD8SP-FMC63(vL-vH)-Myc-z-P2A-FKBP-K13- FLAG-T2A-EGFP |
| CD19 | 120314-J03 | 1756 | 3501 | CD8SP-FMC63(vL-vH)-Myc-z-P2A-FKBPx2-K13- FLAG-T2A-EGFP |
| CD19 | 120314-N07 | 1757 | 3502 | CD8SP-FMC63(vL-vH)-Myc-z-P2A-Myr-FKBPx2- K13-FLAG-T2A-EGFP |
| CD19 | 012315-001 | 1758 | 3503 | CD8SP-FMC63(vL-vH)-Myc-BBz-P2A-FKBPx2- HTLV2-Tax-RS-T2A-EGFP |
| CD19 | 033115-P06 | 1759 | 3504 | CD8SP-FMC63-(vL-vH)-Myc-CD28z-P2A-K13-Flag- T2A-PAC |
| CD19 | 032415-Q03 | 1760 | 3505 | CD8SP-FMC63-(vL-vH)-Myc-CD28z-P2A-MC159- Flag-T2A-PAC |
| CD19 | 022415-S03 | 1761 | 3506 | CD8SP-FMC63-(vL-vH)-Myc-CD28z-P2A-K13-Flag- T2A-EGFP |
| CD19 | 011315-H06 | 1762 | 3507 | CD8SP-FMC63(vL-vH)-BBz-P2A-IgHSP-IL6- 19A(vL-vH)-Flag-T2A-PAC |
| CD19 | 011315-K04 | 1763 | 3508 | CD8SP-FMC63(vL-vH)-BBz-P2A-IgHSP-IL6R- M83(vL-vH)-Flag-T2A-PAC |
| CD19 | 012115-N04 | 1764 | 3509 | CD8SP-FMC63-(vL-vH)-BBz-P2A-IgHSP-Fx06-Flag- T2A-PAC |
| CD19 | 091715-C01 | 1765 | 3510 | CD8SP-FMC63-(vL-vH)-Myc-z-P2A-HTLV2-TAX2- FLAG-T2A-Pac |
| CD19 | 091715-D05 | 1766 | 3511 | CD8SP-FMC63-(vL-vH)-Myc-z-P2A-HTLV2-TAX- RS-T2A-Pac |
| CD19 | 091715-L04 | 1767 | 3512 | CD8SP-FMC63-(vL-vH)-Myc-z-P2A-FLAG-HTLV2- TAX-RS-T2A-Pac |
| CD19 | 091715-004 | 1768 | 3513 | CD8SP-FMC63-(vL-vH)-MYC-z-P2A-HTLV2-TAX- T2A-Pac |
| CD19 | 033115-T01 | 1769 | 3514 | CD8SP-FMC63-(vL-vH)-Myc-CD28z-T2A-PAC |
| CD19 | 112316-S06 | 1770 | 3515 | CD8SP-FMC63(vL-vH)-Myc-z-P2A-Myr-FKBPx2- K13-FLAG-T2A-PAC |
| GRP78 | 031615-005 | 1771 | 3516 | CD8SP-GRP78-GD4-(vL-vH)-MYC-CD28z-T2A-Pac |
| CD19 | 093015-D10 | 1772 | 3517 | CD8SP-hCD19-Bu12-(vL-vH)-MYC-CD28z-T2A-Pac |
| Lym1 | 031615-M03 | 1773 | 3518 | CD8SP-Lym1-(vL-vH)-Myc-CD28z-T2A-Pac |
| Lym2 | 031615-N03 | 1774 | 3519 | CD8SP-Lym2(vL-vH)-Myc-CD28z-T2A-Pac |
| GRP78 | 031615-P03 | 1775 | 3520 | CD8SP-GRP78-GC18-(vL-vH)-Myc-CD28z-T2A-Pac |
| FLT3 | 032415-L05 | 1776 | 3521 | CD8SP-FLT3-NC7-(vL-vH)-Myc-CD28z-T2A-Pac |
| CD79b | 031915-D05 | 1777 | 3522 | CD8SP-huMA79bv28-(vL-vH)-Myc-CD28z-T2A-Pac |
| CS1 | 031915-F03 | 1778 | 3523 | CD8SP-Luc90-(vL-vH)-MYC-CD28z-T2A-PAC |
| CD4 | 101415-004 | 1779 | 3524 | SP-CD4-8-03F1-(vHH)-MYC-BBZ-T2A-Pac |
| CXCR4 | 101415-U01 | 1780 | 3525 | CD8SP-CXCR4-1-(vHH)-MYC-BBZ-T2A-Pac |
| KSHV-K8.1 | 031615-W05 | 3534 | 3540 | CD8SP-4C3-(vL-vH)-Myc-CD28z-T2A-Pac |
| CD19 | 070814-D06 | 3535 | 3541 | CD8SP-FMC63-(vL-vH)-Myc-BBz-T2A-EGFP |
| MPL | 031615-B06 | 3536 | 3542 | CD8SP-MPL-VB22Bw4-(vL-vH)-Myc-CD28z-T2A- PAC |
| KSHV-K8.1 | 100814-K06 | 3537 | 3543 | CD8SP-4C3-(vL-vH)-Myc-BBz-T2A-EGFP |
| CD19 | 111214-D05 | 3538 | 3544 | CD8SP-FMC63-(vL-vH)-Myc-z-T2A-PAC |
| CD19 | 012315-M03 | 3539 | 3545 | CD8SP-FMC63(vL-vH)-Myc-BBz-P2A-FKBPx2- FLAG-TAX2U2RS -T2A-eGFP |

**Table 23. HLA-A2 restricted peptides used for generaton of CAR**

| **Protein** | **Fragment Name** | **Amino Acid Seq** |
|---|---|---|
| gp100 | G9-209 | (IMDQVPFSV) |
| gp100 | G9-280 | (YLEPGPVTV) |
| gp100 | G9-154 | (KTWGQYWQV) |
| MUC1-A7 (130-138) | A7 | (NLTISDVSV) |
| MUC1-D6 (13-21) | D6 | (LLLTVLTVV) |
| TAX (11-19) | | (LLFGYPVYV) |
| hTERT(540-548) | T540 | (ILAKFLHWL) |
| hTERT (865-873) | T865 | (RLVDDFLLV) |
| HIV1 gag (77-85) | SL9 | (SLYNTVATL) |
| CMV-pp65(495-503) | | (NLVPMVATV) |
| MART (26-35) | | (EAAGIGILTV) |
| EBNA-3A (596-604) | | (SVRDRLARL) |
| EBNA-3c | | (LLDFVRFMGV) |
| WT1 | | (RMFPNAPYL) |
| PR1 | | (VLQELNVTV) |
| AFP | AFP-158 | (FMNKFIYEI) |
| HPV16-E7 | E7 (11-19) | (YMLDLQPET) |
| Tyrosinase | Tyr (369-377) | (YM**D**GTMSQV) |

### EXAMPLES

The following examples are not intended to limit the scope of the claims to the invention, but are rather intended to be exemplary of certain embodiments. Any variations in the exemplified methods which occur to the skilled artisan are intended to fall within the scope of the present invention.

### MPL (TPO Receptor) as target for CAR-T cell therapy

Thrombopoietin and its cognate receptor, c-Mpl, are the primary molecular regulators of megakaryocytopoiesis(Zhan et al., Monoclonal antibodies in immunodiagnosis and immunotherapy 32:149-61, 2013). Binding of TPO to cell surface c-Mpl activates a cascade of signaling events that result in the proliferation and differentiation of megakaryocytic progenitor cells and ultimately platelet production. TPO and c-Mpl also regulate hematopoietic stem cell self-renewal. *Consistent with these biological functions of TPO in the megakaryocytic lineage, c-Mpl expression is mostly restricted to non-lymphoid hematopoietic tissues.* c-Mpl mRNA and cell surface protein have been identified in human CD34+ cells, megakaryocytes, platelets, primary leukemia samples of myeloid origin, and megakaryocytic and erythroid leukemia cell lines. c-Mpl mRNA has not been detected in most normal non-hematopoietic tissues or in lymphoid cell lines.

We examined the public gene expression databases for MPL expression. As shown in **Fig. 2A****-****Fig. 2D****,** we confirmed extremely restrictive expression of MPL in normal tissues, with only one organ showing low level expression. In contrast, MPL was significantly expressed in most patient samples of Acute myeloid leukemia (AML), myelodysplastic syndrome (MDS) and chronic myeloid leukemia (CML). These results suggest that MPL is an excellent therapeutic target for patients with myeloid malignancies, such as AML, MDS and CML, using chimeric antigen receptor modified T cells.

### Cell lines and cells

The cell lines used in this invention and their growth media are shown in the **Table 24.** Cells were cultured at 37°C, in a 5% CO2 humidified incubator. The cell lines were obtained from ATCC, NIH AIDS reagent program or were available in our laboratory or obtained from other laboratories.

**Table 24: Cell lines and growth media**

| **Cell lines** | **Media** | | **Cell lines** | **Media** |
|---|---|---|---|---|
| BC-1 | RPMI, 20% FCS | | THP-1 | RPMI, 10% FCS |
| BC-3 | RPMI, 20% FCS | | U87MG | DMEM, 10% FCS |
| BCBL-1 | RPMI, 20% FCS | | NCI-H540 | DMEM, 10% FCS |
| JSC-1 | RPMI, 20% FCS | | LoVo | DMEM, 10% FCS |
| UMPEL-1 | RPMI, 20% FCS | | SKOV-3 | DMEM, 10% FCS |
| MM1S | RPMI, 10% FCS | | NCI-H1993 | DMEM, 10% FCS |
| U266 | RPMI, 10% FCS | | Kasumi-1 | RPMI, 20% FCS |
| L363 | RPMI, 10% FCS | | Jeko-1 | RPMI, 20% FCS |
| K562 | RPMI, 10% FCS | | PC-3 | DMEM, 10% FCS |
| BV173 | RPMI, 10% FCS | | HeLa | DMEM, 10% FCS |
| Nalm6 | RPMI, 10% FCS | | NCI-H2452 | DMEM, 10% FCS |
| HL60 | RPMI, 10% FCS | | LnCap | DMEM, 10% FCS |
| U937 | RPMI, 10% FCS | | SNU-5 | RPMI, 20% FCS |
| RS:411 | RPMI, 20% FCS | | OVCAR-3 | DMEM, 10% FCS |
| MV:411 | RPMI, 10% FCS | | MEL-624 | DMEM, 10% FCS |
| Raji | RPMI, 10% FCS | | LS174-T | DMEM, 10% FCS |
| HEL-92.1.7 | RPMI, 10% FCS | | MEL-526 | DMEM, 10% FCS |
| Meg-01 | RPMI, 10% FCS | | MDA-MB231 | DMEM, 10% FCS |
| Jurkat | RPMI, 10% FCS | | L1236 | RPMI, 20% FCS |
| MM1 | DMEM, 10% FCS | | L428 | RPMI, 20% FCS |
| Daudi | RPMI, 10% FCS | | Molt-16 | RPMI, 20% FCS |
| REC-1 | RPMI, 10% FCS | | RPMI8402 | RPMI, 20% FCS |
| U2932 | RPMI, 10% FCS | | KN5-5F2 | RPMI, 20% FCS |
| H929 | RPMI, 10% FCS beta Mercaptoethanol (BME) | | CCRF-CEM (ATCC) | RPMI, 10% FCS |
| KMS28 | RPMI, 10% FCS | | MG-63 | DMEM, 10% FCS |
| EJM | RPMI, 10% FCS | | Karpass-299 | RPMI, 20% FCS |
| MRC-5 | DMEM, 10% FCS | | MCF7 | DMEM, 10% FCS |
| CMK | RPMI, 20% FCS | | SUDHL-1 | RPMI, 10% FCS |
| TF-1 | RPMI, 10% FCS + GMCSF | | AA-2 | RPMI, 10% FCS |
| ML-2 | RPMI, 20% FCS | | HL2/3 | DMEM, 10% FCS |
| A20 | RPMI, 10% FCS + BME | | TF228.1.16 | DMEM, 10% FCS |
| KMS28BM | RPMI, 10% FCS | | MT-4 | RPMI, 10% FCS |
| KG-1 | RPMI, 20% FCS | | Sup-T1 | RPMI, 10% FCS |
| CEM | RPMI, 10% FCS | | HuT-78 | RPMI, 10% FCS |
| U937 | RPMI, 10% FCS | | TT | DMEM, 10% FCS |
| LAMA5 | RPMI, 10% FCS | | DMS79 | RPMI, 10% FCS |
| A549 | DMEM, 10% FCS | | LAN-5 | DMEM, 10% FCS |
| HT29 | DMEM, 10% FCS | | PEER1 | RPMI, 10% FCS |
| Molm-13 | RPMI, 20% FCS | | SK-MEL-37 | DMEM, 10% FCS |
| A431 | DMEM, 10% FCS | | Jurkat-NFAT-GFP | RPMI, 10% FCS |
| P19 | DMEM, 10% FCS | | F9 | DMEM, 10% FCS |

Jurkat cell line (clone E6-1) engineered with a NFAT-dependent EGFP (or GFP) reporter gene was a gift from Dr. Arthur Weiss at University of California San Francisco and have been described to study CAR-signaling ((Wu, CY et al., Science 350:293-302,2015). Jurkat cells were maintained in RPMI-1640 medium supplemented with 10% FBS, penicillin and streptomycin.

### Generation of lentiviral vectors encoding chimeric antigen receptors against MPL

The pLENTI-Blast vector was derived from pLenti6v5gw_lacz vector (Invitrogen; ThermoFisher Scientific) by removal of the LacZ gene. pLenti-MP2 was a gift from Pantelis Tsoulfas (Addgene plasmid # 36097) and was used to generate pLenti-EF1a or pLenti-EF1α [SEQ ID NO:905] lentiviral vector by replacement of the CMV promoter with human EF1α promoter using standard molecular biology techniques. pLenti-EF1a-DWPRE [SEQ ID NO:906] was derived from the pLENTI-EFIα vector by deletion of WPRE sequence. The psPAX2 vector was a gift from Didier Trono (Addgene plasmid # 12260). The pLP/VSVG envelope plasmid and 293FT cells were obtained from Invitrogen (ThermoFisher Scientific). The retroviral transfer vector MSCVneo, MSCVhygro, and MSCVpac and the packaging vector pKAT were obtained from Dr. Robert Illaria's laboratory. phRGTK Renilla Luciferase plasmid was from Promega.

Several monoclonal antibodies targeting human MPL have been described (US20120269814A1, US 6,342,220 B1, EP1616881A1 and WO 02568109). The nucleotide sequences of the 1.6.1 monoclonal antibody described in US20120269814A1 was used for the synthesis of its corresponding scFV fragment consisting from 5' to 3' ends of a nucleotide sequences encoding a signal peptide derived from human CD8 molecule [SEQ ID NO: 1-4], the vL fragment, a (Gly4Ser)x3 linker and the vH fragment. The sequence of the scFV fragment was codon optimized using GeneArtTM software (Thermo Fisher Scientific) and gene-fragments encoding the optimized sequences synthesized by GeneArtTM or Integrated DNA Technologies (IDT). The gene fragment was used as templates in PCR reaction using custom primers containing NheI and MluI restriction enzyme sites and then cloned in a modified pLenti-EF1a vector containing the hinge, transmembrane and cytosolic domains of the CAR cassette by standard molecular biology techniques. The final CAR construct named CD8SP-161-(vL-vH)-Myc-CD8TM-BBz-T2A-EGFP(090814-C03)[SEQ ID NO: 1732] consisted of human CD8 signal peptide (hCD8SP), fused in frame to the scFv (vL-vH) fragment derived from the monoclonal antibody 1.6.1 against human MPL [SEQ ID NO:730], a Myc epitope tag [SEQ ID NO:553], the hinge and transmembrane domain of human CD8 [SEQ ID NO:842] , the cytosolic domain of human 41BB (CD137) receptor [SEQ ID NO:845], the cytosolic domain of human CD3z, [SEQ ID NO:846], a T2A ribosomal skip sequence [SEQ ID NO:832] and a cDNA encoding enhanced Green Fluorescent Protein (EGFP). The CAR construct CD8SP-MPL-161-(vL-vH)-Myc-BBz-T2A-PAC(021015-R07)[SEQ ID NO:1658] is nearly identical to the above except EGFP cDNA is replaced by cDNA encoding a variant of puromycin resistance gene (SEQ ID NO: 850). In the CAR construct CD8SP-161-(vL-vH)-Myc-CD28z-T2A-PAC(021715-Z07)[SEQ ID NO:1731], the hinge and transmembrane domain of human CD8 and the cytosolic domain of human 41BB (CD137) receptor are replaced by the human CD28-hinge, -transmembrane and -cytosolic domains (SEQ ID NO: 848). Essentially a similar approach was used to generate several additional CARs against multiple antigens in which the 161 scFv fragment was replaced by the scFv fragments of antibodies targeting different antigens. The SEQ ID NOs of the nucleotide and protein sequences of the different scFv fragments and the name of their target antigens are provided in **Table 11.** Additionally, two constructs targeting MPL were made in which the scFv region was replaced by the extracellular domains of human and mouse thrombopoietin (TPO) (SEQ ID NOs: 553 and 554). These constructs, hTPO-(1-187)-Myc-CD28z-T2A-PAC(031915-U04)[SEQ ID NO:1738] and mTPO-(1-187)-Myc-CD28z-T2A-PAC(031915-V03)[SEQ ID NO:1739], consisted of the extracellular receptor binding domains of human and mouse thrombopoietin (TPO) fused to the CD28-hinge, - transmembrane and -cytosolic domains and CD3z cytosolic domains. The SEQ ID NOs of the nucleotide and protein sequences of the different camelid vHH fragments, non-immunoglobulin binding scaffolds and ligands that can be used to generate CAR and the name of their target antigens are provided in **Tables 7, 8** and **10.**

In the CAR construct CD8SP-161-(vL-vH)-Myc-CD28z-P2A-K13-Flag-T2A-EGFP(022415-T02)[SEQ ID NO:1747], the cDNA encoding K13-vFLIP from the Kaposi's sarcoma associated herpesvirus containing a carboxy-terminal Flag epitope tag is inserted downstream of the nucleotide sequence encoding the CD8SP-161-(vL-vH)-Myc-CD28z CAR and is separated from it by nucleotide sequence encoding P2A cleavable linker. The CAR construct CD8SP-MPL-161-(vL-vH)-Myc-BBz-P2A-K13-Flag-T2A-PAC[SEQ ID NO:1388] is similar in design to SEQ ID NO: 1747 except that it contains the CD8 hinge and transmembrane domains and the 41BB costimulatory domain. Essentially, a similar approach was used to clone other accessory modules, such as MC159-vFLIP, cFLIP-L, HTLV1-Tax and HTLV2-Tax, in the CAR-encoding lentiviral vectors. The construct CD8SP-MPL-161-(vL-vH)-Myc-BBz-P2A-K13-Flag-T2A-PAC[SEQ ID NO:1388] comprises a CAR containing a 41BB costimulatory domain (BB) and a CD3z activation domain (z). This construct also co-expresses a Flag-epitope tagged K13-vFLIP downstream of the CAR-encoding sequence and separated from it by a P2A cleavable linker. The construct CD8SP-MPL-161-(vL-vH)-Myc-z-P2A-K13-Flag-T2A-PAC(111516-N05)[SEQ ID NO:1118] is similar in design to SEQ ID NO: 1388 except that it lacks the 41BB costimulatory domain. The SEQ ID NOs of the nucleotide and protein sequences of the different CAR constructs and the name of their target antigens are provided in **Tables 19-22.** All the CAR constructs were generated using standard molecular biology techniques and their sequences confirmed using automated sequencing.

### Lentivirus and retrovirus vectors

Lentiviruses were generated by calcium phosphate based transfection in 293FT cells essentially as described previously (Matta H et al, Cancer biology and therapy. 2(2):206-10. 2003). 293FT cells were grown in DMEM with 10% FCS 4 mM L-Glutamine, 0.1 mM MEM Non-Essential Amino Acids, and 1 mM MEM Sodium Pyruvate (hereby referred to as DMEM-10). For generation of lentivirus, 293FT cells were plated in 10 ml of DMEM-10 medium without antibiotics in a 10 cm tissue culture plate so that they will be approximately 80 confluent on the day of transfection. The following day, the cells were transfected by calcium phosphate transfection method using 10 µg of lentiviral expression plasmid encoding different genes, 7.5 µg of PSPAX2 plasmid and 2 µg of PLP/VSVG plasmid. Approximately 15-16 hours post-transfection, 9 ml of media was removed and replaced with 5 ml of fresh media. Approximately, 48 hours post- transfection, 5 ml of supernatant was collected (first collection) and replaced with fresh 5 ml media. Approximately 72 hrs post-transfection, all media was collected (second collection, usually around 6 ml). The collected supernatants were pooled and centrifuged at 1000 rpm for 1 minute to remove any cell debris and non-adherent cells. The cell-free supernatant was filtered through 0.45 µm syringe filter. In some cases, the supernatant was further concentrated by ultra-centrifugation at 18500 rpm for 2 hours at 4oC. The viral pellet was re-suspended in 1/10 of the initial volume in XVIVO medium. The virus was either used fresh to infect the target cells or stored frozen in aliquots at -80°C.

### Infection of T cells and PBMC

Buffy coat cells were obtained from healthy de-identified adult donors from the Blood Bank at Children Hospital of Los Angeles and used to isolate peripheral blood mononuclear cells (PBMC) by Ficoll-Hypaque gradient centrifugation. PBMC were either used as such or used to isolate T cells using CD3 magnetic microbeads (Miltenyi Biotech) and following the manufacturer's instructions. PBMC or isolated T cells were re-suspended in XVIVO medium (Lonza) supplanted with 10 ng/ml CD3 antibody, 10ng/ml CD28 antibody and 100 IU recombinant human-IL2. Cells were cultured at 37°C, in a 5% CO2 humidified incubator. Cells were activated in the above medium for 1 day prior to infection with lentiviral vectors. In general, primary cells (e.g. T cells) were infected in the morning using spin-infection (1800 rpm for 90 minutes at 37°C with 300µl of concentrated virus that had been re-suspended in XVIVO medium in the presence of 8 µg/ml of Polybrene^{®} (Sigma, Catalog no. H9268). The media was changed in the evening and the infection was repeated for two more days for a total of 3 infections. After the 3rd infection, the cells were pelleted and resuspended in fresh XVIVO media containing 10ng/ml CD3 antibody, 10ng/ml CD28 antibody and 100 IU recombinant human-IL2 and supplemented with respective antibiotics (if indicated) and place in the cell culture flask for selection, unless indicated otherwise. Cells were cultured in the above medium for 10-15 days in case no drug selection was used and for 20-30 days in case drug-selection was used. In cases, where cells were infected with a lentivirus expressing EGFP, they were expanded without drug-selection or flow-sorted to enrich for EGFP-expressing cells. For infection of cancer cell lines, approximately 500,000 cells were infected with 2 ml of the un-concentrated viral supernatant in a total volume of 3 ml with Polybrene^{®} (Sigma, Catalog no. H9268). Then next morning, the cells were pelleted and resuspended in the media with respective antibiotics and place in the cell culture flask for selection.

Essentially a similar procedure as described above for lentivirus vector production was used for generation of retroviral vectors with the exception that 293FT cells were generally transfected in 10 cm tissue culture plates in 10 ml of DMEM-10 medium using 10 µg of retroviral construct, 4µg of pKAT and 2µg of VSVG plasmid. The virus collection and infection of target cells was carried out essentially as described above for lentiviral vectors.

### Antibodies and drugs

Blinatumomab was obtained from Amgen. Digitonin was purchased from Sigma (Cat. no D141) and a stock solution of 100mg/ml was made in DMSO. A diluted stock of 1 mg/ml was made in PBS. Final concentration of digitonin used for cell lysis was 30µg/ml unless indicated otherwise.

### ELISA

Human IL2, IFNγ, IL6 and TNFα were measured in the cell culture supernatant of CAR-expressing Jurkat-NFAT-GFP effector cells or T cells that had been co-cultured with the specific target cell lines for 24 to 96 hours using commercially available ELISA kits from R&D systems (Minneapolis, MN) and BD Biosciences and following the recommendations of the manufacturer.

### FACS analysis for detecting expression of CAR

Mouse Anti-Human c-Myc APC-conjugated Monoclonal Antibody (Catalog # IC3696A) was from R&D Systems (Minneapolis, MN). Biotinylated protein L was purchased from GeneScript (Piscataway, NJ), reconstituted in phosphate buffered saline (PBS) at 1 mg/ml and stored at 4°C. Streptavidin-APC (SA1005) was purchased from ThermoFisher Scientific.

For detection of CARs using Myc staining, 1 × 10⁶ cells were harvested and washed three times with 3 ml of ice-cold 1 × PBS containing 4% bovine serum albumin (BSA) wash buffer. After wash, cells were resuspended in 0.1 ml of the ice-cold wash buffer containing 10 µl of APC-conjugated Myc antibody and incubated in dark for 1 hour followed by two washings with ice cold wash buffer.

For detection of CARs using Protein L staining, 1 × 10⁶ cells were harvested and washed three times with 3 ml of ice-cold 1 × PBS containing 4% bovine serum albumin (BSA) wash buffer. After wash, cells were resuspended in 0.1 ml of the ice-cold wash buffer containing 1 µg of protein L at 4°C for 1 hour. Cells were washed three times with ice-cold wash buffer, and then incubated (in the dark) with 10µl of APC-conjugated streptavidin in 0.1 ml of the wash buffer for 30 minutes followed by two washings with ice cold wash buffer. FACS was done using FACSVerse analyzer from BD Biosciences.

### Cell death assay

To measure cell death, a novel assay based on ectopic cytosolic expression of Gluc, NLuc and other luciferases was utilized as described in US provisional patent application 62/396,650 "A Non-Radioactive Cytotoxicity Assay". The method involves expression of a reporter in a target cells in a manner so that it is preferentially retained within the healthy cells but is either released from dead and dying cells or whose activity can be preferentially measured in dead and dying cells. The preferred reporter for this assay are 1) non-secreted forms of luciferases from the copepods, such as Gaussia princeps, Pleuromamma abdominalis, Metridia pacifica, Metridia curticauda, Metridia asymmetrica, Metridia okhotensis, Metridia longa, Lucicutia ovaliformis, Heterorhabdus tanneri, and Pleuromamma scutullata 2) engineered luciferase reporters from deep sea shrimp, such as NanoLuc. The sequence of several such exemplary reporter vectors is provided in SEQ ID NO: 912 to SEQ ID NO: 918. The above vectors were used to generate retrovirus and lentiviruses which in turn were used to generate polyclonal population of several target cell lines stably expressing GLuc, NLuc, or TurboLuc following selection with appropriate antibiotics. Unless indicated otherwise, the target cells stably expressing the different luciferases were plated in triplicate in a 384 well plate in the media used for growing the target cells. Target cells which grow in suspension were generally plated at a concentration of 2-3 x 10⁴ per well, while target cells which grow as adherent monolayers were plated at a concentration of 1-2 x 10⁴ per well. Unless indicated otherwise, the target cells were cocultured with the genetically modified T cells (i.e. those expressing CAR) at an Effector: Target (E:T) ratio varying from 1: 1 to 10:1 for 4 hours to 96 hours. In the case target cells grow as adherent cells (e.g., HeLa cells), they were allowed to attach to the bottom of the wells overnight before the T cells were added. T cells mediated induction of lysis of target cells was assayed by increase of luciferase activity as measured by BioTek synergy plate reader by directly injecting 0.5 x CTZ assay buffer containing native coeloentrazine (Nanaolight) as described below.

### CTZ assay

A 100X stock solution of native coelenterazine (CTZ; Nanolight, cat # 303) was made by dissolving 1mg of lyophilized CTZ powder in 1.1 ml of 100% Methanol supplemented with 30µl of 6N HCl to avoid oxidation of CTZ with time. To make CTZ assay buffer, the 100X stock solution of CTZ was diluted to 0.5X concentration in PBS. Unless indicated otherwise, a total volume of 15µl of the CTZ assay buffer (as prepared above) was added to each well of a 384-well white plate (Greiner, 384 well white plate cat # 781075) containing cells expressing the non-secretory form of the luciferase in approximately 50-60µl volume of medium and plates were read for luminescence in endpoint mode using BioTek synergyH4 plate reader. For 96 well plates, cells were plated in 200µl of media and approximately 50µl of 0.5X CTZ assay buffer was added. Unless indicated otherwise, the 0.5X CTZ assay buffer was used for assaying the activity of GLuc, TurboLuc16, and MLuc7. The CTZ assay buffer (diluted to 0.125X concentration) was also used for measurement of NLuc activity in some experiments (see below). In general, unless indicated otherwise, the volume of 0.5X CTZ assay buffer added was approximately 1/4th of the volume of the liquid in the well containing the cells, although the assay also worked when the 0.5X CTZ assay was added to the media containing the cells in 1:1 volume. Gluc activity in wells containing media alone (Med) and in wells in which target cells were incubated with T cells that were not infected with any SIR construct (T-UI) were used as controls, where indicated.

### NLuc assay:

Nano-Glo^{®} Luciferase Assay System (Promega) was used for measurement of NLuc activity by following the manufacturer's instructions. Briefly, Nano-Glo Reagent was prepared by adding 5µl of Nano-Glo substrate in 1ml of Nano-Glo assay buffer (Promega). Unless indicated otherwise, 15µl of Nano-Glo Reagent prepared above was manually added directly to each well of a 384-well white plate containing cells in 60µl of media and plates. For 96 well plates, cells were plated in 200µl of media and approximately 50µl of assay buffer was added.

Plates were read for luminescence in endpoint mode using BioTek synergyH4 plate reader without prior cell lysis. In some experiments, NLuc activity was measured using CTZ assay buffer but here the buffer was diluted to final concentration of 0.125X. When CTZ assay buffer was used for measurement of NLuc activity, a total volume of approximately 15µl (unless indicated otherwise) of the 0.125X CTZ assay buffer was added by injector to each well of a 384-well white plate (Greiner, 384 well white plate cat # 781075) containing cells in approximately 50-60µl volume of medium and plates were read for luminescence in endpoint mode using BioTek synergyH4 plate reader. For 96 well plates, cells were generally plated in 200 µl of media and approximately 50µl of 0.125X CTZ assay buffer was added.

### Assay to detect the expression of antigens on target cells

Both CD19 and MPL (also known as Thrombopoietin receptor or TPO-R) are expressed on hematopoietic cells but show differential expression in cells of different lineages. FMC63 is a well characterized mouse monoclonal antibody that specifically recognizes human CD19. Similarly, 161 (also designated as 1.6.1) is a monoclonal antibody that recognizes human MPL and is described in U.S. patent application US 2012/0269814 A1. We generated a FMC63 single chain Fv (scFv) fragment based on the known sequence of FMC63 vL and vH fragments. The cDNA encoding FMC63 scFv fragment consisted from 5' to 3' ends a nucleotide sequences encoding a signal peptide derived from human CD8 molecule, FMC63 vL fragment, a (Gly4Ser)x3 linker and FMC63-vH fragment. The cDNA encoding the FMC63 scFv fragment was then fused in-frame at its 3' end to cDNA encoding AcV5-tagged NLuc through a Gly-Gly-Ser-Gly linker to generate FMC63-GGSG-NLuc. The resulting fragment was then cloned down-stream of the human EF1α promoter into the lentiviral vector pLenti-EF1a (SEQ ID NO: 905) to make the construct Plenti-EF1a-FMC63(vL-vH)-GGSG-NLuc-AcV5-U09. The DNA and PRT sequences of the insert fragment are provided in SEQ ID NO: 923 and SEQ ID NO: 2668, respectively. A construct encoding 161-GGSG-NLuc was similarly generated using the vL and vH fragment of 1.6.1 monoclonal antibody against MPL. The DNA and PRT sequences of the insert fragment are provided in SEQ ID NO: 924 and SEQ ID NO: 2669, respectively. The pLenti-EF1a-FMC63(vL-vH)-GGSG-NLuc-AcV5 and pLenti-EF1a-161(vL-vH)-GGSG-NLuc-AcV5 plasmids were transfected into 293FT cells by calcium phosphate co-precipitation method. Approximately 20h post-transfection, the cell culture media was replaced with XVIVO medium. The conditioned media containing the secreted FMC63(vL-vH)-GGSG-NLuc-AcV5 (also referred to as FMC63-GGSG-NLuc-AcV5) and 161(vL-vH)-GGSG-NLuc-AcV5 (also referred to as 161-GGSG-NLuc-AcV5) proteins was collected 48-72h later.

The supernatant containing FMC63-GGSG-NLuc-AcV5 and 161-GGSG-NLuc-AcV5 proteins were used to detect the expression of CD19 and MPL on the surface of Jurkat, K562, RAJI, RS-4-11 (RS411) and HEL.92.1.7 (HEL) cells that had been engineered to express a c-MPL cDNA by transducing these cells with a lentiviral vector expressing human c-MPL cDNA or an empty vector. The cells also expressed a humanized Gluc cDNA lacking its signal peptide. The vector- and MPL-expressing Jurkat-Gluc, K562-Gluc, HEL.92.1.7-Gluc, RAJI-Gluc and RS411-Gluc cells were incubated with the FMC63-GGSG-NLuc-AcV5 and 161-GGSG-NLuc-AcV5 supernatants at 4°C for 1h followed by extensive washings with cold PBS supplemented with 0.1% BSA. The cells were re-suspended in cold PBS and 30µl of cell suspension was plated per well in a flat-bottom 384 well plate (Greiner, 384 well white plate cat. # 781075) in triplicate. NLuc assay buffer containing native coelenterazine (CTZ) as NLuc substrate (30µl/well of native coelenterazine diluted in PBS) was added to each well by an automatic dispenser in a well mode using a BioTek synergy H4 plate reader and light emission as a measure of NLuc activity was measured. Fig. 3A shows that strong binding with 161-GGSG-NLuc-AcV5 was observed on HEL.92.1.7-Gluc-vector cells suggesting significant expression of MPL endogenously. Ectopic expression of MPL in HEL.92.1.7-Gluc-MPL cells led to a modest increase in 161-GGSG-NLuc-AcV5 binding. In contrast, very weak binding with 161-GGSG-NLuc-AcV5 was observed on vector-expressing Jurkat, RAJI and RS411 cells and was only modestly increased upon ectopic expression of MPL. Finally, weak but stronger binding of 161-GGSG-NLuc-AcV5 was observed on K562-vector cells, and was significantly increased on K562-MPL cells. In contrast to161-GGSG-NLuc-AcV5, the FMC63-GGSG-NLuc-AcV5 supernatant showed strongest binding on vector- and MPL-expressing RAJI cells, modestly strong binding on RS411 cells and very weak to negligible binding on the other cells.

### Assay to detect the expression of chimeric antigen receptors targeting CD19 and MPL (Thrombopoietin receptor)

A frequent problem in the field of chimeric antigen receptors is lack of a sensitive and specific assay that can detect cells that express chimeric antigen receptors. To detect the expression of CAR targeting CD19 and MPL, we fused the extracellular domains (ECD) of human CD19 and human MPL, including their signal peptides, in frame with nucleotide sequence encoding a Gly-Gly-Ser-Gly linker, NLuc (without a secretory signal) and an AcV5 epitope tag. In the case of CD19 construct, a FLAG tag was inserted between the signal peptide and the beginning of the extracellular domain. The whole cassette was cloned downstream of the human EF1α promoter into the lentiviral vector pLenti-EF1 to make constructs pLenti-EF1-FLAG-CD19-ECD-GGSG-NLuc-AcV5 and pLenti-EF1-MPL-ECD-GGSG-NLuc-AcV5, respectively. The nucleic acid sequences of the insert fragments are provided in SEQ ID NO: 926 and SEQ ID NO: 925, respectively. The protein sequences of the insert fragments are provided in SEQ ID NO: 2671 and SEQ ID NO: 2670, respectively. The constructs were transfected into 293FT cells by calcium phosphate co-precipitation method. Approximately 20 h post-transfection, the cell culture media was replaced with fresh medium. The conditioned media containing the secreted FLAG-CD19-ECD-GGSG-NLuc-AcV5 (also referred to as CD19-GGSG-NLuc-AcV5) and MPL-ECD-GGSG-NLuc-AcV5 (also referred to as MPL-GGSG-NLuc-AcV5) proteins was collected 48-72 h later.

293FT-cells were transiently transfected (in a 24-well plate, 500µl volume) with lentiviral constructs expressing chimeric antigen receptors CD8SP-FMC63-(vL-vH)-Myc-BBz-T2A-PAC(112014-A13)[SEQ ID NO:1467], CD8SP-MPL-161-(vL-vH)-Myc-BBz-T2A-PAC(021015-R07)[SEQ ID NO:1658] or CD8SP-161-(vL-vH)-Myc-CD28z-T2A-PAC(021715-Z07)[SEQ ID NO:1731] using calcium phosphate co-transfection method or left untransfected. Next day morning, approximately 18 hours post-transfection, cells were collected by pipetting up and down in 1.5 ml tubes. The tubes were spun down at 1500 RPM for 5 minutes. Then the cells were washed once with wash buffer (1% FBS in PBS), followed by incubation with 100µl of indicated secretory forms of GGS NLuc supernatants. The cells were incubated at 4°C for 1 hour.

After the incubation, cells were washed 5 times with wash buffer (1 ml each wash). Finally the pellet was resuspended in 200µl wash buffer. Resuspended cells were placed in a 384 well plate in triplicate (25µl each). Luciferase activity was measured using a BioTek synergy H4 plate reader after addition of NLuc assay buffer (Promega) containing native coelenterazine (25µl each well) directly to each well (one at a time).

As shown in the **Fig. 4A****,** 293FT cells expressing the CAR CD8SP-FMC63-(vL-vH)-Myc-BBz-T2A-PAC(112014-A13)[SEQ ID NO:1467] that targets CD19 demonstrated strong binding to FLAG-CD19-ECD-GGSG-NLuc-AcV5[SEQ ID NO: 926] as measured by NLuc assay while very little binding was seen on uninfected T cells (UI) or those expressing the control CAR CD8SP-MPL-161-(vL-vH)-Myc-BBz-T2A-PAC(021015-R07)[SEQ ID NO:1658]. Similarly, 293FT cells expressing the CAR CD8SP-161-(vL-vH)-Myc-CD28z-T2A-PAC(021715-Z07)[SEQ ID NO:1731] showed strong binding with MPL-ECD-GGSG-NLuc-AcV5[SEQ ID NO: 925] supernatant as compared to un-transfected 293FT cells or those transfected with the CAR CD8SP-FMC63-(vL-vH)-Myc-BBz-T2A-PAC(112014-A13)[SEQ ID NO:1467] **(****Fig. 4B****).**

### Assay to detect the expression of chimeric antigen receptors targeting MPL (Thrombopoietin receptor) on 293FT cells that had been transfected with the different CAR constructs

The construct hTPO-(1-187)-Myc-CD28z-T2A-PAC(031915-U04)[SEQ ID NO:1738], mTPO-(1-187)-Myc-CD28z-T2A-PAC(031915-V03)[SEQ ID NO:1739], CD8SP-MPL-161-(vL-vH)-Myc-BBz-T2A-PAC(021015-R07)[SEQ ID NO:1658] and a control CAR construct containing a scFv derived from an irrelevant antibody were transiently transfected into 293FT cells and incubated with MPL-ECD-GGSG-NLuc-AcV5[SEQ ID NO: 925] supernatant essentially as described in the preceding example. **Fig. 5** shows modest binding of MPL-ECD-GGSG-NLuc-AcV5 to 293FT cells transfected with hTPO-(1-187)-Myc-CD28z-T2A-PAC(031915-U04)[SEQ ID NO:1738] and mTPO-(1-187)-Myc-CD28z-T2A-PAC(031915-V03)[SEQ ID NO:1739] constructs and strong binding to 293FT cells transfected with CD8SP-MPL-161-(vL-vH)-Myc-BBz-T2A-PAC(021015-R07)[SEQ ID NO:1658] construct as compared to untransfected cells or cells that had been transfected with control CAR construct.

### MPL-CARs are expressed on the surface of T cells

Purified T cells were infected with lentiviruses encoding the different Myc-tagged CAR constructs targeting MPL. The constructs also co-expressed EGFP. The expression of CAR on T cells was examined by EGFP fluorescence and immunofluorescence staining with an APC-conjugated Myc-antibody followed by FACS analysis on a BD verse flow cytometer. **Fig. 6** shows expression of MPL CARs on the surface of T cells as determined by increased EGFP fluorescence and increased staining with APC-Myc as compared to the uninfected T cells (UI).

### T cells expressing MPL CARs are capable of binding MPL-GGSG-NLuc fusion protein

T cells expressing the different MPL CAR constructs were incubated with MPL-GGSG-NLuc-AcV5 and CD19-GGSG-NLuc-AcV5 supernatants and after extensive washes assayed for NLuc activity essentially as described in the preceding example. Figure 7 shows strong binding of T cells expressing CD8SP-MPL-161-(vL-vH)-Myc-BBz-T2A-PAC(021015-R07)[SEQ ID NO:1658], CD8SP-175-(vL-vH)-Myc-CD28z-T2A-PAC(031615-Q04)[SEQ ID NO:1733], CD8SP-MPL-VB22Bw4-(vL-vH)-Myc-CD28z-T2A-PAC(031615-B06)[SEQ ID NO:3536] CARs and modest binding of T cells expressing CD8SP-161-(vL-vH)-Myc-CD28z-T2A-PAC(021715-Z07)[SEQ ID NO:1731], CD8SP-AB317-(vL-vH)-Myc-CD28z-T2A-PAC(031615-T04)[SEQ ID NO:1735] and CD8SP-12E10-(vL-vH)-Myc-CD28z-T2A-PAC(031615-S03)[SEQ ID NO:1736] to MPL-GGSG-NLuc AcV5 supernatant, while no significant binding was observed on uninfected T cells or those expressing the control CAR CD8SP-4C3-(vL-vH)-Myc-CD28z-T2A-Pac(031615-WO5)[SEQ ID NO:3534]. Similarly, no specific binding was observed on any MPL CAR-T cells with CD19-GGSG-NLuc-AcV5 supernatant, thereby demonstrating the specificity of the assay.

### MPL CARs activate T cell signaling in Jurkat cells upon antigen stimulation.

Jurkat cells stably expressing an NFAT-EGFP reporter construct were stably transduced with lentiviral vectors expressing the different CAR constructs targeting MPL followed by selection with puromycin. Cells were subsequently co-cultured with MPL-expressing HEL.92.1.7 (HEL) cells. Co-culturing of MPL-CAR expressing Jurkat-NFAT-EGFP cells with HEL cells resulted in increase in EGFP expression as determined by FACS analysis, which was evident as early as 4 h after co-culture. The increase in EGFP expression was strongest in Jurkat-NFAT-EGFP cells expressing the CD8SP-MPL-161-(vL-vH)-Myc-BBz-T2A-PAC(021015-R07)[SEQ ID NO:1658], CD8SP-161-(vL-vH)-Myc-CD28z-T2A-PAC(021715-Z07)[SEQ ID NO:1731] and CD8SP-175-(vL-vH)-Myc-CD28z-T2A-PAC(031615-Q04)[SEQ ID NO:1733] constructs. In an independent experiment, significant EGFP induction was also observed upon culturing of Jurkat-NFAT-EGFP cells expressing the CD8SP-2-MPL-111-(vL-vH)-Myc-BBz-T2A-PAC(041316-A03)[SEQ ID NO:1660] CAR construct with HEL cells. The experiment was repeated by co-culturing the Jurkat-NFAT-EGFP cells with either RAJI cells, which have weak MPL expression, or with RAJI-MPL cells that were engineered to ectopically express human MPL receptor. Jurkat-NFAT-EGFP cells expressing CAR directed against MPL showed more increase in EGFP expression when incubated with RAJI-MPL cells as compared with RAJI-vector cells. The increase in EGFP expression was again strongest in Jurkat-NFAT-EGFP cells expressing the CD8SP-MPL-161-(vL-vH)-Myc-BBz-T2A-PAC(021015-R07)[SEQ ID NO:1658], CD8SP-161-(vL-vH)-Myc-CD28z-T2A-PAC(021715-Z07)[SEQ ID NO:1731] and CD8SP-175-(vL-vH)-Myc-CD28z-T2A-PAC(031615-Q04)[SEQ ID NO:1733] constructs.

**Table 25: EGFP induction in Jurkat-NFAT-eGFP cells expressing different CARs**

| **S.No.** | **Jurkat-NFAT-EGFP** | **GFP induction (%) TARGET CELL LINE** | | | |
|---|---|---|---|---|---|
| | | NONE | HEL | RAJI-vector | RAJI-Mpl |
| I | Jurkat-NFAT-EGFP (Parental cells) | 3 | 1 | 3 | 3 |
| 2 | CD8SP-MPL-161-(vL-vH)-Myc-BBz-T2A- | 4 | 27 | 4 | 23 |
| | PAC(021015-R07)[SEQ ID NO:1658] | | | | |
| 3 | CD8SP-161-(vL-vH)-Myc-CD28z-T2A-PAC(021715- Z07)[SEQ ID NO:1731] | 4 | 14 | 5 | 11 |
| 4 | CD8SP-175-(vL-vH)-Myc-CD28z-T2A-PAC(031615- Q04)[SEQ ID NO:1733] | 6 | 33 | 11 | 18 |
| 5 | CD8SP-178-(vL-vH)-Myc-CD28z-T2A-PAC(031615- R04)[SEQ ID NO:1734] | 4 | 2 | 4 | 3 |
| 6 | CD8SP-12E10-(vL-vH)-Myc-CD28z-T2A- PAC(031615-S03)[SEQ ID NO:1736] | 2 | 5 | 4 | 6 |
| 7 | CD8SP-AB317-(vL-vH)-Myc-CD28z-T2A- PAC(031615-T04)[SEQ ID NO:1735] | I | I | 3 | 6 |

### T cells expressing MPL CAR induce cytotoxicity in MPL-expressing target leukemia cells

Human peripheral blood T cells isolated using CD3 magnetic beads were infected with lentiviruses expressing the CAR construct CD8SP-161-(vL-vH)-Myc-CD8TM-BBz-T2A-EGFP(090814-C03)[SEQ ID NO:1732] targeting MPL or left uninfected (UI). The indicated number of HEL cells stably expressing GLuc were cocultured with T cells expressing the CD8SP-161-(vL-vH)-Myc-CD8TM-BBz-T2A-EGFP(090814-C03)[SEQ ID NO:1732] in a 384 well plate at an Effector: Target (E:T) ratio of 1:1 for 4 hours. CAR-T cells mediated induction of lysis of target cells was assayed by increase of GLuc activity as measured by BioTek synergy plate reader by directly injecting 0.5X CTZ assay buffer containing native coeloentrazine (Nanaolight). **Fig. 8** shows increase in GLuc activity following co-culture with T cells expressing MPL-specific CD8SP-161-(vL-vH)-Myc-CD8TM-BBz-T2A-EGFP(090814-C03)[SEQ ID NO:1732] CAR as compared to the uninfected T cells indicating lysis of target cells by the different MPL-CAR-T cells.

### PBMC expressing MPL CAR induce lysis of Target Leukemia cells expressing MPL

The blood samples to isolate Peripheral blood mononuclear cells (PBMCs) were obtained from healthy de-identified adult donors. PBMC were isolated from buffy coats by Ficoll-Hypaque gradient centrifugation and re-suspended in XVIVO medium (Lonza) supplanted with 10 ng/ml soluble anti-CD3, 10 ng/ml soluble anti-CD28 and 100 IU recombinant human-IL2. PBMCs were engineered to express CD8SP-MPL-161-(vL-vH)-Myc-BBz-T2A-PAC(021015-R07)[SEQ ID NO:1658]CAR targeting MPL and CD8SP-FMC63-(vL-vH)-Myc-BBz-T2A-PAC(112014-A13)[SEQ ID NO:1467] targeting CD19 by infection with the respective lentiviral vectors followed by selection with puromycin. The PBMC expressing the indicated CARs and uninfected PBMC were co-cultured with HEL-GLuc target cells for 4 h and lysis of target cells measured by the single-step homogenous GLuc assay by injecting 0.5X CTZ assay buffer as described above. **Fig. 9** shows increased killing of HEL-GLuc cells by CD8SP-MPL-161-(vL-vH)-Myc-BBz-T2A-PAC(021015-R07)[SEQ ID NO:1658] CAR-expressing PBMC as compared to the FMC63-(vL-vH)-Myc-BBz-T2A-PAC(112014-A13)[SEQ ID NO:1467]CAR-expressing PBMC or uninfected PBMC.

### PBMC expressing MPL CAR induce lysis of Target Leukemia cells expressing MPL

The blood samples to isolate Peripheral blood mononuclear cells (PBMCs) were obtained from healthy de-identified adult donors. PBMC were isolated from buffy coats by Ficoll-Hypaque gradient centrifugation and re-suspended in XVIVO medium (Lonza) supplanted with 10 ng/ml soluble anti-CD3, 10 ng/ml soluble anti-CD28 and 100 IU recombinant human-IL2. PBMCs were engineered to express CD8SP-175-(vL-vH)-Myc-CD28z-T2A-PAC(031615-Q04)[SEQ ID NO:1733]CAR targeting MPL by infection with the lentiviral vector followed by selection with puromycin. The PBMCs expressing the indicated CAR and uninfected PBMCs were co-cultured with HEL-GLuc target cells for 4 h and lysis of target cells measured by the single-step homogenous GLuc assay by injecting 0.5X CTZ assay buffer as described above. **Fig. 10** shows increased killing of HEL-GLuc cells by the CD8SP-175-(vL-vH)-Myc-CD28z-T2A-PAC(031615-Q04)[SEQ ID NO:1733] CAR expressing PBMC as compared to uninfected PBMC.

### T cells expressing MPL CARs expressing CD28 costimulatory domain induce cytotoxicity in MPL-expressing target leukemia cells

Human peripheral blood T cells isolated using CD3 magnetic beads were infected with lentiviruses expressing the CAR constructs CD8SP-161-(vL-vH)-Myc-CD28z-T2A-PAC(021715-Z07)[SEQ ID NO:1731], CD8SP-175-(vL-vH)-Myc-CD28z-T2A-PAC(031615-Q04)[SEQ ID NO:1733], CD8SP-178-(vL-vH)-Myc-CD28z-T2A-PAC(031615-R04)[SEQ ID NO:1734], and CD8SP-AB317-(vL-vH)-Myc-CD28z-T2A-PAC(031615-T04)[SEQ ID NO:1735] targeting MPL.. Cells were selected with puromycin and expanded. HEL cells stably expressing GLuc were cocultured with T cells expressing the CARs at an Effector: Target (E:T) ratio of 10:1 for 4 hours. CAR-T cells mediated induction of lysis of target cells was assayed by increase of GLuc activity. **Fig. 11** shows increase in GLuc activity, indicating lysis of target cells, following co-culture with T cells expressing MPL-specific CARs, which was stronger with the CD8SP-161-(vL-vH)-Myc-CD28z-T2A-PAC(021715-Z07)[SEQ ID NO:1731], CD8SP-175-(vL-vH)-Myc-CD28z-T2A-PAC(031615-Q04)[SEQ ID NO:1733], and CD8SP-AB317-(vL-vH)-Myc-CD28z-T2A-PAC(031615-T04)[SEQ ID NO:1735] constructs and modest with the CD8SP-178-(vL-vH)-Myc-CD28z-T2A-PAC(031615-R04)[SEQ ID NO:1734] construct as compared to the uninfected T cells.

### T cells expressing MPL CARs expressing CD28 costimulatory domain induce cytotoxicity in MPL-expressing target leukemia cells

Human peripheral blood T cells isolated using CD3 magnetic beads were infected with lentiviruses expressing the CAR constructs CD8SP-161-(vL-vH)-Myc-CD28z-T2A-PAC(021715-Z07)[SEQ ID NO:1731], CD8SP-175-(vL-vH)-Myc-CD28z-T2A-PAC(031615-Q04)[SEQ ID NO:1733], CD8SP-178-(vL-vH)-Myc-CD28z-T2A-PAC(031615-R04)[SEQ ID NO:1734], CD8SP-AB317-(vL-vH)-Myc-CD28z-T2A-PAC(031615-T04)[SEQ ID NO:1735], CD8SP-12E10-(vL-vH)-Myc-CD28z-T2A-PAC(031615-S03)[SEQ ID NO:1736] and CD8SP-VB22Bw4-(vL-vH)-Myc-CD28z-T2A-PAC(031615-B06)[SEQ ID NO:3536] targeting MPL. Cells were selected with puromycin and expanded. Jurkat cells stably expressing MPL and GLuc were cocultured with T cells expressing the CARs at an Effector: Target (E:T) ratio of 10:1 for 4 hours. CAR-T cells mediated induction of lysis of target cells was assayed by increase of GLuc activity as measured by BioTek synergy plate reader by directly injecting 0.5 x CTZ assay buffer containing native coeloentrazine (Nanaolight). **Fig. 12** shows increase in GLuc activity, indicating lysis of target cells, following co-culture with T cells expressing MPL-specific CARs, which was stronger with the CD8SP-161-(vL-vH)-Myc-CD28z-T2A-PAC(021715-Z07)[SEQ ID NO:1731], CD8SP-175-(vL-vH)-Myc-CD28z-T2A-PAC(031615-Q04)[SEQ ID NO:1733], CD8SP-AB317-(vL-vH)-Myc-CD28z-T2A-PAC(031615-T04)[SEQ ID NO:1735], and CD8SP-12E10-(vL-vH)-Myc-CD28z-T2A-PAC(031615-S03)[SEQ ID NO:1736] constructs and modest with the CD8SP-178-(vL-vH)-Myc-CD28z-T2A-PAC(031615-R04)[SEQ ID NO:1734] and CD8SP-VB22Bw4-(vL-vH)-Myc-CD28z-T2A-PAC(031615-B06)[SEQ ID NO:3536] constructs as compared to the uninfected T cells.

### NK92MI cells expressing MPL-specific 161-CAR induce cytotoxicity in K562 and HEL.92.1.7 cells

Natural Killer cell derived NK92MI (ATCC) cells were stably transduced with lentiviral vectors expressing the indicated CAR constructs targeting CD8SP-161-(vL-vH)-Myc-CD8TM-BBz-T2A-EGFP(090814-C03)[SEQ ID NO:1732] and CD8SP-FMC63-(vL-vH)-Myc-BBz-T2A-EGFP(070814-D06)[SEQ ID NO:3535]. Cells were subsequently co-cultured overnight with HEL.92.1.7 cells that had been engineered to express GLuc. CAR-expressing NK92MI cells-mediated induction of lysis of target cells was assayed by increase of GLuc activity as measured by directly injecting 0.5X CTZ assay buffer containing native coeloentrazine (Nanaolight). **Fig. 13** shows increase in GLuc activity, indicating lysis of target cells, following co-culture with NK92MI cells expressing MPL-specific CD8SP-161-(vL-vH)-Myc-CD8TM-BBz-T2A-EGFP(090814-C03)[SEQ ID NO:1732]CAR.

### NK92MI cells expressing MPL-specific CARs induce cytotoxicity in K562-MPL cells

Natural Killer cell derived NK92MI (ATCC) cells were stably transduced with lentiviral vectors expressing the indicated CAR constructs targeting MPL followed by selection with puromycin. Cells were subsequently co-cultured overnight with K562 cells that had been engineered to express MPL and GLuc. The effector to target (E:T) ratio was 5:1. MPL-CAR-expressing NK92MI cells-mediated induction of lysis of target cells was assayed by increase of GLuc activity as measured by directly injecting 0.5X CTZ assay buffer containing native coeloentrazine (Nanaolight). **Fig. 14** shows increase in GLuc activity, indicating lysis of target cells, following co-culture with NK92MI cells expressing MPL-specific CARs.

### Cytokine Production by T cells expressing MPL-specific CAR constructs on exposure to target cells expressing MPL

Human peripheral blood T cells isolated using CD3 magnetic beads were infected with lentiviruses expressing the CD8SP-MPL-161-(vL-vH)-Myc-BBz-T2A-PAC(021015-R07)[SEQ ID NO:1658] and CD8SP-161-(vL-vH)-Myc-CD28z-T2A-PAC(021715-Z07)[SEQ ID NO:1731] CAR constructs targeting MPL. Cells were selected with puromycin and expanded. HEL cells were co-cultured with T cells expressing the CARs for approximately 96 hours and secretion of TNFα in the supernatant measured by ELISA. **Fig. 15** shows increased secretion of TNFα upon co-culture of T cells expressing CAR targeting MPL with HEL cells as compared to uninfected T cells used as controls.

### In vivo efficacy of MPL CAR-expressing immune effector cells against leukemia

The in vivo efficacy of MPL CAR-expressing NK92MI cells against HEL leukemia cell line model was tested in NOD/SCID/γ^{-/-} (NSG) mouse model. On day 0, the NSG mice (Jackson Lab) were sub-lethally irradiated at a dose of 275 cGy. 24 hours post irradiation (day 1), mice were injected with 1 x 10⁶ HEL cells intravenously. On day 9 and day 19, the mice were treated with 2 million NK92MI cells expressing the indicated CAR constructs intravenously and followed for survival. **Fig. 16** shows that the median survival of mice given CD8SP-161-(vL-vH)-Myc-CD8TM-BBz-T2A-EGFP(090814-C03)[SEQ ID NO:1732]-expressing NK92MI cells was 31.5 days, which was significantly higher than mice given unmodified NK92MI cells (28.5 days; *p* = 0.03) or those given NK92MI cells expressing a control CAR CD8SP-4C3-(vL-vH)-Myc-BBz-T2A-EGFP(100814-K06)[SEQ ID NO:3537]; 28 days; *p=.*04).

### Chimeric Antigen Receptors Targeting CD19 with scFV fragments derived from a humanized CD19 antibody

The CD19 CARs in current clinical trials contain scFV fragments derived from murine monoclonal antibodies. The development of antibodies against the murine antibody fragments not only contributes to lack of persistence of T cells expressing such CARs but could also lead to allergic reactions, including anaphylaxis. To overcome this limitation, a CD19 CARs was generated containing an scFv fragment (SEQ ID NO: 566) derived from a humanized CD19 monoclonal antibody huCD19-Bu12 (or Bu12) described in PCT/US2008/080373. Another CAR was constructed containing an scFv (SEQ ID NO: 567) fragment derived from a murine antibody described in US7112324 and was designated CD19MM.

The scFV fragments based on CD19-Bu12 and CD19MM were designed consisting from 5' to 3' ends of a nucleotide sequences encoding a signal peptide derived from human CD8 molecule, the vL fragment, a (Gly₄Ser)x3 linker and the vH fragment. The sequence of the scFV fragments were codon-optimized using GeneArt^{™} software (Thermo Fisher Scientific) and gene-fragments encoding the optimized sequences synthesized using a commercial vendor. The gene fragments were used as templates in PCR reaction using custom primers and then cloned in a modified pLENTI-EF1α vector containing the hinge, transmembrane and cytosolic domains of the CAR cassette by standard molecular biology techniques. The final CAR construct CD8SP-2-CD19MM-(vL-vH)-Myc-BBz-T2A-PAC(062915-D03)[SEQ ID NO:1471] consisted of human CD8 signal peptide, fused in frame to the CD19MM scFv fragment (SEQ ID NO: 567), a Myc epitope tag, the hinge and transmembrane domain of human CD8 (SEQ ID NO: 842), the cytosolic domain of human 41BB (CD137) receptor (SEQ ID NO: 845), the cytosolic domain of human CD3z (SEQ ID NO: 846), a T2A ribosomal skip sequence (SEQ ID NO: 832) and a cDNA encoding a variant of puromycin resistance gene (SEQ ID NO: 850). A CAR construct CD8SP-CD19Bu12-(vL-vH)-Myc-BBz-T2A-PAC(082815-P08)[SEQ ID NO:1470] based on CD19Bu12 scFv fragment (SEQ ID NO: 566) was designed similarly. The CAR construct CD8SP-FMC63-(vL-vH)-Myc-BBz-T2A-PAC(112014-A13)[SEQ ID NO:1467] was designed based on the previously described CD19 CAR containing the scFV fragment (SEQ ID NO: 564) derived from mouse monoclonal antibody FMC63. Finally, the CAR construct CD8SP-KSHV-4C3-(vL-vH)-Myc-BBz-T2A-PAC(042315-N01)[SEQ ID NO:1643] contains an scFV fragment (SEQ ID NO: 718) derived from an antibody against a KSHV encoded K8.1 protein and was used as a negative control. Lentiviruses encoding the above CARs were generated using 293FT and used to infect T cells as described previously for MPL CARs.

### CD19-CARs are expressed on the surface of T cells

Purified T cells were infected with lentiviruses encoding the different Myc-tagged CAR constructs targeting MPL (CD8SP-FMC63-(vL-vH)-Myc-BBz-T2A-PAC(112014-A13)[SEQ ID NO:1467], CD8SP-CD19Bul2-(vL-vH)-Myc-BBz-T2A-PAC(082815-P08)[SEQ ID NO:1470], CD8SP-2-CD19MM-(vL-vH)-Myc-BBz-T2A-PAC(062915-D03)[SEQ ID NO:1471] and CD8SP-KSHV-4C3-(vL-vH)-Myc-BBz-T2A-PAC(042315-N01)[SEQ ID NO:1643]) and selected with puromycin. The expression of CAR on T cells (both puromycin selected and unselected) was examined by immunofluorescence staining with an APC-conjugated Myc-antibody followed by FACS analysis as described previously. **Fig. 17** shows increased expression of the different CD19 CARs (shown with grey lines) on the surface of T cells as determined by staining with APC-Myc as compared to the uninfected T cells (UI; shown with dotted lines). The expression of the different CAR constructs was further increased following puromycin selection.

### Jurkat cells expressing CD19 CARs are capable of binding CD19-GGSG-NLuc fusion protein

Jurkat-NFAT-Luc cells were stably transduced with the different CD19 targeted CAR constructs and selected in puromycin. Cells were incubated with FLAG-CD19-ECD-GGSG-NLuc-AcV5 [SEQ ID NO:2671] supernatant and after extensive washes assayed for NLuc activity essentially as described previously. **Fig. 18** shows strong binding of Jurkats expressing CD8SP-CD19Bul2-(vL-vH)-Myc-BBz-T2A-PAC(082815-P08)[SEQ ID NO:1470], CD8SP-2-CD19MM-(vL-vH)-Myc-BBz-T2A-PAC(062915-D03)[SEQ ID NO:1471] and CD8SP-FMC63-(vL-vH)-Myc-BBz-T2A-PAC(112014-A13)[SEQ ID NO:1467], and CD19Bul2(vL-vH)-Myc-BBz-PAC-P08 CAR constructs to FLAG-CD19-ECD-GGSG-NLuc-AcV5 [SEQ ID NO:2671] supernatant, while no significant binding was observed on parental cells or those expressing CD8SP-KSHV-4C3-(vL-vH)-Myc-BBz-T2A-PAC(042315-N01)[SEQ ID NO:1643] control CAR.

### T cells expressing CD19 CARs are capable of binding CD19-GGSG-NLuc fusion protein

Purified T cells were stably transduced with the different CD19-targeted CAR constructs and selected in puromycin. Cells were incubated with FLAG-CD19-ECD-GGSG-NLuc-AcV5 [SEQ ID NO:2671] supernatant and after extensive washes assayed for NLuc activity essentially as described previously. **Fig. 19** shows strong binding of T cells expressing CD8SP-CD19Bul2-(vL-vH)-Myc-BBz-T2A-PAC(082815-P08)[SEQ ID NO:1470], CD8SP-2-CD19MM-(vL-vH)-Myc-BBz-T2A-PAC(062915-D03)[SEQ ID NO:1471] and CD8SP-FMC63-(vL-vH)-Myc-BBz-T2A-PAC(112014-A13)[SEQ ID NO:1467], and CD19Bu12(vL-vH)-Myc-BBz-PAC-P08 CAR constructs to FLAG-CD19-ECD-GGSG-NLuc-AcV5 [SEQ ID NO:2671] supernatant, while no significant binding was observed on uninfected T cells (UI) or those expressing CD8SP-KSHV-4C3-(vL-vH)-Myc-BBz-T2A-PAC(042315-N01)[SEQ ID NO:1643] control CAR.

### T cells expressing CD19 CARs induce cytotoxicity in CD19-expressing RAJI lymphoma cells

Human peripheral blood T cells isolated using CD3 magnetic beads were infected with lentiviruses expressing the indicated CAR constructs targeting CD19. Cells were left unselected or selected with puromycin and expanded. RAJI cells stably expressing GLuc were cocultured with T cells expressing the CARs at an Effector: Target (E:T) ratio of 10:1 for 96 hours. CAR-T cells mediated induction of lysis of target cells was assayed by increase of GLuc activity. **Fig. 20** shows increase in GLuc activity, indicating lysis of target cells, following co-culture with T cells expressing CD19-specific CARs as compared to uninfected T cells (T-UI) or those expressing the control CAR 4C3. The increased cell death was seen in CD19 CAR-expressing T cells that had been selected with puromycin as well as those that had not been selected.

### T cells expressing CD19 CARs induce cytotoxicity in CD19-expressing RAJI lymphoma cells

Human peripheral blood T cells isolated using CD3 magnetic beads were infected with lentiviruses expressing the indicated CAR constructs targeting CD19. Cells were left unselected or selected with puromycin and expanded. RAJI cells stably expressing GLuc were cocultured with T cells expressing the CARs at the indicated Effector: Target (E:T) ratios for 4 hours. CAR-T cells mediated induction of lysis of target cells was assayed by increase of GLuc activity. **Fig. 21** shows increase in GLuc activity, indicating lysis of target cells, following co-culture with T cells expressing CD19-specific CARs as compared to uninfected T cells (T-UI) or those expressing the control 4C3 CAR. In contrast to the results in the preceding section, the increased cell death was seen primarily in CD19 CAR-expressing T cells that had been selected with puromycin. This could be attributable to the fact that cell death was measured after 96 h of co-culture in the preceding example while it was measured after only 4 h in the current example.

### In vivo efficacy of CD19MM-BBz and CD19Bul12-BBz CAR T cells

Human peripheral blood T cells isolated using CD3 magnetic beads were infected with lentiviruses expressing the indicated CAR constructs targeting CD19 or the control CAR (4C3) and expanded in vitro with (with puro) or without (No puro) puromycin selection. NSG mice (Jackson Lab) were sub-lethally irradiated at a dose of 175 cGy. 24 hours post irradiation (day2), mice were injected with 2.5 x 10⁴ RAJI cells via tail-vein. On day3, the mice (n =5 for each group) were treated with 1 million T cells that had been infected with the CD8SP-CD19Bul2-(vL-vH)-Myc-BBz-T2A-PAC(082815-P08)[SEQ ID NO:1470], CD8SP-2-CD19MM-(vL-vH)-Myc-BBz-T2A-PAC(062915-D03)[SEQ ID NO:1471] encoding lentiviruses and either selected with puromycin (with puro) or left unselected (No puro). Control mice (n =5) were injected with 1 million T cells expressing the CD8SP-KSHV-4C3-(vL-vH)-Myc-BBz-T2A-PAC(042315-N01)[SEQ ID NO:1643] CAR or given no T cells. **Fig. 22** shows the survival of mice in each group. The median survival of mice given RAJI cells alone and CD8SP-KSHV-4C3-(vL-vH)-Myc-BBz-T2A-PAC(042315-N01)[SEQ ID NO:1643]CAR-T cells were 17 days and 21 days respectively. In contrast, the median survival of mice which received CD8SP-2-CD19MM-(vL-vH)-Myc-BBz-T2A-PAC(062915-D03)[SEQ ID NO:1471] CAR-T cells with and without puromycin selection were 26 and 30 days, respectively. Similarly, the median survival of mice which received CD8SP-CD19Bu12-(vL-vH)-Myc-BBz-T2A-PAC(082815-P08)[SEQ ID NO:1470] CAR-T cells with and without puromycin selection were 30 and 31 days, respectively. Thus, infusion of T cells expressing CD8SP-CD19Bu12-(vL-vH)-Myc-BBz-T2A-PAC(082815-P08)[SEQ ID NO:1470], CD8SP-2-CD19MM-(vL-vH)-Myc-BBz-T2A-PAC(062915-D03)[SEQ ID NO:1471] CARs lead to statistically significant improvement in survival of mice in this RAJI xenograft model of lymphoma as compared to mice given no T cells or those given T cells expressing the CD8SP-KSHV4C3-(vL-vH)-Myc-BBz-T2A-PAC(042315-NOI)[SEQ ID NO:1643] control CAR.

### Applications of anti-CD19 humanized CAR constructs when used alone or in combination with other strategies

The humanized CARs of this invention can be expressed in the following way to enhance their safety and efficacy:
Although we have used lentiviral methods for expression of the humanized CARs, alternate methods such as use of mRNA/DNA transfection by electroporation or by transient perturbation in cell membrane can be used to express them in the target cells. The mRNA transfection may involve natural or stabilizing nucleotides to enhance the longevity of expression. Alternate methods for gene delivery, such as use of retroviral, adenoviral and adeno-associated viral vectors have been well described in the literature and are known to those skilled in the art. Alternate promoters for expression of the humanized CAR including CMV, MSCV etc. have been described in the literature.

The CARs of the present invention could be also expressed with suitable selection markers, such as extracellular domain sequences of EGFR, tEGFR, CD19, BCMA (SEQ ID NOs: 853-856), to positively select CAR-expressing cells and to reduce the clonal diversity of the final T cell product before it is used for infusion into the patient. Reduction in clonal-diversity of the CAR-T cell product will lead to reduction in the incidence and severity of Graft versus Host Disease (GVHD) as it will reduce the probability and number of host-reactive T cell clones in the product . Alternate resistance gene, such as CNB30 (SEQ ID NO: 852), which confers resistance to calcineurin inhibitors, can be included to positively select CAR-expressing cells and to reduce the clonal diversity of the final T cell product before it is used for infusion into the patient to reduce the incidence of GVHD.

One of the limitations of CD19 CAR-T cell therapy is long-term depletion of normal B cells, which also express CD19 antigen. To circumvent this problem, CD19 CAR-T cell therapy can be combined with knock-out or mutation of endogenous CD19 in normal hematopoietic stem cells using CRIPS/Cas9, TALONS or other suitable gene editing methods which are known in the art. The epitope of CD19 bound by the CD19 CAR-T cells in current clinical use has been mapped to exons 2-4. Thus, missense or nonsense mutations can be generated in exon 2 (or other suitable exons/regions that are recognized by CD19Bu12 and CD19MM CAR T-cells) of autologous or allogeneic hematopoietic stem cells using CRISP/Cas9, Zn finger nucleases, Talons or other methods known in the art. The patient can be given CD19 CAR-T cells infusion to control his/her disease and an autologous or allogeneic transplant using CD19 deleted/mutated hematopoietic stem cells. As the B cells that will originate from the modified stem cells will not be targeted by CD19-CAR-T cells, the patient will escape B cell aplasia which is a common side effect of CD19 CAR-T cells.

The CD19 targeted humanized CARs of the present invention can be also combined with CAR targeting other antigens, such as CD22 and CD20, to overcome resistance due to loss of CD19 antigens. Such bispecific CARs have been described in the literature and are known to one skilled in the art.

The humanized CAR constructs of the present invention can be expressed not only in T cells but in defined subsets of T cells such as CD4 and CD8 T cells, central memory T cells, naive T cells and regulatory T cells. They can be also expressed in NK cells, NK92 cell line, hematopoietic stem cells, and iPSC derived stem cells.

To control their activity, the humanized CAR of the current invention can be expressed in an inducible fashion as is known in the art (Roybal et al., Cell 164:770-9, 2016; Wu et al., Curr Opin Immunol 35:123-30, 2015; Wu et al., Science 350:aab4077, 2015)). Alternatively, suicide genes (such as icaspase 9), can be used to control the activity of CAR-T cells in vivo.

The activity of the CAR-T cells of this invention can be further modulated by use of inhibitors of various signaling pathways, such as mTOR and PI3-AKT pathways. Furthermore, various cytokines, chemokines and their receptors, and costimulatory ligands (e.g. 41BB) and their receptor (e.g. 41BB-receptor or CD137) can be coexpressed with the humanized CAR of the current invention.

### Novel Chimeric Antigen Receptors expressing viral and cellular signaling proteins

As discussed previously, the CAR constructs in clinical trials include one or more costimulatory domains derived from 41BB, CD28 or both. These costimulatory domains are generally derived from the cytosolic signaling domains of TNF (Tumor Necrosis Factor) family receptors and are believed to activate signaling pathways, such as NF-κB and AKT, which are believed to contribute to increased proliferation and survival of CAR-T cells, resulting in their expansion and persistence in vivo. However, tonic signaling through these costimulatory domains when expressed as part of an artificial receptor, such as a CAR, is not physiological and is not under the control of feedback regulatory mechanisms that are normally present to control signaling via the TNF family receptors from which they are derived. Therefore, it is conceivable that robust and persistent activation of signaling pathways by the costimulatory domains present in the current CAR constructs contributes to the cytokine release syndrome and neurological toxicity, which re a major side effect of current CAR-T cells based therapies. It is important to note in this context that cytokine release syndrome was a major side effect in human clinical trials of a CD28 super-agonist antibody.

The present invention circumvents the above limitation of the current CAR vectors by deleting the costimulatory domains. The costimulatory function instead is provided by viral and cellular proteins that are known to constitutively activate various pro-survival signaling pathways and/or block cell death. The viral proteins suitable for this purpose include viral FLICE Inhibitory Protein (vFLIP) K13 encoded by the Kaposi's sarcoma associated herpesvirus (also known as human herpesvirus 8), vFLIP MC159 and MC160 from the molluscum contagiosum virus (MCV), E8 from the equine herpesvirus 2 (EHV2), vFLIP encoded by the bovine herpesvirus 4, vFLIP encoded by herpesvirus mecaca, vFLIP encoded by herpesvirus saimiri, cFLIP-L isoform or MRIT-α, cFLIP-p22 deletion mutant or MRIT-p22 , HTLV1-Tax, HTLV2-Tax and HTLV2-Tax-RS mutant (Jeang, Cytokine Growth Factor Rev 12:207-17., 2001; Sieburg et al., J Virol 78:10399-409, 2004). In contrast to artificial and non-physiological nature of the pro-survival signal provided by the costimulatory domains that are components of the CAR constructs in common use today, these signaling proteins have evolved to provide constitutive pro-survival signals to the cells in which they are expressed.

### KSHV-encoded viral FLICE inhibitory Protein K13 immortalizes T cells

vFLIP K13 was originally believed to protect KSHV-infected cells from death receptor-induced apoptosis (Bertin et al., Proc Natl Acad Sci U S A 94:1172-6, 1997; Hu et al., J Biol Chem 272:9621-4, 1997; Thome et al., Nature 386:517-21, 1997)). However, it was subsequently reported that K13 selectively activates the NF-κB pathway (Chaudhary et al., Oncogene 18:5738-46, 1999; Chugh et al., Proc Natl Acad Sci U S A 102:12885-90, 2005) by interacting with a ~700 kDa IκB kinases (IKK) complex which consists of IKK1/IKKα, IKK2/IKKβ and NEMO/IKKγ (Chaudhary et al., Oncogene 18:5738-46, 1999; Liu et al., J Biol Chem 277:13745-51., 2002). HTLV1-encoded Tax protein is also known to activate the NF-κB pathway by binding to NEMO/IKKγ. Tax has been also shown to immortalize T cells (Ren et al., J Biol Chem 287:34683-93, 2012; Robek and Ratner, J Virol 73:4856-65, 1999).

Human peripheral blood mononuclear cells (PBMC), CD3+ve T cells and CD3-ve cells (designated B cells) were cultured in XVIVO medium (Lonza) supplanted with 10ng/ml soluble anti-CD3, 10 ng/ml soluble anti-CD28 and 100 IU recombinant human-IL2. Cells were cultured at 37°C, in a 5% CO2 humidified incubator, and after 1 day infected with an empty lentiviral vector (pLENTI) carrying blasticidin resistance gene and lentiviral vectors expressing C-terminal Flag-Tagged K13 (pLENTI-K13-FLAG), a deletion mutant of K13 (pLENTI-K13Δ45-48-FLAG) that is known to lack NF-κB activity (Matta et al., Oncogene 27:5243-53, 2008), and HTLV1-Tax protein (pLENTI-Tax). The nucleotide and protein sequences of K13 are provided in SEQ ID NOs: 866 and 2629, respectively. Approximately 1 day post-infection, cells were selected with blasticidin. After selection, the cells were grown in XVIVO medium and IL2 withdrawn. **Table** 26 shows that both PBMC and T cells infected with K13-Flag and HTLV1-Tax encoding vectors continued to proliferate in culture long after IL2 withdrawal, while no long term proliferating cells were obtained with cultures infected with empty lentiviral vector or those infected with K13Δ45-48-FLAG encoding vector. These results demonstrate that similar to HTLV1-Tax, K13 can promote long term proliferation and immortalization of primary T cells and PBMC and its NF-κB activity is essential for this process.

**Table 26**

| Table K13-induced immortalization of primary lymphocytes | | | |
|---|---|---|---|
| | PBMCs | T-cells | B-cells |
| Vector | - | - | - |
| K13 | + | + | - |
| K13-△45-48 | - | - | - |
| HTLV-Tax | + | + | - |

Expression of K13-FLAG in the surviving culture of T cells was examined by immunofluorescence staining with FITC-conjugated FLAG antibody followed by FACS analysis. **Fig. 23** shows increased staining with FITC-FLAG antibody in K13-expressing T cells as compared to uninfected T cells. The immunophenotype of K13 immortalized T cells was examined by staining with CD4 and CD8 antibodies. **Fig. 23** shows that K13 immortalized T cells are CD8+/CD4- (49%), CD8-/CD4+ (21%) and CD8+/CD4+ (29%). In contrast, a majority of uninfected T cells persisting in culture were CD8+/CD4- (94%). There was no significant difference in the expression of CD69 between uninfected and K13-immortalized T cells.

### FMC63 CAR expressing K13 retain the capacity to induce lysis of RS4;11 target cells

T cells were infected with lentiviral vectors encoding GFP, CD8SP-FMC63-(vL-vH)-Myc-BBz-T2A-EGFP(070814-D06)[SEQ ID NO:3535], CD8SP-FMC63-(vL-vH)-Myc-BBz-T2A-PAC(112014-A13)[SEQ ID NO:1467], and CD8SP-FMC63-(vL-vH)-Myc-BBz-P2A-K13-Flag-T2A-PAC(111014-Y11)[SEQ ID NO:1197]. The cells infected with PAC containing vectors were selected with puromycin or left unselected. **Fig. 24** shows that CD8SP-FMC63-(vL-vH)-Myc-BBz-T2A-PAC(112014-A13)[SEQ ID NO:1467], and CD8SP-FMC63-(vL-vH)-Myc-BBz-P2A-K13-Flag-T2A-PAC(111014-Y11)[SEQ ID NO:1197]-expressing T cells that have been selected with puromycin induce specific lysis of RS411 cells as measured by Gluc assay.

### T cells expressing CAR containing K13, MC159 or Tax2 induce specific lysis of RAJI-Gluc cells

Human peripheral blood T cells isolated using CD3 magnetic beads were infected with lentiviruses expressing the following constructs targeting CD19. The features of the constructs are as follows:
CD8SP-FMC63-(vL-vH)-Myc-BBz-T2A-PAC(112014-A13)[SEQ ID NO:1467]: A standard FMC63 based conventional CAR II containing a 41BB costimulatory domain and a CD3z domain separated by a T2A sequence from a Puromycin resistance gene.

CD8SP-FMC63-(vL-vH)-Myc-z-T2A-PAC(111214-D05)[SEQ ID NO:3538]: A FMC63-based conventional CAR I that lacks a 41BB co-stimulatory domain and contains a CD3z activation domain.

CD8SP-FMC63-(vL-vH)-Myc-BBz-P2A-K13-Flag-T2A-PAC(111014-Y11)[SEQ ID NO:1197]: A standard FMC63 based conventional CAR II containing a 41BB costimulatory domain and a CD3z domain, a P2A cleavable linker, a K13-Flag sequence, a T2A cleavable linker, and a Puromycin resistance gene.

CD8SP-FMC63-(vL-vH)-Myc-z-P2A-K13-Flag-T2A-PAC(111614-B05)[SEQ ID NO:927]: A standard FMC63 based conventional CAR I containing a CD3z domain, a P2A cleavable linker, a K13-Flag sequence, a T2A cleavable linker, and a Puromycin resistance gene.

CD8SP-FMC63(vL-vH)-Myc-BBz-P2A-MC159-Flag-T2A-PAC(111014-Z13)[SEQ ID NO:1751]: A standard FMC63 based conventional CAR II containing a 41BB costimulatory domain and a CD3z domain, a P2A cleavable linker, a MC159-Flag sequence, a T2A cleavable linker, and a Puromycin resistance gene.

CD8SP-FMC63(vL-vH)-Myc-z-P2A-MC159-Flag-T2A-PAC(112614-C06)[SEQ ID NO:1750] A standard FMC63 based conventional CAR I containing a CD3z domain, a P2A cleavable linker, a MC159-Flag sequence, a T2A cleavable linker, and a Puromycin resistance gene.

CD8SP-FMC63(vL-vH)-Myc-BBz-P2A-Flag-HTLV2-TAX-RS-T2A-PAC(010100-N01)[SEQ ID NO:1752]: A standard FMC63 based conventional CAR II containing a 41BB costimulatory domain and a CD3z domain, a P2A cleavable linker, an RS mutant of HTLV2-encoded TAX, a T2A cleavable linker, and a Puromycin resistance gene.

Cells were left unselected or selected with puromycin and expanded. RAJI cells stably expressing Gluc were cocultured with T cells expressing the CARs at the Effector: Target (E:T) ratio of 10:1 for 4 hours. CAR-T cells mediated induction of lysis of target cells was assayed by increase of GLuc activity. **Fig. 25** shows increase in GLuc activity, indicating lysis of target cells, following co-culture with T cells expressing CD19-specific CARs as compared to uninfected T cells (T-UI) or those expressing the control CAR targeting 4C3 (SEQ ID NO: 1643). These results demonstrate that incorporation of K13, MC159 or HTLV2-Tax (Tax2) does not negatively impact the killing ability of CARs with or without the presence of 41BB domain.

The above experiment was repeated at E:T ratio of 1:1 and cell death was assayed after 48h. **Fig. 26** confirms that incorporation of K13, MC159 or HTLV2-Tax (Tax2) does not negatively impact the killing ability of CARs with or without the presence of 41BB domain.

The experiment was repeated with cells that had been selected with puromycin and were in culture for approximately 2 months. **Fig. 27** shows that the T cells expressing CAR containing MC159 and HTLV2-Tax continue to exert cytotoxicity after 2 months in culture while the T cells expressing the other CARs lost this ability.

### In vivo efficacy of CAR T cells containing vFLIPs and Tax

Human peripheral blood T cells isolated using CD3 magnetic beads were infected with lentiviruses expressing the indicated CAR constructs targeting CD19. NSG mice (Jackson Lab) were sub-lethally irradiated at a dose of 175 cGy. 24 hours post irradiation (day2), mice were injected with 2.5 x 10⁴ RAJI cells via tail-vein. On day3, the mice (n =3 for each group) were treated with 1 million T cells that had been infected with the CAR encoding lentiviruses. **Table 27** shows the survival of mice in each group. These results demonstrate that highest survival (38 days) is observed in mice injected with T cells expressing FMC63 based CAR CD8SP-FMC63-(vL-vH)-Myc-z-P2A-K13-Flag-T2A-PAC(111614-B05)[SEQ ID NO:927] that lacks 41BB costimulatory domain but expresses K13.

**Table 27**

| **CAR** | **Median Survival** |
|---|---|
| CD8SP-FMC63-(vL-vH)-Myc-BBz-T2A-PAC(112014-A13)[SEQ ID NO: 1467] | 32 |
| CD8SP-FMC63-(vL-vH)-Myc-z-T2A-PAC(111214-D05)[SEQ ID NO:3538] | 32 |
| CD8SP-FMC63-(vL-vH)-Myc-BBz-P2A-K13-Flag-T2A- PAC(111014-Y11)[SEQ ID NO:1197] | 32 |
| CD8SP-FMC63-(vL-vH)-Myc-z-P2A-K13-Flag-T2A-PAC(111614- B05)[SEQ ID NO:927] | 38 |
| CD8SP-FMC63(vL-vH)-Myc-BBz-P2A-MC159-Flag-T2A- PAC(111014-Z13)[SEQ ID NO:1751] | 24 |
| CD8SP-FMC63(vL-vH)-Myc-z-P2A-MC159-Flag-T2A- PAC(112614-C06)[SEQ ID NO:1750] | 20 |
| CD8SP-FMC63(vL-vH)-Myc-BBz-P2A-Flag-HTLV2-TAX-RS-T2A- PAC(010100-N01)[SEQ ID NO:1752] | 25 |
| Control | 20 |

### Inducible expression of K13 by fusion with FKBP

To control the expression of K13, different vectors were generated expressing K13 in fusion with one or two copies of N-terminal FKBP-based switch or dimerization domains. In addition, a construct was constructed in which 2 copies of FKBP domain were fused at the N-terminal with a myrisotylation signal and at C-terminus with K13. The above constructs of K13 were either expressed alone or in constructs expressing different CAR constructs.

NF-κB reporter assay was carried out essentially as described (Chaudhary et al., Oncogene 18:5738-46, 1999). The indicated constructs were transiently transfected in 293FT cells along with a luciferase reporter construct driven by 4 copies of NF-κB binding site and a RSV-LacZ reporter construct. Cells were left untreated or treated with AP20187, which dimerizes the FKBP domains, resulting in activation of K13 signaling. Approximately 24hr later, cells were lysed and NF-κB-Luc and LacZ activities measured in cell lysates. NF-κB-Luc activity was normalized relative to LacZ activity to control for difference in transfection efficiency. As shown in **Fig. 28A****,** treatment with AP20187 (100 nM) led to increase in K13-induced NF-κB-Luc activity in all constructs in which K13 was expressed in fusion with FKBP. Essentially similar results were obtained when RS mutant of HTLV2 encoded Tax was expressed in fusion with FKBP **(****Fig. 28B****).** Thus, activity of K13 and Tax can be controlled by fusion with FKBP and subsequent administration of AP20187 when they are expressed alone or as part of CAR constructs.

### Activation of K13 activity by AP20187 does not impair the ability of CARs to induce cell death

Human peripheral blood T cells isolated using CD3 magnetic beads were infected with lentiviruses expressing the constructs CD8SP-FMC63(vL-vH)-Myc-z-P2A-FKBP-K13-FLAG-T2A-PAC(112316-S06) [SEQ ID NO:1770] and CD8SP-161-(vL-vH)-Myc-z-P2A-FKBP-K13-FLAG-T2A-PAC(112916-U06) [SEQ ID NO: 1741] and selected with puromycin as described previously. RAJI and HEL cells stably expressing Gluc were cocultured with uninfected T cells (UI) or T cells expressing the indicated constructs [SEQ ID NO:1770] and [SEQ ID NO: 1741], respectively, at the Effector: Target (E:T) ratio of 10:1 for 4 hours in the absence and presence of 100nM AP20187 (AP) compound. CAR-T cells mediated induction of lysis of target cells was assayed by increase of GLuc activity. **Fig. 29A and 29B** show that activation of K13-mediated NF-κB activity by addition of AP20187 does not adversely affect cell killing induced by the two CAR constructs that coexpress FKBP-K13.

### Cytotoxic activity of CARs I and II coexpressing K13, MC159, HTLV2-Tax and HTLV2-Tax RS mutant

Human peripheral blood T cells isolated using CD3 magnetic beads were infected with lentiviruses expressing the indicated constructs expressing CAR I and II targeting MPL and coexpressing different accessory modules **(****Fig. 30A****).** Cells were selected with puromycin as described previously. HEL cells stably expressing Gluc were cocultured with uninfected T cells (UI) or T cells expressing the indicated constructs at the Effector: Target (E:T) ratio of 10:1 for 4 hours. CAR-T cells mediated induction of lysis of target cells was assayed by increase of GLuc. **Fig. 30A** shows induction of target cell death by different MPL-directed CAR constructs that coexpress K13, MC159, HTLV2-Tax and HTLV2-Tax RS mutant.

Essentially a similar experiment was conducted with a construct (SEQ ID NO: 983) expressing CAR I targeting CD32 and coexpressing K13. **Fig. 30B** shows effective induction of HEL cell death by T cells expressing this construct as measured by Gluc assay.

### Cytotoxic activity of Backbone 1 constructs comprising a conventional CARs I targeting TROP2 and coexpressing K13

Human peripheral blood T cells isolated using CD3 magnetic beads were infected with lentiviruses expressing the construct CD8SP-TROP2-h7E6-SVG-(vL-vH)-Myc-z-P2A-K13-Flag-T2A-PAC(052616-D04)[SEQ ID NO:1166] targeting TROP2 and selected with puromycin and tested for the ability to kill PC3 (prostate cancer) cells expressing GLuc using the assay described previously. **Fig. 31** shows effective killing of PC3 cells by T cells expressing the CD8SP-TROP2-h7E6-SVG-(vL-vH)-Myc-z-P2A-K13-Flag-T2A-PAC(052616-D04)[SEQ ID NO:1166] construct.

### Cytotoxic activity of Backbone 1 constructs comprising a conventional CARs I targeting LAMP1 and coexpressing K13

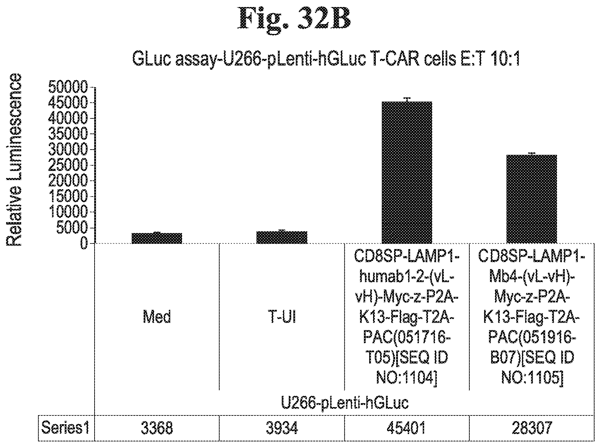

Human peripheral blood T cells isolated using CD3 magnetic beads were infected with lentiviruses expressing the indicated constructs targeting LAMP1 and selected with puromycin. Cells were tested for the ability to kill L363 and U266 cells expressing GLuc using the assay described previously. **Fig. 32A** **and** **32B** shows effective killing of L363 and U266 cells by T cells expressing the CD8SP-LAMP1-humabl-2-(vL-vH)-Myc-z-P2A-K13-Flag-T2A-PAC(051716-T05)[SEQ ID NO:1104] and CD8SP-LAMP1-Mb4-(vL-vH)-Myc-z-P2A-K13-Flag-T2A-PAC(051916-B07)[SEQ ID NO:1105] constructs.

### Activation of K13 signaling by addition of AP20187 in Jurkat cells expressing a CD19-directed CAR and coexpressing FKBP-K13fusion protein

The effect of induction of K13 induced NF-κB activity on CAR-induced NFAT signaling was studied by stably expressing the construct CD8SP-FMC63(vL-vH)-Myc-z-P2A-FKBP-K13-FLAG-T2A-PAC(112316-S06)[SEQ ID NO:1770], which encodes a conventional CAR I targeting CD19 and expresses an FKBP-K13-FLAG fusion protein, in Jurkat-NFAT-GFP cells (JP). The parental Jurkat-NFAT-GFP cells (JP) cells and those expressing the construct CD8SP-FMC63(vL-vH)-Myc-z-P2A-FKBP-K13-FLAG-T2A-PAC(112316-S06)[SEQ ID NO:1770] were cocultured with RAJI cells in the absence and presence of 100 nM AP20187 for approximately 18 hours and induction of NFAT-driven GFP expression examined by FACS analysis as described previously. **Fig. 33** shows effective induction of GFP expression in Jurkat cells expressing the CD8SP-FMC63(vL-vH)-Myc-z-P2A-FKBP-K13-FLAG-T2A-PAC(112316-S06)[SEQ ID NO:1770] construct upon coculture with RAJI cells in the absence (38.7% GFP-expressing cells) and presence (45.85% GFP expressing cells) of AP20187 compound. In contrast, no significant GFP induction was observed upon co-culture of parental Jurkat-NFAT-GFP cells (JP) cells with RAJI cells either in the absence or presence of the AP20187 compound. These results demonstrate that activation of K13 signaling in T cells expressing the FKBP-K13 fusion protein by addition of AP20187 compound does not adversely affect activation of CAR-induced NFAT signaling.

### Generation of K13 mutants with varying level of NF-κB activity

It would be useful to have novel mutants of K13 with varying levels of NF-κB activity for the purpose of adoptive cellular therapy and other applications. Site-directed mutagenesis was conducted using Quickchange site-directed kit (Stratagene) to generate several point mutants of K13 in which the different amino acids of K13 (SEQ ID NO:2629) were mutated. For example, in the mutant K19A the amino acid Lysine at position 19 is mutated to alanine. The wild type and the various mutant constructs were transfected in 293FT cells along with a luciferase reporter construct driven by 4 copies of NF-κB binding site and a RSV-LacZ reporter construct essentially as described (Chaudhary et al., Oncogene 18:5738-46, 1999) . Approximately 24hr later, cells were lysed and NF-κB-Luc and LacZ activities measured in cell lysates. NF-κB-Luc activity was normalized relative to LacZ activity to control for difference in transfection efficiency (Chaudhary et al., Oncogene 18:5738-46, 1999). **Fig. 34** shows that many mutants have either increase (e.g. E48A mutant) or decrease (e.g., E47A mutant) in NF-κB activity as compared to the wild-type K13. These mutants will have application in adoptive cellular therapy and other applications where varying level of NF-κB activities are required. Thus, mutant E48A can be coexpressed in CAR constructs where strong NF-κB activity is desirable while mutant R50A can be coexpressed in CAR constructs where only modest NF-κB activity is desirable. The activity of these mutants can be also controlled by fusing them with FKBP domains (SEQ ID NOs: 2621 and 2622) as described above for wild-type K13.

### A method of controlling the activity of CAR-T cells, Bispecific T cell engagers (BiTEs) and DARTs (Dual Affinity Re-targeting proteins) with Src Inhibitors Dasatinib, Ponatinib and A-770041

### CAR

CARs are synthetic receptors which combine antibody binding specificity with T cell functionality. CARs are generated by fusing a single chain antibody (scFv) that dictates the specific recognition of desired tumor antigen with the intracellular signaling components of CD3z chain of T cell receptor to enable activation of T cell effector function. The HLA- independent recognition of TAA by CAR not only minimize tolerance induction mechanisms, but also allow the versatile design of CARs recognizing virtually any antigen including non-protein moieties on cell surface. Although CARs activate T cells via the CD3z chain, signaling via CARs is different from signaling via the normal T cell receptor (TCR) as they do not engage all the components of the TCR complex. For example, signaling via a normal TCR results in engagement of several molecules, such as TCRα, TCRβ, CD3γ, CD3δ, CD3e, CD3ζ, and CD4 or CD8 as well as their associated downstream signaling proteins, such as Lck, LAT, ZAP70 and SLP-76 (Chakraborty, AK and Weiss, A Nat Immunol 15:798-807,2014). It is unclear if any of these receptors and signaling intermediates are involved in signaling via a chimeric antigen receptor (CAR). The notion that signaling via CAR is distinct from signaling via a normal TCR is evident from the side effects observed following the infusion of CAR versus those observed after infusion of TCR in patients. While cytokine release syndrome (CSR) and neurological complications are frequently observed following the infusion of CAR-T cells, they are not seen following the infusion of tumor infiltrating lymphocytes or after infusion of T cells that had been engineered to express a TCR against a particular antigen, such as NY-ESO (Brentjens, Blood 118:4817-28, 2011; Rapoport et al., Nat Med 21:914-21, 2015; Robbins et al., Clin Cancer Res 21:1019-27, 2015; Sadelain et al., Curr. Opin. Immunol. 21:215-23, 2009; Turtle et al., Curr. Opin. Immunol. 24:633-39, 2012).

### Bispecific T cell engager (BiTE)

Bispecific T cell engager (BiTE^{®}) antibody constructs are a type of immunotherapy being investigated for fighting cancer by helping the body's immune system to detect and target malignant cells. The modified antibodies are designed to engage two different targets simultaneously, thereby juxtaposing T cells (a type of white blood cell capable of killing other cells perceived as threats) to cancer cells. BiTE^{®} antibody constructs help place the T cells within reach of the targeted cell, with the intent of allowing T cells to inject toxins and trigger the cancer cell to die (apoptosis). BiTE^{®} antibody constructs are currently being investigated for their potential to treat a wide variety of cancers. BLINCYTO (Blinatumomab) is a bispecific CD19-directed CD3 T cell engager (BiTE^{®}) antibody construct that binds specifically to CD19 expressed on the surface of cells of B-lineage origin and CD3 expressed on the surface of T cells. Although BiTEs, such as Blinatumomab, activate T cells, they bind to the CD3 chain of TCR rather than TCRα and TCRβ chains, and therefore signaling through BiTEs is not physiological and is not identical to signaling via the normal T cells.

### Limitation of CAR T cells and Bispecific antibodies

In majority of patients who respond to engineered CAR T cells and Blinatumomab, excessive release of proinflammatory cytokines causes symptoms that include fevers, hypotension, hypoxemia, cardiac dysfunction, kidney failure and electrolyte abnormalities, collectively termed as "Cytokine release syndrome' (CRS). A number of these patients require admission to intensive care units for pressure support and artificial ventilation. In significant number of cases (up to 50%), CAR T cells and Blinatumomab therapy can lead to neurologic symptoms including tremor, seizures and can be fatal. For example, approximately 50% of patients receiving Blinatumomab in clinical trials experienced neurological toxicities. Severe, life-threatening, or fatal neurological toxicities occurred in approximately 15% of patients, including encephalopathy, convulsions, speech disorders, disturbances in consciousness, confusion and disorientation, and coordination and balance disorders. Strategies to counteract CRS include treatment with immunosuppressive agents and antibodies to cytokines to block cytokine release and/or activity. Nevertheless, treatment-related deaths have occurred at many institutions. Therefore, there is an unmet and urgent need for new drugs/agents to control the activity of CAR T cells and Blinatumomab.

### Dasatinib as an inhibitor of physiological T cell and NK function

The dual BCR-ABL/Src kinase inhibitor dasatinib is FDA approved for the treatment of chronic myeloid leukemia and Ph+ acute lymphoblastic leukemia. Src kinases are known to play an important role in physiological T-cell activation. Consistent with this, dasatinib has been shown to profoundly inhibit antigen specific physiological T-cell activation, proliferation, cytokine production, and degranulation in a dose-dependent manner (Schade et al., Blood 111:1366-77, 2008; Weichsel et al., Clin Cancer Res 14:2484-91, 2008). Dasatinib has been also shown to inhibit the activation of NK cells. However, since the CAR modified T cells and BiTE do not engage the physiological T cell receptor (see above), it is not clear whether dasatinib can be used to modulate the activity of CAR-T cells and T cells activated via BiTE.

Human peripheral blood T cells isolated using CD3 magnetic beads were infected with lentiviruses expressing the indicated CAR construct targeting CD8SP-2-CD19MM-(vL-vH)-Myc-BBz-T2A-PAC(062915-D03)[SEQ ID NO:1471] and selected with puromycin. CAR-T cells were pre-incubated with the indicated concentrations of Dasatinib and Imatinib for approximately 30 min at 37°C. The drug-treated and untreated T cells were plated in a white 384-cell plate. RAJI cells stably expressing GLuc were added to the wells containing the T cells at a concentration of 30K cells/30µl/ well to give an E:T ratio was 5:1. Dasatinib (Das) and Imatinib (Imat) were added to the wells to maintain the final concentrations as indicated. After 4 h of co-culture, CAR-T cells mediated induction of lysis of target cells was assayed by increase of GLuc activity by directly injecting 0.5X CTZ assay buffer containing native coeloentrazine. **Fig. 35** shows inhibition of CAR CD8SP-2-CD19MM-(vL-vH)-Myc-BBz-T2A-PAC(062915-D03)[SEQ ID NO:1471]-induced cell death by 50nM and 100nM Dasatinib, while Imatinib has no effect.

### Effect of different Tyrosine Kinase inhibitors on cell death induced by CAR modified T cells

Human peripheral blood T cells isolated using CD3 magnetic beads were infected with lentiviruses expressing the indicated CAR constructs targeting human andCD8SP-FMC63-(vL-vH)-Myc-BBz-T2A-PAC(112014-A13)[SEQ ID NO:1467] and CD8SP-2-CD19MM-(vL-vH)-Myc-BBz-T2A-PAC(062915-D03)[SEQ ID NO:1471]). RAJI cells stably expressing GLuc were co-cultured for approximately 4 h with T cells expressing the CARs in the presence and absence of indicated concentrations of the tyrosine kinase inhibitors (TKI) Dasatinib, Imatinib, Nilotinib, Ponatinib and Axitinib essentially as described above. Among these inhibitors, Dasatinib and Ponatinib have been reported to have activity against Src kinase, while the others lack this activity. CAR-T cells mediated induction of lysis of target cells was assayed by increase of GLuc activity as measured by BioTek synergy plate reader by directly injecting 0.5X CTZ assay buffer containing native coeloentrazine (Nanolight). For measurement of maximum cell death, cells were treated with 30µl of digitonin (final concentration 30 µg/ml) for approximately 90 min prior to measurement of Gluc activity. The % specific lysis was calculated by taking digitonin-induced cell death as 100% and untreated cells as 0%. The formula for % specific lysis calculation is: % Specific lysis = 100 x [(experimental data) - (spontaneous cell death))] / [(maximum cell death)-spontaneous cell death)]. **Fig. 36** shows increase in GLuc activity following co-culture with T cells expressing CD19-specific CARs CD8SP-FMC63-(vL-vH)-Myc-BBz-T2A-PAC(112014-A13)[SEQ ID NO:1467] and CD8SP-2-CD19MM-(vL-vH)-Myc-BBz-T2A-PAC(062915-D03)[SEQ ID NO:1471]as compared to the uninfected T cells (T-UI) indicating lysis of target cells by the different CD19-CAR-T cells. However, CD19-CAR-T cells induced cell death was partially blocked by treatment with 50nM Dasatinib and completely blocked by treatment with 100nM Dasatinib. Similarly, CD19-CAR-T cells-induced cell death was partially blocked by treatment with 100 nM Ponatinib. However, treatment with the indicated doses of Imatinib (50nM and 500nM), Nilotinib (10nM and 100nM), and Axitinib (1µM and 10µM) had no effect on CAR-T cell induced cell death. These results demonstrate that TKI with activity against Src can effectively block CAR-T cell induced cell death. It is also possible that treatment with higher doses of Imatinib and Nilotinib than those used in the above study could block CAR-T cell-induced cell death.

### Dasatinib blocks cell death induced by CAR targeting CD22

Human peripheral blood T cells isolated using CD3 magnetic beads were infected with lentivirus expressing the a CAR construct targeting human CD22 (CD8SP-CD22-m971-(vL-vH)-Myc-BBz-T2A-PAC(091515-A02)[SEQ ID NO:1519]) and selected with puromycin. The effect of dasatinib on puromycin selected CD22 CAR-T cells induced death of RAJI-GLuc cells was examined after 96 h of co-culture of effector and target cells. **Fig. 37** shows that Dasatinib effectively blocks killing of RAJI-GLuc cells by T cells expressing the CD8SP-CD22-m971-(vL-vH)-Myc-BBz-T2A-PAC(091515-A02)[SEQ ID NO:1519] CAR as determined by GLuc assay.

### Dasatinib blocks cytotoxicity induced by parental NK92MI cells and those modified to express a CAR

NK92 cells are under clinical development for the treatment of a number of cancers. To test if Dasatinib can block the activity of NK92 cells, NK92MI cells (a derivative of NK92 cells expressing IL2) were co-cultured with K562-Gluc cells in the absence and presence of indicated concentrations of Dasatinib. The target cells were plated a concentration of 100K cells/100µl/ well in a black 96-cell plate and either left untreated or treated with two different doses of Dasatinib (50nM and 100nM) for 30 min followed by co-culture of target cells with NK92MIMI effector cells at an E:T ratio of 0.5:1 for 4 h. **Fig. 38A** shows that Dasatinib blocks NK92 cells mediated death of K562 cells as determined by Gluc assay.

To test if Dasatinib would also block cell death induced by NK92 cells which have been engineered to express a CAR, NK92MI cells were engineered to express CAR CD8SP-FMC63-(vL-vH)-Myc-BBz-T2A-EGFP(070814-D06)[SEQ ID NO:3535] targeting CD19. NK92MI cells expressing the CD8SP-FMC63-(vL-vH)-Myc-BBz-T2A-EGFP(070814-D06)[SEQ ID NO:3535] CAR were co-cultured with CD19 positive RAJI-GLuc target cells in the absence and presence of Dasatinib for 4 h and cell death measured by GLuc assay. **Fig. 38B** shows that Dasatinib blocks death of RAJI-GLuc cells by CD8SP-FMC63-(vL-vH)-Myc-BBz-T2A-EGFP(070814-D06)[SEQ ID NO:3535] CAR-expressing NK92MI cells as determined by Gluc assay.

### Dasatinib blocks Blinatumomab (Blincyto) induced cell death and cytokine production

Effector T-cells (isolated by CD3 microbeads from Miltenyi Biotech) were left untreated or preincubated with 50nM or 100nM Dasatinib for 1 hour at 37°C followed by plating cells in a U-bottom 96-well plates. RAJI-MSCVhygro-Gluc target cells were incubated with a bispecific antibody (Blinatumomab or BLINCYTO; AMGEN) at 100ng/10⁶ cells for 30 min at 37°C in PBS/2% FBS. Next, Blinatumomab-treated RAJI-Gluc or untreated (control) RAJI-Gluc cells were washed three times in PBS, re-suspended in XVIVO medium and 100 µl (100K) cells were plated per well of a 96-well U-bottom plate (Effector:Target ratio of 1:1) in the absence and presence of indicated concentrations of Dasatinib. Total volume in each well was adjusted to 200 µl with medium. Plates were quickly centrifuged to settle cells, and incubated at 37°C in a 5% CO₂ incubator for 96 hours. For maximum cell death, one hour prior to doing GLuc cell death assay, a total volume of 6 µl of Digitonin (stock 1mg/ml, Sigma) was added to wells containing target cells alone to achieve a final concentration of 30µg/ml of Digitonin. The plates were centrifuge at 1,000 rpm for 5 min and 50µl of supernatants were transferred to a Greiner 384-well flat bottom white opaque plate. The luciferase activity was measured by BioTek synergy plate reader by directly injecting 0.5X CTZ assay buffer containing native coeloentrazine (Nanaolight) into the 384 well plates in a well mode. **Fig. 39A** shows that treatment with Blinatumomab in the presence of T cells result in strong induction of death of RAJI-GLuc cells which is effectively blocked by 100nM Dasatinib. The supernatant from the above experiment was also assayed for measurement of IFNγ production by ELISA. **Fig. 39B** shows that co-culture of RAJI-GLuc cells with Blinatumomab in the presence of T cells result in strong induction of IFNγ production which is effectively blocked by 100nM Dasatinib.

### Effect of Ruxotitinib, Fosatamatinib and Alisertib on CAR T cells and Blinatumomab induced cell death

The effect of Ruxolitinib (JAK-1/2 inhibitor), Fosatamatinib (syk inhibitor) and Alisertib (Aurara Kinase inhibitor) on CD8SP-CD19Bu12-(vL-vH)-Myc-BBz-T2A-PAC(082815-P08)[SEQ ID NO:1470] -expressing CAR T cells- and Blinatumomab-induced death of RAJI-pLenti-GLuc cells was studied using the assay as described above. **Fig. 40A** shows no significant toxic effect of the various drugs on RAJI-pLenti-Gluc cells that had not been co-cultured with CAR T cells. **Fig. 40B** shows significant induction of cell death, as measured by increase in Gluc activity, when RAJI--pLenti-Gluc cells are incubated with the CAR-T cells. Fig. 40B also shows mild to modest inhibition of -CAR-T cell-induced death of RAJI-pLenti-GLuc cells by Ruxolitinib, Fosatamatinib and Alisertib as compared to cells treated with media (Med) alone. Dasatinib was used as a positive control and led to significant inhibition of CAR-T cell-induced death of RAJI cells. **Fig. 40C** shows that the above compound have only a minimal effect on Blinatumomab induced cell death mediated by T cells at the indicated concentrations.

### Effect of A-770041, Saracatinib, Avasimibe on CAR T cells- and Blinatumomab-induced cell death

A-770041 is a selective small molecule inhibitor of LCK, one of the 8 members of the src family, that is required for T cell activation and IL2 production (Burchat et al., Bioorg Med Chem Lett 16:118-22, 2006). A770041 is a 147 nM inhibitor of Lck (1 mM ATP) and is 300 fold selective against Fyn, the other src family kinase involved in T cell signaling. Saracatinib (AZD0530) is a src family that can be administered orally. Avasimibe is an inhibitor of ACAT1, a key cholesterol esterification enzyme that has been shown to augment anti-tumor response of CD8 cells.

The effect of A-770041, Saracatinib and Avasimibe on CD19Bu12(vL-vH)-BBz-Pac-P08 expressing CAR T cells- and Blinatumomab-induced death of RAJI-pLenti-GLuc cells was studied next using the assay as described above. **Fig. 41A** shows the release of Gluc in cells treated with the indicated drugs in the presence of media alone without any CAR-T cells. **Fig.41B** shows near complete inhibition of CD8SP-CD19Bu12-(vL-vH)-Myc-BBz-T2A-PAC(082815-P08)[SEQ ID NO:1470] CAR-T cells induced death of RAJI-pLenti-GLuc cells by A-770041 at 100nM and 200nM while Saracatinib had no significant effect. Dasatinib was used as a positive control in the above experiment and as expected blocked CAR-T cells induced cell death. Avasimibe had no significant effect on CAR-T cells induced cell death at 100nM but completely blocked it at 1µM. **Fig. 41C** shows that treatment with 100nM A770041 partially blocked cell death induced by combination of Blinatumomab with T cells while treatment with 200nM A770041 led to complete inhibition. Similarly, treatment with 1µM Avasimibe blocked Blinatumomab induced cell death mediated by T cells. Collectively, the above studies demonstrate that inhibitors of LCK kinase can block the effect of CAR-T cells and Blinatumomab and therefore can be used for the treatment of cytokine release syndrome and neurological complications associated with treatment with CAR-T cells and Blinatumomab.

### Generation of CAR constructs coexpressing scFv targeting IL6R and IL6R

One of the major limitations of CAR therapy is cytokine release syndrome (CRS). One approach currently being used in clinical practice to control CRS is administration of anti-IL-6R monoclonal antibody tocilizumab. To control CRS associated with CAR, we constructed exemplary CARs against CD19 (CD8SP-FMC63(vL-vH)-BBz-P2A-IgHSP-IL6R-M83(vL-vH)-Flag-T2A-PAC(011315-K04)[SEQ ID NO:1763]) ) and MPL (CD8SP-161(vL-vH)-BBz-P2A-IgHSP-IL6R-M83(vL-vH)-Flag-T2A-EGFP(012015-I29)[SEQ ID NO:1746])) which expresses a scFv against IL6R, designated M83, along with a C-terminal Flag tag. To control CRS, we also constructed exemplary CARs against CD19 (CD8SP-FMC63(vL-vH)-BBz-P2A-IgHSP-IL6-19A(vL-vH)-Flag-T2A-PAC(011315-H06)[SEQ ID NO:1762]) and MPL (CD8SP-161(vL-vH)-BBz-P2A-IgHSP-IL6-19A(vL-vH)-Flag-T2A-EGFP(011315-F02)[SEQ ID NO:1745]) which co-expresses an scFv against IL6, designated IL6-19A. The scFv fragments in these constructs are separated from the CAR fragment by a P2A cleavable linker. Jurkat-NFAT-EGFP (J-N-G) cells were stably transduced with the lentivirus encoding the CAR constructs CD8SP-FMC63(vL-vH)-BBz-P2A-IgHSP-IL6-19A(vL-vH)-Flag-T2A-PAC(011315-H06)[SEQ ID NO:1762] and CD8SP-FMC63(vL-vH)-BBz-P2A-IgHSP-IL6R-M83(vL-vH)-Flag-T2A-PAC(011315-K04)[SEQ ID NO:1763]. The parental and CAR-expressing Jurkats were subsequently cocultured with RAJI cells and induction of EGFP expression monitored by FACS analysis after 4 h. **Fig. 42A** shows that coculturing of Jurkat cells expressing CD8SP-FMC63(vL-vH)-BBz-P2A-IgHSP-IL6-19A(vL-vH)-Flag-T2A-PAC(011315-H06)[SEQ ID NO:1762] with RAJI led to increase in EGFP expression as compared to cells that had not been exposed to RAJI cells. Essentially similar results were obtained with Jurkats expressing the CAR construct CD8SP-FMC63(vL-vH)-BBz-P2A-IgHSP-IL6R-M83(vL-vH)-Flag-T2A-PAC(011315-K04)[SEQ ID NO:1763]. No increase in EGFP expression was observed in parental Jurkats (J-N-G-P) without or with culturing with RAJI **(****Fig. 42B****).** Thus, a CAR can be coexpressed with a scFV against IL6 and IL6R from a single vector without loss of activity.

Due to their small size, one of the limitations of the scFv is their short half-life due to their rapid clearance by the kidneys. Therefore, we also constructed a CAR against MPL (161) that expresses a bispecific antibody consisting of a camel-derived IL6R single chain antibody (vHH) designated 304 which is fused via a (GGGGS)x3 linker to a single chain camel-derived antibody (vHH) against human serum albumin (Alb8). The IL6R-Alb8 cassette carries a human IgH signal peptide at the N-terminus and a Flag epitope tag at the C-terminus. The secreted bispecific IL6R-304-Alb8 antibody will bind to human serum albumin through its Alb8 region, which will prevent its excretion via the kidneys and thereby prolong its half-life.

### CAR expressing Fx06 peptide to block increased vascular leakiness during CRS

Capillary leak syndrome is another major complication of CARs. A natural plasmin digest product of fibrin, peptide Bβ15-42 (also called FX06), has been shown to significantly reduce vascular leak (Groger et al., PLoS ONE 4:e5391, 2009). We generated vectors that coexpress CARs targeting CD19 or MPL with FX06 peptide. To facilitate extracellular secretion of FX06, a signal peptide derived from human IgH was fused to its N-terminus. The coexpression of FX06 peptide with CARs will help ameliorate the capillary leak syndrome seen as part of cytokine release syndrome following infusion of CAR modified T cells.

Jurkat-NFAT-EGFP cells were stably transduced with the lentivirus CD8SP-FMC63-(vL-vH)-BBz-P2A-IgHSP-Fx06-Flag-T2A-PAC(012115-N04)[SEQ ID NO:1764] that expresses FMC63 CAR against CD19 along with FX06. The parental and CAR expressing Jurkats were subsequently cocultured with RAJI cells and induction of EGFP expression monitored by flow cytometry. Coculturing of Jurkat-NFAT-EGFP cells expressing CD8SP-FMC63-(vL-vH)-BBz-P2A-IgHSP-Fx06-Flag-T2A-PAC(012115-N04)[SEQ ID NO:1764] with RAJI led to increase in EGFP expression as compared to cells that had not been exposed to RAJI cells. Essentially similar results were obtained upon culture with Bv173 and NALM6 cells. Thus, a CAR can be expressed along with FX06 from a single vector without loss of activity.

### CARs targeting multiple diverse antigens

We next generated lentiviral constructs encoding for CARs targeting many diverse antigens that are overexpressed in cancer and other diseased causing or disease associated cells. The lentiviruses were generated and used to infect the Jurkat-NFAT-EGFP reporter cell line. The Jurkat-NFAT-GFP cells were engineered to express the different CARs and backbone targeting the different antigens and were tested for their ability to activate NFAT-dependent EGFP reporter upon coculture with the target cells expressing their respective target antigen. The Jurkat-NFAT-EGFP (parental) cells were used as control. The results with different CARs are summarized in the following summary **Table** 28. A CAR is considered positive in the assay in case the CAR-expressing Jurkat-NFAT-GFP cells show greater % GFP positive cells when cultured with the target cell line as compared to parental Jurkat-NFAT-GFP cells. Thus, the Jurkat-NFAT-GFP cells expressing the CAR CD8SP-FMC63(vL-vH)-Myc-BBz-P2A-cFLIP-L-Flag-T2A-PAC(092215-A06)[SEQ ID NO:1754] showed greater induction of EGFP expression when co-cultured with RAJI cell as compared to EGFP expresson induced in parental Jurkat-NFAT-EGFP cells upon co-culture with RAJI cells. These results also demonstrate that cFLIP-L, an agent known to block activation induced cell death, can be co-expressed with a CAR without adversely affecting its signaling activity. The results in **Table** 28 demonstrate the functional activity of several conventional CAR II constructs comprising 41BB costimualatory domain and CD3z activation domain and targeting novel antigens, such as PTK7, DLL3, cMET, TSHR, CD22, Muc1/MHC class I complex, tyrosinase/MHC class I complex. The data in **Table** 28 also demonstrate the functional activity of several backbone 1 constructs comprising a conventional CAR I and K13-vFLIP and targeting novel antigens, such as CD34, CD179b, CLEC5A, CSF2RA, CD200R, LAMP1, TROP2, TnAg, CDH6, CDH17, CDH19, GD3, GFRalpha4, Her2, IL11Ra, IL13Ra2, NYBR-1, Slea, and TGFBR2. Additionally, several bispecific constructs, such as those targeting EGFR and CEA and those targeting cMet and Her3 were positive in this assay. Essentially a similar experimental approach can be used to test the functional activity of other CARs and backbones described in Tables 19-22.

**Table 28: Exemplary CARs positive on Jurkat-NFAT-GFP Assay**

| **No.** | **Target** | **SEQUENCE NAME** | **Positive Cell Line on co-culture assay** |
|---|---|---|---|
| 1 | CD34 | CD8SP-CD34-hu4C7-(vL-vH)-Myc-z-P2A-K13-Flag- T2A-PAC(051616-I02)[SEQ ID NO:992] | HL60 |
| 2 | CD179b | CD8SP-CD179b-(vL-vH)-Myc-z-P2A-K13-Flag-T2A- PAC(051716-N05)[SEQ ID NO:1012] | HL60 |
| 3 | CD200R | CD8SP-CD200R-huDx182-(vL-vH)-Myc-z-P2A-K13- Flag-T2A-PAC(051716-W05)[SEQ ID NO:1190] | HEL |
| 4 | CDH6 | CD8SP-CDH6-NOV710-(vL-vH)-Myc-z-P2A-K13- Flag-T2A-PAC(051716-I05 )[SEQ ID NO:1016] | SKOV3 |
| 5 | CDH7 | CD8SP-CDH6-NOV712-(vL-vH)-Myc-z-P2A-K13- Flag-T2A-PAC(051716-J05)[SEQ ID NO:1017] | SKOV3 |
| 6 | CDH17 | CD8SP-CDH17-PTA001A4-(vL-vH)-Myc-z-P2A-K13- Flag-T2A-PAC(051716-M06)[SEQ ID NO:1018] | LoVo |
| 7 | CDH19 | CD8SP-CDH19-4B10-(vL-vH)-Myc-z-P2A-K13-Flag- T2A-PAC(051716-U05)[SEQ ID NO:1180] | MEL624 |
| 8 | CLEC5A | CD8SP-CLEC5A-3E12A2-(vL-vH)-Myc-z-P2A-K13- Flag-T2A-PAC(051016-F06)[SEQ ID NO:1022] | Kasumi |
| 9 | CSF2RA | CD8SP-CSF2RA-Ab1-(vL-vH)-Myc-z-P2A-K13-Flag- T2A-PAC(051616-G01)[SEQ ID NO:1041] | THP1 |
| 10 | CSF2RA | CD8SP-CSF2RA-Ab6-(vL-vH)-Myc-z-P2A-K13-Flag- T2A-PAC(051616-H05)[SEQ ID NO:1040] | THP1 |
| 11 | EGFR & CEA | CD8SP-EGFR1-vHH-Gly-Ser-Linker-CEA1-vHH- Myc-z-P2A-K13-Flag-T2A-PAC(040716-O08)[SEQ ID NO:1049] | Hela |
| 12 | GD3 | CD8SP-GD3-KM-641-(vL-vH)-Myc-z-P2A-K13-Flag- T2A-PAC(051016-D04)[SEQ ID NO:1065] | MEL624 |
| 13 | GFRa4 | CD8SP-GFRa4-P4-10-(vL-vH)-Myc-z-P2A-K13-Flag- T2A-PAC(051316-L02)[SEQ ID NO:1067] | LAN5 |
| 14 | GFRa4 | CD8SP-GFRa4-P4-10-(vL-vH)-Myc-z-P2A-K13-Flag- T2A-PAC(051716-L05)[SEQ ID NO:1067] | LAN5, TT |
| 15 | Her2 | CD8SP-Her2-Hu4D5-(vL-vH)-Myc-z-P2A-K13-Flag- T2A-PAC(050516-I03)[SEQ ID NO:1084] | MCF7 |
| 16 | IL11Ra | CD8SP-IL11Ra-8E2-Ts107-(vL-vH)-Myc-z-P2A-K13- Flag-T2A-PAC(050516-D01)[SEQ ID NO:1099] | K562 |
| 17 | IL13Ra | CD8SP-IL13Ra2-Hu108-(vL-vH)-Myc-z-P2A-K13- Flag-T2A-PAC(050516-F04)[SEQ ID NO:1102] | U87MG |
| 18 | LAMP1 | CD8SP-LAMP1-humab1-2-(vL-vH)-Myc-z-P2A-K13- Flag-T2A-PAC(051716-T05)[SEQ ID NO:1104] | L363, U266 |
| 19 | cMET-Her3 | CD8SP-cMET-171-vHH-Gly-Ser-Linker-Her3-21F06- vHH-Myc-z-P2A-K13-Flag-T2A-PAC(041116- B05)[SEQ ID NO:1116] | U266; MCF7 |
| 20 | MPL | CD8SP-2-MPL-111-(vL-vH)-Myc-z-P2A-K13-Flag- T2A-PAC(040716-M02)[SEQ ID NO:1120] | HEL |
| 21 | NYBR1 | CD8SP-NYBR1-(vL-vH)-Myc-z-P2A-K13-Flag-T2A- PAC(051716-Q05)[SEQ ID NO:1131] | L363 |
| 22 | Slea | CD8SP-SLea-5B1-(vL-vH)-Myc-z-P2A-K13-Flag- T2A-PAC(051716-X05)[SEQ ID NO:1149] | LAN5 |
| 23 | TGFBetaR2 | CD8SP-TGFBR2-Ab1-(vL-vH)-Myc-z-P2A-K13-Flag- T2A-PAC(051716-O06)[SEQ ID NO:1158] | NALM6 |
| 24 | Tim1 | CD8SP-TIM1-HVCR1-ARD5-(vL-vH)-Myc-z-P2A- K13-Flag-T2A-PAC(051716-P05)[SEQ ID NO:1161] | A549; SKOV3 |
| 25 | TnAg | CD8SP-TnAg-(vL-vH)-Myc-z-P2A-K13-Flag-T2A- PAC(051616-F05)[SEQ ID NO:1162] | K562 |
| 26 | TROP2 | CD8SP-TROP2-ARA47-HV3KV3-(vL-vH)-Myc-z- P2A-K13-Flag-T2A-PAC(051716-G05)[SEQ ID NO:1165] | PC3 |
| | | | |
| 27 | CD23 | CD8SP-CD23-p5E8-(vL-vH)-Myc-BBz-T2A- PAC[SEQ ID NO:1727] | L1236 |
| 27 | CD19 | CD8SP-FMC63-(vL-vH)-Myc-BBz-T2A-PAC(112014- A13)[SEQ ID NO:1467] | RAJI, NALM6 |
| 27 | CD19 | CD8SP-FMC63(vL-vH)-BBz-P2A-IgHSP-IL6- 19A(vL-vH)-Flag-T2A-PAC(011315-H06)[SEQ ID NO:1762] | RAJI |
| 27 | CD19 | CD8SP-FMC63(vL-vH)-Myc-BBz-P2A-cFLIP-L-Flag- T2A-PAC(092215-A06)[SEQ ID NO:1754] | RAJI, NALM6, Jeko1. MOLM13 |
| 27 | CD19 | CD8SP-FMC63-(vL-vH)-BBz-P2A-IgHSP-Fx06-Flag- T2A-PAC(012115-N04)[SEQ ID NO:1764] | RAJI, Bv173, NALM6 |
| 27 | CD19 | CD8SP-FMC63(vL-vH)-BBz-P2A-IgHSP-IL6R- M83(vL-vH)-Flag-T2A-PAC(011315-K04)[SEQ ID NO:1763] | RAJI, NALM6,U266 |
| 27 | CD19 | CD8SP-FMC63(vL-vH)-BBz-P2A-IgHSP-IL6- 19A(vL-vH)-Flag-T2A-PAC(011315-H06)[SEQ ID NO:1762] | RAJI, NALM6 |
| 27 | CD22 | CD8SP-CD22-m971-(vL-vH)-Myc-BBz-T2A- PAC(091515-A02)[SEQ ID NO:1519] | RAJI, NALM6 |
| 27 | CD324 | CD8SP-CD324-hSC10-17-(vL-vH)-Myc-BBz-T2A- PAC(052516-A07)[SEQ ID NO:1555] | MDAMB-231 |
| 27 | DLL3 | CD8SP-DLL3-hSC16-56-(vL-vH)-Myc-BBz-T2A- PAC(052516-C07)[SEQ ID NO:1585] | SKMEL31, SKMEL37. LAN5 |
| 27 | DLL3 | CD8SP-DLL3-hSC16-13-(vL-vH)-Myc-BBz-T2A- PAC(052516-D07)[SEQ ID NO:1584] | LAN5, SKMEL31, SKMEL317 |
| 27 | GCC | CD8SP-GCC-5F9-(vL-vH)-Myc-BBz-T2A-PAC[SEQ ID NO:1728] | HCT116 |
| 27 | MPL | CD8SP-MPL-161-(vL-vH)-Myc-BBz-T2A- PAC(021015-R07)[SEQ ID NO:1658] | HEL, RAJI-MPL |
| 27 | MPL | CD8SP-2-MPL-111-(vL-vH)-Myc-BBz-T2A- PAC[SEQ ID NO:1660] | HEL |
| 27 | Mucl/MHC class I | CD8SP-Muc1-D6-M3B8-(vL-vH)-Myc-BBz-T2A- PAC(052316 A02)[SEQ ID NO:1665] | SKOV3 |
| 27 | PTK7 | CD8SP-PTK76-10-2-(vL-vH)-Myc-BBz-T2A- PAC(052516-E07)[SEQ ID NO:1684] | LAN5 |
| 27 | TSHR | CD8SP-TSHR-KB1-(vL-vH)-Myc-BBz-T2A- PAC(052616-E05)[SEQ ID NO:1709] | RAJI |
| 27 | Tyrosinase | CD8SP-Tyros-TA2-(vL-vH)-Myc-BBz-T2A-PAC[SEQ ID NO:1714] | MEL624 |

### Use of PI3K inhibitors to expand T cells expressing conventional CARs and backbones 1-62

Human peripheral blood T cells will be isolated using CD3 magnetic beads and infected with lentiviruses expressing the CAR constructs (SEQ ID NO: 111614-B05) targeting CD19. Cells will be left unselected or selected with puromycin and expanded using the standard protocol described previously using CD3/CD28 beads and IL2 but in the absence or presence of dual PI3K/mTOR inhibitor PF-04691502 (0.10 µM to 0.5 µM). NSG mice (Jackson Lab) will be sub-lethally irradiated at a dose of 175 cGy. 24 hours post irradiation (day2), mice will be injected with 2.5 x 10⁴ RAJI cells via tail-vein. On day 3, the mice (n =5 for each group) will be treated with 5 million T cells that had been infected with the indicated CAR encoding lentiviruses and expanded in the absence or presence of PF-04691502. Control mice (n =5) will be injected with 5 million uninfected T cells. Mice are given human IL2 (400 IU i.p.) on alternate days till the death of all mice in control group. The median survival of mice which received CAR-T cells expanded in the presence of PI3K/AKT inhibitor is expected to be higher than the mice which received CAR-T cells that had been expanded in the absence of PI3K/AKT inhibitors.

### Use of IL7 along with CAR-T cells

The study will be conducted as described in the preceding example with the exception that starting 1 day after the infusion of CAR-T cells, mice will be administered exogenous recombinant human IL-7 at a dosage of 200 ng/mouse by intraperitoneal injection three times, while the control mice receive normal saline. The mice that receive IL-7 are expected to reject their tumor and survive longer than the control mice.

### Use of autologous CAR-T cells for adoptive cell therapy

CAR-T cells of the invention can be used for adoptive cell therapy. As an example, patients with relapsed Acute lymphocytic Leukemia, chronic lymphocytic leukemia, or high-risk intermediate grade B-cell lymphomas may receive immunotherapy with adoptively transferred autologous CAR-T cells targeting CD19. A leukapheresis product collected from each patient will undergo selection of CD3-positive T lymphocytes using the CliniMACS Prodigy^{®} System from Miltenyi Biotec and following the manufacturer's recommendations.. Cells will be transduced with clinical-grade CD8SP-CD19Bu12-(vL-vH)-Myc-BBz-T2A-PAC(082815-P08)[SEQ ID NO: 1470]. Alternatively, clinical grade CD8SP-CD19Bul2-(vL-vH)-Myc-z-P2A-K13-Flag-T2A-PAC[SEQ ID NO:930] virus will be used. The selection and expansion of the CAR-T cells will occur in a closed system. After the resulting cell products has undergone quality control testing (including sterility and tumor specific cytotoxicity tests), they will be cryopreserved. Meanwhile, following leukapheresis, study participants will commence with lymphodepletive chemotherapy (30 mg/m²/day fludarabine plus 500 mg/m²/day cyclophosphamide x 3 days). One day after completion of their lymphodepleting regimen, the previously stored CAR-T cell product will be transported, thawed and infused at the patient's bedside. The study participant may also receive CAR-transduced lymphocytes infused intravenously followed by high-dose (720,000 IU/kg) IL-2 (Aldesleukin; Prometheus, San Diego, CA) every 8 hours to tolerance. The dose of CAR-T product will vary from 1 x 10⁴ CAR+ve CD3 cells/kg to 5 x 10⁹ CAR+ve CD3 cells/kg as per the study protocol. The CAR-T product may be administered in a single infusion or split infusions. Research participants will be pre-medicated at least 30 minutes prior to T cell infusion with 15 mg/kg of acetaminophen P.O. (max. 650 mg.) and diphenhydramine 0.5-1 mg/kg I.V. (max dose 50 mg). The study participant will optionally receive daily injections of human IL-2. Clinical and laboratory correlative follow-up studies will then be performed at the physician's discretion, and may include quantitative RT-PCR studies for the presence of CD19-expressing ALL/lymphoma cells and/or the adoptively transferred T cells; FDG-PET and/or CT scans; bone marrow examination for disease specific pathologic evaluation; lymph node biopsy; and/or long-term follow up per the guidelines set forth by the FDA's Biologic Response Modifiers Advisory Committee that apply to gene transfer studies. Essentially a similar approach will be used to treat other diseases using autologous immune cells (e.g., T cells) that have been engineered to express the conventional CARs of the invention or conventional CARs with accessory module (i.e. backbones 1-62) where the CAR targets an antigen or antigens expressed on the disease causing or disease-associated cells.

### Use of autologous T cells expressing conventional CARs and backbones 1-62 targeting multiple antigens for adoptive cell therapy

Patients with many different diseases, including infectious diseases (e.g., HIV1, EBB, CMV, HTLV1, etc), degenerative diseases (e.g., Alzheimer's disease), allergic diseases (e.g., chronic idiopathic urticarial) and multiple cancers will be enrolled in an IRB approved phase I clinical trial of immunotherapy with adoptively transferred autologous CAR-T cells coexpressing K13-vFLIP (backbone 1) targeting different disease-causing or disease-associated antigens. The CAR for different diseases will be selected based on the known expression of their target antigen in the disease-causing or disease-associated cells. Where possible, the expression of the CAR target on the disease causing or disease associated cells will be confirmed by binding with Antigen binding domain-GGS-NLuc fusion protein in which the antigen binding domain of the CAR is fused to non-secretory form of NLuc protein via a flexible linker. Alternatively, immunohistochemistry or flow cytometry using commercially available antibodies will be used to confirm the expression of the target antigen of the CAR on disease-causing or disease-associated cells. T cells will be collected from the subjects using leukopheresis, transduced with the appropriate lentivirus vectors and expanded ex vivo using CD3/CD28 beads in a closed system. After the resulting cell products have undergone quality control testing (including sterility and tumor specific cytotoxicity tests), they will be cryopreserved. CAR-T cell products will be administered to the subjects as described in the preceding example. Clinical and laboratory correlative follow-up studies can then be performed at the physician's discretion.

### Use of an mTOR inhibitor RAD001 in combination with T cells expressing conventional CARs and backbones 1-62

The study is conducted as described in the preceding examples with the exception that starting 1 day after the infusion of CAR -T cells, study participants will be administered an mTOR inhibitor, e.g., an allosteric inhibitor, e.g., RAD001, at a dosage that provides a target trough level 0.1 to 3 ng/ml, where the trough level" refers to the concentration of a drug in plasma just before the next dose, or the minimum drug concentration between two doses.

### Use of Ibrutinib in combination with T cells expressing conventional CARs and backbones 1-62

The study will be conducted as described in the preceding examples with the exception that starting 1 day after the infusion of CAR-T cells, study participants will be administered oral ibrutinib at dose of 140 mg/d to 420 mg/d. It is expected that the study participant receiving ibrutinib will have lower incidence of severe cytokine release syndrome as compared to participants who received CAR-T cells without ibrutinib.

### CAR-T Cell Hepatic Arterial Infusion

In addition to intravenous infusion, T cells expressing the conventional CARs and backbones 1-62 described in this invention can be infused intra-arterially to provide high concentration of CAR-T cells in a local area or organ involved with a disease. In the following example, this approach is used in case of a patient with hepatic metastases from a gastrointestinal cancer which expresses Folate Receptor alpha (FR1). Essentially a similar approach can be used for intra-arterial infusion of T cells expressing conventional CARs and backbones 1-62 targeting other tumor antigens.

A mapping angiogram will be performed via a right common femoral artery approach at baseline. The gastroduodenal and right gastric arteries, in addition to other potential sources of extrahepatic perfusion, will be embolized with microcoils. The same arterial access procedure will be carried out for administration of T cells expressing the construct CD8SP-FR1-huMov19-(vL-vH)-Myc-z-P2A-K13-Flag-T2A-PAC[SEQ ID NO:1060]. The T cells will be collected from the patient on day 0 and will be infected with lentivirus encoding the construct CD8SP-FR1-huMovl9-(vL-vH)-Myc-z-P2A-K13-Flag-T2A-PAC[SEQ ID NO:1060] and expanded as described in the previous examples. The CAR-T cells will be given in a dose escalating fashion on day 14 (10⁷ CAR-T cells), day 28 (10⁸ CAR-T cells) and day 44 (10⁹ CAR-T cells). The CAR- T cells will be injected manually via a 60cc syringe at a rate of <2 cc/second. The total volume of infusion will be approximately 100 cc. Angiography with calibrated contrast rate will be performed after the first infusion of 50 cc and at completion of the CAR-T infusion to confirm preserved arterial flow. Infusions will be delivered into the proper hepatic artery when possible. Certain patients may have aberrant hepatic arterial anatomy, where either the right or left hepatic artery does not arise from the proper hepatic artery. In such cases the dose of CAR-T cells will be split based upon lobar volume calculations. In such cases, split doses will be delivered separately into the right and left hepatic arteries to ensure proportionate CAR-T delivery to both hepatic lobes.

### Intraperitoneal administration of CAR-T cells

CAR-T cells can also be administered intraperitoneally, essentially as described in Koneru M et al (Journal of Translational Medicine; 2015; 13:102). In the following example, this approach is used in patients with peritoneal involvement with ovarian cancer which expresses Folate Receptor alpha (FR1). Essentially a similar approach can be used for intra-peritoneal infusion of CAR-T cells targeting other tumor antigens.

A screening informed consent will be offered to patients with recurrent high-grade serous ovarian cancer to test their cancer for the expression of FR1. After expression of FR1 is confirmed by immunohistochemistry, then patients will have a leukapheresis product obtained from peripheral blood. Excess platelet and red blood cell contamination will be removed from the leukapheresis product and the product will be frozen. In the treatment phase of the study, the leukapheresis product will be thawed and washed. Subsequently, CD3+ T cells will be isolated from the thawed leukapheresis product by magnetic separation using CD3/CD28 beads. Activated T cells will be lentivirally transduced with the CD8SP-FR1-huMov19-(vL-vH)-Myc-z-P2A-K13-Flag-T2A-PAC[SEQ ID NO:1060] construct and further expanded using CD3/CD28 bead expansion protocol.

Patients with recurrent high-grade serous ovarian, primary peritoneal or fallopian tube carcinoma shown to express FR1 antigen confirmed by immunohistochemistry (IHC) analysis of banked (paraffin embedded) or freshly biopsied tumor will potentially be eligible for the study.

The phase I dose-escalation dosing will be used in the trial. Cohorts of 3-6 patients will be infused with escalating doses of modified T cells to establish the maximum tolerated dose (MTD). There will be four planned dose levels: 3 × 10⁵, 1 × 10⁶, 3 × 10⁶, and 1 × 10⁷ CAR-T cells/kg. Cohorts I and II will be treated with 3 × 10⁵ CAR-T cells/kg but patients in cohort II will also receive lymphodepleting cyclophosphamide. Cohorts II-V will receive escalating doses of the modified T cells following pretreatment with cyclophosphamide. Lymphodepleting cyclophosphamide dosed at 750 mg/m² will be administered 2-4 days prior to the initial T cell infusion. A standard 3 + 3 dose escalation schema will be followed.

An IP catheter will be placed prior to T cell infusion. Patients will be admitted to the inpatient unit of the hospital prior to their first infusion of CAR T cells and will remain hospitalized until at least 3 days after the second infusion of CAR T cells. The first cohort of patients to be treated, and the first patient treated in each subsequent cohort, will be admitted to the intensive care unit (ICU); subsequent patients may be admitted to the medical oncology inpatient service (subject to the clinical judgment of the treating physician).

Patients will receive a single dose of lymphodepleting cyclophosphamide (750 mg/m² IV) chemotherapy 2 to 4 days prior to initiating treatment with CAR-modified T cells. The transduced T cells will be quality tested for number, purity, viability, and sterility prior to infusion. All patients will receive 50% of the genetically modified T cell dose intravenously. Patients will be closely monitored for toxicities. One to 3 days later, the remaining dose of T cells will be administered as an IP infusion. At least 3 patients will be treated at dose level 1, with an accrual of no more than 2 patients per month within each dose level. All patients treated in the preceding cohort will be observed for a minimum of 4 weeks from the day of the initial T cell infusion before escalation to the next cohort occurs. Blood samples will be obtained from all patients prior to and following treatment to assess toxicity, therapeutic effects, and survival of the genetically modified T cells.

### Use of CAR-T cells for intratumoral injection

CAR-T cells can also be administered intra-tumorally, essentially as described in Brown CE, et al, Clin Cancer Res. 2015 September 15; 21(18): 4062-4072. In the following example, this approach will be used in case of patients with recurrent glioblastoma (GBM) which expresses IL13Ra2. Essentially a similar approach can be used for intratumoral injection of T cells expressing conventional CARs or conventional CARs expressing accessory modules (backbones 1-62) targeting other tumor antigens.

A pilot safety and feasibility study will be conducted to test CD8SP-IL13Ra2-Hu108-(vL-vH)-Myc-z-P2A-K13-Flag-T2A-PAC(050516-F04)[SEQ ID NO:1102] expressing T cells in recurrent GBM. All participating patients will be required to give written informed consent. The clinical protocol will be approved by the University of Southern California Institutional Review Board and conducted under an Investigational New Drug Application, and registered at ClinicalTrials.gov. Eligible patients will include adults (18-70 yrs) with recurrent or refractory unifocal supratentorial grade III or IV glioma whose tumors do not show communication with ventricles/CSF pathways and are amenable to resection. Participation in this trial will be independent of IL13Rα2 (or IL13Ra2) tumor antigen status. Patients will be enrolled following initial diagnosis of high-grade glioma (WHO grade III or IV), at which time they will undergo leukapheresis for collection of peripheral blood mononuclear cells (PBMC). These cells will be used to engineer T cells to express the construct CD8SP-IL13Ra2-Hu108-(vL-vH)-Myc-z-P2A-K13-Flag-T2A-PAC(050516-F04)[SEQ ID NO:1102] containing the puromycin resistance gene (PAC) following infection with the corresponding lentiviral vector as described in the previous examples. Alternatively, the CAR-T cells could be generated following infection with a retroviral vector or using sleeping beauty transposon or by transfection of IVT mRNA. Subsequently, the release tested therapeutic CAR-T cells will be cryopreserved and stored for later use. At the time of first recurrence of the tumor, the research participant will undergo resection of tumor along with placement of a Rickham reservoir/catheter. Concurrently, the therapeutic CAR-T cells will be thawed, re-expanded in vitro using CD3/CD28 beads based rapid expansion protocol. Following recovery from surgery and post baseline MR imaging, the CAR-T cells will be administered directly into the resection cavity via the indwelling catheter, essentially as described (Brown CE, et al, Clin Cancer Res. 2015 21(18): 4062-4072). Cells will be manually injected into the Rickham reservoir using a 21 gauge butterfly needle to deliver a 2 mL volume over 5-10 minutes, followed by 2 mL flush with preservative free normal saline over 5 minutes. The protocol treatment plan will specify an intra-patient dose escalation schedule with a target of 12 CAR T cell doses administered intracranially over a 5 week period comprised of weekly treatment cycles. During cycles 1, 2, 4 and 5, T cell infusions will be performed on days 1, 3 and 5 of the cycle week, and week 3 will be a rest cycle. For safety, in cycle 1 an intrapatient dose escalation strategy, with CAR T cell doses of 10⁷, 5 × 10⁷ and 10⁸ cells per infusion administered on days 1, 3 and 5 respectively, will be used and this will be followed by 9 additional CAR T cell infusions of 10⁸ cells over 4 weeks. Imaging to assess response will be performed during the week 3 rest cycle and after week 5. The guidelines provided in the NCI Common Toxicity Criteria version 2.0 (https://ctep.ifo.nih.gov/l) will be followed for the monitoring of toxicity and adverse event reporting

### Use of CAR-T cells for ex-vivo purging of bone marrow or peripheral blood stem cell preparation prior to transplant

CAR T cells can be used to purge the bone marrow or peripheral blood stem cell preparation of cancer cells prior to stem cell transplant. In the following example, CD8SP-CS1-HuLuc64-(vL-vH)-Myc-z-P2A-K13-Flag-T2A-PAC[SEQ ID NO:1032] expressing T cells will be used to purge bone marrow or peripheral blood stem cells obtained from a patient with multiple myeloma prior to autologous stem cell (or bone marrow) transplant.

Patient will undergo leukopheresis to collect peripheral blood mononuclear cells (PBMC). T cells will be purified using CD3 beads. These cells will be used to engineer T cells to express the CD8SP-CS1-HuLuc64-(vL-vH)-Myc-z-P2A-K13-Flag-T2A-PAC[SEQ ID NO:1032] CAR following infection with the corresponding lentiviral vector as described in the previous examples. Subsequently, the release-tested therapeutic CAR-T cells will be cryopreserved and stored for later use or used fresh. Bone marrow cells and peripheral blood progenitor cell products will be collected from a patient with multiple myeloma following standard procedures. For mobilization of peripheral blood stem cells, patients will receive cyclophosphamide, 3 gm/m² followed by G-CSF, 10 µg/kg subcutaneously each day beginning 24 h after cyclophosphamide until pheresis is complete. Peripheral blood stem cells will be collected once the peripheral blood CD34+-cell count is 15 cells/µl. The collection goal will be to process three blood volumes per day until a minimum of 2.0 times 10⁶ CD34+ cells/kg are reached after processing. The bone marrow and peripheral blood stem cell products will be optionally depleted of Red Blood Cells and/or enriched for CD34 expressing cells using CliniMACS Prodigy^{®} System from Miltenyi Biotec and following the manufacturer's recommendations. The products will be used for *ex-vivo* purging fresh or cryopreserved. For purging, the bone marrow or peripheral blood stem cell products will be cocultured with thawed CAR-T cells at an effector to target ratio ranging from 5: 1 to 30:1 for 4 to 24 hours in XVIVO medium (Lonza) supplanted with 100 IU recombinant human-IL2. Cells will be cultured at 37°C, in a 5% CO2 humidified incubator. At the end of the coculture period, an aliquot of the cells will be taken for sterility and quality testing (including measurement of CFU-GM and flow cytometry for CD34 and CD138 positive cells). The remaining sample will be administered intravenously to the patient who has previously received myeloablative chemotherapy (e.g., high dose Melphalan in two divided doses of 70 mg/m² for a total dose of 140 mg/m²).

### References

1. Bertin, J., et al. (1997), Death effector domain-containing herpesvirus and poxvirus proteins inhibit both Fas- and TNFR1-induced apoptosis, Proc Natl Acad Sci U S A, 94(4), 1172-6.
2. Brentjens, R. J. (2011), Safety and persistence of adoptively transferred autologous CD19-targeted T cells in patients with relapsed or chemotherapy refractory B-cell leukemias, Blood, 118, 4817-28.
3. Burchat, A., D. W. Borhani, D. J. Calderwood, G. C. Hirst, B. Li, and R. F. Stachlewitz (2006), Discovery of A-770041, a src-family selective orally active lck inhibitor that prevents organ allograft rejection, Bioorg Med Chem Lett, 16(1), 118-22.
4. Chaudhary, P. M., A. Jasmin, M. T. Eby, and L. Hood (1999), Modulation of the NF-kappa B pathway by virally encoded death effector domains-containing proteins, Oncogene, 18(42), 5738-46.
5. Chugh, P., H. Matta, S. Schamus, S. Zachariah, A. Kumar, J. A. Richardson, A. L. Smith, and P. M. Chaudhary (2005), Constitutive NF-kappaB activation, normal Fas-induced apoptosis, and increased incidence of lymphoma in human herpes virus 8 K13 transgenic mice, Proc Natl Acad Sci U S A, 102(36), 12885-90.
6. Groger, M., et al. (2009), Peptide Bbeta(15-42) preserves endothelial barrier function in shock, PLoS ONE, 4(4), e5391.
7. Hu, S., C. Vincenz, M. Buller, and V. M. Dixit (1997), A novel family of viral death effector domain-containing molecules that inhibit both CD-95- and tumor necrosis factor receptor-1-induced apoptosis, J Biol Chem, 272(15), 9621-4.
8. Jeang, K. T. (2001), Functional activities of the human T-cell leukemia virus type I Tax oncoprotein: cellular signaling through NF-kappa B, Cytokine Growth Factor Rev, 12(2-3), 207-17.
9. Liu, L., M. T. Eby, N. Rathore, S. K. Sinha, A. Kumar, and P. M. Chaudhary (2002), The Human Herpes Virus 8-encoded Viral FLICE Inhibitory Protein Physically Associates with and Persistently Activates the Ikappa B Kinase Complex, J Biol Chem, 277(16), 13745-51.
10. Matta, H., V. Punj, S. Schamus, L. Mazzacurati, A. M. Chen, R. Song, T. Yang, and P. M. Chaudhary (2008), A nuclear role for Kaposi's sarcoma-associated herpesvirus-encoded K13 protein in gene regulation, Oncogene, 27(39), 5243-53.
11. Rapoport, A. P., et al. (2015), NY-ESO-1-specific TCR-engineered T cells mediate sustained antigen-specific antitumor effects in myeloma, Nat Med, 21(8), 914-21.
12. Ren, T., W. Dong, Y. Takahashi, D. Xiang, Y. Yuan, X. Liu, T. P. Loughran, Jr., S. C. Sun, H. G. Wang, and H. Cheng (2012), HTLV-2 Tax immortalizes human CD4+ memory T lymphocytes by oncogenic activation and dysregulation of autophagy, J Biol Chem, 287(41), 34683-93.
13. Robbins, P. F., et al. (2015), A pilot trial using lymphocytes genetically engineered with an NY-ESO-1-reactive T-cell receptor: long-term follow-up and correlates with response, Clin Cancer Res, 21(5), 1019-27.
14. Robek, M. D., and L. Ratner (1999), Immortalization of CD4(+) and CD8(+) T lymphocytes by human T-cell leukemia virus type 1 Tax mutants expressed in a functional molecular clone, J Virol, 73(6), 4856-65.
15. Roybal, K. T., L. J. Rupp, L. Morsut, W. J. Walker, K. A. McNally, J. S. Park, and W. A. Lim (2016), Precision Tumor Recognition by T Cells With Combinatorial Antigen-Sensing Circuits, Cell, 164(4), 770-9.
16. Sadelain, M., R. Brentjens, and I. Riviere (2009), The promise and pitfalls of chimeric antigen receptors, Curr. Opin. Immunol., 21, 215-23.
17. Schade, A. E., G. L. Schieven, R. Townsend, A. M. Jankowska, V. Susulic, R. Zhang, H. Szpurka, and J. P. Maciejewski (2008), Dasatinib, a small-molecule protein tyrosine kinase inhibitor, inhibits T-cell activation and proliferation, Blood, 111(3), 1366-77.
18. Sieburg, M., A. Tripp, J. W. Ma, and G. Feuer (2004), Human T-cell leukemia virus type 1 (HTLV-1) and HTLV-2 tax oncoproteins modulate cell cycle progression and apoptosis, J Virol, 78(19), 10399-409.
19. Thome, M., et al. (1997), Viral FLICE-inhibitory proteins (FLIPs) prevent apoptosis induced by death receptors, Nature, 386(6624), 517-21.
20. Turtle, C. J., M. Hudecek, M. C. Jensen, and S. R. Riddell (2012), Engineered T cells for anti-cancer therapy, Curr. Opin. Immunol., 24, 633-39.
21. Weichsel, R., et al. (2008), Profound inhibition of antigen-specific T-cell effector functions by dasatinib, Clin Cancer Res, 14(8), 2484-91.
22. Wu, C. Y., L. J. Rupp, K. T. Roybal, and W. A. Lim (2015), Synthetic biology approaches to engineer T cells, Curr Opin Immunol, 35, 123-30.
23. Wu, C. Y., K. T. Roybal, E. M. Puchner, J. Onuffer, and W. A. Lim (2015), Remote control of therapeutic T cells through a small molecule-gated chimeric receptor, Science, 350(6258), aab4077.
24. Zhan, J., B. Felder, A. R. Ellison, A. Winters, H. Salimi-Moosavi, S. Scully, J. R. Turk, and P. Wei (2013), Novel anti-c-Mpl monoclonal antibodies identified multiple differentially glycosylated human c-Mpl proteins in megakaryocytic cells but not in human solid tumors, Monoclonal antibodies in immunodiagnosis and immunotherapy, 32(3), 149-61.

The various methods and techniques described above provide a number of ways to carry out the application. Of course, it is to be understood that not necessarily all objectives or advantages described can be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as taught or suggested herein. A variety of alternatives are mentioned herein. It is to be understood that some preferred embodiments specifically include one, another, or several features, while others specifically exclude one, another, or several features, while still others mitigate a particular feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be employed in various combinations by one of ordinary skill in this art to perform methods in accordance with the principles described herein. Among the various elements, features, and steps some will be specifically included and others specifically excluded in diverse embodiments.

Although the application has been disclosed in the context of certain embodiments and examples, it will be understood by those skilled in the art that the embodiments of the application extend beyond the specifically disclosed embodiments to other alternative embodiments and/or uses and modifications and equivalents thereof.

Preferred embodiments of this application are described herein, including the best mode known to the inventors for carrying out the application. Variations on those preferred embodiments will become apparent to those of ordinary skill in the art upon reading the foregoing description. It is contemplated that skilled artisans can employ such variations as appropriate, and the application can be practiced otherwise than specifically described herein. Accordingly, many embodiments of this application include all modifications and equivalents of the subject matter recited in the claims appended hereto as permitted by applicable law. Moreover, any combination of the above-described elements in all possible variations thereof is encompassed by the application unless otherwise indicated herein or otherwise clearly contradicted by context.

All patents, patent applications, publications of patent applications, and other material, such as articles, books, specifications, publications, documents, things, and/or the like, referenced herein are hereby incorporated herein by this reference in their entirety for all purposes, excepting any prosecution file history associated with same, any of same that is inconsistent with or in conflict with the present document, or any of same that may have a limiting affect as to the broadest scope of the claims now or later associated with the present document. By way of example, should there be any inconsistency or conflict between the description, definition, and/or the use of a term associated with any of the incorporated material and that associated with the present document, the description, definition, and/or the use of the term in the present document shall prevail.

It is to be understood that the embodiments of the application disclosed herein are illustrative of the principles of the embodiments of the application. Other modifications that can be employed can be within the scope of the application. Thus, by way of example, but not of limitation, alternative configurations of the embodiments of the application can be utilized in accordance with the teachings herein. Accordingly, embodiments of the present application are not limited to that precisely as shown and described.

Various embodiments of the invention are described above in the Detailed Description. While these descriptions directly describe the above embodiments, it is understood that those skilled in the art may conceive modifications and/or variations to the specific embodiments shown and described herein. Any such modifications or variations that fall within the purview of this description are intended to be included therein as well. Unless specifically noted, it is the intention of the inventors that the words and phrases in the specification and claims be given the ordinary and accustomed meanings to those of ordinary skill in the applicable art(s).

The foregoing description of various embodiments of the invention known to the applicant at this time of filing the application has been presented and is intended for the purposes of illustration and description. The present description is not intended to be exhaustive nor limit the invention to the precise form disclosed and many modifications and variations are possible in the light of the above teachings. The embodiments described serve to explain the principles of the invention and its practical application and to enable others skilled in the art to utilize the invention in various embodiments and with various modifications as are suited to the particular use contemplated. Therefore, it is intended that the invention not be limited to the particular embodiments disclosed for carrying out the invention.

While particular embodiments of the present invention have been shown and described, it will be obvious to those skilled in the art that, based upon the teachings herein, changes and modifications may be made without departing from this invention and its broader aspects and, therefore, the appended claims are to encompass within their scope all such changes and modifications as are within the true spirit and scope of this invention.

The present application and invention further includes the subject matter of the following numbered clauses.
1. A chimeric antigen receptor comprising: an antigen-specific targeting domain; a hinge region; a transmembrane domain; and an intracellular signaling domain, wherein each antigen-specific targeting region comprises an antigen-specific single chain Fv (scFv) fragment or an antigen specific ligand .
2. The chimeric antigen receptor of clause1, further comprising one or more co-stimulatory domains.
3. The chimeric antigen receptor of clause 1, wherein the antigen-specific targeting domain targets any one or more of MPL receptor, CD19, GRP-78, CD79b, Lym-1, Lym-2, FLT3 or combinations thereof.
4. The chimeric antigen receptor of clause 1, wherein the hinge region comprises any one or more of human CD8 hinge region, an Fc fragment of an antibody or a functional equivalent, fragment or derivative thereof, a hinge region of an antibody or a functional equivalent, fragment or derivative thereof, a CH2 region of an antibody, a CH3 region of an antibody, an artificial spacer sequence and combinations thereof.
5. The chimeric antigen receptor of clause 4, wherein the hinge region comprises any one or more of (i) a hinge, CH2 and CH3 region of IgG4, (ii) a hinge region of IgG4, (iii) a hinge and CH2 region of IgG4, (iv) a hinge region of CD8α, (v) a hinge, CH2 and CH3 region of IgG1, (vi) a hinge region of IgG1, (vi) a hinge and CH2 region of IgG1, or (vii) combinations thereof.
6. The chimeric antigen receptor of clause 1, wherein the transmembrane domain comprises any one or more of a transmembrane domain of a zeta chain of a T cell receptor complex, CD28, CD8a, and combinations thereof.
7. The chimeric antigen receptor of clause 2, wherein the co-stimulatory domain comprises a signaling domain from any one or more of CD28, CD137 (4-1BB), CD134 (OX40), Dap10, CD27, CD2, CD5, ICAM-1, LFA-1, Lck, TNFR-I, TNFR-II, Fas, CD30, CD40 and combinations thereof.
8. The chimeric antigen receptor of clause 1, wherein the intracellular signaling domain comprises a signaling domain of one or more of a human CD3 zeta chain, FcgRIII, FceRI, a cytoplasmic tail of a Fc receptor, an immunoreceptor tyrosine-based activation motif (ITAM) bearing cytoplasmic receptors, and combinations thereof.
9. The chimeric antigen receptor of clause 1, further coexpressed with scFv or vHH or bispecific antibody specific for cytokines or cytokine receptor.
10. The chimeric antigen receptor of clause 9, wherein the coexpressed cytokines or cytokine receptor are any one or more of , IL-6, IL6R, IFNγ, TNFα or combinations thereof.
11. The chimeric antigen receptor of clause 1, further coexpressing the peptide FX06 so as to mitigate capillary leak associated with CAR therapy.
12. The chimeric antigen receptor of clause 1, further coexpressing a viral or cellular signaling protein to stimulate T cell activation, proliferation and/or to block T cell activation induced cell death.
13. The chimeric antigen receptor of clause 12, wherein the signaling protein is any one or more of vFLIP K13, vFLIP MC159, vFLIP MC160, vFLIP E8, HSV vFLIP, BHV vFLIP, Herpes virus Mecaca vFLIP, cFLIP/MRITα, cFLIP-p22, HTLV1 Tax, HTLV2 Tax, HTLV1- Tax RS mutant, HTLV2 Tax RS mutant, Tio, StpC, Tip or mutants or combinations thereof.
14. The chimeric antigen receptor of clause 12, wherein the signaling protein is expressed in a constitutive or inducible manner.
15. The chimeric antigen receptor of clause 12, where activity of the signaling protein is controlled at the post-translational level by administration of a compound.
16. The chimeric antigen receptor of clause 12, where the signaling protein is expressed in fusion with one or more copies of FKBP domain.
17. The chimeric antigen receptor clause 16, where the activity of the protein is controlled at the post-translational level by administration of therapeutically effective amount of a chemical compound that induces dimerization of the FKBP domain.
18. A chimeric antigen receptor of clauses 1 or 3, where the antigen specific targeting domain targeting MPL comprises a scFV fragment of 161, 175, 178, AB317, 12E10, or VB22 or human TPO or mouse TPO or mutants thereof.
19. A chimeric antigen receptor of clauses 1 or 3, where the antigen specific targeting domain targeting CD19 comprises a scFV fragment of CD19Bu12 or CD19MM or mutants thereof.
20. A chimeric antigen receptor of clauses 1 or 3, where the antigen specific targeting domain targeting CD79b comprises a scFV fragment of huMA79bv28 or CD79b-2F2 or mutants thereof.
21. A chimeric antigen receptor of clauses 1 or 3, wherein the antigen specific targeting domain targeting FLT3 comprises a scFV fragment of NC7 or mutants thereof.
22. A chimeric antigen receptor of clauses 1 or 3, wherein the antigen specific targeting domain targeting Lym1 antigen comprises a scFV fragment of Lym1 or mutants thereof.
23. A chimeric antigen receptor of clauses 1 or 3, wherein the antigen specific targeting domain targeting Lym2 antigen comprises a scFV fragment of Lym2 or mutants thereof.
24. A chimeric antigen receptor of clauses 1 or 3, wherein the antigen specific targeting domain targeting GRP-78 comprises a scFV fragment of GRP78-GC18 or mutants thereof.
25. A polynucleotide encoding the chimeric antigen receptor of any one of clauses 1, 12 and 18-24.
26. A polypeptide encoding the polynucleotide of clause 25.
27. A vector comprising the polynucleotide of clause 25.
28. A virus comprising the polynucleotide of clause 25.
29. The virus of clause 25, wherein the virus is a retrovirus, an adenovirus, an adeno-associated virus, a lentivirus, a pox virus or a herpes virus.
30. A genetically engineered cell, comprising the polynucleotide of clause 25 or the chimeric antigen receptor of clause 1.
31. The genetically engineered cell of clause 30, wherein the cell is a T-lymphocyte (T-cell).
32. The genetically engineered cell of clause 31, wherein the cell is a naive T cells, a central memory T cells, an effector memory T cell, Treg or a combination thereof.
33. The genetically engineered cell of clause 21, wherein the cell is a natural killer (NK) cell, a hematopoietic stem cell (HSC), an embryonic stem cell, or a pluripotent stem cell.
34. A pharmaceutical composition comprising: any one or more of the chimeric antigen receptor of clause 1, the polynucleotide of clause 25, the polypeptide of clause 26, the vector of clause 27, the virus of clause 28, the genetically engineered cell of clause 30 and combination thereof; and a pharmaceutically acceptable carrier.
35. A method for treating cancer comprising: providing the composition of clause 34; and administering a therapeutically effective amount of the composition to the subject so as to treat cancer.
36. The method of clause 35, wherein the cancer is blood cancer.
37. The method of clause 36, wherein the blood cancer is any one or more of acute myeloid leukemia, chronic myeloid leukemia, myelodysplastic syndrome, lymphoma, multiple myleoma and acute lymphocytic leukemia.
38. A method of controlling the activity of T cells expressing the CAR of clause 1, comprising administering a therapeutically effective amount of an inhibitor of tyrosine kinases wherein the kinase are selected from the src family of kinases.
39. A method of treating CRS associated with treatment with CAR-T cells comprising administering a therapeutically effective amount of an inhibitor of tyrosine kinases wherein the kinase are selected from the src family of kinases.
40. A method of treating neurological complications associated with treatment with CAR-T cells comprising administering a therapeutically effective amount of an inhibitor of tyrosine kinases wherein the kinase are selected from the src family of kinases.
41. The method of clauses 38, 39 or 40, wherein the inhibitor inhibits the src kinase Lck.
42. The method of clause 41, wherein the inhibitor is Dasatinib.
43. The method of clause 41, wherein the inhibitor is Ponatinib.
44. The method of clause 41, wherein the inhibitor is A-770041.
45. A method of controlling the activity of T cells activated by a BiTE comprising administering a therapeutically effective amount of an inhibitor of tyrosine kinases wherein the kinase are selected from the src family of kinases.
46. A method of treating CRS caused by treatment with BiTE comprising administering a therapeutically effective amount of an inhibitor of tyrosine kinases wherein the kinase are selected from the src family of kinases.
47. A method of treating neurological complications caused by treatment with BiTE comprising administering a therapeutically effective amount of an inhibitor of tyrosine kinases wherein the kinase are selected from the src family of kinases.
48. The method of clauses 45, 46 or 47, wherein the BiTE is Blinatumomab (Blincyto).
49. The method clauses 45, 46 or 47, wherein the inhibitor inhibits the src kinase Lck.
50. The method of clause 49, wherein the inhibitor is Dasatinib.
51. The method of clause 49, wherein the inhibitor is Ponatinib.
52. The method of clause 49, wherein the inhibitor is A-770041.
53. A method of treating complications of CAR and BiTE comprising administering a therapeutically effective amount of an inhibitor of tyrosine kinases wherein the kinase are selected from the src family of kinases.
54. The method of clause 53, wherein the BiTE is Blinatumomab (Blincyto).
55. The method of clause 53, wherein the inhibitor inhibits the src kinase Lck.
56. The method of clause 55, wherein the inhibitor is Dasatinib.
57. The method of clause 55, wherein the inhibitor is Ponatinib.
58. The method of clause 55, wherein the inhibitor is A-77041.
59. A method of treating CRS, neurological complications and other complications of CAR-T cells and BiTE by administration of therapeutic effective amount of Avasimibe.
60. A method of immortalizing human peripheral blood mononuclear cells and T cells by expressing vFLIP K13 in the cells.
61. The method clause 60, wherein vFLIP K13 is expressed constitutively.
62. The method clause 60, wherein vFLIP K13 is expressed in a manner that allows control of its activity at the transcriptional or post-translational level.
63. The method clause 60, wherein vFLIP K13 is expressed in fusion with one or more domains of FKBP.
64. The method of clause 63, where FKBP-vFLIP-K13 fusion protein further comprises an N-terminal Myristoylation sequence.
65. The method of clause 64, wherein the activity of FKBP-vFLIP-K13 fusion protein is controlled by a chemical that induces dimerization of FKBP domain and activation of K13 induced NF-kB signaling.
66. The method of clause 65, wherein the peripheral blood mononuclear cells and/or T cells expressing K13 or FKBP-K13 fusion protein are administered to a patient.
67. The method of clause 65, wherein the peripheral blood mononuclear cells and/or T cells expressing K13 or FKBP-K13 fusion protein also co-express a chimeric antigen receptor, a native T cell receptor or an artificial T cell receptor.
68. A method of immortalizing human peripheral blood mononuclear cells and T cells by expressing HTLV2 Tax protein in the cells.
69. A method of immortalizing human peripheral blood mononuclear cells and T cells by expressing HTLV2 Tax RS mutant protein in the cells.
70. The method clauses 68 or 69, wherein the HTLV2 Tax and its RS mutants are expressed in fusion with one or more domains of FKBP.
71. A method of clause 70, wherein FKBP-Tax fusion proteins further comprises an N-terminal Myristoylation sequence.
72. The method of clause 71, wherein the activity of FKBP-Tax and FKBP-Tax-RS fusion proteins is controlled by a chemical that induces dimerization of FKBP domain and activation of Tax induced NF-kB signaling.
73. The method of clause72, wherein the peripheral blood mononuclear cells and/or T cells expressing HTLV2 Tax HTLV2 Tax-RS mutant, FKBP-Tax, or FKBP-Tax-RS mutant proteins are administered to a patient.
74. The method of clause 72, wherein the peripheral blood mononuclear cells and/or T cells expressing HTLV-Tax or FKBP-Tax fusion protein further co-express a chimeric antigen receptor, a native T cell receptor or an artificial T cell receptor.

In addition, the present application and invention also include the subject matter of the following further items:
1. A cell comprising nucleic acids encoding a chimeric antigen receptor (CAR) and K13- vFLIP signaling protein, wherein the CAR comprises an a) extracellular antigen specific domain, b) a transmembrane domain and c) an intracellular signaling domain comprising an immunoreceptor tyrosine-based activation motif (ITAM); wherein c) is located at the C-terminus of the chimeric receptor.
2. The cell of item 1, further comprising nucleic acid encoding MC159-vFLIP signaling protein.
3. The cell of item 1, wherein the CAR further comprises one or more co-stimulatory domains.
4. The cell of item 1, wherein the cell further comprises nucleic acids encoding scFvs targeting IL6 and/or IL6 receptor alpha.
5. The cell of any of items 1-4, further comprising nucleic acid encoding peptide FX06 so as to mitigate capillary leak associated with CAR therapy.
6. The cells of any of items 1-4, wherein the signaling protein is expressed in fusion with one or more copies of FKBP domain.
7. The cells of item 6, wherein the activity of the signaling protein is controlled post-tranlationally by dimerization of the FKBP domain in the presence of a dimerizing agent.
8. The cell of item 7, wherein the dimerizing agent is AP20187.
9. The cell of item 1, wherein the antigen specific domain of the CAR targets MPL.
10. The cell of item 1, wherein the antigen specific domain targets two antigens.
11. The cell of item 10, wherein the two antigens are MPL and CD 123.
12. The cell of item 1, wherein the antigen specific domain of the CAR targets CD19, CD23, Lyml, Lym2, CLEC5A, CDH179b, FLT3, GCC, Mucl, CSF2RA, GFRa4, CD32, IL11Ra, IL13Ra, NYBR1, SLea, CD200R, TGFBetaR2, CD276, TROP2, LAMP1, PTK7, DLL3, CDH1, CDH6, CDH17, CDH19, TSHR and tyrosinase.
13. The cell of item 9, wherein the antigen specific domain of the CAR targets MPL and comprises one or more scFv fragments selected from 161 (SEQ ID NO: 2500 and 2501), 175 (SEQ ID NO: 2499), 178 (SEQ ID NO: 2503), 111 (SEQ ID NO: 2502), AB317 (SEQ ID NO: 2404), 12E10 (SEQ ID NO: 2505) or huVB22Bw5 (SEQ ID NO: 2506) or ligands selected from extracellular receptor binding domains of hTPO (SEQ ID NO: 2323) or mTPO (SEQ ID NO: 2323).
14. The cell of item 12, wherein the antigen specific domain of the CAR targets CD19 and comprises one or more scFv fragments selected from CD19Bu12 or CD19MM.
15. The cell of item 1, wherein the cell is a T-lymphocyte (T-cell).
16. The cells of item 1, wherein the cells is a Natural Killer (NK) cell.
17. Nucleic acids comprising a first polynucleotide encoding the CAR of item 1 and a second polynucleotide encoding the signaling protein of item 1.
18. The nucleic acid of item 17, further comprising a third polynucleotide encoding the MC159-vFLIP signaling protein.
19. Polypeptides encoded by the nucleic acids of items 17 or 18.
20. Vectors comprising nucleic acids of items 17 or 18.
21. A pharmaceutical composition, comprising the cell of any of items 1-16, nucleic acid of any of items 17-18, polypeptides of any of item 19 or a vector of items 20, and a pharmaceutically acceptable carrier.
22. A method of treating a MPL-expressing cancer comprising administering to the subject, a therapeutically effective amount of the cell of item 13.
23. The method of item 23, wherein the cancer is a blood cancer.
24. The method of item 23, wherein the blood cancer is any one or more of acute myeloid leukemia, chronic myeloid leukemia, myelodyplastic syndrome, lymphoma, multiple myeloma and acute lymphocytic leukemia.
25. The method of item 22, further comprising administering to the subject a tyrosine kinase inhibitor, wherein the inhibitor inhibits the src family of kinases.
26. The method of item 25, wherein the inhibitor inhibits Lck.
27. The method of item 26, wherein the inhibitor is any one or more of Dasatinib, Ponatinib or A-770041.

## Claims

1. An inhibitor of tyrosine kinase for use in a method of controlling the activity of an immunotherapy in a subject, or for use in a method of treating or preventing a complication and/or side effect of an immunotherapy in a subject, the method comprising administering the inhibitor to the subject, wherein the immunotherapy is selected from: an immune effector cell comprising a CAR; a bi-specific T-cell engager antibody; or a dual affinity re-targeting protein.

2. An inhibitor of tyrosine kinase and an immunotherapy for use in combination in a method of treating or preventing a disease, the method comprising administering the inhibitor and the immunotherapy separately, simultaneously, or sequentially, to a subject, wherein:
(i) the immunotherapy is selected from: an immune effector cell comprising a CAR; a bi-specific T-cell engager antibody; or a dual affinity re-targeting protein; and
(iii) the inhibitor is administered to control the activity of the immunotherapy in the subject and/or treat or prevent a complication and/or side effect of the immunotherapy in the subject.

3. The inhibitor for use according to claim 1, or the inhibitor and immunotherapy for use according to claim 2, wherein the tyrosine kinase is a Scr family kinase.

4. An inhibitor of tyrosine kinase for use in a method of preventing and/or treating cytokine release syndrome or neurological complications associated with the use of a genetically modified T cell, a bi-specific T cell engager antibody, or a dual affinity re-targeting protein, wherein the tyrosine kinase is Scr, optionally wherein the genetically modified T cell is a T cell expressing a CAR, an exogenous TCR or a chimeric TCR.

5. The inhibitor for use or the inhibitor and immunotherapy for use according to any of claims 1 to 4, wherein: (i) the inhibitor inhibits the src kinase Lck; or (ii) the inhibitor is selected from Dasatinib, Ponatinib, and A-770041.

6. The inhibitor for use or the inhibitor and immunotherapy for use according to any of claims 1 to 5, wherein the method is for controlling the activity of T cells activated by the bi-specific T-cell engager antibody, optionally wherein the bi-specific T-cell engager antibody is blinatumomab.

7. The inhibitor for use or the inhibitor and immunotherapy for use according to any of claims 1 to 6, wherein the immune effector cell comprising a CAR is a CAR-T cell.

8. The inhibitor for use or the inhibitor and immunotherapy for use according to any of claims 1 to 7, wherein the immunotherapy is for treating a disease associated with expression of a disease-associated antigen, wherein the CAR targets one or more antigens selected from the group consisting of: CD19; CD123; CD22; CD23, CD30; CD32, CD99; Tissue Factor; MPL; CD171; CS-1 (also referred to as CD2 subset 1, CRACC, SLAMF7, CD319, and 19A24); C-type lectin-like molecule-1 (CLL-1 or CLECL1); CD33; epidermal growth factor receptor variant III (EGFRvIII); ganglioside G2 (GD2); ganglioside GD3(aNeu5Ac(2-8)aNeu5Ac(2-3)bDGalp(l-4)bDGlcp(l-l)Cer); TNF receptor family member B cell maturation (BCMA); Tn antigen ((Tn Ag) or (GalNAca-Ser/Thr)); prostate-specific membrane antigen (PSMA); Receptor tyrosine kinase-like orphan receptor 1 (ROR1); Fms- Like Tyrosine Kinase 3 (FLT3); Tumor-associated glycoprotein 72 (TAG72); CD38; CD44v6; Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Mesothelin; Interleukin 11 receptor alpha (IL-11Ra); prostate stem cell antigen (PSCA); Protease Src Family Tyrosine 21 (Testisin or PRSS21); vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha; Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CAFX); Proteasome (Prosome, Macropain) Subunit, Beta Type, 9 (LMP2); glycoprotein 100 (gp100); oncogene fusion protein consisting of breakpoint cluster region (BCR) and Abelson murine leukemia viral oncogene homolog 1 (Abl) (bcr-abl); tyrosinase; ephrin type-A receptor 2 (EphA2); Fucosyl GM1; sialyl Lewis adhesion molecule (sLe); ganglioside GM3 (aNeu5Ac(2-3)bDGalp(l- 4)bDGlcp(l-l)Cer); transglutaminase 5 (TGS5); high molecular weight-melanoma- associated antigen (HMWMAA); o-acetyl-GD2 ganglioside (OAcGD2); Folate receptor beta; tumor endothelial marker 1 (TEM1/CD248); tumor endothelial marker 7-related (TEM7R); claudin 6 (CLDN6); thyroid stimulating hormone receptor (TSHR); G protein- coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCRl); adrenoceptor beta 3 (ADRB3); pannexin 3 (PANX3); G protein-coupled receptor 20 (GPR20); lymphocyte antigen 6 complex, locus K 9 (LY6K); Olfactory receptor 51 E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-la); Melanoma-associated antigen 1 (MAGE-A1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen- 1 (MAD-CT-1); melanoma cancer testis antigen-2 (MAD-CT-2); Fos-related antigen 1; tumor protein p53 (p53); p53 mutant; prostein; surviving; telomerase; prostate carcinoma tumor antigen- 1 (PCTA-1 or Galectin 8), melanoma antigen recognized by T cells 1 (MelanA or MARTI); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin Bl; v-myc avian myelocytomatosis viral oncogene neuroblastoma derived homolog (MYCN); Ras Homolog Family Member C (RhoC); Tyrosinase-related protein 2 (TRP-2); Cytochrome P450 1B1 (CYP1B1); CCCTC-Binding Factor (Zinc Finger Protein)-Like (BORIS or Brother of the Regulator of Imprinted Sites), Squamous Cell Carcinoma Antigen Recognized By T Cells 3 (SART3); Paired box protein Pax-5 (PAX5); proacrosin binding protein sp32 (OY-TES1); lymphocyte- specific protein tyrosine kinase (LCK); A kinase anchor protein 4 (AKAP-4); synovial sarcoma, X breakpoint 2 (SSX2); Receptor for Advanced Glycation Endproducts (RAGE-1); renal ubiquitous 1 (RU1); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin- like hormone receptor- like 2 (EMR2); lymphocyte antigen 75 (LY75); Glypican-3 (GPC3); Fc receptor-like 5 (FCRL5); immunoglobulin lambda-like polypeptide 1 (IGLL1); PTK7; LAMP1; TROP2; Tim1; and HIV1-envelop glycoprotein gp120.

9. The inhibitor for use or the inhibitor and immunotherapy for use according to any of claims 1 to 8, wherein the inhibitor and the immunotherapy are to be administered concurrently or sequentially.

10. The inhibitor for use or the inhibitor and immunotherapy for use according to any of claims 1 to 9, wherein the side effect and/or complication is selected from cytokine release syndrome, capillary leak syndrome, and a neurological complication.

11. The inhibitor for use or the inhibitor and immunotherapy for use according to any of claims 1 to 9, wherein the immunotherapy is a CAR-expressing cell, and the inhibitor is for reducing or ameliorating a side effect of the CAR-expressing cell, and wherein the side effect is selected from cytokine release syndrome, hemophagocytic lymphohistiocytosis (HLH), and neurological complications.

12. The inhibitor for use or the inhibitor and immunotherapy for use according to claim 11, wherein the inhibitor is for:
(i) reducing or ameliorating a symptom selected from: fever, fatigue, nausea, vomiting, diarrhea, anorexia, arthralgia, myalgia, hypotension, tachypnea, hypoxemia, cardiac dysfunction, kidney dysfunction, electrolyte abnormalities, tachycardia, widened pulse pressure, increased or decreased cardiac output, elevated D-dimer, hypofibrinogenemia, bleeding, azotemia, transaminitis, hyperbilirubinemia, mental status changes, confusion, delirium, word-finding difficulty, aphasia, hallucinations, tremor, dysmetria, altered gait, seizures, or headache,
(ii) treating a side effect of the immunotherapy which is an elevated levels of one or more soluble factors selected from: IFN-γ, TNF-α, IL-2, IL-6, IL-1, GM-CSF, IL-10, IL-8, IL-5, and fractalkine.

13. The inhibitor for use or the inhibitor and immunotherapy for use according to any of claims 1 to 12, wherein the inhibitor dasatinib or ponatinib is to be administered to the subject, wherein:
(i) the dasatinib is to be administered after the administration of CAR-expressing cells to control or terminate the activity of CAR-expressing cells, wherein the dasatinib is to be administered orally at a dose of at least 10 mg/day, 20 mg/day, 40 mg/day, 60 mg/day, 70 mg/day, 90 mg/day, 100 mg/day, 140 mg/day, 180 mg/day, 210 mg/day;
(ii) the dasatinib is to be administered to manage elevated levels of a soluble factor resulting from treatment with a CAR-expressing cell, and is to be administered at a dose of 10 mg/day to 300 mg/day; or
(iii) the ponatinib is to be administered after the administration of CAR-expressing cells to control or terminate the activity of CAR-expressing cells, wherein the ponatinib is to be administered orally at a dose of at least 15 mg/day, 30 mg/day, 45 mg/day, or 60 mg/day.

14. The inhibitor for use or the inhibitor and immunotherapy for use according to any of claims 1 to 13, wherein the immunotherapy is for the treatment of cancer.

15. The inhibitor for use or the inhibitor and immunotherapy for use according to any of claims 1 to 13, wherein the immunotherapy is an immune effector cell comprising a CAR and the immunotherapy is for the treatment of a cancer, an infectious disease, an autoimmune disease, or a degenerative disease.
